(19) Europäisches Patentamt
European Patent Office
Office européen des brevets

(11) EP 1 565 442 B1

(12) **EUROPEAN PATENT SPECIFICATION**

(45) Date of publication and mention of the grant of the patent:
**14.11.2007 Bulletin 2007/46**

(21) Application number: **03750676.3**

(22) Date of filing: **25.09.2003**

(51) Int Cl.:
***C07D 213/82*** (2006.01) ***C07D 401/12*** (2006.01)
***A61K 31/44*** (2006.01) ***A61K 31/444*** (2006.01)
***A61K 31/4439*** (2006.01) ***A61P 29/00*** (2006.01)
***C07D 407/12*** (2006.01) ***C07D 409/12*** (2006.01)

(86) International application number:
**PCT/EP2003/010930**

(87) International publication number:
**WO 2004/029026 (08.04.2004 Gazette 2004/15)**

(54) **PYRIDINE DERIVATIVES AS CB2 RECEPTOR MODULATORS**

PYRIDIN-DERIVATE ALS CB2-REZEPTOR-MODULATOREN

DERIVES DE PYRIDINE MODULATEURS DU RECEPTEUR CB2

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **27.09.2002 GB 0222493**

(43) Date of publication of application:
**24.08.2005 Bulletin 2005/34**

(73) Proprietor: **GLAXO GROUP LIMITED**
**Greenford,**
**Middlesex UB6 0NN (GB)**

(72) Inventors:
- **EATHERTON, Andrew, John,**
  **c/o GlaxoSmithKline**
  **Welwyn, Hertfordshire AL6 9AR (GB)**
- **GIBLIN, Gerard, Martin, Paul,**
  **c/o GlaxoSmithKline**
  **Welwyn, Hertfordshire AL6 9AR (GB)**
- **JANDU, Karamjit, Singh,**
  **c/o GlaxoSmithKline**
  **Welwyn, Hertfordshire AL6 9AR (GB)**
- **MITCHELL, William, Leonard,**
  **c/o GlaxoSmithKline**
  **Welwyn, Hertfordshire AL6 9AR (GB)**
- **NAYLOR, Alan,**
  **c/o GlaxoSmithKline**
  **Stevenage, Hertfordshire SG1 2NY (GB)**
- **PALOMBI, Giovanni,**
  **c/o NiKem Research S.r.L.**
  **I-20021 Milan (IT)**
- **RAWLINGS, Derek, Anthony,**
  **c/o GlaxoSmithKline**
  **Welwyn, Hertfordshire AL6 9AR (GB)**
- **SLINGSBY, Brian, Peter,**
  **c/o GlaxoSmithKline**
  **Welwyn, Hertfordshire AL6 9AR (GB)**
- **WHITTINGTON, Andrew, Richard,**
  **c/o GlaxoSmithKline**
  **Stevenage, Hertfordshire SG1 2NY (GB)**

(74) Representative: **Billson, Siân Catherine et al**
**GlaxoSmithKline**
**Corporate Intellectual Property (CN9.25.1)**
**980 Great West Road**
**Brentford, Middlesex TW8 9GS (GB)**

(56) References cited:
**WO-A-02/062750**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 565 442 B1

## Description

The present invention relates to novel pyridine derivatives, pharmaceutical compositions containing these compounds and their use in the treatment of diseases, particularly pain, which diseases are caused directly or indirectly by an increase or decrease in activity of the cannabinoid receptor.

Cannabinoids are a specific class of psychoactive compounds present in Indian cannabis (*Cannabis sativa*), including about sixty different molecules, the most representative being cannabinol, cannabidiol and several isomers of tetrahydrocannabinol. Knowledge of the therapeutic activity of cannabis dates back to the ancient dynasties of China, where, 5,000 years ago, cannabis was used for the treatment of asthma, migraine and some gynaecological disorders. These uses later became so established that, around 1850, cannabis extracts were included in the US Pharmacopaeia and remained there until 1947.

Cannabinoids are known to cause different effects on various systems and/or organs, the most important being on the central nervous system and on the cardiovascular system. These effects include alterations in memory and cognition, euphoria, and sedation. Cannabinoids also increase heart rate and vary systemic arterial pressure. Peripheral effects related to bronchial constriction, immunomodulation, and inflammation have also been observed. The capability of cannabinoids to reduce intraocular pressure and to affect respiratory and endocrine systems is also well documented. See e.g. L.E. Hollister, Health Aspects of Cannabis, Pharmacological Reviews, Vol. 38, pp. 1-20, (1986). More recently, it was found that cannabinoids suppress the cellular and humoral immune responses and exhibit antiinflammatory properties. Wirth et al., Antiinflammatory Properties of Cannabichrome, Life Science, Vol. 26, pp. 1991-1995, (1980).

In spite of the foregoing benefits, the therapeutic use of cannabis is controversial, both due to its relevant psychoactive effects (causing dependence and addiction), and due to manifold side effects that have not yet been completely clarified. Although work in this field has been ongoing since the 1940's, evidence indicating that the peripheral effects of cannabinoids are directly mediated, and not secondary to a CNS effect, has been limited by the lack of receptor characterization, the lack of information concerning an endogenous cannabinoid ligand and, until recently, the lack of receptor subtype selective compounds.

The first cannabinoid receptor was found to be mainly located in the brain, in neural cell lines, and, only to a lesser extent, at the peripheral level. In view of its location, it was called the central receptor ("CB1"). See Matsuda et al., "Structure of a Cannabinoid Receptor and Functional Expression of the Cloned cDNA," Nature, Vol. 346, pp. 561-564 (1990. The second cannabinoid receptor ("CB2") was identified in the spleen, and was assumed to modulate the non psychoactive effects of the cannabinoids. See Munro et el., "Molecular Characterization of a Peripheral Receptor for Cannabinoids," Nature, Vol. 365, pp. 61-65 (1993).

Recently, some compounds have been prepared which are capable of acting as agonists on both the cannabinoid receptors. For example, use of derivatives of dihydroxypyrrole-(1,2,3-d,e)-1,4-benzoxazine in the treatment of glaucoma and the use of derivatives of 1,5-diphenyl-pyrazole as immunomodulators or psychotropic agents in the treatment of various neuropathologies, migraine, epilepsy, glaucoma, etc are known. See U.S. Patent No. 5,112,820 and EP 576357, respectively. However, because these compounds are active on both the CB1 and CB2 receptor, they can lead to serious psychoactive effects.

The foregoing indications and the preferential localization of the CB2 receptor in the immune system confirms a specific role of CB2 in modulating the immune and antiinflammatory response to stimuli of different sources.

The total size of the patient population suffering from pain is vast (almost 300 million), dominated by those suffering from back pain, osteo-arthritic pain and postoperative pain. Neuropathic pain (associated with neuronal lesions such as those induced by diabetes, HIV, herpes infection, or stroke) occurs with lower, but still substantial prevalence, as does cancer pain.

The pathogenic mechanisms that give rise to pain symptoms can be grouped into two main categories:

- those that are components of inflammatory tissue responses (Inflammatory Pain);
- those that result from a neuronal lesion of some form (Neuropathic Pain).

Chronic inflammatory pain consists predominantly of osteoarthritis, chronic low back pain and rheumatoid arthritis. The pain results from acute and on-going injury and/or inflammation. There may be both spontaneous and provoked pain.

There is an underlying pathological hypersensitivity as a result of physiological hyperexcitability and the release of inflammatory mediators which further potentiate this hyperexcitability. CB2 receptors are expressed on inflammatory cells (T cells, B cells, macrophages, mast cells) and mediate immune suppression through inhibition of cellular interaction/ inflammatory mediator release. CB2 receptors may also be expressed on sensory nerve terminals and therefore directly inhibit hyperalgesia.

The role of CB2 in immunomodulation, inflammation, osteoporosis, cardiovascular, renal and other disease conditions is now being examined. In light of the fact that cannabinoids act on receptors capable of modulating different

functional effects, and in view of the low homology between CB2 and CB1, the importance of developing a class of drugs selective for the specific receptor sub-type is evident. The natural or synthetic cannabinoids currently available do not fulfil this function because they are active on both receptors.

Based on the foregoing, there is a need for compounds which are capable of selectively modulating the receptor for cannabinoids and, therefore, the pathologies associated with such receptors. Thus, CB2 modulators offer a unique approach toward the pharmacotherapy of immune disorders, inflammation, osteoporosis, renal ischemia and other pathophysiological conditions.

The present invention provides novel pyridine derivatives of formula (I) and pharmaceutically acceptable derivatives thereof, pharmaceutical compositions containing these compounds or derivatives, and their use as CB2 receptor modulators, which are useful in the treatment of a variety of disorders.

The present invention further comprises a method for treating disease mediated by CB2 receptors in an animal, including humans, which comprises administering to an animal in need thereof an effective amount of a compound of formula (I) or a pharmaceutically acceptable derivative thereof.

The invention provides compounds of formula (I):

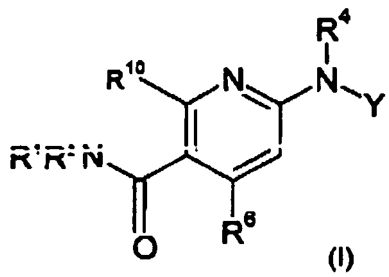

(I)

wherein:

Y is phenyl, unsubstituted or substituted with one, two or three substituents selected from $C_{1-6}$ alkyl, halosubstituted$C_{1-6}$ alkyl, $C_{1-6}$ alkoxy, a hydroxy group, a cyano group, halo, a $C_{1-6}$alkylsulfonyl group, $-CONH_2$, $-NHCOCH_3$, -COOH, halosubstituted$C_{1-6}$ alkoxy, $SO_2NR^{8a}R^{8b}$ wherein $R^{8a}$ and $R^{8b}$ are each independently H, $C_{1-6}$alkyl or $C_{1-6}$ alkynyl;

$R^1$ is selected from hydrogen, $C_{1-6}$ alkyl, $C_{3-6}$cycloalkyl, or halosubstituted$C_{1-6}$ alkyl;

$R^2$ is $(CH_2)_mR^3$ where m is 0 or 1;

or $R^1$ and $R^2$ together with N to which they are attached form a 4- to 8- membered non-aromatic heterocyclyl ring which is unsubstituted or substituted with 1, 2 or 3 substituents selected from: $C_{1-6}$ alkyl, $C_{1-6}$ alkoxy, a hydroxy group, a cyano group, halo, a sulfonyl group, methylsulfonyl, $NR^{8a}$ $R^{8b}$, $CH_2$phenyl, $NHCOCH_3$, (=O), $CONHCH_3$ or $NHSO_2CH_3$ wherein $R^{8a}$ and $R^{8b}$ are as defined above;

$R^3$ is a 4- to 8- membered non-aromatic heterocyclyl group, a $C_{3-8}$ cycloalkyl group, a straight or branched $C_{1-10}$ alkyl, a $C_{2-10}$alkenyl, a $C_{3-8}$cycloalkenyl, a $C_{2-10}$alkynyl, or a $C_{3-8}$ cycloalkynyl any of which can be unsubtituted or substituted with 1, 2 or 3 substituents preferably selected from: $C_{1-6}$ alkyl, $C_{1-6}$ alkoxy, a hydroxy group, a cyano group, halo, a sulfonyl group, methylsulfonyl, $NR^{8a}$ $R^{8b}$, $CH_2$phenyl, $NHCOCH_3$, (=O), $CONHCH_3$ or $NHSO_2CH_3$ wherein $R^{8a}$ and $R^{8b}$ are as defined above, or $R^5$;

$R^4$ is selected from hydrogen, $C_{1-6}$ alkyl, $C_{3-6}$ cycloalkyl, or halosubstituted$C_{1-6}$ alkyl, $COCH_3$, or $SO_2Me$;

$R^5$ is

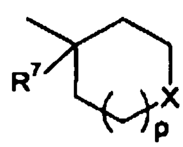

wherein p is 0, 1 or 2, and X is $CH_2$, O, or S;

$R^6$ is chloro or a $(C_{1-6})$alkyl optionally substituted with 1, 2 or 3 substitutents selected from hydroxy, $C_{1-6}$alkyoxy, cyano, halo, $NR^{8a}$ $R^{8b}$,

$CONR^{8a}R^{8b}$, $SO_2NR^{8a}R^{8b}$, $NR^{8a}COR^{8b}$ or $NR^{8a}$ $SO_2R^{8b}$ wherein $R^{8a}$ and $R^{8b}$ are as defined above and $R^{10}$ is hydrogen, or $R^{10}$ is chloro or $(C_{1-6})$alkyl optionally substituted with 1, 2 or 3 substitutents selected from hydroxy, $C_{1-6}$alkyoxy, cyano, halo, $NR^{8a}$ $R^{8b}$, $CONR^{8a}R^{8b}$, $SO_2NR^{8a}R^{8b}$, $NR^{8a}COR^{Bb}$ or $NR^{8a}$ $SO_2R^{8b}$ wherein $R^{8a}$ and $R^{8b}$

are as defined above and $R^6$ is hydrogen;
$R^7$ is OH, $C_{1-6}$alkoxy, $NR^{8a}R^{8b}$, $NHCOR^9$, $NHSO_2R^8$ or $SOqR^9$:
$R^{8a}$ is H or $C_{1-6}$alkyl;
$R^{8b}$ is H or $C_{1-6}$alkyl;
$R^9$ is $C_{1-6}$alkyl;
q is 0, 1 or 2;

and pharmaceutically acceptable derivatives thereof.

In one particular embodiment Y is a substituted phenyl. In one particular embodiment Y is substituted by 1 or 2 substituents. If mono- substituted, in one particular embodiment, the substituents is in the 3 position.

When Y is substituted, the substituent or substituents are preferably selected from: $C_{1-6}$ alkyl, halosubstituted$C_{1-8}$ alkyl, $C_{1-8}$ alkoxy, a hydroxy group, a cyano group, halo, a $C_{1-6}$alkylsulfonyl group, $-CONH_2$, $-NHCOCH_3$ or -COOH. Further the substituent or substituents can be selected from halosubstituted$C_{1-6}$ alkoxy, $SO_2NR^{8a}R^{8b}$ wherein $R^{8a}$ and $R^{8b}$ are as defined above or $C_{1-6}$ alkynyl. In one particular embodiment Y is substituted by halo, cyano, methoxy, trifluoromethoxy or methyl.

A further aspect of the invention are compounds of formula (Ia):

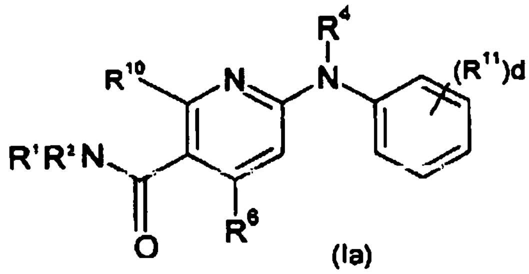

(Ia)

wherein:

$R^1$ is selected from hydrogen, $C_{1-6}$ alkyl, $C_{3-6}$ cycloalkyl, or halosubstituted$C_{1-6}$ alkyl;
$R^2$ is $(CH_2)_mR^3$ where m is 0 or 1;
or $R^1$ and $R^2$ together with N to which they are attached form a non-aromatic heterocyclyl ring selected from azetidinyl, pyrrolidinyl, morpholinyl, piperazinyl, piperidinyl, tetrahydropyridinyl, azapine, oxapine, azacyclooctanyl, azaoxacyclooctanyl and azathiacyclooctanyl, any of which can be unsubstituted or substituted with 1, 2 or 3 substituents selected from; $C_{1-6}$ alkyl, $C_{1-6}$ alkoxy, hydroxy, cyano, halo, sulfonyl, methylsulfonyl, $NR^{8a}R^{8b}$, $CH_2$phenyl, $NHCOCH_3$, (=O), $CONHCH_3$ and $NHSO_2CH_3$;
$R^3$ is 2- or 3- azetidinyl, oxetanyl, thioxetanyl, thioxetanyl-s-oxide, thioxetanyl-s,s-dioxide, dioxalanyl, pyrrolidinyl, tetrahydrofuranyl, tetrahydrothiophenyl, tetrahydrothiophenyl-s,s-dioxide, morpholinyl, piperidinyl, piperazinyl, tetrahydropyranyl, tetrahydrothiopyranyl, thiomorpholinyl, thiamorpholinyi-s,s-dioxide, tetrahydropyridinyl, dioxanyl, tetrahydro-thiopyran 1,1 dioxide, azapine, oxapine, azacyclooctanyl, azaoxacyclooctanyl, azathiacyclooctanyl, oxacylcooctanyl, thiacyclooctanyl, a $C_{3-8}$ cycloalkyl group, a straight or branched $C_{1-10}$ alkyl, a $C_{2-10}$alkenyl, a $C_{3-8}$cycloalkenyl, a $C_{2-10}$ alkynyl, or a $C_{3-8}$cycloalkynyl; any of which can be unsubstituted or substituted with 1, 2 or 3 substituents selected from $C_{1-6}$ alkyl, $C_{1-6}$ alkoxy, hydroxy, cyano, halo, sulfonyl, methylsulfonyl, $NR^{8a}R^{8b}$, $CH_2$phenyl, $NHCOCH_3$, (=O), $CONHCH_3$ and $NHSO_2CH_3$; or $R^3$ is $R^5$;
$R^4$ is selected from hydrogen, $C_{1-6}$ alkyl, $C_{3-6}$ cycloalkyl, or halosubstituted$C_{1-6}$ alkyl, $COCH_3$, or $SO_2Me$;
$R^5$ is

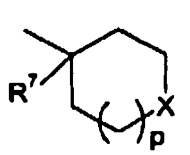

wherein p is 0, 1 or 2, and X is $CH_2$, O or S;
$R^6$ is chloro or $(C_{1-6})$alkyl optionally substituted with 1, 2 or 3 substitutents selected from hydroxy, $C_{1-6}$alkyoxy, cyano, halo, $NR^{8a}R^{8b}$, $CONR^{8a}R^{8b}$, $SO_2NR^{8a}R^{8b}$, $NR^{8a}COR^{8b}$ or $NR^{8a}SO_2R^{8b}$ wherein $R^{8a}$ and $R^{8b}$ are as defined

above and $R^{10}$ is hydrogen, or $R^{10}$ is chloro or $(C_{1-6})$alkyl optionally substituted with 1, 2 or 3 substitutents selected from hydroxy, $C_{1-6}$alkyoxy, cyano, halo, $NR^{8a}R^{8b}$, $CONR^{8a}R^{8b}$, $SO_2NR^{8a}R^{8b}$, $NR^{8a}COR^{8b}$ or $NR^{8a}SO_2R^{8b}$ wherein $R^{8a}$ and $R^{8b}$ are as defined above and $R^6$ is hydrogen; $R^7$ is OH, $C_{1-6}$ alkoxy, $NR^{8a}R^{8b}$, $NHCOR^9$, $NHSO_2R^9$ or $SOqR^9$;

$R^{8a}$ is H or $C_{1-6}$alkyl;

$R^{8b}$ is H or $C_{1-6}$alkyl;

$R^8$ is $C_{1-6}$alkyl;

$R^{11}$ is $C_{1-6}$ alkyl, halosubstituted$C_{1-6}$ alkyl, $C_{1-6}$ alkoxy, hydroxy, cyano, halo, $C_{1-6}$ alkylsulfonyl group, $-CONH_2$, $-NHCOCH_3$, -COOH, halosubstituted $C_{1-6}$ alkoxy $SO_2NR^{8a}R^{8b}$ or $C_{1-6}$ alkynyl;

q is 0, 1 or 2;

d is 0,1, 2, or 3;

and pharmaceutically acceptable derivatives thereof.

In one particular embodiment $R^1$ is hydrogen.

In one particular embodiment $R^4$ is $C_{1-6}$ alkyl or hydrogen, more preferably methyl or hydrogen, even more preferably hydrogen.

In one particular embodiment X is $CH_2$ or O.

When $R^1$ and $R^2$ together with N to which they are attached form a 4- to 8-membered non-aromatic heterocyclyl ring which is substituted, or when $R^3$ is substituted, they may be substituted with 1, 2 or 3 substituents preferably selected from: $C_{1-6}$ alkyl, $C_{1-6}$ alkoxy, a hydroxy group, a cyano group, halo or a sulfonyl group. Additionally the optional substituent (s) can be selected from methylsulfonyl, $NR^{8a}R^{8b}$, $CH_2$phenyl, $NHCOCH_3$, (=O), $CONHCH_3$ or $NHSO_2CH_3$ wherein $R^{8a}$ and $R^{8b}$ are as defined for formula (I).

When $R^6$ or $R^{10}$ are substituted alkyl groups, they can be substituted with 1, 2 or 3 substitutents selected from hydroxy, $C_{1-6}$alkyoxy, cyano, halo, $NR^{8a}R^{8b}$, $CONR^{8a}R^{8b}$, $SO_2NR^{8a}R^{8b}$, $NR^{8a}COR^{8b}$ or $NR^{8a}SO_2R^{8b}$, preferably hydroxy or fluorine.

In one particular embodiment $R^1$ and $R^2$ together with the N to which they are attached form an optionally substituted 5-or 6- membered non-aromatic heterocyclyl ring.

In one particular embodiment $R^8$ is a substituted or unsubstituted $(C_{1-6})$alkyl, chloro or CHxFn wherein n is 1, 2, or 3, x is 0, 1 or 2 and n and x add up to 3 and $R^{10}$ is hydrogen or $R^{10}$ is a substituted or unsubstituted $(C_{1-6})$alkyl, chloro or CHxFn wherein n is 1, 2, or 3, x is 0, 1 or 2 and n and x add up to 3 and $R^6$ is hydrogen

In one particular embodiment $R^6$ is *t*-butyl, isopropyl or CHxFn, more preferably $R^6$ is isopropyl or CHxFn even more preferably isopropyl or $CF_3$ and $R^{10}$ is hydrogen or $R^{10}$ is *t*-butyl, Isopropyl or CHxFn, more preferably $R^{10}$ is isopropyl or CHxFn, more preferably isopropyl or $CF_3$ and $R^6$ is hydrogen.

In one particular embodiment $R^{10}$ is hydrogen.

In one particular embodiment $R^7$ is OH.

In one particular embodiment $R^6$ is

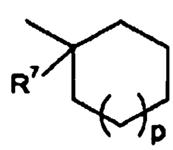

wherein p is 0,1 or 2.

In one particular embodiment when $R^3$ is an optionally substituted $C_{3-8}$cycloalkyl group or an optionally substituted 4- to 8- membered nonaromatic heterocyclyl, m is 1.

In one particular embodiment $R^3$ is an optionally substituted $C_{3-8}$cycloalkyl group or an optionally substituted 4- or 6- membered nonaromatic heterocyclyl.

In one particular embodiment when $R^1$ and $R^2$ taken together with the N to which they are attached form an optionally substituted heterocyclyl ring, the ring may be selected from pyrrolidinyl, morpholinyl, piperazinyl, piperidinyl and tetrahydropyridinyl.

In one particular embodiment when $R^3$ is an optionally substituted non-aromatic heterocyclyl group selected from dioxalanyl, pyrrolidinyl, tetrahydrofuranyl, tetrahydrothiophenyl, tetrahydrothiophenyl-s,s-dioxide, morpholinyl, piperidinyl, piperazinyl, tetrahydropyranyl, tetrahydrothiopyranyl, dioxanyl, thiomorpholinyl, dioxanyl, thiomorpholinyl-s,s-dioxide and tetrahydropyridinyl.

A further aspect of the invention are compounds of formula (Ib):

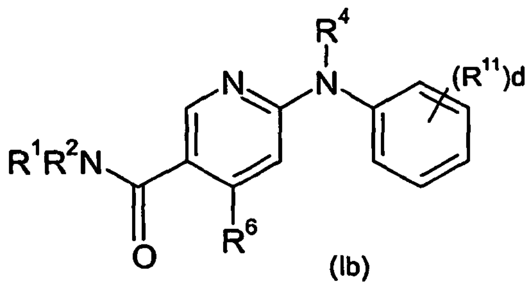

(Ib)

wherein:

$R^1$ is selected from hydrogen;
$R^2$ is $(CH_2)_mR^3$where m is 0 or 1;
or $R^1$ and $R^2$ together with N to which they are attached form pyrrolidinyl, morpholinyl, piperazinyl, piperidinyl, tetrahydropyridinyl, any of which can be unsubstituted or substituted with 1, 2 or 3 substituents selected from $C_{1-6}$ alkyl, $C_{1-6}$ alkoxy, hydroxy, cyano, halo, sulfonyl, methylsulfonyl, $NR^{8a}R^{8b}$, $CH_2$phenyl, $NHCOCH_3$, (=O), $CONHCH_3$ and $NHSO_2CH_3$;
$R^3$ is dioxalanyl, pyrrolidinyl, tetrahydrofuranyl, tetrahydrothiophenyl, tetrahydrothiophenyl-s,s-dioxide, morpholinyl, piperidinyl, piperazinyl, tetrahydropyranyl, tetrahydrothiopyranyl, thiomorpholinyl, thiomorpholinyl-s,s-dioxide, dioxanyl, tetrahydropyridinyl, a $C_{3-8}$ cycloalkyl group, a straight or branched $C_{1-10}$ alkyl; any of which can be unsubstituted or substituted with 1, 2 or 3 substituents selected from $C_{1-6}$ alkyl, $C_{1-6}$ alkoxy, hydroxy, cyano, halo, sulfonyl, methylsulfonyl, $NR^{8a}R^{8b}$, $CH_2$phenyl, $NHCOCH_3$, (=O), $CONHCH_3$ or $NHSO_2CH_3$; or $R^5$.
$R^4$ is selected from hydrogen, $C_{1-6}$ alkyl, $C_{3-6}$ cycloalkyl, or halosubstituted$C_{1-6}$ alkyl, $COCH_3$, or $SO_2Me$;
$R^5$ is

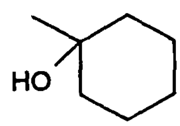

$R^6$ is a substituted or unsubstituted $(C_{1-6})$alkyl or chloro;
$R^{8a}$ is H or $C_{1-6}$alkyl;
$R^{8b}$ is H or $C_{1-6}$alkyl;
$R^{11}$ is $C_{1-6}$ alkyl, halosubstituted$C_{1-6}$ alkyl, $C_{1-6}$ alkoxy, hydroxy, cyano, halo, $C_{1-6}$ alkylsulfonyl group, $-CONH_2$, $-NHCOCH_3$, -COOH, halosubstituted $C_{1-6}$ alkoxy, $SO_2NRSaR^{8a}R^{8b}$ or $C_{1-6}$ alkynyl;
d is 0,1, 2, or 3;

and pharmaceutically acceptable derivatives thereof.

Alternatively compounds of formula (I) can be selected from compounds of formula (Ic);

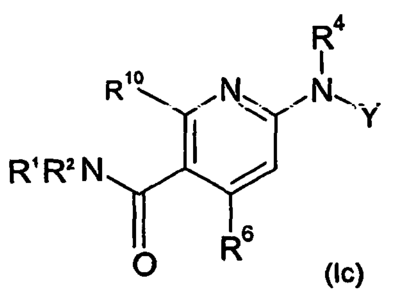

(Ic)

wherein:

Y is phenyl, optionally substituted with one, two or three substituents;

$R^1$ is selected from hydrogen, $C_{1-6}$ alkyl, $C_{3-6}$ cycloalkyl, or halosubstituted$C_{1-6}$ alkyl;
$R^2$ is $(CH_2)_mR^3$ where m is 0 or 1;
or $R^1$ and $R^2$ together with N to which they are attached form an optionally substituted 5- or 6- membered non-aromatic heterocyclyl ring;
$R^3$ is an optionally substituted 4- to 8- membered non-aromatic heterocyclyl group, an optionally substituted $C_{3-8}$ cycloalkyl group, an optionally substituted straight or branched $C_{1-10}$ alkyl or $R^5$;
$R^4$ is selected from hydrogen, $C_{1-6}$ alkyl, $C_{3-6}$ cycloalkyl, or halosubstituted$C_{1-6}$ alkyl, $COCH_3$, or $SO_2Me$;
$R^5$ is

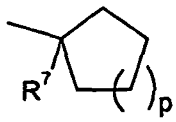

wherein p is 0, 1 or 2;
$R^6$ is $(C_{1-6})$alkyl, chloro or CHxFn wherein n is 1, 2, or 3, x is 0, 1 or 2 and n and x add up to 3 and $R^{10}$ is hydrogen or $R^{10}$ is $(C_{1-6})$alkyl, chloro or CHxFn wherein n is 1, 2, or 3, x is 0, 1 or 2 and n and x add up to 3 and $R^6$ is hydrogen;
$R^7$ is OH, $C_{1-6}$alkoxy, $NR^{8a}R^{8b}$, $NHCOR^9$, $NHSO_2R^9$, $SOqR^9$;
$R^{8a}$ is H or $C_{1-6}$alkyl;
$R^{8b}$ is H or $C_{1-6}$alkyl;
$R^9$ is $C_{1-6}$alkyl;
q is 0, 1 or 2;

and pharmaceutically acceptable derivatives thereof.

In one particular embodiment the compounds are selective for CB2 over CB1. Preferably the compounds are 100 fold selective i.e. compounds of formula (I) have an EC50 value at the cloned human cannabinoid CB2 receptor of at least 100 times the $EC_{50}$ values at the cloned human cannabinoid CB1 receptor or have less than 10% efficacy at the CB1 receptor.

The invention is described using the following definitions unless otherwise indicated.

The term "pharmaceutically acceptable derivative" means any pharmaceutically acceptable salt, ester, salt of such ester or solvate of the compounds of formula (I), or any other compound which upon administration to the recipient is capable of providing (directly or indirectly) a compound of formula (I) or an active metabolite or residue thereof.

It will be appreciated by those skilled in the art that compounds of formula (I) may be modified to provide pharmaceutically acceptable derivatives thereof at any of the functional groups in the compounds, and that the compounds of formula (I) may be derivatised at more than one position.

It will be appreciated that, for pharmaceutical use, the salts referred to above will be physiologically acceptable salts, but other salts may find use, for example in the preparation of compounds of formula (I) and the physiological acceptable salts thereof. Pharmaceutically acceptable salts include those described by Berge, Bighley and Monkhouse, J. Pharm. Sci., 1977, 66, 1-19. The term "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropyl amine, tromethamine, and the like. When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like.

Preferred examples of pharmaceutically acceptable salts include the ammonium, calcium, magnesium, potassium, and sodium salts, and those formed from maleic, fumaric, benzoic, ascorbic, pamoic, succinic, hydrochloric, sulfuric, bismethylenesalicylic, methanesulfonic, ethanedisulfonic, propionic, tartaric, salicylic, citric, gluconic, aspartic, stearic, palmitic, itaconic, glycolic, p-aminobenzoic, glutamic, benzenesulfonic, cyclohexylsulfamic, phosphoric and nitric acids.

The terms 'halogen or halo' are used to represent fluorine, chlorine, bromine or iodine.

The term 'alkyl' as a group or part of a group means a straight or branched chain alkyl group or combinations thereof, for example a methyl, ethyl, n-propyl, i-propyl, n-butyl, s-butyl, t-butyl, pentyl, hexyl, 1,1-dimethylethyl, or combinations thereof.

The term 'alkoxy' as a group or as part of a group means a straight, branched or cyclic chain alkyl group having an oxygen atom attached to the chain, for example a methoxy, ethoxy, n-propoxy, i-propoxy, n-butoxy, s-butoxy, t-butoxy group, pentoxy, hexyloxy group, cyclopentoxy or cyclohexyloxy group.

The term 'cycloalkyl' means a closed non-aromatic carbon ring, for example cyclobutyl, cyclopentyl, cyclohexyl or cycloheptyl, or cyclooctyl.

The term 'alkenyl' as a group or part of a group means a straight or branched chain carbon chain or combinations containing 1 or more double bonds for example an ethenyl, n-propenyl, i-propenyl, butenyl, pentenyl, hexenyl or combinations thereof.

The term 'cycloalkenyl' means a closed non-aromatic carbon ring containing 1 or more double bonds, for example cyclobutenyl, cyclopentenyl, cyclohexenyl or cycloheptenyl, or cyclooctenyl.

The term 'alkynyl' as a group or part of a group means a straight or branched chain carbon chain or combinations containing 1 or more triple carbon bonds for example a ethynyl, propynyl, butynyl, pentynyl, hexynyl or combinations thereof.

The term 'cycloalkynyl' means a closed non-aromatic carbon ring containing 1 or more triple bonds, for example cyclobutynyl, cyclopentynyl, cyclohexynyl or cycloheptynyl, or cyclooctynyl.

When $R^1$ and $R^2$ taken together with the N to which they are attached form an optionally substituted heterocyclyl ring, the ring may optionally contain 1, 2, 3 or 4 further hetero atoms. The ring may be saturated or unsaturated. Preferably the further hetero atoms are selected from oxygen, nitrogen or sulphur. An example of a 4- membered heterocyclyl ring is azetidinyl Examples of 5- membered heterocyclyl rings include pyrrolidinyl, Examples of 6-membered heterocyclyl rings are morpholinyl, piperazinyl or piperidinyl. An additional example is tetrahydropyridinyl. Examples of a 7- membered heterocyclyl ring are azapine or oxapine. Examples of 8-membered heterocyclyl rings are azacyclooctanyl, azaoxacyclooctanyl or azathiacyclooctanyl.

When $R^3$ is an optionally substituted non-aromatic heterocyclyl group, the ring may contain 1, 2, 3, or 4 hetero atoms. Preferably the hetero atoms are selected from oxygen, nitrogen or sulphur. Examples of 4- membered groups are 2- or 3- azetidinyl, oxetanyl, thioxetanyl, thioxetanyl-s-oxide and thioxetanyl-s,s-dioxide. Examples of 5- membered heterocyclyl groups in this instance include dioxalanyl, pyrrolidinyl, tetrahydrofuranyl and tetrahydrothiophenyl. Additionally it can be tetrahydrothiophenyl-s,s-dioxide. Examples of 6-membered heterocyclyl groups are morpholinyl, piperidinyl, piperazinyl, tetrahydropyranyl, tetrahydrothiopyranyl, thiomorpholinyl and thiomorpholinyl-s,s-dioxide. Additional examples are tetrahydropyridinyl, dioxanyl, and tetrahydro-thiopyran 1,1 dioxide. Examples of a 7- membered heterocyclyl ring are azapine or oxapine.

Examples of 8- membered groups are azacyclooctanyl, azaoxacyclooctanyl or azathiacyclooctanyl, oxacylcooctanyl, or thiacyclooctanyl.

In one particular embodiment compounds of the present invention can be selected from:

6-(3-Chloro-phenyl-amino)-4-isopropyl-N-(tetrahydro-pyran-4-ylmethyl)-nicotinamide;
6-(3-Bromo-phenyl-amino)-4-isopropyl-N-(tetrahydro-pyran-4-ylmethyl)-nicotinamide;
6-(2,4-Dichloro-phenylamino)-4-isopropyl-N-(tetrahydro-pyran-4-ylmethyl)-nicotinamide;
4-Isopropyl-N-(tetrahydro-pyran-4-ylmethyl)-6-(3-trifluoromethoxy-phenylamino)-nicotinamide;
4-*tert*-Butyl-6-(2,4-di-chloro-phenylamino)-N-(tetrahydro-pyran-4-ylmethyl)-nicotinamide;
6-(3-Chloro-4-cyano-phenylamino)-4-isopropyl-N-(tetrahydro-pyran-4-ylmethyl)-nicotinamide;
6-(2-Fluoro-3-trifluoromethyl-phenylamino)-4-isopropyl-N-(tetrahydro-pyran-4-ylmethyl)-nicotinamide;
6-(4-Bromo-2-chloro-phenylamino)-4-isopropyl-N-(tetrahydro-pyran-4-ylmethyl)-nicotinamide;
6-(3,4-Dichloro-phenylamino)-4-isopropyl-N-(tetrahydro-pyran-4-ylmethyl)-nicotinamide;
6-(2-Bromo-4-trifluoromethoxy-phenylamino)-4-isopropyl-*N*-(tetrahydro-pyran-4-ylmethyl)-nicotinamide;
6-(3,5-Difluoro-phenylamino)-4-isopropyl-*N*-(tetrahydro-pyran-4-ylmethyl)-nicotinamide;
6-(2,4-Dichloro-phenylamino)-N-(tetrahydro-pyran-4-ylmethyl)-4-trifluoromethyl-nicotinamide;

and pharmaceutically acceptable derivatives thereof.

Compounds of formula (1) can be prepared as set forth in scheme 1:

**Scheme 1**

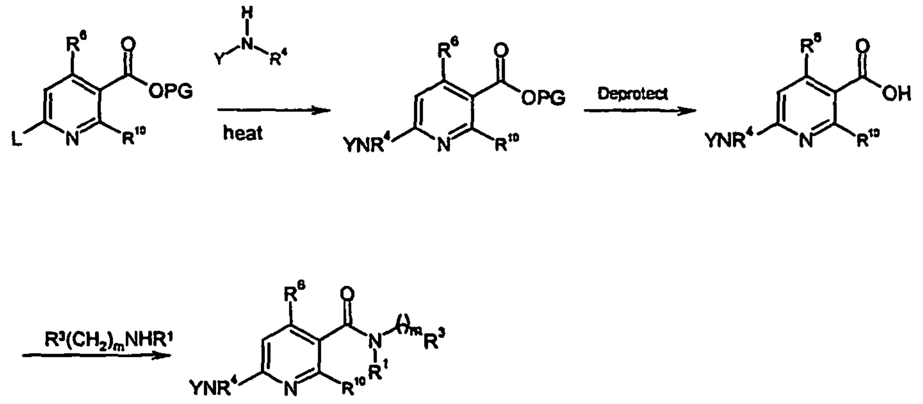


wherein L is a leaving group, for example halo, PG is a protecting group for example methyl, ethyl or benzyl, and $R^1$, $R^3$, $R^4$, $R^6$, $R^{10}$, m and Y are as defined for compounds of formula (I).

Alternatively compounds of formula (I) can be prepared as shown in scheme 2.

**Scheme 2**

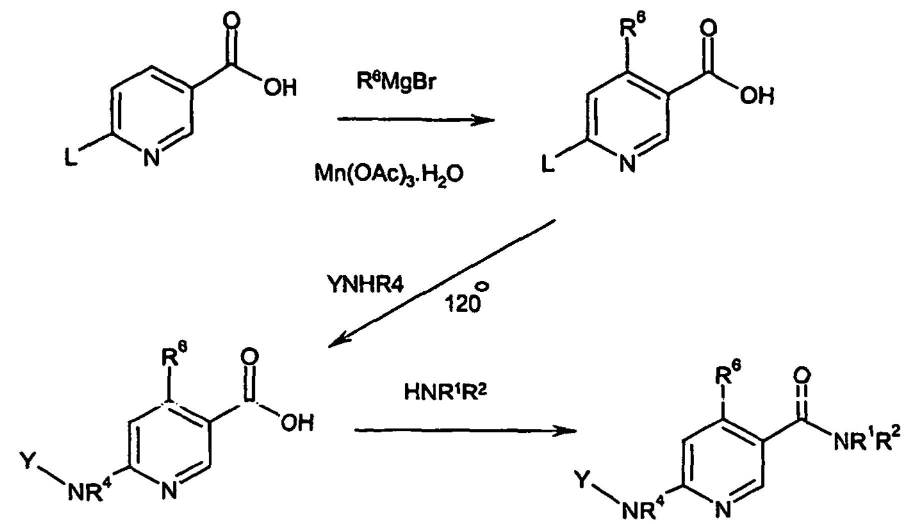


wherein L is a leaving group, for example halo e.g. chloro, and $R^1$, $R^2$, $R^4$ and Y are as defined for compounds of formula (I).

Furthermore compounds of formula (I) when $R^{10}$ is unsubstituted or substituted $(C_{1-6})$ alkyl or chloro and $R^6$ is hydrogen can be prepared as shown in scheme 3.

**Scheme 3**

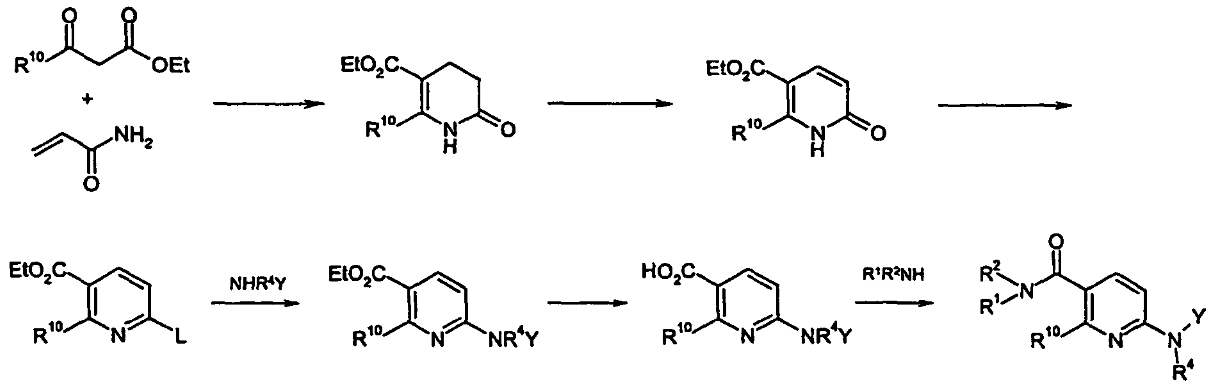


wherein L is a leaving group for example halogen, e.g. chloro, $R^1$, $R^2$, Y, $R^4$ are as defined for compounds of formula (I).

Furthermore compounds of formula (I) when $R^{10}$ is unsubstituted or substituted ($C_{1-6}$) alkyl or chloro and $R^6$ is hydrogen can be prepared as shown in scheme 4.

**Scheme 4:**

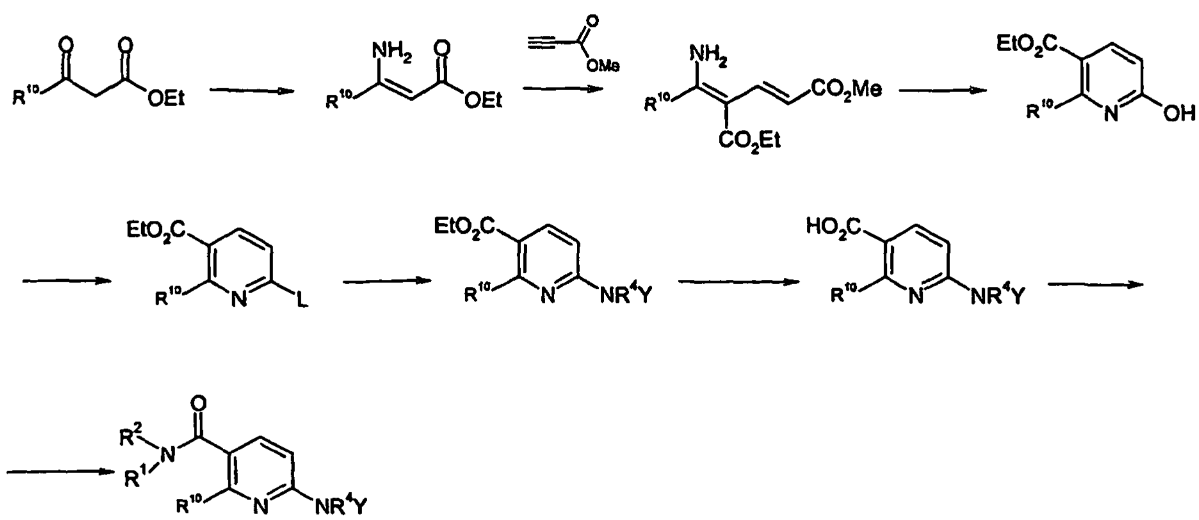

wherein L is a leaving group for example halogen, e.g. chloro, $R^1$, $R^2$, Y, $R^4$ are as defined for compounds of formula (I).

It is to be understood that the present invention encompasses all isomers of compounds of formula (I) and their pharmaceutically acceptable derivatives, including all geometric, tautomeric and optical forms, and mixtures thereof (e.g. racemic mixtures). Where additional chiral centres are present in compounds of formula (I), the present invention includes within its scope all possible diastereoismers, including mixtures thereof. The different isomeric forms may be separated or resolved one from the other by conventional methods, or any given isomer may be obtained by conventional synthetic methods or by stereospecific or asymmetric syntheses.

The subject invention also includes isotopically-labeled compounds, which are identical to those recited in formulas I and following, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as $^{3}H$, $^{11}C$, $^{14}C$, $^{18}F$, $^{123}I$ and $^{125}I$.

Compounds of the present invention and pharmaceutically acceptable salts of said compounds that contain

the aforementioned isotopes and/or other isotopes of other atoms are within the scope of the present invention. Isotopically-labeled compounds of the present invention, for example those into which radioactive isotopes such as $^3H$, $^{14}C$ are incorporated, are useful in drug and/or substrate tissue distribution assays. Tritiated, i.e., $^3H$, and carbon-14, i.e., $^{14}C$, isotopes are particularly preferred for their ease of preparation and detectability. $^{11}C$ and $^8F$ isotopes are particularly useful in PET (positron emission tomography), and $^{125}I$ isotopes are particularly useful in SPECT (single photon emission computerized tomography), all useful in brain imaging. Further, substitution with heavier isotopes such as deuterium, i.e., $^2H$, can afford certain therapeutic advantages resulting from greater metabolic stability, for example increased *in vivo* half-life or reduced dosage requirements and, hence, may be preferred in some circumstances. Isotopically labeled compounds of formula I and following of this invention can generally be prepared by carrying out the procedures disclosed in the Schemes and/or in the Examples below, by substituting a readily available isotopically labeled reagent for a non-isotopically labeled reagent.

The compounds of formula (I) may be prepared in crystalline or non-crystalline form, and, if crystalline, may optionally be hydrated or solvated. This invention includes within its scope stoichiometric hydrates or solvates as well as compounds containing variable amounts of water and/or solvent.

The compounds of the invention bind selectively to the CB2 receptor, and are therefore useful in treating CB2 receptor mediated diseases.

In view of their ability to bind to the CB2 receptor, the compounds of the invention may be useful in the treatment of the disorders that follow. Thus, the compounds of formula (I) may be useful as analgesics. For example they may be useful in the treatment of chronic inflammatory pain (e.g. pain associated with rheumatoid arthritis, osteo-arthritis, rheumatoid spondylitis, gouty arthritis and juvenile arthritis) including the property of disease modification and joint structure preservation; musculoskeletal pain; lower back and neck pain; sprains and strains; neuropathic pain; sympathetically maintained pain; myositis; pain associated with cancer and fibromyalgia; pain associated with migraine; pain associated with influenza or other viral infections, such as the common cold; rheumatic fever; pain associated with functional bowel disorders such as non-ulcer dyspepsia, non-cardiac chest pain and irritable bowel syndrome; pain associated with myocardial ischemia; post operative pain; headache; toothache; dysmenorrhea, chronic pain, dental pain algesia, pelvic pain, poststroke pain and menstrual pain.

The compounds of the invention may also be useful disease modification or joint structure preservation in multiple sclerosis, rheumatoid arthritis, osteo-arthritis, rheumatoid spondylitis, gouty arthritis and juvenile arthritis.

The compounds of the invention may be particularly useful in the treatment of neuropathic pain. Neuropathic pain syndromes can develop following neuronal injury and the resulting pain may persist for months or years, even after the original injury has healed. Neuronal injury may occur in the peripheral nerves, dorsal roots, spinal cord or certain regions in the brain. Neuropathic pain syndromes are traditionally classified according to the disease or event that precipitated them. Neuropathic pain syndromes include: diabetic neuropathy; sciatica; non-specific lower back pain; multiple sclerosis pain; fibromyalgia; HIV-related neuropathy; post-herpetic neuralgia; trigeminal neuralgia; and pain resulting from physical trauma, amputation, cancer, toxins or chronic inflammatory conditions. These conditions are difficult to treat and although several drugs are known to have limited efficacy, complete pain control is rarely achieved. The symptoms of neuropathic pain are incredibly heterogeneous and are often described as spontaneous shooting and lancinating pain, or ongoing, burning pain. In addition, there is pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

The compounds of formula (I) may also be useful in the treatment of fever.

The compounds of formula (I) may also be useful in the treatment of inflammation, for example in the treatment of skin conditions (e.g. sunburn, bums, eczema, dermatitis, psoriasis); ophthalmic diseases such as glaucoma, retinitis, retinopathies, uveitis and of acute injury to the eye tissue (e.g. conjunctivitis); lung disorders (e.g. asthma, bronchitis, emphysema, allergic rhinitis, respiratory distress syndrome, pigeon fancier's disease, farmer's lung, chronic obstructive pulmonary disease, (COPD); cough, gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastroesophageal reflux disease emesis, oesophagitis, organ transplantation; other conditions with an inflammatory component such as vascular disease, migraine, periarteritis nodosa, thyroiditis, aplastic anaemia, Hodgkin's disease, sclerodoma, myaesthenia gravis, multiple sclerosis, sorcoidosis, nephrotic syndrome, Bechet's syndrome, polymyositis, gingivitis, myocardial ischemia, pyrexia, systemic lupus erythematosus, tendinitis, bursitis, and Sjogren's syndrome.

The compounds of formula (I) may also be useful in the treatment of bladder hyperrelexia following bladder inflammation.

The compounds of formula (I) are also useful in the treatment of immunological diseases such as autoimmune diseases, immunological deficiency diseases or organ transplantation. The compounds of formula (I) are also effective

in increasing the latency of HIV infection.

The compounds of formula (I) are also useful in the treatment of diseases of abnormal platelet function (e.g. occlusive vascular diseases).

The compounds of formula (I) are also useful in the treatment of neuritis, heart bum, dysphagia, pelvic hypersensitivity, urinary incontinence, cystitis or pruritis.

The compounds of formula (I) are also useful for the preparation of a drug with diuretic action.

The compounds of formula (I) are also useful in the treatment of impotence or erectile dysfunction.

The compounds of formula (I) are also useful for attenuating the hemodynamic side effects of non-steroidal anti-inflammatory drugs (NSAID's) and cyclooxygenase-2 (COX-2) inhibitors.

The compounds of formula (I) are also useful in the treatment of neurodegenerative diseases and neurodegeneration such as dementia, particularly degenerative dementia (including senile dementia, Alzheimer's disease, Pick's disease, Huntingdon's chorea, Parkinson's disease and Creutzfeldt-Jakob disease, motor neuron disease); vascular dementia (including multi-infarct dementia); as well as dementia associated with intracranial space occupying lesions; trauma; infections and related conditions (including HIV infection); dementia in Parkinson's disease; metabolism; toxins; anoxia and vitamin deficiency; and mild cognitive impairment associated with ageing, particularly Age Associated Memory Impairment. The compounds may also be useful for the treatment of amyotrophic lateral sclerosis (ALS) and neuroinflamation.

The compounds of formula (I) are also useful in neuroprotection and in the treatment of neurodegeneration following stroke, cardiac arrest, pulmonary bypass, traumatic brain injury, spinal cord injury or the like.

The compounds of formula (I) are also useful in the treatment of tinnitus.

The compounds of formula (I) are also useful in the treatment of psychiatric disease for example schizophrenia, depression (which term is used herein to include bipolar depression, unipolar depression, single or recurrent major depressive episodes with or without psychotic features, catatonic features, melancholic features, atypical features or postpartum onset, seasonal affective disorder, dysthymic disorders with early or late onset and with or without atypical features, neurotic depression and social phobia, depression accompanying dementia for example of the Alzheimer's type, schizoaffective disorder or the depressed type, and depressive disorders resulting from general medical conditions including, but not limited to, myocardial infarction, diabetes, miscarriage or abortion, *etc*), anxiety disorders (including generalised anxiety disorder and social anxiety disorder), panic disorder, agoraphobia, social phobia, obsessive compulsive disorder and post-traumatic stress disorder, memory disorders, including dementia, amnesic disorders and age-associated memory impairment, disorders of eating behaviours, including anorexia nervosa and bulimia nervosa, sexual dysfunction, sleep disorders (including disturbances of circadian rhythm, dyssomnia, insomnia, sleep apnea and narcolepsy), withdrawal from abuse of drugs such as of cocaine, ethanol, nicotine, benzodiazepines, alcohol, caffeine, phencyclidine (phencyclidine-like compounds), opiates (e.g. heroin, morphine), amphetamine or amphetamine-related drugs (e.g. dextroamphetamine, methylamphetamine) or a combination thereof.

The compounds of formula (I) are also useful in preventing or reducing dependence on, or preventing or reducing tolerance or reverse tolerance to, a dependence - inducing agent. Examples of dependence inducing agents include opioids (e.g. morphine). CNS depressants (e.g. ethanol), psychostimulants (e.g. cocaine) and nicotine.

The compounds of formula (I) are also useful in the treatment of kidney dysfunction (nephritis, particularly mesangial proliferative glomerulonephritis, nephritic syndrome), liver dysfunction (hepatitis, cirrhosis), gastrointestinal dysfunction (diarrhoea) and colon cancer.

The compounds of formula (I) may useful for the treatment of bladder hyper-reflexia following bladder inflammation.

It is to be understood that references to treatment includes both treatment of established symptoms and prophylactic treatment unless explicitly stated otherwise.

According to a further aspect of the invention, we provide a compound of formula (I) or a pharmaceutically acceptable derivative thereof for use in human or veterinary medicine.

According to another aspect of the invention, we provide a compound of formula (I) or a pharmaceutically acceptable derivative thereof for use in the treatment of a condition which is mediated by the activity of cannabinoid 2 receptors.

The compounds of the invention may be used in a method of treating a human or animal subject suffering from a condition which is mediated by the activity of cannabinoid 2 receptors which comprises administering to said subject a therapeutically effective amount of a compound of formula (I) or a pharmaceutically acceptable derivative thereof.

The compounds of the invention may be used in a method of treating a human or animal subject suffering from an immune disorder, an inflammatory disorder, pain, rheumatoid arthritis, multiple sclerosis, osteoarthritis or osteoporosis which method comprises administering to said subject an effective amount of a compound of formula (I) or a pharmaceutically acceptable derivative thereof. Preferably the pain is selected from inflammatory pain, viseral pain, cancer pain, neuropathic pain, lower back pain, muscular sceletal, post operative pain, acute pain and migraine. More preferably the inflammatory pain is pain associated with rheumatoid arthritis or osteoarthritis.

According to another aspect of the invention is provided the use of a compound of formula (I) or a pharmaceutically acceptable derivative thereof for the manufacture of a therapeutic agent for the treatment or prevention of a condition such as an immune disorder, an inflammatory disorder, pain, rheumatoid arthritis, multiple sclerosis, osteoarthritis or osteoporosis

Preferably the pain is selected from inflammatory pain, viseral pain, cancer pain, neuropathic pain, lower back pain, muscular sceletal, post operative pain, acute pain and migraine. More preferably the inflammatory pain is pain associated with rheumatoid arthritis or osteoarthritis.

In order to use a compound of formula (I) or a pharmaceutically acceptable derivative thereof for the treatment of humans and other mammals it is normally formulated in accordance with standard pharmaceutical practice as a pharmaceutical composition. Therefore in another aspect of the invention is provided a pharmaceutical composition comprising a compound of formula (I) or a pharmaceutically acceptable derivative thereof adapted for use in human or veterinary medicine.

As used herein, "modulator" means both antagonist, full or partial agonist and inverse agonist. In one embodiment of the present modulators are agonists.

The term "treatment" or "treating" as used herein includes the treatment of established disorders and also includes the prophylaxis thereof. The term " prophylaxis" is used herein to mean preventing symptoms in an already afflicted subject or preventing recurrence of symptoms in an afflicted subject and is not limited to complete prevention of an affliction.

Compounds of formula (I) and their pharmaceutically acceptable derivatives may be administered in a standard manner for the treatment of the Indicated diseases, for example orally, parentarally, sub-lingually, dermally, intranasally, transdermally, rectally, via inhalation or via buccal administration.

Compositions of formula (I) and their pharmaceutically acceptable derivatives which are active when given orally can be formulated as syrups, tablets, capsules and lozenges. A syrup formulation will generally consist of a suspension or solution of the compound or salt in a liquid carrier for example, ethanol, peanut oil, olive oil, glycerine or water with a flavouring or colouring agent. Where the composition is in the form of a tablet, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils, and are incorporated in a soft gelatin capsule shell.

Typical parenteral compositions consist of a solution or suspension of a compound or derivative in a sterile aqueous or non-aqueous carrier optionally containing a parenterally acceptable oil, for example polyethylene glycol, polyvinylpyrrolidone, lecithin, arachis oil or sesame oil.

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of formula (I) or a pharmaceutically acceptable derivative thereof which is active when administered in this way, with a binding and/or lubricating agent, for example polymeric glycols, gelatins, cocoa-butter or other low melting vegetable waxes or fats or their synthetic analogs.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

Each dosage unit for oral administration contains suitably from 0.01mg/Kg to 500 mg/Kg for example 0.1 mg to 500 mg/Kg, and preferably from 0.01 mg to 100 mg/Kg for example 1mg/Kg to 100mg/Kg, and each dosage unit for parenteral administration contains suitably from 0.1 mg to 100 mg/Kg, of a compound of formula (I) or a pharmaceutically acceptable derivative thereof calculated as the free acid. Each dosage unit for intranasal administration contains suitably 1-400 mg and preferably 10 to 200 mg per person. A topical formulation contains suitably 0.01 to 5.0% of a compound of formula (I).

The daily dosage regimen for oral administration is suitably about 0.01 mg/Kg to 40 mg/Kg, of a compound of formula (I) or a pharmaceutically acceptable derivative thereof calculated as the free acid. The daily dosage regimen for parenteral administration is suitably about 0.001 mg/Kg to 40 mg/Kg, of a compound of formula (I) or a pharmaceutically acceptable derivative thereof calculated as the free acid. The daily dosage regimen for intranasal administration and oral inhalation is suitably about 10 to about 500 mg/person. The active ingredient may be administered from 1 to 6 times a day, sufficient to exhibit the desired activity.

It may be advantageous to prepare the compounds of the present invention as nanoparticles. This may improve the oral bioavailability of the compounds. For the purposes of the present invention "nanoparticulate" is defined as solid

particles with 50% of the particles having a particle size of less than 1μm, more preferably less than 0.75μm

The particle size of the solid particles of compound (I) may be determined by laser diffraction. A suitable machine for determining particle size by laser diffraction is a Lecotrac laser particle size analyser, using an HELOS optical bench fitted with a QUIXEL dispersion unit.

Numerous processes for the synthesis of solid particles in nanoparticulate form are known. Typically these processes involve a milling process, preferably a wet milling process in the presence of a surface modifying agent that inhibits aggregation and/or crystal growth of the nanoparticles once created. Alternatively these processes may involve a precipitation process, preferably a process of precipitation in an aqueous medium from a solution of the drug in a non-aqueous solvent.

Accordingly, in a further aspect, the present invention provides a process for preparing compound (I) in nanoparticulate form as hereinbefore defined, which process comprises milling or precipitation.

Representative processes for the preparation of solid particles in nanoparticulate form are described in the patents and publications listed below.

U.S. Patent No. 4,826,689 to Violanto & Fischer, U. S. Patent No. 5,145,684 to Liversidge et al
U.S Patent No. 5,298,262 to Na & Rajagopalan, U.S. Patent No. 5,302,401 Liversidge et al
U.S. Patent No. 5,336,507 to Na & Rajagopalan, U.S. Patent No. 5,340,564 to Illig & Sarpotdar
U.S. Patent No. 5,346,702 to Na Rajagopalan, U.S. Patent No. 5,352,459 to Hollister et al U.S. Patent No. 5,354,560 to Lovrecich, U.S. Patent No. 5,384,124 to Courteille et al, U.S. Patent No. 5,429,824 to June, U.S. Patent No. 5,503,723 to Ruddy et al, U.S. Patent No. 5,510 118 to Bosch et al, U.S. Patent No. 5,518 to Bruno et al, U.S. Patent No. 5,518,738 to Eickhoff et al, U.S. Patent No. 5,534,270 to De Castro, U.S. Patent No. 5,536,508 to Canal et al, U.S. Patent No. 5,552,160 to Liversidge et al, U.S. Patent No. 5,560,931 to Eickhoff et al, U.S. Patent No. 5,560,932 to Bagchi et al, U.S. Patent No. 5,565,188 to Wong et al, U.S. Patent No. 5,571,536 to Eickhoff et al, U.S. Patent No. 5,573,783 to Desieno & Stetsko, U.S Patent No. 5,580,579 to Ruddy et al, U.S. Patent No 5,585,108 to Ruddy et al, U.S. Patent No. 5,587,143 to Wong, U.S. Patent No. 5,591456 to Franson et al, U.S. Patent No. 5,622,938 to Wong, U.S. Patent No 5,662,883 to Bagchi et al, U.S. Patent No. 5,665,331 to Bagchi et al, U.S Patent No. 5,718,919 to Ruddy et al, U.S. Patent No. 5,747,001 to Wiedmann et al, WO93/25190, WO96/24336, WO 97/14407, WO 98/35666, WO 99/65469, WO 00/18374, WO 00/27369, WO 00/30615 and WO 01/41760.

Such processes may be readily adapted for the preparation of compound (I) in nanoparticulate form. Such processes form a further aspect of the invention.

The process of the present invention preferably uses a wet milling step carried out in a mill such as a dispersion mill in order to produce a nanoparticulate form of the compound. The present invention may be put into practice using a conventional wet milling technique, such as that described in Lachman et al., The Theory and Practice of Industrial Pharmacy, Chapter 2, "Milling" p.45 (1986).

In a further refinement, WO02/00196 (SmithKline Beecham plc) describes a wet milling procedure using a mill in which at least some of the surfaces are made of nylon (polyamide) comprising one or more internal lubricants, for use in the preparation of solid particles of a drug substance in nanoparticulate form.

In another aspect the present invention provides a process for preparing compounds of the invention in nanoparticulate form comprising wet milling a suspension of compound in a mill having at least one chamber and agitation means, said chamber(s) and/or said agitation means comprising a lubricated nylon, as described in WO02/00196.

The suspension of a compound of the invention for use in the wet milling is typically a liquid suspension of the coarse compound in a liquid medium. By "suspension" is meant that the compound is essentially insoluble in the liquid medium. Representative liquid media include an aqueous medium. Using the process of the present invention the average particle size of coarse compound of the invention may be up to 1 mm in diameter. This advantageously avoids the need to pre-process the compound.

In a further aspect of the invention the aqueous medium to be subjected to the milling comprises compound (1) present in from about 1% to about 40% w/w, preferably from about 10% to about 30% w/w, more preferably about 20% w/w.

The aqueous medium may further comprise one or more pharmaceutically acceptable water-soluble carriers which are suitable for steric stabilisation and the subsequent processing of compound (I) after milling to a pharmaceutical composition, e.g. by spray drying. Pharmaceutically acceptable excipients most suitable for steric stabilisation and spray-drying are surfactants such as poloxamers, sodium lauryl sulphate and polysorbates etc; stabilisers such as celluloses e.g. hydroxypropylmethyl cellulose; and carriers such as carbohydrates e.g. mannitol.

In a further aspect of the invention the aqueous medium to be subjected to the milling may further comprise hydroxypropylmethyl cellulose (HPMC) present from about 0.1 to about 10% w/w.

The process of the present invention may comprise the subsequent step of drying compound of the invention to yield a powder.

Accordingly, in a further aspect, the present invention provides a process for preparing a pharmaceutical composition contain a compound of the present invention which process comprises producing compound of formula (I)

in nanoparticulate form optionally followed by drying to yield a powder.

A further aspect of the invention is a pharmaceutical composition comprising a compound of formula (I) or a pharmaceutically acceptable derivative thereof in which the compound of formula (I) or a pharmaceutically acceptable derivative thereof is present in solid particles in nanoparticulate form, in admixture with one or more pharmaceutically acceptable carriers or excipients.

By "drying" is meant the removal of any water or other liquid vehicle used during the process to keep compound of formula (1) in liquid suspension or solution. This drying step may be any process for drying known in the art, including freeze drying, spray granulation or spray drying. Of these methods spray drying is particularly preferred. All of these techniques are well known in the art. Spray drying/fluid bed granulation of milled compositions is carried out most suitably using a spray dryer such as a Mobile Minor Spray Dryer [Niro, Denmark], or a fluid bed drier, such as those manufactured by Glatt, Germany.

In a further aspect the invention provides a pharmaceutical composition as hereinbefore defined, in the form of a dried powder, obtainable by wet milling solid particles of compound of formula (I) followed by spray-drying the resultant suspension.

Preferably, the pharmaceutical composition as hereinbefore defined, further comprises HPMC present in less than 15% w/w, preferably in the range 0.1 to 10% w/w.

The $CB_2$ receptor compounds for use in the instant invention may be used in combination with other therapeutic agents, for example COX-2 inhibitors, such as celecoxib, deracoxib, rofecoxib, valdecoxib, parecoxib or COX-189; 5-lipoxygenase inhibitors; NSAID's, such as aspirin, diclofenac, indomethacin, nabumetone or ibuprofen; leukotriene receptor antagonists; DMARD's such as methotrexate; adenosine A1 receptor agonists; sodium channel blockers, such as lamotrigine; NMDA receptor modulators, such as glycine receptor antagonists; gabapentin and related compounds; tricyclic antidepressants such as amitriptyline; neurone stabilising antiepileptic drugs; mono-aminergic uptake inhibitors such as venlafaxine; opioid analgesics; local anaesthetics; $5HT_1$ agonists, such as triptans, for example sumatriptan, naratriptan, zolmitriptan, eletriptan, frovatriptan, almotriptan or rizatriptan; $EP_1$ receptor ligands, $EP_4$ receptor ligands; $EP_2$ receptor ligands; $EP_3$ receptor ligands; $EP_4$ antagonists; $EP_2$ antagonists and $EP_3$ antagonists; bradykinin receptor ligands and vanilloid receptor ligand, antirheumatoid arthritis drugs, for example anti TNF drugs e.g. enbrel, remicade, anti-IL-1 drugs, or DMARDS e.g. leflunamide. When the compounds are used in combination with other therapeutic agents, the compounds may be administered either sequentially or simultaneously by any convenient route.

Additional COX-2 inhibitors are disclosed in US Patent Nos. 5,474,995 US5,633,272; US5,466,823, US6,310,099 and US6,291,523; and in WO 96/25405, WO 97/38986, WO 98/03484, WO 97/14691, WO99/12930, WO00/26216, WO00/52008, WO00/38311, WO01/58881 and WO02/18374.

The compound of the present invention may be administered in combination with other active substances such as 5HT3 antagonists, NK-1 antagonists, serotonin agonists, selective serotonin reuptake inhibitors (SSRI), noradrenaline re-uptake inhibitors (SNRI), tricyclic antidepressants and/or dopaminergic antidepressants.

Suitable 5HT3 antagonists which may be used in combination of the compound of the inventions include for example ondansetron, granisetron, metoclopramide.

Suitable serotonin agonists which may be used in combination with the compound of the invention include sumatriptan, rauwolscine, yohimbine, metoclopramide.

Suitable SSRIs which may be used in combination with the compound of the invention include fluoxetine, citalopram, femoxetine, fluvoxamine, paroxetine, indalpine, sertraline, zimeldine.

Suitable SNRIs which may be used in combination with the compound of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

It will be appreciated that the compounds of any of the above combinations or compositions may be administered simultaneously (either in the same or different pharmaceutical formulations), separately or sequentially.

The invention thus provides, in a further aspect, a combination comprising a compound of formula (I) or a pharmaceutically acceptable derivative thereof together with a further therapeutic agent or agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

### Determination of cannabinoid CB1 Receptor Agonist Activity

The cannabinoid CB1 receptor agonist activity of the compounds of formula (I) was determined in accordance with the following experimental method.

### Experimental Method

Yeast (*Saccharomyces cerevisiae*) cells expressing the human cannabinoid CB1 receptor were generated by integration of an expression cassette into the *ura3* chromosomal locus of yeast strain MMY23. This cassette consisted of DNA sequence encoding the human CB1 receptor flanked by the yeast GPD promoter to the 5' end of CB1 and a yeast transcriptional terminator sequence to the 3' end of CB1. MMY23 expresses a yeast/mammalian chimeric G-protein alpha subunit in which the C-terminal 5 amino acids of Gpa1 are replaced with the C-terminal 5 amino acids of human Gαi3 (as described in Brown et al. (2000), Yeast 16:11-22). Cells were grown at 30˚C in liquid Synthetic Complete (SC) yeast media (Guthrie and Fink (1991), Methods in Enzymology, Vol. 194) lacking uracil, tryptophan, adenine and leucine to late logarithmic phase (approximately 6 $OD_{600}$/ml).

Agonists were prepared as 10 mM stocks in DMSO. $EC_{50}$ values (the concentration required to produce 50% maximal response) were estimated using dilutions of between 3- and 5-fold (BiomekFX, Beckman) into DMSO. Agonist solutions in DMSO (1% final assay volume) were transferred into black, clear bottom, microtitre plates from NUNC (96- or 384-well). Cells were suspended at a density of 0.2 $OD_{600}$/ml in SC media lacking histidine, uracil, tryptophan, adenine and leucine and supplemented with 10mM 3-aminotriazole, 0.1 M sodium phosphate pH 7.0, and 20uM fluorescein di-β-D-glucopyranoside (FDGlu). This mixture (50ul per well for 384-well plates, 200ul per well for 96-well plates) was added to agonist in the assay plates (Multidrop 384, Labsystems). After incubation at 30˚C for 24 hours, fluorescence resulting from degradation of FDGlu to fluorescein due to exoglucanase, an endogenous yeast enzyme produced during agonist-stimulated cell growth, was determined using a Spectrofluor microtitre plate reader (Tecan; excitation wavelength: 485nm; emission wavelength: 535nm). Fluorescence was plotted against compound concentration and iteratively curve fitted using a four parameter fit to generate a concentration effect value. Efficacy ($E_{max}$) was calculated from the equation

$$E_{max} = Max_{[compound\ X]} - Min_{[compound\ X]} / Max_{[HU210]} - Min_{[HU210]} \times 100\%$$

where $Max_{[compound\ X]}$ and $Min_{[compound\ X]}$ are the fitted maximum and minimum respectively from the concentration effect curve for compound X, and $Max_{[HU210]}$ and $Min_{[HU210]}$ are the fitted maximum and minimum respectively from the concentration effect curve for (6aR,10aR)-3-(1,1'-Dimethylheptyl)-6a,7,10,10a-tetrahydro-1-hydroxy-6,6-dimethyl-6H-dibenzo[b,d]pyran-9-methanol (HU210; available from Tocris). Equieffective molar ratio (EMR) values were calculated from the equation

$$EMR = EC_{50\ [compound\ X]} / EC_{50\ [HU210]}$$

Where $EC_{50\ [compound\ X]}$ is the $EC_{50}$ of compound X and $EC_{50\ [HU210]}$ is the $EC_{50}$ of HU210.

Compounds of the Examples tested according to this method had $EC_{50}$ values >30,OOOnM at the cloned human cannabinoid CB1 receptor.

### Determination of cannabinoid CB2 Receptor Agonist Activity

The cannabinoid CB2 receptor agonist activity of the compounds of formula (I) was determined in accordance with the following experimental method.

### Experimental Method

Yeast (*Saccharomyces cerevisiae*) cells expressing the human cannabinoid CB2 receptor were generated by integration of an expression cassette into the *ura3* chromosomal locus of yeast strain MMY23. This cassette consisted of DNA sequence encoding the human CB2 receptor flanked by the yeast GPD promoter to the 5' end of CB2 and a yeast transcriptional terminator sequence to the 3' end of CB2. MMY23 expresses a yeast/mammalian chimeric G-protein alpha subunit in which the C-terminal 5 amino acids of Gpa1 are replaced with the C-terminal 5 amino acids of human Gαi3 (as described in Brown et al. (2000), Yeast 16:11-22). Cells were grown at 30˚C in liquid Synthetic Complete (SC) yeast media (Guthrie and Fink (1991), Methods in Enzymology, Vol. 194) lacking uracil, tryptophan, adenine and

leucine to late logarithmic phase (approximately 6 $OD_{600}$/ml).

Agonists were prepared as 10 mM stocks in DMSO. $EC_{50}$ values (the concentration required to produce 50% maximal response) were estimated using dilutions of between 3- and 5-fold (BiomekFX, Beckman) into DMSO. Agonist solutions in DMSO (1% final assay volume) were transferred into black, clear bottom, microtitre plates from NUNC (96- or 384-well). Cells were suspended at a density of 0.2 $OD_{600}$/ml in SC media lacking histidine, uracil, tryptophan, adenine and leucine and supplemented with 10mM 3-aminotriazole, 0.1 M sodium phosphate pH 7.0, and 20M fluorescein di-p-D-glucopyranoside (FDGlu). This mixture (50ul per well for 384-well plates, 200ul per well for 96-well plates) was added to agonist in the assay plates (Multidrop 384, Labsystems). After incubation at 30˚C for 24 hours, fluorescence resulting from degradation of FDGlu to fluorescein due to exoglucanase, an endogenous yeast enzyme produced during agonist-stimulated cell growth, was determined using a Spectrofluor microtitre plate reader (Tecan; excitation wavelength: 485nm; emission wavelength: 535nm). Fluorescence was plotted against compound concentration and iteratively curve fitted using a four parameter fit to generate a concentration effect value. Efficacy ($E_{max}$) was calculated from the equation

$$E_{max} = Max_{[compound\ X]} - Min_{[compound\ X]} / Max_{[HU210]} - Min_{[HU210]} \times 100\%$$

where $MaX_{[compound\ X]}$ and $Min_{[compound\ X]}$ are the fitted maximum and minimum respectively from the concentration effect curve for compound X, and $Max_{[HU210]}$ and $Min_{[HU210]}$ are the fitted maximum and minimum respectively from the concentration effect curve for (6aR,10aR)-3-(1,1'-Dimethylheptyl)-6a,7,10,10a-tetrahydro-1-hydroxy-6,6-dimethyl-6H-dibenzo[b,d]pyran-9-methanol (HU210; available from Tocris). Equieffective molar ratio (EMR) values were calculated from the equation

$$EMR = EC_{50\ [compound\ X]} / EC_{50\ [HU210]}$$

Where $EC_{50\ [compound\ X]}$ is the $EC_{50}$ of compound X and $EC_{50\ [HU210]}$ is the $EC_{50}$ of HU210.

The compounds of Examples 1 to 38, 50 to 55, 69 to 93, 104 to 172, 204, 208 to 220, 223, 224, 234 to 279, 293, 295 to 297 tested according to this method had an $EC_{50}$ values of <300nM and efficacy value of >50% at the cloned human cannabinoid CB2 receptor.

The compounds of Examples 39 to 45, 56 to 62, 94 to 102, 173 to 177, 280 to 292, 294 and 298 to 304 tested according to this method had an $EC_{50}$ values of <1000nM and efficacy value of >50% at the cloned human cannabinoid CB2 receptor.

The compounds of Examples 46 to 49, 63 to 68, 103, 178 to 203, 205 to 207, 222, 225 to 233 and 305 tested according to this method had an $EC_{50}$ values of > 1000nM and/or efficacy value of <50% at the cloned human cannabinoid CB2 receptor.

The compound of Examples 221 tested according to this method had an $EC_{50}$ value of between 300 and 1000nM and an efficacy value of <30% at the cloned human cannabinoid CB2 receptor.

The following examples are illustrative, but not limiting of the embodiments of the present invention.

The following abbreviations are used herein

MDAP represents mass-directed auto-purification;

THF represents tetrahydrofuran;

DCM represents dichloromethane;

DMSO represents dimethyl sulfoxide;

TFA represents trifluoroacetic acid.

All NMR experimental data was recorded at 400MHz unless indicated.

Conditions, Hardware, and Software used for Mass-directed Autopurification

Hardware

Waters 600 gradient pump, Waters 2700 sample manager, Waters Reagent Manager, Micromass ZMD mass spectrometer, Gilson 202 - fraction collector, Gilson Aspec - waste collector.

Software

Micromass Masslynx version 3.5

Column

The column used is typically a Supelco ABZ+ column whose dimensions are 10mm internal diameter by 100mm in length. The stationary phase particle size is 5μm.

Solvents

A. Aqueous solvent = Water +0.1% Formic Acid
B. Organic solvent = MeCN: Water 95:5 +0.05% Formic Acid

Make up solvent = MeOH: Water 80:20 +50mMol Ammonium Acetate
Needle rinse solvent = MeOH: Water: DMSO 80:10:10

Methods

Five methods are used depending on the analytical retention time of the compound of interest.
They all have a flow rate of 20ml/min and a 15-minute runtime, which comprises of a 10-minute gradient followed by a 5-minute column flush and re-equilibration step.
Method 1 MDAP 1.5-2.2 = 0-30%B
Method 2 MDAP 2.0-2.8 = 5-30% B
Method 3 MDAP 2.5-3.0 = 15-55%B
Method 4 MDAP 2.8-4.0 = 30-80% B
Method 5 MDAP 3.8-5.5 = 50-90% B

Method Used for Purification Using the Biotage Horizon System.

Column: Biotage C18HS 25+S
Fraction volume: 9ml ; UV Threshold : 0.03AU
Solvent A= Water, B= Acetonitrile, Gradient :

| Volume(ml) | A | B |
|---|---|---|
| 0 | 70% | 30% |
| 240 | 0% | 100% |

**Description 1: Methyl 6-(3-chlorophenylamino)-4-(trifluoromethyl)-nicotinate**

A mixture of methyl 6-chloro-4-(trifluoromethyl)-nicotinate (0.7 g, ex Fluorochem) and 3-chloroaniline (0.62 mL) was heated at 120°C for 6 h. The reaction mixture solidified and the crude crystals were used for the next step without further purification.
LC-MS (ESI+): t = 10.20 min,(MH+) 331 and 333.

**Description 2: 6-(3-Chlorophenylamino)-4-(trifluoromethyl)-nicotinic acid hydrochloride**

To a suspension of methyl 6-(3-chlorophenylamino)-4-(trifluoromethyl)-nicorinate (Description 1) (1.0 g) in ethanol (5 mL) was added a solution of potassium hydroxide (510 mg) in water (5 mL) and the solution was stirred at reflux for 30 min. After removal of the ethanol under reduced pressure, the mixture was diluted with water (10 mL) and washed twice with dichloromethane. Concentrated hydrochloric acid was added to adjust pH to 1 and the precipitated solid was filtered and dried *in vacuo* at 60 °C to afford 6-(3-chlorophenylamino)-4-(trifluoromethyl)-nicotinic acid as its hydrochloride salt (0.62 g).
LC-MS (ESI+): t = 8.51 min, (MH+) 317 and 319.

**Description 3: 6-Chloro-*N*-cyclohexylmethyl-nicotinamide**

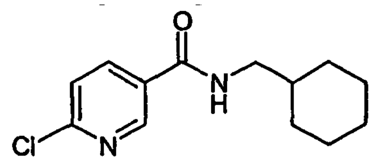

To a solution of 6-chloronicotinoyl chloride (1.5 g, ex Lancaster) in dry dichloromethane (15 ml) was added dropwise at 0° under nitrogen a solution of cyclohexanemethanamine (1.11 ml, ex Lancaster) and triethylamine (1.5 ml) in dry dichloromethane (15 ml) over 1 hour. The solution was stirred at 0° for 1 hour. Dichloromethane was removed under reduced pressure and ethyl acetate (30 ml) added. The solution was washed with water (3 x 20 ml), dried ($MgSO_4$) and evaporated to afford 6-chloro-*N*-cyclohexylmethyl-nicotinamide (1.96g).
NMR (DMSO-d6) δ 0.85-1.0 (2H, m), 1.1-1.25 (3H, m), 1.54 (1H, m), 1.55-1.75 (5H, m), 3.11 (2H, t), 7.64 (1H, d), 8.23 (1H, d of d), 8.69 (1H, t), 8.82 (1H, s).
LC/MS t = 2.9 min, Molecular ion observed [$MH^+$] 253 consistent with molecular formula $C_{13}H_{17}\,^{35}ClN_2O$

**Description 4: 6-Chloro-*N*-cyclohexylmethyl-4-isopropyl-nicotinamide**

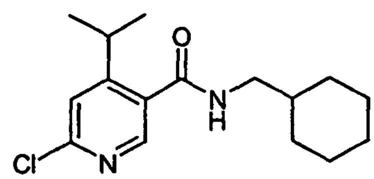

To a solution of 6-chloro-*N*-cyclohexylmethyl-nicotinamide (Description 3) (0.89 g) in dry tetrahydrofuran (5 ml) was added dropwise at 0° under nitrogen a 2.0M solution of isopropylmagnesium chloride (5.3 ml, ex Aldrich) and the solution stirred at room temperature for 15 hours. It was cooled to 0° and dry methanol (0.86 ml) added dropwise and the solution stirred for 15 minutes. 2,3-Dichloro-5,6-dicyano-1,4-benzoquinone (0.88 g) was added and the mixture stirred at room temperature for 30 minutes then evaporated under reduced pressure to ca. 6 ml. The residual liquid was warmed to 50° and t-butyl methyl ether (20 ml) added. The mixture was stirred under reflux for 1 hour then at room temperature for 1 hour and filtered. The filtrate was evaporated and the residue purified using Biotage chromatography (Merck 9385 silica gel) with 1:4 ethyl acetate:isohexane to afford 6-chloro-*N*-cyclohexylmethyl-4-isopropyl-nicotinamide (886 mg).
NMR (DMSO-d6) δ 0.85-1.0 (2H, m), 1.1-1.25 (3H, m), 1.19 (6H, d), 1.50 (1H, m), 1.55-1.75 (5H, m), 3.08 (2H, t), 3.22 (1H, m), 7.53 (1H, s), 8.24 (1H, s), 8.57 (1H, t).
LC/MS, t = 3.2 min, Molecular ion observed [$MH^+$] = 295 consistent with the molecular formula $C_{16}H_{23}\,^{35}ClN_2O$.

**Description 5: 6-Chloro-N-cyclobutylmethyl-nicotinamide**

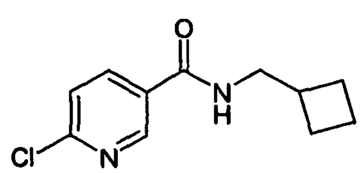

Prepared in a manner similar to Description 3 from 6-chloronicotinoyl chloride (1.9g, ex-Lancaster), C-cyclobutyl-methylamine hydrochloride (1.52g), and triethylamine (3.4ml), to give the title compound (2.02g).
NMR (DMSO-d6) δ 1.71 (2H, m), 1.82 (2H, m), 1.99 (2H, m), 2.52 (1H, m excess), 3.31 (2H, t), 7.64 (1H, d), 8.22 (1H, d of d), 8.71 (1H, t), 8.81 (1H, d).
LC/MS t = 2.51 min, Molecular ion observed [$MH^+$] = 225 consistent with the molecular formula $C_{11}H_{13}\,^{35}ClN_2O$

**Description 6: 6-Chloro-N-cyclobutylmethyl-4-isopropyl-nicotinamide**

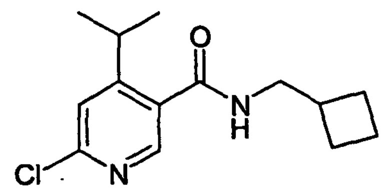

Prepared in a manner similar to Description 4 from 6-chloro-N-cyclobutylmethyl-nicotinamide (Description 3) (2.00g), and 2.0M isopropylmagnesium chloride in THF (13.5 mi), iu give the title compound (1.31g).
NMR (DMSO-d6) δ 1.19 (6H, d), 1.72 (2H, m), 1.82 (2H, m), 1.98 (2H, m), 2.50 (1H, m excess), 3.20 (1H, m), 3.27 (2H, t), 7.53 (1H, s), 8.23 (1H, s), 8.58 (1H, t).
LC/MS t = 3.07 min, [MH+] = 267 consistent with the molecular formula $C_{14}H_{19}{}^{35}ClN_2O$

**Description 7: 6-Chloro-N-(tetrahydro-pyran-4-ylmethyl)-nicotinamide**

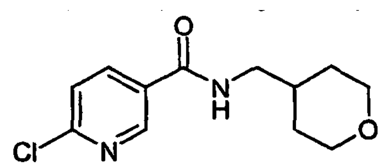

In a manner similar to Description 5, 6-chloronicotinoyl chloride (1.90 g) and C-(tetrahydro-pyran-4-yl)-methylamine (1.65 g) afforded the title compound (1.46 g).
NMR (DMSO-d6) δ 1.1-1.25 (2H, m), 1.60 (2H, d), 1.79 (1H, m), 3.17 (2H, t), 3.26 (2H, t), 3.83 (2H, d of d), 7.64 (1H, d), 8.23 (1H, d of d), 8.75 (1H, t), 8.82 (1H, s).
LC/MS t = 2.1 min, [$MH^+$] 255 consistent with the molecular formula $C_{12}H_{15}{}^{35}ClN_2O_2$

**Description 8: 6-Chloro-4-isopropyl-N-(tetrahydro-pyran-4-ylmethyl)-nicotinamide**

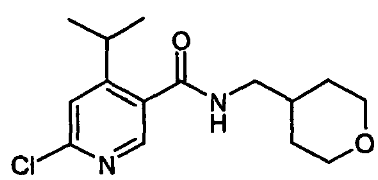

In a manner similar to Description 4, 6-chloro-N-(tetrahydro-pyran-4-ylmethyl)-nicotinamide (Description 7) (1.46 g) and 2.0M isopropylmagnesium chloride in tetrahydrofuran (8.5 ml) afforded the title compound (624 mg).
NMR (DMSO-d6) δ 1.1-1.25 (2H, m), 1.19 (6H, d), 1.60 (2H, d), 1.75 (1H, m), 3.14 (2H, t), 3.21 (1H, m), 3.27 (2H, t), 3.85 (2H, d of d), 7.54 (1H, d), 8.26 (1H, s), 8.63 (1H, t).
LC/MS t = 2.4 min, [$MH^+$] 297 consistent with the molecular formula $C_{15}H_{21}{}^{35}ClN_2O_2$

**Description 9: 6-Chloro-N-cyclopentylmethyl-nicotinamide**

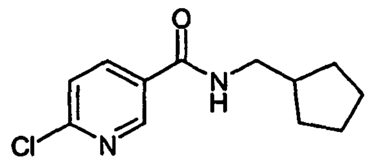

In a manner similar to Description 3, 6-chloronicotinoyl chloride (0.50 g) and cyclopentanemethylamine hydrochloride (385 mg) afforded the title compound (534 mg).
NMR (DMSO-d6) δ 1.2-1.3 (2H, m), 1.45-1.65 (4H, m), 1.65-1.75 (2H, m), 2.13 (1H, m), 3.20 (2H, t), 7.64 (1H, d), 8.23 (1H, d of d), 8.74 (1H, t), 8.82 (1H, s).
LC/MS t = 2.7 min, [MH$^+$] 239, consistent with the moiecuiar formula $C_{12}H_{15}{}^{35}ClN_2O$

**Description 10: 6-Chloro-N-cyclopentylmethyl-4-isopropyl-nicotinamide**

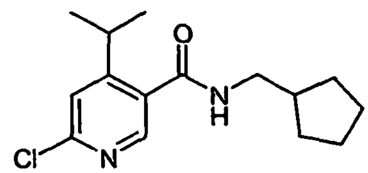

In a manner similar to Description 4, 6-chloro-N-cyclopentyl-nicotinamide (Description 9)(532 mg) and 2.0M isopropylmagnesium chloride in tetrahydrofuran (3.4 ml) afforded the title compound (166 mg).
NMR (DMSO-d6) δ 1.19 (6H, d), 1.2-1.3 (2H, m), 1.45-1.65 (4H, m), 1.65-1.75 (2H, m), 2.10 (1H, m), 3.17 (2H, t), 3.21 (1H, m), 7.53 (1H, s), 8.23 (1H, s), 8.61 (1H, t).
LC/MS t = 3.1 min, [MH$^+$] 281, consistent with the molecular formula $C_{15}H_{21}{}^{35}ClN_2O$.

**Description 11: 1-(6-Chloro-4-isopropyl-pyridin-3-yl)-1-morpholin-4-yl-methanone**

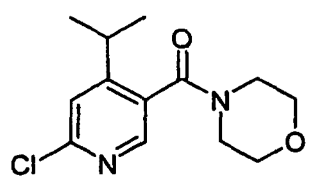

In a manner similar to Description 4, 1-(6-chloro-pyridin-3-yl)-1-morpholin-4-yl-methanone (534 mg, Ref: US Patent Application 2002183309 (2002), and 2.0M isopropyl-magnesium chloride in tetrahydrofuran (3.6 ml) afforded the title compound (169 mg).
NMR (DMSO-d6) δ 1.19 (6H, t), 2.89 (1H, m), 3.1-3.25 (2H, m), 3.45 (1H, m), 3.55-3.75 (5H, m), 7.60 (1H, s), 8.26 (1H, s).
LC/MS t = 2.3 min, [MH$^+$] 269, consistent with the molecular formula $C_{13}H_{17}{}^{35}ClN_2O_2$

**Description 12: 6-Chloro-4-isopropyl-nicotinic acid.**

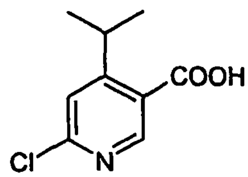

2M Isopropylmagnesium bromide in tetrahydrofuran (48 ml) was added dropwise over 1 hour to a solution of 6-chloronicotinic acid (Aldrich) (6.0 g) in dry tetrahydrofuran (100 ml) at 0° under nitrogen and the solution stirred at 0° for 3 hours then at room temperature for 15 hours. It was cooled to -60° and acetic acid (48 ml), tetrahydrofuran (40 ml) and manganese (III) acetate dihydrate (20.4 g) added successively. The mixture was stirred at -70° for 30 minutes then at room temperature for 1 hour. The suspension was filtered through Celite and the filtrate evaporated under reduced pressure. The residue was partitioned between dichloromethane (150 ml) and water (120 ml) and the aqueous layer separated and washed with dichloromethane (2 x 50 ml). The combined organic layers were dried ($MgSO_4$) and evaporated under reduced pressure to afford, after silica gel chromatography using 3:1 isohexane:ethyl acetate, 6-chloro-4-isopropyl-nicotinic acid (2.31 g).
NMR (DMSO-d6)8 1.21 (6H, d), 3.76 (1H, m), 7.60 (1H, s), 8.67 (1H, s), 13.55 (1H, br s).
LC/MS t = 2.6 min, [$MH^+$] 200 consistent with molecular formula $C_9H_{10}{}^{35}ClNO_2$

**Description 13: 6-(3-Chloro-phenylamlno)-4-isopropyl-nicotinic acid.**

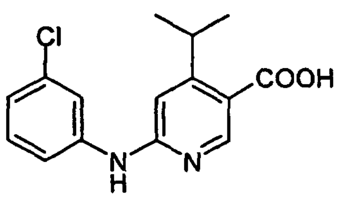

A mixture of 6-chloro-4-isopropyl-nicotinic acid (Description 12) (0.50 g) and 3-chloroaniline (265 mg) was stirred at 120° for 1½ hours. Isopropanol was added and the mixture chilled. Insoluble solid was filtered off, washed successively with isopropanol and ether and dried *in vacuo* at 50° to afford 6-(3-chloro-phenylamino)-4-isopropyl-nicotinic acid (0.51 g).
NMR (DMSO-d6) δ 1.19 (6H, d), 3.93 (1H, m), 6.85 (1H, s), 6.99 (1H, d), 7.31 (1H, t), 7.53 (1H, d), 8.00 (1H, s), 8.64 (1H, s), 9.73 (1H, s), 12.6 (1H, br s).
LC/MS t = 3.63 min, [$MH^+$] 291, consistent with molecular formula $C_{15}H_{15}{}^{35}ClNO_2$

**Description 14: 4-*tert*-Butyl-6-chloro-N-cyclohexylmethyl-nicotinamide**

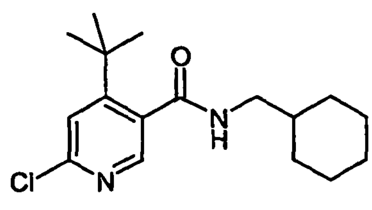

1.6 M n-Butyllithium in hexane (2.7 ml) was added dropwise to a stirred solution of 6-chloro-N-cyclohexylmethyl-4-isopropyl-nicotinamide (Description 4) (0.50 g) in dry tetrahydrofuran (3 ml) at -70° under nitrogen. The solution was stirred for 15 minutes then warmed to 0° and a solution of methyl iodide (0.11 ml) in dry tetrahydrofuran (2 ml) added, followed by stirring for a further 30 minutes. Solvent was removed under reduced pressure and ethyl acetate (10 ml)

added. The solution was washed with water (10 ml), dried ($MgSO_4$) and evaporated under reduced pressure. The residue was purified using silica gel chromatography with 17:3 isohexane:ethyl acetate and further purified by MDAP to afford the title compound (83 mg).

NMR ($CDCl_3$) δ 0.95-1.05 (2H, m), 1.15-1.3 (4H, m), 1.42 (9H, s), 1.65-1.8 (5H, m), 3.28 (2H, t), 5.81 (1H, br s), 7.36 (1H, s), 8.21 (1H, s).

LC/MS t = 3.6 min, [$MH^+$] 309, consistent with $C_{17}H_{25}{}^{35}ClN_2O$

**Description 15: 4-*tert*-Butyl-6-chloro-N-(tetrahydro-pyran-4-ylmethyl)-nicotinamide**

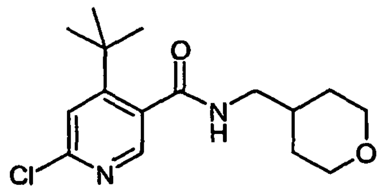

In a manner similar to Description 14, 6-chloro-4-isopropyl-N-(tetrahydro-pyran-4-ylmethyl)-nicotinamide (Description 8) (1.0 g), 1.6 M n-butyllithium in hexane (2.7 ml) and methyl iodide (0.22 ml) afforded, after silica gel chromatography, eluting with 1:1 isohexane:ethyl acetate and MDAP purification, the title compound (116 mg).

NMR ($CDCl_3$) δ 1.3-1.45 (2H, m), 1.42 (9H, s), 1.68 (2H, d), 1.91 (1H, m), 3.34 (2H, t), 3.40 (2H, t), 4.00 (2H, d of d), 6.04 (1H, br s), 7.36 (1H, s), 8.18 (1H, s).

LC/MS t = 2.4 min, [$MH^+$] 311 consistent with molecular formula $C_{16}H_{23}{}^{35}ClN_2O_2$

**Description 16: 4-Aminomethyltetrahydropyran-4-ol hydrochloride**

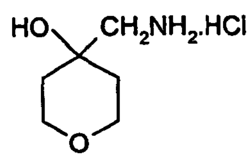

To a solution of 1.0M lithium aluminium hydride in tetrahydrofuran (20 ml) was added under a nitrogen atmosphere a solution of 4-hydroxytetra-hydropyran-4-carbonitrile (0.50 g, prepared as described in Eiden et al., Arch. Pharm., 320, 348, (1987)) in tetrahydrofuran (2 ml) and the solution stirred at reflux for 6 hours. Water (1 ml) and 2N sodium hydroxide solution (1 ml) were added cautiously and the resultant solid filtered and washed with ether. The filtrate was dried ($MgSO_4$), evaporated and the residue dissolved in ethanol (3 ml) and concentrated hydrochloric acid (0.5 ml) added. Solvent was removed under reduced pressure and the resultant solid washed with ether and dried in vacuo at 40˚C to afford the title compound (234 mg).

NMR (DMSO-d6) 1.45-1.6 (4H, m), 2.78 (2H, q), 3.61 (4H, m). 5.07 (1H, br s), 7.89 (3H, br s).

**Description 17: 6-(2,3-Dichloro-phenylamino)-4-trifluoromethyl-nicotinic acid methyl ester**

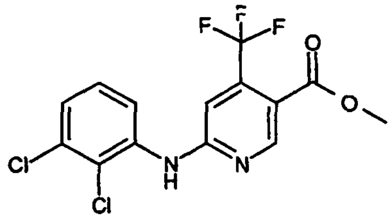

A mixture of methyl-6-chloro-4-(trifluoromethyl)-nicotinate (2.0 g, 8.37 mmol, ex Fluorochem) and 2,3-dichloroaniline (4.06 g, 25 mmol) was heated at 130˚C for 18 h, to afford the title compound.
MS m/z (ESI+): 365, 367 and 369 (isomeric peaks) (MH+).

**Description 18: 6-(2,3-Dichloro-phenylamino)-4-trifluoromethyl-nicotinic acid**

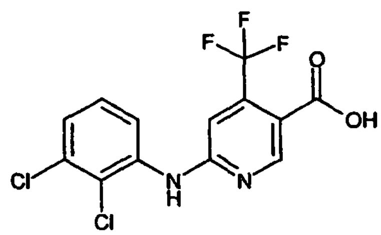

A solution of KOH (1.4 g, 25 mmol) in 20 mL of EtOH / $H_2O$ (1:1) was added to the crude mixture from Description 17 and the resulting mixture was stirred under reflux for 3h. The solution was concentrated in vacuo, diluted with water and washed three times (3 x 15 mL) with diethyl ether. Upon acidification of the aqueous layer to pH1 with HCl 37%, the title compound precipitated out as the hydrochloride salt that was filtered and dried under vacuum. The solid (2.7 g, 7 mmol) was then suspended in dichloromethane (20 mL), in the presence of PS-diisopropylethylamine (1.80 g, 7 mmol, loading 3.88 mmol/g, ex Argonaut Technologies) and stirred at room temperature for 30 min. After filtration of the resin and evaporation in vacuo of the solvent, the title compound was isolated as a white solid (2.45 g).
$^1H$ NMR (300 MHz, DMSO-$d_6$) δ: 13.17 (s br, 1H); 9.61 (s, 1H); 8.68 (s, 1H); 7.88 (dd, 1H); 7.44 (dd, 1H); 7.42 (s, 1H); 7.37 (dd, 1H).
MS m/z (ESI+): 351, 353 and 355 (isomeric peaks) (MH+).

**Description 19: 6-(2,4-Dichloro-phenylamino)-4-trifluoromethyl-nicotinic acid methyl ester**

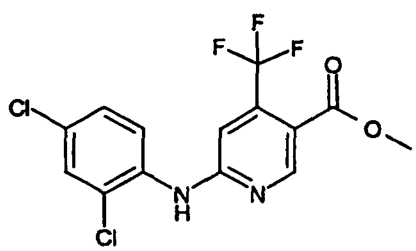

A mixture of methyl-6-chloro-4-(trifluoromethyl)-nicotinate (2.0 g, 8.37 mmol ex Fluorochem) and 2,4-dichloroaniline (4.05 g, 25 mmol) was heated at 130˚C for 15 h, to afford the title compound.
MS m/z (ESI+): 365, 367 and 369 (isomeric peaks) (MH+).

**Description 20: 6-(2,4-Dichloro-phenylamino)-4-trifluoromethyl-nicotinic acid**

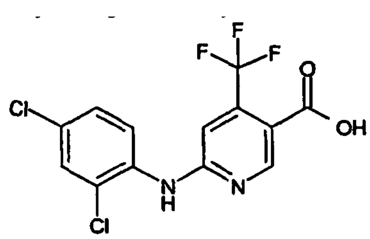

The title compound was prepared in a manner analogous to Description 18 from the product of Description 19 and isolated as a white solid (2.62 g).
$^1$H NMR (300 MHz, DMSO-$d_6$) δ: 13.16 (s br, 1H); 9.49 (s, 1H); 8.67 (s, 1H); 7.94 (d, 1H); 7.67 (d, 1H); 7.43 (dd, 1H); 7.40 (s, 1H).
MS m/z (ESI+): 351, 353 and 355 (MH+).

**Description 21: 6-(3-Chloro-phenylamino)-4-trifluoromethyl-nicotinic acid methyl ester**

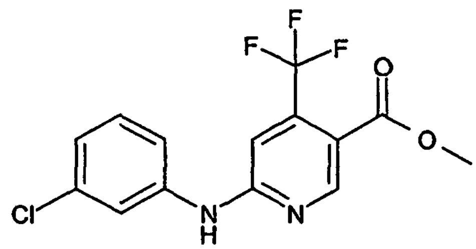

A mixture of methyl-6-chloro-4-(trifluoromethyl)-nicotinate (2.5 g, 10.5 mmol) and 3-chloroaniline (2.2 mL, 20.1 mmol) was heated at 120°C for 18 h, to afford the title compound.
MS m/z (ESI+): 331 (MH+).

**Description 22: 6-(3-Chloro-phenylamino)-4-trifluoromethyl-nicotinic acid**

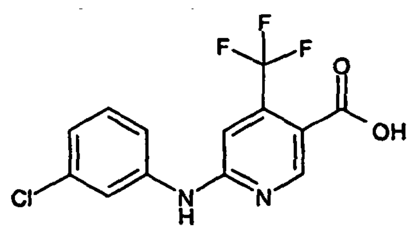

The title compound was prepared in a manner analogous to Description 18 from the product of Description 21 and was isolated as a white solid (1.5 g).
$^1$H NMR (300 MHz, DMSO-$d_6$) δ: 13.16 (s br, 1H); 10.28 (s, 1H); 8.80 (s, 1H); 8.01 (dd, 1H); 7.58 (ddd, 1H); 7.35 (dd, 1H); 7.28 (s, 1H); 7.06 (ddd, 1H).
MS m/z (ESI+): 317 (MH+).

**Description 23: 4-Aminomethyl-pyrrolidin-2-one**

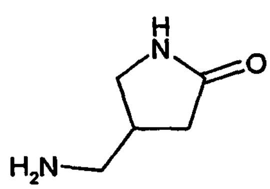

Sodium (0.1 g, 4.34 mmol) was added portionwise to a solution of 4-aminomethyl-1-benzyl-pyrrolidin-2-one (0.3 g, 1.47 mmol, CAS Registry N.: 97205-34-0) in 10 mL of liquid ammonia, at -50°C and the mixture was stirred at -50°C for 1 h. EtOH (10 mL) was slowly added and the reaction mixture was allowed to reach room temperature and stirred for 1 h at RT. Evaporation of the solvent in vacuo afforded the title compound (0.21 g), which was used for coupling

with the acids above mentioned, without further purification.
$^1H$ NMR (300 MHz, DMSO-$d_6$) δ: 3.28 (dd, 1H); 2.89 (dd, 1H); 2.45 (m, 2H); 2.18-1.93 (m, 2H); 1.68 (m, 1H).

**Description 25: 6-Hydroxy-2-trifluoromethyl-4,5-dihydro-pyridine-3-carboxylic acid ethyl ester**

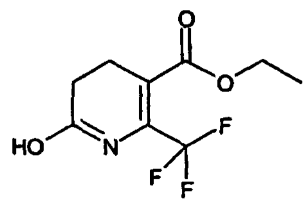

A mixture of ethyl 4,4,4-trifluoroacetoacetate (14.7 mL, 0.1 mol, 1.6 eq), acrylamide (4.5 g, 0.063 mol, 1.0 eq) and p-toluenesulphonic acid (0.156 g, 0.82 mmol, 0.013 eq) in toluene (60 mL) was refluxed for 38 h with azeotropic removal of water (Dean-Stark conditions). The reaction mixture was then concentrated to a small volume, by slow distillation of toluene at atmospheric pressure. Toluene (60 mL) was added and again the reaction mixture was concentrated, through slow distillation of toluene. After repeating this operation three times, the reaction mixture was concentrated in vacuo and the solid residue was purified by flash chromatography (silica gel, eluent gradient: from hexane / ethyl acetate 9:1 to hexane / ethyl acetate 8:2). The title compound was obtained as a brownish solid (3.8 g, yield = 25%).
LC-MS (ESI+), MH+: 238, 210, 190.

**Description 26: 6-Hydroxy-2-trifluoromethyl-nicotinic acid ethyl ester**

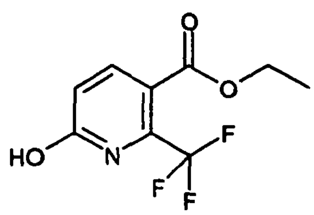

A solution of 6-hydroxy-2-trifluoromethyl-4,5-dihydro-pyridine-3-carboxylic acid ethyl ester (Description 25) (4.7 g, 19.8 mmol, 1 eq) and N-bromo succinimide (3.51 g, 19.8 mmol, 1 eq) in 15 mL of carbon tetrachloride was heated under reflux for 20 h. The resulting precipitate was filtered off and the filtrate was concentrated under reduced pressure to afford a brownish solid that was purified by flash chromatography (silica gel, eluent gradient: from hexane / ethyl acetate 9:1 to hexane / ethyl acetate 8:2). The title compound was obtained as a white solid (4.3 g, yield = 92%).
LC-MS (ESI+), MH+: 236.

**Description 27: 6-Chloro-2-trifluoromethyl-nlcotinic acid ethyl ester**

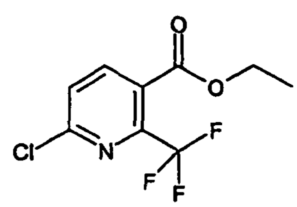

A mixture of 6-hydroxy-2-trifluoromethyl-nicotinic acid ethyl ester (Description 26) (2.6 g, 11.0 mmol, 1.0 eq) and phenyl dichlorophosphate (2.47 mL, 16.5 mmol, 1.5 eq) was heated under microwave irradiation for 30 min (170˚C,

power = 70 W). The reaction mixture was poured into ice, stirred for 20 min and diluted with ethyl acetate (50 mL). The pH was adjusted to 10, by addition of a saturated aqueous solution of sodium bicarbonate (50 mL) and then the organic layer was separated, washed with water, dried over $Na_2SO_4$ and concentrated in vacuo. The resulting solid residue was purified by flash chromatography (silica gel, eluent gradient: from hexane to hexane / ethyl acetate 98:2) to give 1.7 g of the title compound (yield = 61%).
LC-MS (ESI+), MH+: 254 and 256.

**Description 28: 6-(3-Chloro-phenylamino)-2-trifluoromethyl-nicotinic acid ethyl ester**

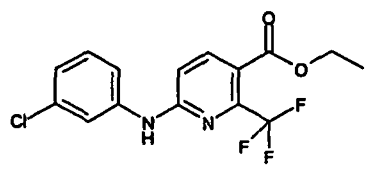

A mixture of 6-chloro-2-trifluoromethyl-nicotinic acid ethyl ester (Description 27) (1.4 g, 5.53 mmol, 1.0 eq) and 3-chloro aniline (2.91 mL, 27.6 mmol, 5.0 eq) was heated at 160˚C for 52 h to afford a black solid which was used for the next step without further purification.
LC-MS (ESI+), MH+: 345 and 347.

**Description 29: 6-(3-Chloro-phenylamino)-2-triftuoromethyl-nicotinic acid hydrochloride**

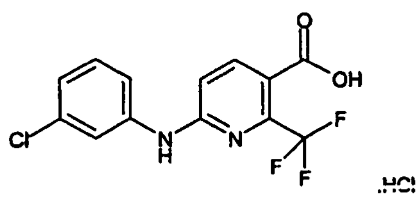

A solution of KOH (1.18 g) in water (25 mL) was added to a mixture of crude 6-(3-chloro-phenylamino)-2-trifluoromethyl-nicotinic acid ethyl ester (Description 28) in ethanol (25 mL) and refluxed for 8h. After evaporation of ethanol under reduced pressure, the reaction mixture was diluted with water (35 mL) and repeatedly washed with diethyl ether (200 mL x 5 times). The aqueous layer was treated with conc. HCl to adjust the pH to 3 and the title compound precipitated out as its hydrochloride salt, was filtered and dried at 40˚C in oven (1.71 g, yield of Description 28 and 29 = 87%).
LC-MS (ESI+), MH+: 317 and 319.

**Description 30: 3-Amino-4-methyl-pent-2-enoic acid ethyl ester**

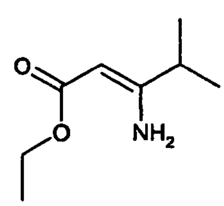

Ammonium acetate (2.44 g, 31.6 mol, 5 eq) was added to a solution of 4-methyl-3-oxo-pentanoic acid ethyl

ester (1.0 g, 6.32 mol, 1 eq) in methanol (10 mL) and the mixture was stirred at room temperature for 3 days. Solvent was evaporated in vacuo and the solid residue was triturated with dichloromethane (20 mL) and filtered off. The filtrate was then washed with water and brine, dried over $Na_2SO_4$ and concentrated in vacuo to afford the title compound as a yellow oil (0.85 g, yield = 85%).

**Description 31: 4-(1-Amino-2-methyl-propylidene)-pent-2-enedioic acid 5-ethyl ester 1-methyl ester**

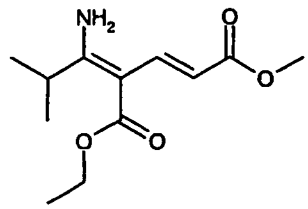

A solution of 3-amino-4-methyl-pent-2-enoic acid ethyl ester (Description 30) (5.0 g, 31.84 mmol, 1 eq) and methyl propiolate (3.08 mL, 36.8 mmol, 1.15 eq) in dry DMSO (20 mL) was heated under microwave irradiation at 170˚C (1st cycle: 20 min, 2nd cycle: 10 min). The reaction mixture was diluted with water (140 mL) and extracted twice with ethyl acetate (80 mL). The organic phase was washed with a saturated aqueous solution of $NaHCO_3$ and with brine, dried over sodium sulphate and concentrated in vacuo to afford 9.5 g of yellow solid, used for the next step without further purification.
LC-MS (ESI+), MH+: 242, 196.

**Description 32: 6-Hydroxy-2-isopropyl-nicotinic acid ethyl ester**

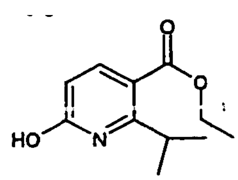

A catalytic amount of sodium tert-butoxide (100 mg) was added to a suspension of crude 4-(1-amino-2-methyl-propylidene)-pent-2-enedioic acid 5-ethyl ester 1-methyl ester (Description 31) (9.5 g) in anhydrous ethanol (100 mL) and the resulting mixture was refluxed for 28 h. Solvent was removed in vacuo, the residue was taken up with ethyl acetate and then washed subsequently with $NaHCO_3$ (aq) and with brine. The organic layer was dried over $Na_2SO_4$ and concentrated in vacuo to afford a reddish resin. Trituration of the resin with hexane / diethyl ether 1:1 yielded the title compound as a solid that was filtered off and dried in oven (1.97 g). The mother liquor was concentrated and purified by flash chromatography (silica gel, eluent gradient: from hexane / ethyl acetate 9:1 to hexane / ethyl acetate 7:3) to yield a second crop of pure title compound (1.6 g, total yield of Descriptions 31 and 32 = 54%).
LC-MS (ESI+), MH+: 210.

**Description 33: 6-Chloro-2-isopropyl-nicotinic acid ethyl ester**

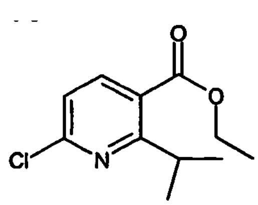

A mixture of 6-hydroxy-2-isopropyl-nicotinic acid ethyl ester (Description 32) 1.0 g, 4.78 mmol, 1.0 eq) and phenyl dichlorophosphate (1.13 mL, 7.56 mmol, 1.5 eq) was heated under microwaves irradiation at 170˚C for 1 min. The reaction mixture was poured into ice-water (25 mL), stirred for 20 min and diluted with ethyl acetate (40 mL). The pH was adjusted to 10, by addition of a saturated aqueous solution of sodium bicarbonate (50 mL) and then the organic layer was separated, washed with water, dried over $Na_2SO_4$ and concentrated in vacuo to give 1.11 g of the crude title compound as a black resin (yield = 99%).
LC-MS (ESI+), MH+: 228 and 230.

**Description 34: 6-(3-Chloro-phenylamino)-2-isopropyl-nicotinic acid ethyl ester**

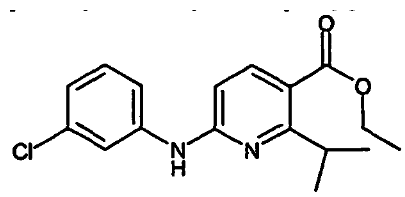

A mixture of 6-chloro-2-isopropyl-nicotinic acid ethyl ester (Description 33) (1.1 g, 4.84 mmol, 1.0 eq) and 3-chloro aniline (1.54 mL, 14.5 mmol, 3.0 eq) was heated at 120˚C for 4h to afford a solid residue which was used for the next step without further purification. LC-MS (ESI+), MH+: 319 and 321.

**Description 35: 6-(3-Chloro-phenylamino)-2-isopropyl-nicotinic acid hydrochloride**

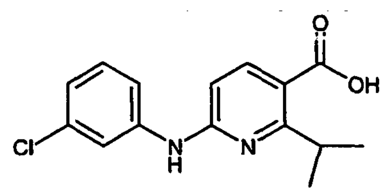

A solution of KOH (1.08 g) in water (10 mL) was added to a mixture of crude 6-(3-chloro-phenylamino)-2-isopropyl-nicotinic acid ethyl ester (Description 34) in ethanol (10 mL) and refluxed for 4h. After evaporation of ethanol under reduced pressure, the reaction mixture was diluted with water (15 mL) and repeatedly washed with diethyl ether (40 mL x 4 times). The aqueous layer was treated with conc. HCl to adjust the pH to 1 and the title compound precipitated out as its hydrochloride salt, was filtered and dried at 40˚C in oven (0.68 g). The aqueous mother liquor was treated with NaCl (s) and repeatedly extracted with ethyl acetate (30 mL x 3 times), the organic layer was dried over sodium sulphate and evaporated in vacuo. The residue was treated with conc. HCl and the title compound that precipitated out was filtered and dried in oven (0.681 g, total yield of Description 34 and 35 =85%).
LC-MS (ESI+), MH+: 291 and 293.

**Description 36: 6-Chloro-N-(1,1-dioxo-tetrahydro-*1*$^6$-thiophen-3-ylmethyl)-4-isopropyl-nicotinamide**

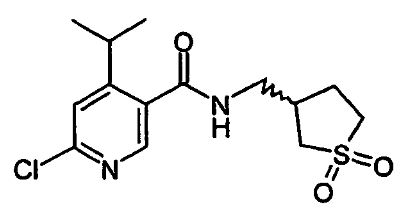

To a solution of 6-chloro-4-isopropyl-nicotinic acid (Description 12) (100 mg) in dimethylformamide (7 ml) was added successively N-ethylmorpholine (0.22 ml), C-(1,1-dioxo-tetrahydro-$1^6$-thiophen-3-ylmethyl)-methylamine hydrochloride (111 mg, Ref.: Argyle et al., J. Chem. Soc., (C), 2156, (1967)), 1-hydroxybenzotriazole hydrate (120 mg) and 1-(3-dimethylamino-propyl)-3-ethylcarbodiimide hydrochloride (120 mg). The solution was stirred for 5 h and allowed to stand overnight. Dimethylformamide was removed under reduced pressure and ethyl acetate (20 ml) added. The solution was washed sequentially with 5% sodium bicarbonate solution (12 ml), water (12 ml) and brine (2 x 12 ml), dried ($MgSO_4$) and evaporated to afford the title compound (150 mg).
LC/MS t = 2.1 min, [$MH^+$] 331 consistent with the molecular formula $C_{14}H_{19}{}^{35}ClN_2O_3S$.

All the amines used in the Examples are commercially available except as follows where the synthesis of the amines is described in the literature or above.

**Amines known in the literature**

| Structure | CAS Registry Number |
|---|---|
| 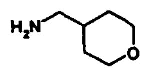 | 130290-79-8 |
| 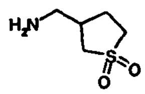 | 45697-13-0 |
| 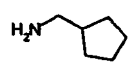 | 6053-81-2 |
| 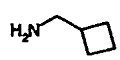 | 4415-83-2 |
| 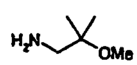 | 89282-70-2 |
| 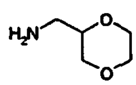 | 88277-83-2 |
| 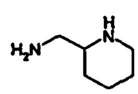 | 22990-77-8 |
| 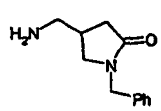 | 97205-34-0 |
| 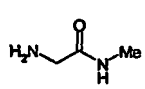 | 22356-89-4 |
| 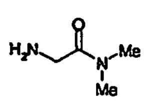 | 1857-19-8 |

**Example 1: 2-(3-Chlorophenylamino)-4-trifluoromethylpyridine-5-carboxylic acid cyclohexylmethyl amide**

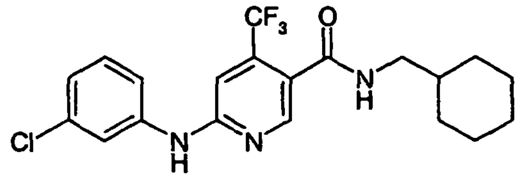

To a solution of 6-(3-chlorophenylamino)-4-(trifluoromethyl)-nicotinic acid hydrochloride (Description 2) (0.2 g) in dimethylformamide (5 mL) were added N-methylmorpholine (283 μL), 4-aminomethylcyclohexane (80 μL), 1-hydroxybenzotriazole hydrate (104 mg), 1-(3-dimethylamino-propyl)-3-ethylcarbodiimide hydrochloride (118 mg). After stirring at room temperature for 6 h, dimethylformamide was evaporated under reduced pressure and dichloromethane added. The solution was washed with a 5% aqueous solution of potassium carbonate (5 mL), then with brine (2 x 3 mL) and was evaporated under reduced pressure. Chromatographic purification (silica gel; hexane, ethyl acetate 8:2) afforded the title compound (35 mg).
$^1$H NMR (300 MHz, DMSO-d6) δ 9.85 (1H, s) 8.45 (2H, m), 8.05 (1H, s), 7.5 (1H, d), 7.35 (1H, t), 7.15 (1H, s), 7.02 (1H, d), 3.1 (2H, t), 0.85-1.8 (11H, m).
MS m/z (EI$^+$): 411 and 413 (MH$^+$.), 328, 315, 299.
IR (KBr): 3412 cm-1, 3309, 2925, 2852, 1648.

**Example 2: 6-(3-Chlorophenylamino)-*N*-cyclohexylmethyl-4-isopropylnicotinamide**

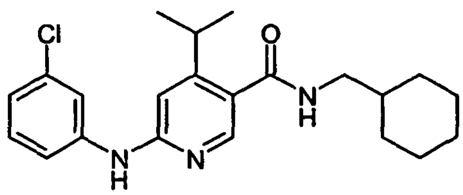

A mixture of 6-chloro-*N*-cyclohexylmethyl-4-isopropyl-nicotinamide (Description 4) (50 mg) and 3-chloroaniline (90 μl) was heated under microwave conditions at 190° for 20 minutes. Ethyl acetate (5 ml) was added and the solution washed with dilute potassium carbonate solution (3 ml) and water (3 ml), dried ($MgSO_4$) and evaporated. The residue was triturated with isohexane to afford the title compound (60 mg).
NMR (DMSO-d6) δ 0.85-1.0 (2H, m), 1.1-1.25 (3H, m), 1.16 (6H, d), 1.51 (1H, m), 1.6-1.8 (5H, m), 3.06 (2H, t), 3.41 (1H, m), 6.78 (1H, s), 6.92 (1H, d), 7.27 (1H, t), 7.46 (1H, d), 8.06 (1H, t), 8.12 (1H, s), 8.33 (1H, t), 9.41 (1H, s).
LC/MS, t = 3.7 min, Molecular ion observed [MH$^+$] = 386 consistent with the molecular formula $C_{22}H_{28}{}^{35}ClN_3O$.

**Example 3: N-Cyclohexylmethyl-6-(3,4-dichloro-phenylamino)-4-isopropyl-nicotinamide**

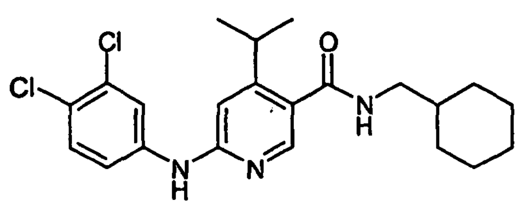

A mixture of 6-chloro-N-cyclohexylmethyl-4-isopropyl-nicotinamide (Description 4) (50 mg), 3,4-dichloroaniline (Aldrich) (33 mg), sodium t-butoxide (46 mg), tris(dibenzylideneacetone)palladium (0) (3.2 mg), 2-(dicyclohexylphosphino)biphenyl (2.6 mg) and dimethoxyethane (1 ml) was irradiated under microwave conditions at 150° for 30 minutes. Solvent was evaporated under reduced pressure and ethyl acetate (5 ml) added. The mixture was washed with water (3 ml), dried ($MgSO_4$) and evaporated. The residue was purified by mass-directed autopurification techniques to afford

the title compound (12.0 mg).
NMR (DMSO-d6) δ 0.85-1.0 (2H, m), 1.1-1.25 (3H, m), 1.16 (6H, d), 1.51 (1H, m), 1.6-1.8 (5H, m), 3.06 (2H, t), 3.41 (1H, m), 6.80 (1H, s), 7.50 (2H, m), 8.13 (1H, s), 8.25 (1H, s), 8.35 (1H, t), 9.62 (1H, s).
LC/MS t = 3.9 min, [MH$^+$] 420, consistent with molecular formula $C_{22}H_{27}{}^{35}Cl_2N_3O$

**Example 4: 6-(3-Bromo-phenylamino)-N-cyclohexylmethyl-4-isopropyl-nicotinamide**

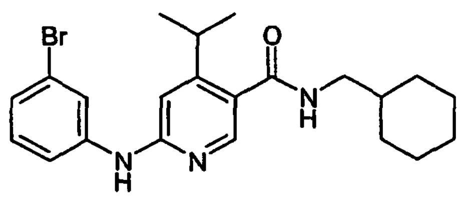

A mixture of 6-chloro-N-cyclohexylmethyl-4-isopropyl-nicotinamide (Description 4) (60 mg) and 3-bromoaniline (Aldrich) (0.5 ml) was irradiated under microwave conditions at 180° for 30 minutes. The mixture was dissolved in dichloromethane and passed down a 10g SepPak column to remove excess 3-bromoaniline. Elution with 9:1 dichloromethane:ether removed the crude product which was further purified by MDAP to afford the title compound (13.6 mg).
NMR (DMSO-d6) δ 0.85-1.0 (2H, m), 1.1-1.25 (3H, m), 1.17 (6H, d), 1.52 (1H, m), 1.6-1.8 (5H, m), 3.06 (2H, t), 3.42 (1H, m), 6.78 (1H, s), 7.06 (1H, d), 7.22 (1H, t), 7.52 (1H, d), 8.13 (1H, s), 8.19 (1H, s), 8.33 (1H, t), 9.40 (1H, s).
LC/MS t = 3.95 min, [MH$^+$] 430, consistent with molecular formula $C_{22}H_{28}{}^{79}BrN_3O$

**Example 5: N-Cyclohexylmethyl-6-(2,4-dichloro-phenylamino)-4-isopropyl-nicotinamide**

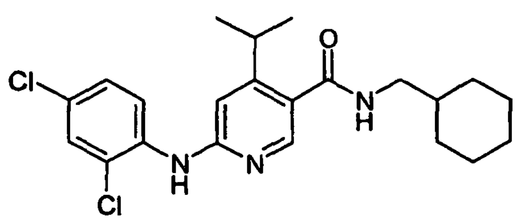

A mixture of 6-chloro-N-cyclohexylmethyl-4-isopropyl-nicotinamide (Description 4) (50 mg), 2,4-dichloroaniline (33 mg), sodium t-butoxide (23 mg), tris(dibenzylideneacetone)palladium(0) (1.6 mg), 2-(dicyclohexylphosphino)biphenyl (1.3 mg) and dimethoxyethane (1 ml) was stirred under reflux for 18 hours. The solvent was evaporated under reduced pressure and ethyl acetate (5 ml) added. The mixture was washed with water (3 ml), dried ($MgSO_4$) and evaporated. The residue was purified by MDAP to afford the title compound (12 mg).
NMR (DMSO-d6) δ 0.8-1.0 (2H, m), 1.1-1.3 (3H, m), 1.17 (6H, d), 1.50 (1H, m), 1.6-1.8 (5H, m), 3.05 (2H, t), 3.38 (1H, m), 7.08 (1H, s), 7.40 (1H, d), 7.65 (1H, s), 8.01 (1H, s), 8.07 (1H, d), 8.37 (1H, t), 8.93 (1H, br s).
LC/MS t = 3.8 min, [MH$^+$] 420, consistent with molecular formula $C_{22}H_{27}{}^{35}Cl_2N_3O$

**Example 6: 6-(3-Chloro-phenylamino)-N-cyclobutylmethyl-4-isopropyl-nicotinamide**

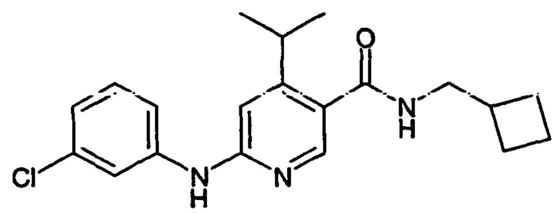

A mixture of 6-chloro-N-cyclobutylmethyl-4-isopropyl-nicotinamide (Description 6) (80mg) and 3-chloroaniline (0.5ml) was irradiated under microwave conditions at 180˚C for 30mins. The mixture was diluted with dichloromethane (2ml) and chromatographed on silica gel. The excess aniline was removed by elution with dichloromethane and then elution with dichloromethane/ether (5:1) gave the title compound (38mg).
NMR (DMSO-d6) δ 1.16 (6H, m), 1.74 (2H, m), 1.82 (2H, m), 2.00 (2H, m), 2.52 (1H, m excess), 3.23 (2H, t), 3.40 (1H, m), 6.78 (1H, s), 6.92 (1H, d), 7.27 (1H, t), 7.46 (1H, d), 8.04 (1H, s), 8.10 (1H, s), 8.33 (1H, t), 9.41 (1H, s)
LC/MS t = 3.65min, [$MH^+$] 358 consistent with the molecular formula $C_{20}H_{24}{}^{35}ClN_3O$

**Table 1**

Preparative Method A: As for Example 2, with temperature and time of reaction, and any other variations included in the table.
Preparative Method B: As for Example 3, with temperature and time of reaction, and any other variations noted in the table.
Preparative Method C: As for Example 6, with temperature and time of reaction, and any other variations noted in the table.
Purification Method E: Purify by mass-directed autopurification techniques.
Purification Method F: The crude product was diluted with dichloromethane (2ml) and the solution applied to a Sep-Pack column of silica gel. This was eluted firstly with dichloromethane, followed by dichloromethane/ether 5:1 to give pure product.

| Ex. No. | Chemical Name | Structure | 1. Preparation method A, B, or C<br>2. Reaction temperature (˚C),<br>3. Time. | Purification method E or F | 1.Retention Time (min).<br>2.[MH+]<br>3. Molecular formula |
|---|---|---|---|---|---|
| 7 | 6-(3-Chloro-phenylamino)-4-isopropyl-N-(tetrahydro-pyran-4-ylmethyl)-nicotinamide | 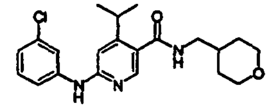 | A<br>200˚<br>1 hr | E | 3.1<br>388<br>$C_{21}H_{26}{}^{35}ClN_3O_2$ |
| 8 | 6-(3-Bromo-phenylamino)-4-isopropyl-N-(tetrahydro-pyran-4-ylmethyl)-nicotinamide | 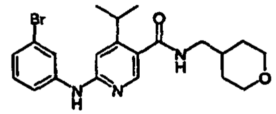 | A<br>200˚<br>30 min | E | 3.1<br>432<br>$C_{21}H_{26}{}^{79}BrN_3\ O_2$ |
| 9 | N-Cyclohexylmethyl-4-isopropyl-6-(3-methoxy-phenylamino)-nicotinamide | 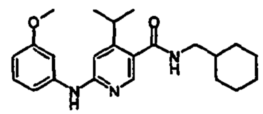 | B<br>150˚<br>30 min | E | 3.4<br>382<br>$C_{23}H_{31}N_3O_2$ |
| 10 | N-Cyclohexylmethyl-6-(3-fluoro-phenylamino)-4-isopropyl-nicotinamide | 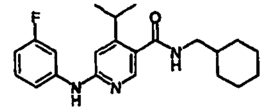 | B<br>150˚<br>30 min | E | 3.6<br>370<br>$C_{22}H_{28}FN_3O$ |
| 11 | 1-[6-(3-Chloro-phenylamino)-4-isopropyl-pyridin-3-yl]-1-morpholin-4-yl-methanone | 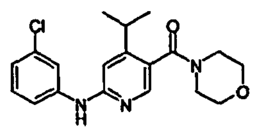 | A<br>180˚<br>30 min | E | 3.1<br>360<br>$C_{19}H_{22}{}^{35}ClN_3O_2$ |
| 12 | 6-(3-Bromo-phenylamino)-N-cyclohexylmethyl-4-isopropyl-nicotinamide | 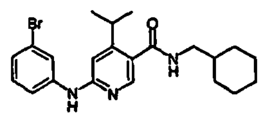 | A<br>180˚<br>30 min | E | 3.95<br>430<br>$C_{22}H_{28}{}^{79}BrN_3\ O$ |
| 13 | N-Cyclohexylmethyl-4-isopropyl-6-m-tolylamino-nicotinamide | 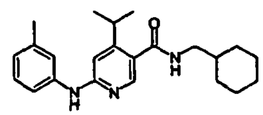 | A<br>180˚<br>(1 hr) | E | 3.68<br>366<br>$C_{23}H_{31}N_3O$ |

(continued)

| Ex. No. | Chemical Name | Structure | 1. Preparation method A, B, or C<br>2. Reaction temperature (˚C),<br>3. Time. | Purification method E or F | 1.Retention Time (min).<br>2.[MH$^+$]<br>3. Molecular formula |
|---|---|---|---|---|---|
| 14 | N-Cyclohexylmethyl-4-isopropyl-6-(3-trifluoromethyl-phenylamino)-nicotinamide | 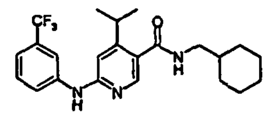 | A<br>180˚<br>1 hr | E | 3.7<br>420<br>$C_{23}H_{28}F_3N_3O$ |
| 15 | N-Cyclohexylmethyl-4-isopropyl-6-(3-trifluoromethoxy-phenylamino)-nicotinamide | 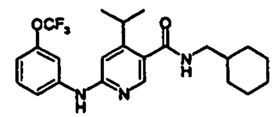 | A<br>180˚<br>30 min | E | 3.8<br>436<br>$C_{23}H_{28}F_3N_3O_2$ |
| 16 | 6-(2,3-Dichloro-phenylamino)-4-isopropyl-N-(tetrahydro-pyran-4-ylmethyl)-nicotinamide | 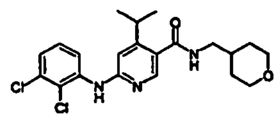 | B<br>150˚<br>30 min | E | 3.34<br>422<br>$C_{21}H_{25}{}^{35}Cl_2N_3O_2$ |
| 17 | 6-(2,4-Dichloro-phenylamino)-4-isopropyl-N-(tetrahydro-pyran-4-ylmethyl)-nicotinamide | 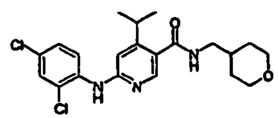 | B<br>150˚<br>30 min | E | 3.39<br>422<br>$C_{21}H_{25}{}^{35}Cl_2N_3O_2$ |
| 18 | 6-(3,4-Dichloro-phenylamino)-4-isopropyl-N-(tetrahydro-pyran-4-ylmethyl)-nicotinamide | 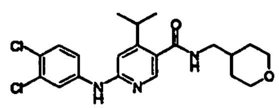 | B<br>150˚<br>30 min | E | 3.51<br>422<br>$C_{21}H_{25}{}^{35}Cl_2N_3\ O_2$ |
| 19 | 4-isopropyl-N-(tetrahydro-pyran-4-ylmethyl)-6-(3-trifluoromethyl-phenylamino)-nicotinamide | 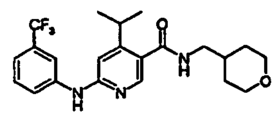 | A<br>180˚<br>1 hr | E | 3.2<br>422<br>$C_{22}H_{26}F_3N_3O_2$ |
| 20 | 4-isopropyl-N-(tetrahydro-pyran-4-ylmethyl)-6-(3-trifluoromethoxy-phenylamino)-nicotinamide | 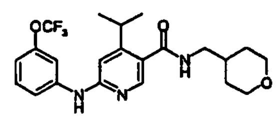 | A<br>180˚<br>30 min | E | 3.3<br>438<br>$C_{22}H_{26}F_3N_3O_3$ |

(continued)

| Ex. No. | Chemical Name | Structure | 1. Preparation method A, B, or C<br>2. Reaction temperature (˚C),<br>3. Time. | Purification method E or F | 1.Retention Time (min).<br>2.[MH+]<br>3. Molecular formula |
|---|---|---|---|---|---|
| 21 | 6-[(3-Chloro-phenyl) amino]-N-(cyclopentylmethyl)-4-isopropyl-nicotinamide | 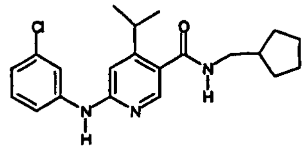 | A<br>180˚<br>30 min | E | 3.76<br>372<br>$C_{21}H_{26}N_3ClO$ |
| 22 | N-Cyclopentylmethyl-6-(3-fluorophenylamino)-4-isopropyl-nicotinamide | 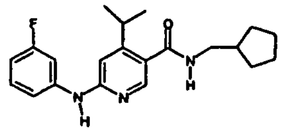 | A<br>180˚<br>30 min | E | 3.69<br>356<br>$C_{21}H_{26}N_3FO$ |
| 23 | N-Cyclopentylmethyl-4-isopropyl-6-(3-trifluoromethyl-phenylamino)-nicotinamide | 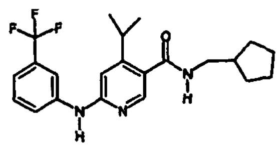 | A<br>180˚<br>30 min | E | 3.82<br>406<br>$C_{21}H_{26}N_3F_3O$ |
| 24 | N-Cyclopentylmethyl-4-isopropyl-6-m-tolylamino-nicotinamide | 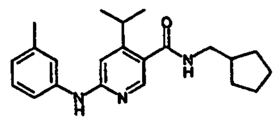 | A<br>180˚<br>30 min | E | 3.52<br>352<br>$C_{22}H_{29}ON_3$ |
| 25 | N-Cyclopentylmethyl-4-isopropyl-6-(3-trifluoromethoxy-phenylamino)-nicotinamide | 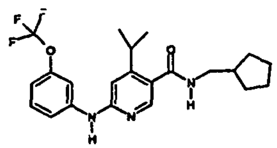 | A<br>180˚<br>30 min | E | 3.86<br>422<br>$C_{22}H_{26}N_3O_2F_3$ |
| 26 | 6-(3-Bromophenylamino)-N-cyclopentylmethyl-4-isopropyl-nicotinamide | 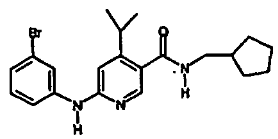 | A<br>180˚<br>30 min | E | 3.86<br>422<br>$C_{21}H_{26}N_3OBr$ |

(continued)

| Ex. No. | Chemical Name | Structure | 1. Preparation method A, B, or C<br>2. Reaction temperature (˚C),<br>3. Time. | Purification method E or F | 1.Retention Time (min).<br>2.[MH+]<br>3. Molecular formula |
|---|---|---|---|---|---|
| 27 | N-Cyclopentylmethyl-4-isopropyl-6-(3-methoxyphenylamino)nicotinamide | 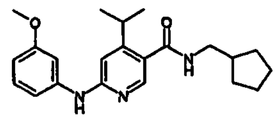 | A<br>180˚<br>30 min | E | 3.81<br>418<br>$C_{22}H_{29}N_3O_2$ |
| 28 | 6-(3-Cyano-phenylamino)-N-cyclopentylmethyl-4-isopropyl-nicotinamide | 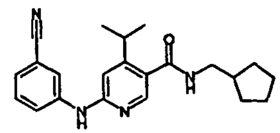 | A<br>180˚<br>30 min | E | 3.55<br>363<br>$C_{22}H_{26}N_4O$ |
| 29 | 6-(2-Chloro-4-fluoro-phenylamino)-N-cyclopentylmethyl-4-isopropyl-nicotinamide | 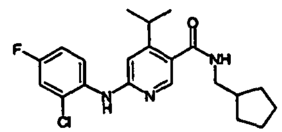 | A<br>180˚<br>30 min | E | 3.6<br>391<br>$C_{21}H_{25}N_3ClFO$ |
| 30 | 6-(2-Chloro-4-cyano-phenylamino)-N-cyclopentylmethyl-4-isopropyl-nicotinamide | 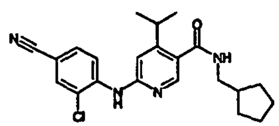 | A<br>180˚<br>30 min | E | 3.76<br>398<br>$C_{22}H_{25}N_4ClO$ |
| 31 | N-Cyclopentylmethyl-6-(2,4-dichloro-phenylamino)-4-isopropyl-nicotinamide | 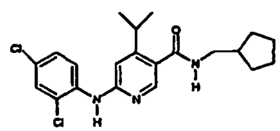 | A<br>180˚<br>30 min | E | 3.70<br>407<br>$C_{21}H_{25}N_3Cl_2O$ |
| 32 | N-Cyclopentylmethyl-6-(3,4-dichlorophenyl)amino)-4-isopropyl-nicotinamide | 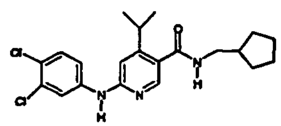 | A<br>180˚<br>30 min | E | 3.80<br>407<br>$C_{21}H_{25}N_3Cl_2O$ |

(continued)

| Ex. No. | Chemical Name | Structure | 1. Preparation method A, B, or C<br>2. Reaction temperature (°C),<br>3. Time. | Purification method E or F | 1.Retention Time (min).<br>2.[MH$^+$]<br>3. Molecular formula |
|---|---|---|---|---|---|
| 33 | 6-(3-Bromo-phenylamino)-N-cyclobutylmethyl-4-isopropyl-nicotinamide | 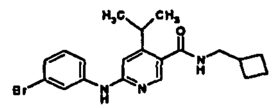 | C<br>180°<br>30 min | F | 3.70<br>402<br>$C_{20}H_{24}{}^{79}BrN_3O$ |
| 34 | N-Cyclobutylmethyl-6-(3-fluoro-phenylamino)-4-isopropyl-nicotinamide | 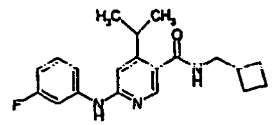 | C<br>180°C<br>30 min | F | 3.49<br>342<br>$C_{20}H_{24}FN_3O$ |
| 35 | N-Cyclobutylmethyl-6-(3-trifluoromethyl-phenylamino)-4-isopropyl-nicotinamide | 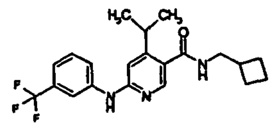 | C<br>180°<br>30 min | F | 3.53<br>392<br>$C_{21}H_{24}F_3N_3O$ |
| 36 | 6-(3-Cyano-phenylamino)-N-cyclobutylmethyl-4-isopropyl-nicotinamide | 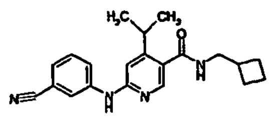 | C<br>180°<br>30 min | F | 3.41<br>349<br>$C_{21}H_{24}N_4O$ |
| 37 | N-Cyclobutylmethyl-4-isopropyl-6-m-tolylamino-nicotinamide | 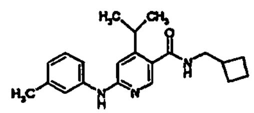 | C<br>180°<br>1 hr | F | 3.39<br>338<br>$C_{21}H_{27}N_3O$ |
| 38 | N-Cyclobutylmethyl-4-isopropyl-6-(3-methoxy-phenylamino)-nicotinamide | 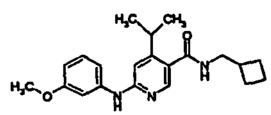 | C<br>180°<br>1hr | F | 3.30<br>354<br>$C_{21}H_{27}N_3O_2$ |

**Table 2**

The Examples 39 to 45 in Table 2 were prepared in a manner similar to as Example 2 with the reaction temperature and time given in the table. An asterisk in the fourth column signifies that the preparative method used was the same as that used in Example 46 and the product was purified by the method given in the 5th column.

Purification Method E: Purify by mass-directed autopurification techniques.

Purification Method F: The crude product was diluted with dichloromethane (2ml) and the solution applied to a Sep-Pack column of silica gel. This was eluted firstly with dichloromethane, followed by dichloromethane/ether 5:1 to give pure product.

| Ex. No | Name | Structure | 1. Reaction Temperature<br>2. Reaction Time | Purificati Time method E, or F | 1.Retention on (min).<br>2.[MH$^+$]<br>3. Molecular formula |
|---|---|---|---|---|---|
| 39 | 6-(3-Fluoro-phenylamino)-4-isopropyl-N-(tetrahydro-pyran-4-ylmethyl)-nicotinamide | 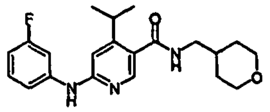 | 200°<br>1 hr | E | 2.9<br>372<br>$C_{21}H_{26}FN_3O_2$ |
| 40 | 1-[6-(3-Fluoro-phenylamino)-4-isopropyl-pyridin-3-yl]-1-morpholin-4-yl-methanone | 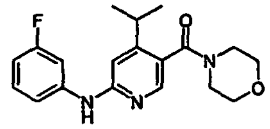 | 180°<br>30 min | E | 2.9<br>344<br>$C_{19}H_{22}FN_3O_2$ |
| 41 | 4-Isopropyl-6-(3-methoxy-phenylamino)-N-(tetrahydro-pyran-4-ylmethyl)-nicotinamide | 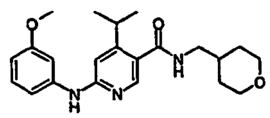 | 180°<br>2 hr | E | 2.7<br>384<br>$C_{22}H_{29}N_3O_3$ |
| 42 | 4-Isopropyl-N-(tetrahydro-pyran-4-ylmethyl)-6-m-tolylamino-nicotinamide | 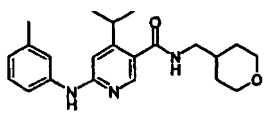 | 180°<br>1 hr | E | 2.93<br>368<br>$C_{22}H_{29}N_3O_2$ |
| 43 | 6-(3-Cyano-phenylamino)-4-isopropyl-N-(tetrahydro-pyran-4-ylmethyl)-nicotinamide | 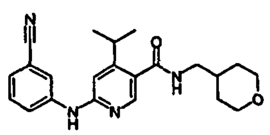 | 180°<br>30 min | E | 2.8<br>379<br>$C_{22}H_{26}N_4O_3$ |
| 44 | 6-[(3,4-Dichloro-phenyl)-methyl-amino]-4-isopropyl-N-(tetrahydro-pyran-4-ylmethyl)-nicotinamide | 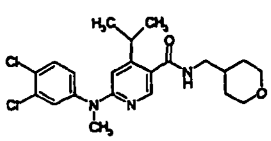 | 180°C<br>2hrs * | E | 3.51<br>436<br>$C_{22}H_{27}{}^{35}Cl_2N_3O_2$ |
| 45 | 6-[(3-Bromo-phenyl)-methyl-amino]-4-isopropyl-N-(tetrahydro-pyran-4-ylmethyl)-nicotinamide | 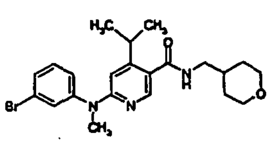 | 180°C<br>2hrs * | F | 3.31<br>446<br>$C_{22}H_{28}{}^{79}BrN_3O_2$ |

**Example 46: 6-[(3-Fluoro-phenyl)-methyl-amino]-4-isopropyl-N-(tetrahydro-pyran-4-ylmethyl)-nicotinamide**

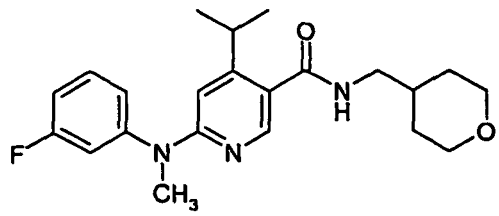

A mixture of 6-chloro-4-isopropyl-N-(tetrahydro-pyran-4-ylmethyl)-nicotinamide (Description 8) (89mg), 3-fluoro-N-methylaniline (75mg) and methanesulphonic acid (72mg) in dioxan (1ml) was heated on microwave at 180˚C for 2 hours. The mixture was diluted with ethyl acetate (20ml) and washed with sodium bicarbonate solution (20ml) and water (2 x 20ml) and evaporated to an oil. Purification by chromatography on silica gel (dichloromethane then dichloromethane/methanol 10:1) gave a solid which was triturated with ether/isohexane 1:1 to give the title compound (63 mg). NMR (DMSO-d6) δ 1.05 (6H, d), 1.15 (2H, m), 1.60 (2H, d), 1.74 (1H, m), 3.10 (2H, t), 3.26 (2H, m), 3.34 (1H, m excess), 3.42 (3H, s), 3.84 (2H, m), 6.64 (1H, s), 7.02 (1H, m), 7.14 (2H, m), 7.43 (1H, q), 8.11 (1H, s), 8.35 (1H, t).
LC/MS t = 2.97 min, Molecular ion observed [$MH^+$] = 386 consistent with the molecular formula $C_{22}H_{28}FN_3O_2$

**Table 3**

All examples prepared in Table 3 were prepared by the same method as given for Example 46, with variations in reaction time, and purification method given in the table. Purification Method E: Purify by mass-directed autopurification techniques.
Purification Method F: The crude product was diluted with dichloromethane (2ml) and the solution applied to a Sep-Pack column of silica gel. This was eluted firstly with dichloromethane, followed by dichloromethane/methanol 10:1 to give pure product.

| Ex. No | Compound Name | Compound Structure | Reaction time | Purification, E or F | 1.Retention Time (min). 2.[$MH^+$] 3. Molecular formula |
|---|---|---|---|---|---|
| 47 | 4-Isopropyl-6-(methyl-phenyl-amino)-N-(tetrahydro-pyran-4-ylmethyl)-nicotinamide | 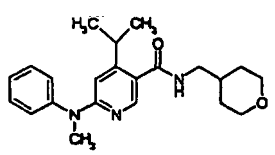 | 1 hr | E, then silica gel chromatography, $CH_2Cl_2$:MeOH, 50:1, then 25:1 | 2.67<br>368<br>$C_{22}H_{29}N_3O_2$ |
| 48 | 6-[(3-Chloro-phenyl)-methyl-amino]-4-isopropyl-N-(tetrahydro-pyran-4-ylmethyl)-nicotinamide | 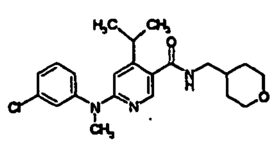 | 2hrs | E | 3.22<br>402<br>$C_{22}H_{28}{}^{35}ClN_3O_2$ |
| 49 | 6-[(4-Chloro-phenyl)-methyl-amino]-4-isopropyl-N-(tetrahydro-pyran-4-ylmethyl)-nicotinamide | 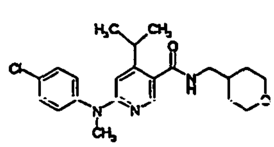 | 2hrs | E | 3.20<br>402<br>$C_{22}H_{28}{}^{35}ClN_3O_2$ |

**Example 50: 6-(3-Chloro-phenylamino)-N-cyclobutyl-4-isopropyl-nicotinamide.**

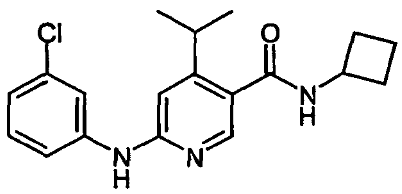

To a solution of 6-(3-chloro-phenylamino)-4-isopropyl-nicotinic acid (Description 13) (48 mg) in dimethylformamide (2.5 ml) was added successively N-ethylmorpholine (69 μl), cyclobutylamine (17 μl), 1-hydroxybenzotriazole hydrate (40 mg) and 1-(3-dimethylaminopropyl)-3-ethylcarbodiimide hydrochloride (40 mg). The solution was stirred for 3 hours and allowed to stand overnight. Dimethylformamide was removed under reduced pressure and ethyl acetate (8 ml) added. The solution was washed sequentially with 5% sodium bicarbonate solution (5 ml), water (5 ml) and brine (2 x 5 ml), dried ($MgSO_4$) and evaporated to afford the title compound (40 mg).
NMR (DMSO-d6) δ 1.16 (6H, d), 1.65 (2H, m), 1.99 (2H, m), 2.2 (2H, m), 3.40 (1H, m), 4.35 (1H, m), 6.77 (1H, s), 6.92 (1H, d), 7.28 (1H, t), 7.46 (1H, d), 8.06 (1H, t), 8.13 (1H, s), 8.56 (1H, d), 9.42 (1H, s).
LC/MS t = 3.51 min, [$MH^+$] 344, consistent with molecular formula $C_{19}H_{22}{}^{35}ClN_3O$

The compounds in Tables 4, 5, and 6 were synthesized by the method used to prepare Example 50.

**Table 4**

| Ex. No | Name | Structure | 1.Retention Time (min). 2.[$MH^+$] 3. Molecular formula |
|---|---|---|---|
| 51 | 6-(3-Chloro-phenylamino)-N-cyclopropylmethyl-4-isopropyl-nicotinamide | 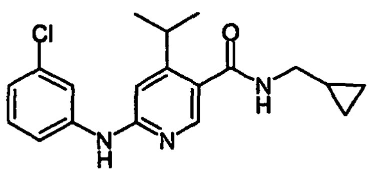 | 3.47<br>344<br>$C_{19}H_{22}{}^{35}ClN_3O$ |
| 52 | 6-(3-Chloro-phenylamino)-N-(2-ethyl-butyl)-4-isopropyl-nicotinamide | 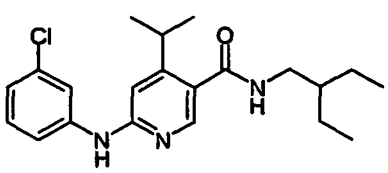 | 3.8<br>374<br>$C_{21}H_{28}{}^{35}ClN_3O$ |
| 53 | 6-(3-Chloro-phenylamino)-N-cyclohexyl-4-isopropyl-nicotinamide | 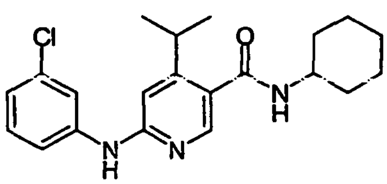 | 3.7<br>372 $C_{21}H_{26}{}^{35}ClN_3O$ |
| 54 | 6-(3-Chloro-phenytamino)-N-(1-hydroxy-cyclohexylmethyl)-4-isopropyl-nicotinamide | 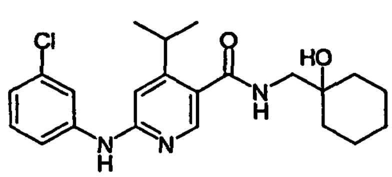 | 3.46<br>402<br>$C_{22}H_{28}{}^{35}ClN_3O_2$ |

(continued)

| Ex. No | Name | Structure | 1.Retention Time (min). 2.[MH+] 3. Molecular formula |
|---|---|---|---|
| 55 | 1-[6-(3-Chloro-phenylamino)-4-isopropyl-pyridin-3-yl]-1-piperidin-1-yl-methanone | 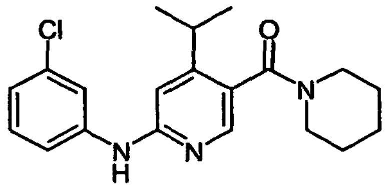 | 3.57<br>358<br>$C_{20}H_{24}{}^{35}ClN_3O$ |

**Table 5**

| Ex. No | Name | Structure | 1.Retention Time (min). 2.[MH+] 3. Molecular formula |
|---|---|---|---|
| 56 | 6-(3-Chloro-phenylamino)-N-(2,2-dimethyl-propyl)-4-isopropyl-nicotinamide | 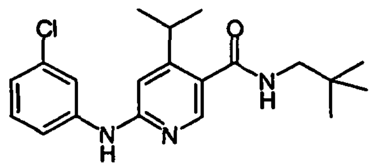 | 3.6<br>360<br>$C_{20}H_{26}{}^{35}ClN_3O$ |
| 57 | 6-(3-Chloro-phenylamino)-4-isopropyl-N-(2-methoxy-ethyl)-nicotinamide | 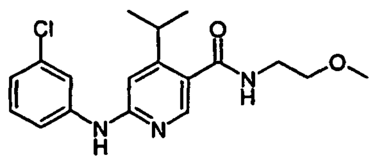 | 3.0<br>348<br>$C_{18}H_{22}{}^{35}ClN_3O_2$ |
| 58 | 6-(3-Chloro-phenylamino)-4-isopropyl-N-(tetrahydro-pyran-4-yl)-nicotinamide | 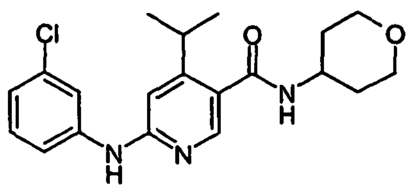 | 3.0<br>374<br>$C_{20}H_{24}{}^{35}ClN_3O_2$ |
| 59 | 6-(3-Chloro-phenylamino)-4-isopropyl-N-[(R)-1-(tetrahydro-furan-2-yl)methyl]-nicotinamide | 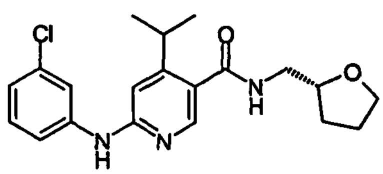 | 3.30<br>374<br>$C_{20}H_{24}{}^{35}ClN_3O_2$ |
| 60 | N-((R)-1-{1-[6-(3-Chloro-phenylamino)-4-isopropyl-pyridin-3-yl]-methanoyl}-pyrrolidin-3-yl)-acetamide | 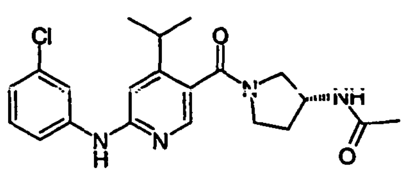 | 2.77<br>401<br>$C_{21}H_{25}{}^{35}ClN_4O_2$ |

(continued)

| Ex. No | Name | Structure | 1.Retention Time (min). 2.[MH+] 3. Molecular formula |
|---|---|---|---|
| 61 | 1-[6-(3-Chloro-phenylamino)-4-isopropyl-pyridin-3-yl]-1-(4-methane-sulfonyl-piperazin-1-yl)-methanone | 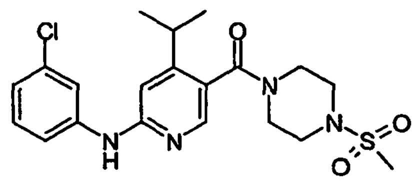 | 3.1<br>437<br>$C_{20}H_{25}{}^{35}ClN_4O_3S$ |
| 62 | 6-(3-Chloro-phenylamino)-N-(1,1-dioxo-tetrahydro-1/6-thiophen-3-yl)-4-isopropyl-nicotinamide | 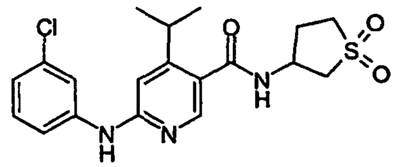 | 3.0<br>408<br>$C_{19}H_{22}{}^{35}ClN_3O_3S$ |

**Table 6**

| Ex. No | Name | Structure | 1.Retention Time (min). 2.[MH+] 3. Molecular formula |
|---|---|---|---|
| 63 | 6-(3-Chloro-phenylamino)-4-isopropyl-N-[(S)-1-(tetrahydro-furan-2-yl)methyl]-nicotinamide | 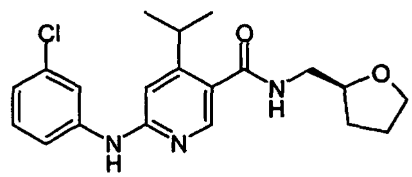 | 3.30<br>374<br>$C_{20}H_{24}{}^{35}ClN_3O_2$ |
| 64 | 6-(3-Chloro-phenylamino)-N-(1,1-dioxo-hexahydro-1/6-thiopyran-4-yl)-4-isopropyl-nicotinamide | 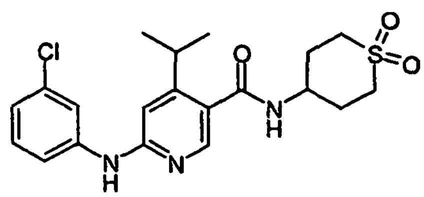 | 2.9<br>422<br>$C_{20}H_{24}{}^{35}ClN_3O_3S$ |
| 65 | 1-[6-(3-Chloro-phenylamino)-4-isopropyl-pyridin-3-yl]-1-(4-methyl-piperazin-1-yl)-methanone | 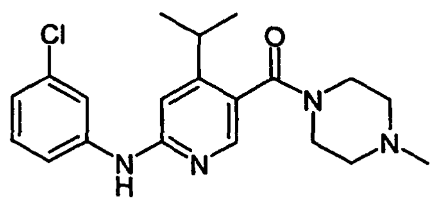 | 2.18<br>373<br>$C_{20}H_{25}{}^{35}ClN_4O$ |
| 66 | 6-(3-Chloro-phenylamino)-N-(2-dimethylamino-ethyl)-4-isopropyl-nicotinamide | 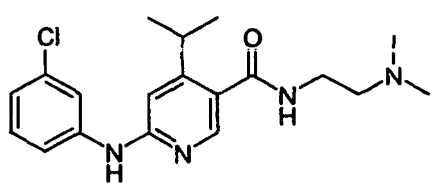 | 2.20<br>361<br>$C_{19}H_{25}{}^{35}ClN_4O$ |

(continued)

| Ex. No | Name | Structure | 1.Retention Time (min). 2.[MH+] 3. Molecular formula |
|---|---|---|---|
| 67 | N-((S)-1-{1-[6-(3-Chloro-phenylamino)-4-isopropyl-pyridin-3-yl]-methanoyl}-pyrrolidin-3-yl)-acetamide | 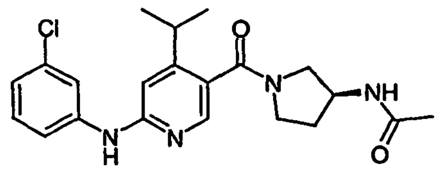 | 2.77<br>401<br>$C_{21}H_{25}{}^{35}ClN_4O_2$ |
| 68 | N-(1-{1-[6-(3-Chloro-phenylamino)-4-isopropyl-pyridin-3-yl]-methanoyl}-piperidin-4-yl)-methanesulfonamide | 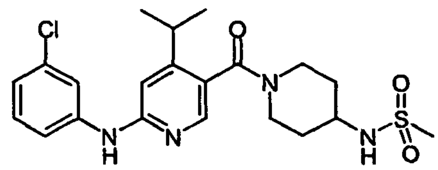 | 2.9<br>451<br>$C_{21}H_{27}{}^{35}ClN_4O_3S$ |

**Example 69: 4-*tert*-Butyl-6-(3-chloro-phenylamino)-N-cyclohexylmethyl-nicotinamide**

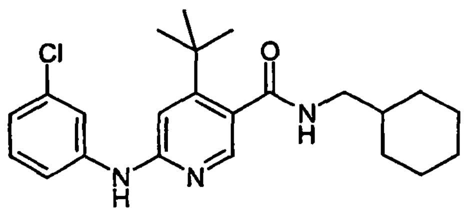

A solution of 4-*tert*-butyl-6-chloro-N-cyclohexylmethyl-nicotinamide (Description 14) (41 mg), 3-chloroaniline (21 μl) and methanesulphonic acid (17μl) in dioxan (0.5 ml) was irradiated under microwave conditions at 180° for 30 minutes. Solvent was evaporated under reduced pressure and the residue purified by MDAP to afford the title compound (35 mg).

NMR (DMSO-d6) δ 0.85-1.0 (2H, m), 1.1-1.25 (3H, m), 1.35 (9H, s), 1.55 (1H, m), 1.6-1.8 (5H, m), 3.03 (2H, t), 6.87 (1H, s), 6.92 (1H, d), 7.27 (1H, t), 7.46 (1H, d), 7.95 (1H, s), 8.03 (1H, t), 8.36 (1H, t), 9.39 (1H, s).

LC/MS t = 4.20 min, [MH+] consistent with molecular formula $C_{23}H_{30}{}^{35}ClN_3O$

**Table 7**

The compounds prepared in Table 7 were prepared in a manner similar to Example 69 from the intermediates in Description 14 or Description 15, with the reaction time given in Table 7.

| Ex. No | Name | Structure | Reaction time (minutes) | 1.Retention Time (min). 2.[MH+] 3. Molecular formula |
|---|---|---|---|---|
| 70 | 4-*tert*-Butyl-6-(2,4-dichloro-phenylamino)-N-cyclohexylmethyl-nicotinamide | 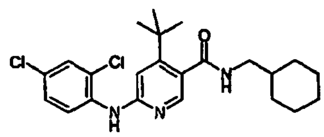 | 75 | 4.35<br>434<br>$C_{23}H_{29}{}^{35}Cl_2N_3O$ |
| 71 | 4-*tert*-Butyl-6-(3-chloro-phenylamino)-N-(tetrahydro-pyran-4-ylmethyl)-nicotinamide | 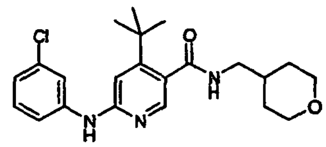 | 30 | 3.40<br>402<br>$C_{22}H_{28}{}^{35}ClN_3O_2$ |
| 72 | 4-*tert*-Butyl-6-(3-fluoro-phenylamino)-N-(tetrahydro-pyran-4-ylmethyl)-nicotinamide | 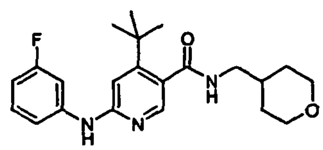 | 30 | 3.21<br>386<br>$C_{22}H_{28}FN_3O_2$ |
| 73 | 4-*tert*-Butyl-6-(2-chloro-3-fluorophenylamino)-N-(tetrahydro-pyran-4-ylmethyl)-nicotinamide | 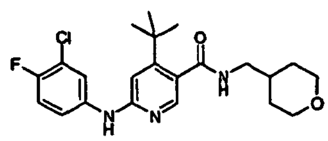 | 30 | 3.40<br>420<br>$C_{22}H_{27}{}^{35}ClFN_3O_2$ |
| 74 | 4-*tert*-Butyl-6-(2,4-di-chloro-phenylamino)-N-(tetrahydro-pyran-4-ylmethyl)-nicotinamide | 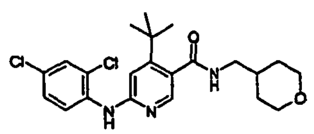 | 60 | 3.40<br>436<br>$C_{22}H_{27}{}^{35}Cl_2N_3O_2$ |

**Example 75: 6-(3,5-Dichloro-phenylamino)-4-isopropyl-N-(tetrahydro-pyran-4-ylmethyl)-nicotinamide**

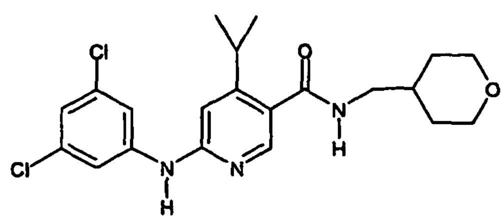

A mixture of 6-chloro-4-isopropyl-N-(tetrahydro-pyran-4-ylmethyl)-nicotinamide (Description 8) (100mg), 3,5-dichloroaniline (ex-Aldrich, 109mg), methanesulfonic acid (44μl) in 1,4-dioxane (1ml) was irradiated under microwave conditions at 180˚C for 30minutes, The crude mixture was purified using MDAP to afford 6-(3,5-dichloro-phenylamino)-4-isopropyl-N-(tetrahydro-pyran-4-ylmethyl)-nicotinamide (50mg)
NMR ($CDCl_3$) δ1.21-1.29 (6H, m), 1.35-1.48 (2H, m), 1.35-1.49 (2H, m), 1.71 (2H, d), 1.86-1.99 (1H, m), 3.34-3.49 (4H, m), 3.50-3.61 (1H, m), 4.03 (2H, d), 6.10 (1H, bs), 6.75 (1H, bs), 7.08 (1H, bs), 7.10-7.16 (1H, m), 7.41-7.45 (2H, m), 8.26 (1H, s)

**Table 8**

Preparative Method B As for the preparation of Example 3
Preparative Method G As for the preparation of Example 75
Purification Method A: Purify by trituration as for Example 2.
Purification Method E: Purify by mass-directed autopreparative technique.
Purification Method H: Purify using the Biotage Horizon system detailed at the beginning of the experimental section.

| | Chemical Name | Structure | Method | Purification method | RT (min), ( MH+), Consistent with the molecular formula |
|---|---|---|---|---|---|
| 76 | 6-(5-Chloro-2-fluoro-phenylamino)-4-isopropyl-N-(tetrahydro-pyran-4-ylmethyl)-nicotinamide | 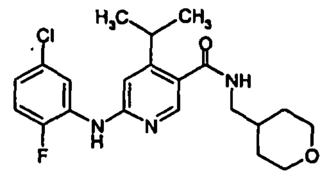 | G | E | 3.13<br>406<br>$C_{21}H_{25}{}^{35}Cl\ FN_3O_2$ |
| 77 | 6-(3-Chloro-4-fluoro-phenylamino)-4-isopropyl-N-(tetrahydro-pyran-4-ylmethyl)-nicotinamide | 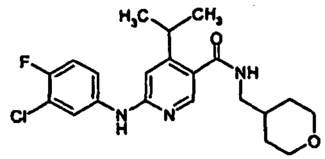 | G | E | 3.13<br>406<br>$C_{21}H_{25}{}^{35}Cl\ FN_3O_2$ |
| 78 | 6-(3-Chloro-4-trifluoromethoxy-phenylamino)-4-isopropyl-N-(tetrahydro-pyran-4-ylmethyl)-nicotinamide | 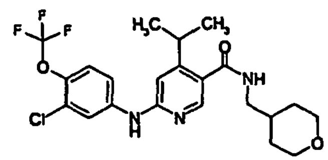 | G | E | 3.62<br>472<br>$C_{22}H_{25}{}^{35}Cl\ F_3N_3O_3$ |
| 79 | 6-(3-Chloro-4-cyano-phenylamino)-4-isopropyl-N-(tetrahydro-pyran-4-ylmethyl)-nicotinamide | 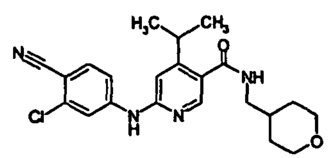 | G | E | 3.10<br>413<br>$C_{22}H_{25}{}^{35}Cl\ N_4O_2$ |
| 80 | 6-(3-Fluoro-5-trifluoromethyl-phenylamino)-4-isopropyl-N-(tetrahydro-pyran-4-ylmethyl)-nicotinamide | 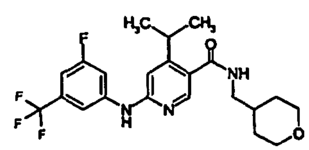 | G | E | 3.20<br>440<br>$C_{22}H_{25}F_4N_3O_2$ |

(continued)

| | Chemical Name | Structure | Method | Purification method | RT (min), ( $MH^+$), Consistent with the molecular formula |
|---|---|---|---|---|---|
| 81 | 6-(2-Fluoro-3-trifluoromethyl-phenylamino)-4-isopropyl-N-(tetrahydro-pyran-4-ylmethyl)-nicotinamide | 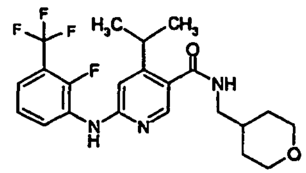 | G | E | 3.40<br>440<br>$C_{22}H_{25}F_4N$ $3^{O}2$ |
| 82 | 6-(4-Bromo-2-chloro-phenylamino)-4-isopropyl-N-(tetrahydro-pyran-4-ylmethyl)-nicotinamide | 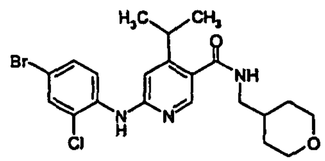 | G | E | 3.41<br>468<br>$C_{21}H_{25}{}^{79}Br\ {}^{35}ClN_3O_2$ |
| 83 | 6-(2-Bromo-4-chloro-phenylamino)-4-isopropyl-N-(tetrahydro-pyran-4-ylmethyl)-nicotinamide | 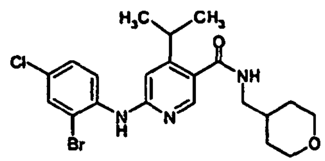 | G | E | 3.39<br>468<br>$C_{21}H_{25}{}^{79}Br\ {}^{35}ClN_3O_2$ |
| 84 | 4-isopropyl-6-(2-methyl-3-trifluoromethyl-phenylamino)-N-(tetrahydro-pyran-4-ylmethyl)-nicotinamide | 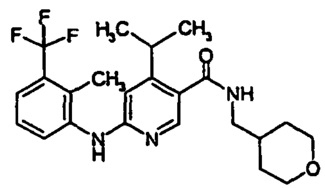 | G | E | 3.09<br>436<br>$C_{23}H_{28}F_3N\ _3O_2$ |
| 85 | 6-(3-chloro-4-methyl-phenylamino)-4-isopropyl-N-(tetrahydro-pyran-4-ylmethyl)-nicotinamide | 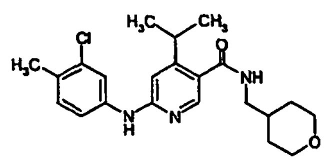 | G | H | 3.24<br>402<br>$C_{22}H_{28}{}^{35}Cl\ N_3O_2$ |

(continued)

| | Chemical Name | Structure | Method | Purification method | RT (min), ( $MH^+$), Consistent with the molecular formula |
|---|---|---|---|---|---|
| 86 | 6-(4-Bromo-3-methyl-phenylamino)-4-isopropyl-N-(tetrahydro-pyran-4-ylmethyl)-nicotinamide | 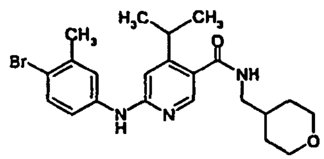 | G | A | 2.48<br>446<br>$C_{22}H_{28}{}^{79}Br\ N_3O_2$ |
| 87 | 6-(2,5-Dichloro-phenylamino)-4-isopropyl-N-(tetrahydro-pyran-4-ylmethyl)-nicotinamide | 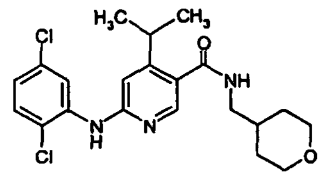 | G<br><br>NB Irradiation time was 60 min. | E | 3.28<br>422<br>$C_{21}H_{25}{}^{35}Cl_2N_3O_2$ |
| 88 | 4-Isopropyl-6-(2-methyl-5-trifluoromethyl-phenylamino)-N-(tetrahydro-pyran-4-ylmethyl)-nicotinamide | 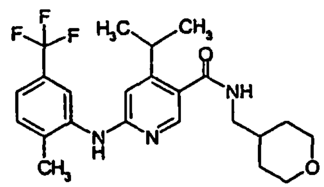 | G | E | 3.23<br>436<br>$C_{23}H_{28}F_3N_3O_2$ |
| 89 | 6-(2-Bromo-4-chloro-phenylamino)-N-cyclopentylmethyl-4-isopropyl-nicotinamide | 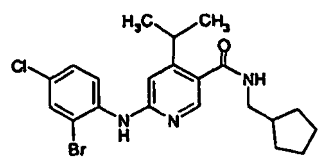 | G | E | 3.97<br>452<br>$C_{21}H_{25}{}^{79}Br\ {}^{35}ClN_3O$ |
| 90 | 6-(4-Bromo-3-chloro-phenylamino)-4-isopropyl-N-(tetrahydro-pyran-4-ylmethyl)-nicotinamide | 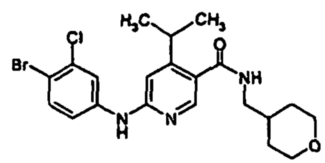 | G | H | 3.48<br>466<br>$C_{21}H_{25}{}^{79}Br\ {}^{35}ClN_3O_2$ |

(continued)

| | Chemical Name | Structure | Method | Purification method | RT (min), ( $MH^+$), Consistent with the molecular formula |
|---|---|---|---|---|---|
| 91 | 6-(4-Chloro-2-fluoro-phenylamino)-N-cyclopentylmethyl-4-isopropyl-nicotinamide | 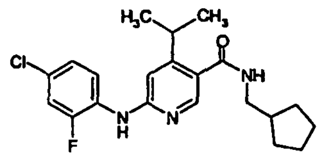 | G | E | 3.7<br>390<br>$C_{21}H_{25}{}^{35}Cl\ FN_3O$ |
| 92 | N-Cyclopentylmethyl-6-(3-fluoro-4-trifluoromethyl-phenylamino)-4-isopropyl-nicotinamide | 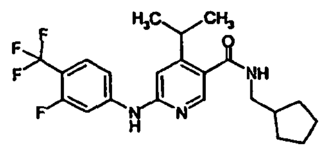 | G | H | 3.8<br>424<br>$C_{22}H_{25}F_4N_3O$ |
| 93 | 6-(4-Cyano-2-methyl-phenylamino)-N-cyclopentylmethyl-4-isopropyl-nicotinamide | 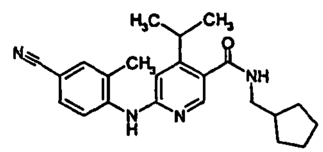 | B | H | 3.43<br>377<br>$C_{23}H_{28}N_4O$ |

**Table 9**

All compounds in table 9 were prepared as for Example 75 and purified by the technique given in the table. Purification Method E: Purify by mass-directed autopreparative technique.
Purification Method H: Purify using the Biotage Horizon system detailed at the beginning of the experimental section.

| Ex. No. | Name | Structure | Purification method | RT (min), (MH+), Consistent with the molecular formula |
|---|---|---|---|---|
| 94 | 6-(3-Chloro-2-fluoro-phenylamino)-4-isopropyl-N-(tetrahydro-pyran-4-ylmethyl)-nicotinamide | 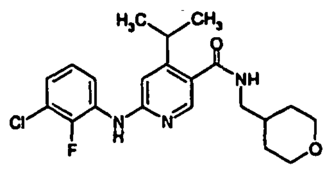 | E | 3.05<br>406<br>$C_{21}H_{25}{}^{35}ClFN_3O_2$ |
| 95 | 6-(3-Fluoro-4-trifluoromethyl-phenylamino)-4-isopropyl-N-(tetrahydro-pyran-4-ylmethyl)-nicotinamide | 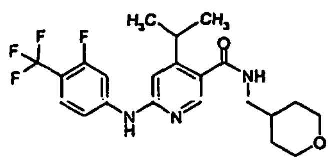 | E | 3.40<br>440<br>$C_{22}H_{25}F_4N_3O_2$ |
| 96 | 6-(4-Cyano-3-trifluoromethyl-phenylamino)-4-isopropyl-N-(tetrahydro-pyran-4-ylmethyl)-nicotinamide | 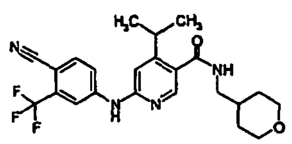 | E | 3.29<br>447<br>$C_{23}H_{25}F_3N_4O_2$ |
| 97 | 6-(4-Cyano-2-fluoro-phenylamino)-4-isopropyl-N-(tetrahydro-pyran-4-ylmethyl)-nicotinamide | 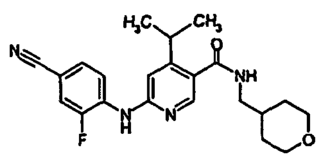 | E | 2.92<br>397<br>$C_{22}H_{25}FN_4O_2$ |
| 98 | 6-(4-fluoro-3-methyl-phenylamino)-4-isopropyl-N-(tetrahydro-pyran-4-ylmethyl)-nicotinamide | 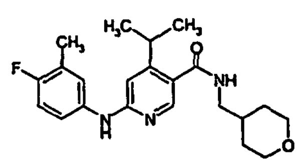 | H | 2.83<br>386<br>$C_{22}H_{28}FN_3O_2$ |
| 99 | 6-(5-Chloro-2-methyl-phenylamino)-4-isopropyl-N-(tetrahydro-pyran-4-ylmethyl)-nicotinamide | 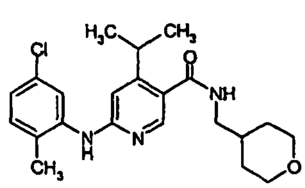 | E | 3.02<br>402<br>$C_{22}H_{28}{}^{35}ClN_3O_2$ |
| 100 | 6-(3-Fluoro-4-methyl-phenylamino)-4-isopropyl-N-(tetrahydro-pyran-4-ylmethyl)-nicotinamide | 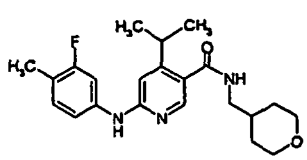 | H | 3.03<br>386<br>$C_{22}H_{28}FN_3O_2$ |

(continued)

| Ex. No. | Name | Structure | Purification method | RT (min), (MH+), Consistent with the molecular formula |
|---|---|---|---|---|
| 101 | 6-(3,4-Dimethyl-phenylamino)-4-isopropyl-N-(tetrahydro-pyran-4-ylmethyl)-nicotinamide | 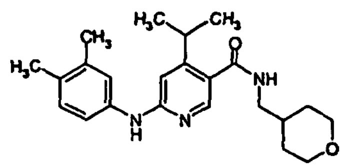 | H | 2.85<br>382<br>$C_{23}H_{31}N_3O_2$ |
| 102 | 6-(3-Bromo-4-methyl-phenylamino)-4-isopropyl-N-(tetrahydro-pyran-4-ylmethyl)-nicotinamide | 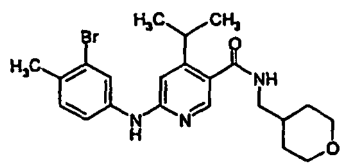 | H | 3.32<br>446<br>$C_{22}H_{28}{}^{79}BrN_3O_2$ |

**Example 103: 6-(3-Chloro-phenylamino)-N-(4-hydroxy-tetrahydro-pyran-4-ylmethyl)-4-isopropyl-nicotinamide**

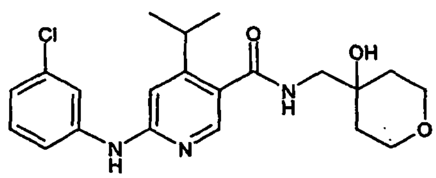

This was prepared by the same method used to prepare Example 50 from Description 16. LC/MS t = 2.89 min, $[MH^+]$ 404 $C_{21}H_{26}{}^{35}ClN_3O_3$

**Example 104: 6-(2,3-Dichloro-phenylamino)-N-(cyclobutyl)-4-trifluoromethyl-nicotinamide**

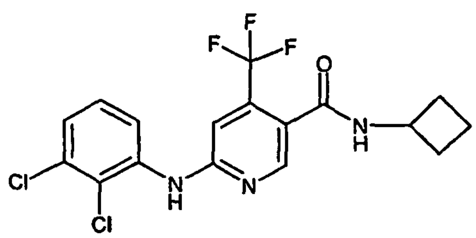

N-methylmorpholine (48 uL, 0.43 mmol), cyclobutylamine (13 mg, 0.18 mmol), 1-hydroxybenzotriazole (30 mg, 0.22 mmol), 1-(3-dimethylamino-propyl)-3-ethylcarbodiimide hydrochloride (32 mg, 0.17 mmol) were added to a solution of 6-(2,3-dichloro-phenylamino)-4-trifluoromethyl nicotinic acid (Description 18) (50 mg, 0.14 mmol) in dimethylformamide (3 mL). After stirring at room temperature for 6 h, dimethylformamide was evaporated under reduced pressure and dichloromethane was added. The solution was washed with an aqueous solution of $NaHCO_3$ 5% (5 mL), with water (10 mL), then with brine (2 x 3 mL) and was evaporated under reduced pressure. The crude residue was triturated with diethyl ether, filtered and dried under vacuum to afford the title compound (46 mg, yield=81 %).
$^1H$ NMR (300 MHz, DMSO-$d_6$) $\delta$: 9.27 (s br, 1H); 8.66 (d br, 1H); 8.27 (s, 1H); 7.90 (dd, 1H); 7.42-7.31 (m, 3H); 4.30 (m, 1H); 2,21 (m, 2H); 1.97 (m, 2H); 1.66 (m, 2H).
MS m/z (EI+); TSQ 700; source 180˚C; 70 V; 200 uA: 403 ($M^{+\cdot}$), 375, 332.

**Example 105: 6-(2,4-Dichloro-phenylamino)-N-(tetrahydropyran-4-ylmethyl)-4-trifluoromethyl-nicotinamide**

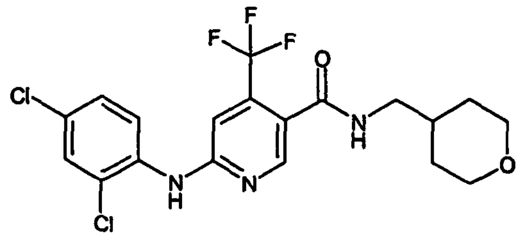

1-Hydroxy-7-azabenzotriazole (33 mg, 0.24 mmol), tetrahydropyran-4-ylmethylamine (17 mg, 0.14 mmol) and PS-carbodiimide (218 mg, 0.28 mmol, loading 1.31 mmol/g, ex Argonaut Technologies) were added to a solution of 6-(2,4-dichloro-phenylamino)-4-trifluoromethyl nicotinic acid (Description 20)(75 mg, 0.21 mmol) in 3 mL of dichloromethane. After orbital shaking at room temperature overnight, the resin was filtered and washed repeatedly with dichloromethane; the filtrate was treated with an aqueous solution of $NaHCO_3$ 5%. The organic layer was separated through Phase Separator cartridge, dried over sodium sulphate and evaporated in vacuo. The solid residue was triturated with acetonitrile, filtered and dried under vacuum to afford the title compound (44 mg, yield=46 %).
$^1$H NMR (300 MHz, DMSO-$d_6$) δ: 9.18 (s, 1H); 8.48 (t br, 1H); 8.27 (s, 1H); 7.98 (d, 1H); 7.66 (d, 1H); 7.42 (dd, 1H); 7.37 (s, 1H); 3.84 (dd, 2H); 3.26 (dd, 2H); 3.10 (dd, 1H); 1.74 (m, 1H); 1.60 (d br, 2H); 1.18 (m, 2H).
MS m/z (EI+); TSQ 700; source 180˚C; 70 V; 200 uA: 447 ($M^+$), 412, 333, 314.

**Example 106: 6-(3-Chloro-phenylamino)-N-(1,1-dioxo-tetrahydrothiophen-3-ylmethyl)-4-trifluoromethyl-nicotinamide**

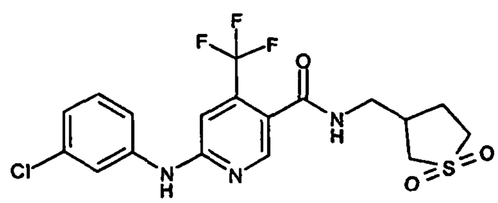

PS-carbodiimide (1.6 g, 2 mmol, loading 1.31 mmol/g, ex Argonaut Technologies) and 1-hydroxy-benzotriazole (0.2 g, 1.5 mmol) were added to a solution of 6-(3-chlorophenylamino)-4-trifluoromethyl nicotinic acid (Description 22) (0.35 g, 1 mmol) in dry dichloromethane (15 mL) and the mixture was stirred at room temperature overnight. The resin was filtered and washed repeatedly with dichloromethane, the solvent was then removed under reduced pressure. The solid residue was dissolved in anhydrous tetrahydrofuran (3.5 mL) and PS-diisopropylethylamine (300 mg, 1.16 mmol, loading 3.88 mmol/g, ex Argonaut Technologies), (1,1-dioxo-tetrahydrothiophen-3-yl)methylamine (0.185 g, 1 mmol) and 1-butyl-3-methylimidazolium hexafluorophosphate (72 uL, 0.35 mmol) were added. The mixture was heated in a sealed tube under microwaves irradiation for 40 min at 140˚C (power=25-30W), then the resin was filtered and washed with THF (15 mL) and dichloromethane (15 mL) and the filtrate was evaporated under reduced pressure. The residue was dissolved in dichloromethane, washed with an aqueous solution of $K_2CO_3$ 10%, dried over magnesium sulphate and evaporated under reduced pressure. Purification by flash chromatography on silica gel (initial eluent: DCM, final eluent: DCM / MeOH 98:2) yielded the title compound (210 mg, yield=47%).
$^1$H NMR (300 MHz, $CDCl_3$) δ: 8.41 (s, 1H); 8.38 (s, 1H); 7.73 (dd, 1H); 7.37 (d br, 1H); 7.36 (t br, 1H); 7.21 (dd, 1H); 7.04 (s, 1H); 6.98 (d br, 1H); 3.60-3.39 (m, 2H); 3.24-3.12 (m, 2H); 3.02 (ddd, 1H); 2.90-2.70 (m, 2H); 2.38-2.26 (m, 1H); 2.09-1.87 (m,1H).
MS m/z (EI+); TSQ 700; source 180˚C; 70 V; 200 uA: 447 ($M^{+\cdot}$); 299; 236.

**Table 10.**

Compounds of Examples 107 to 172 described in Table 10 were prepared as described in Example 104 (Method

A), Example 105 (Method B) and Example 106 (Method C). The method used is indicated in the third column.

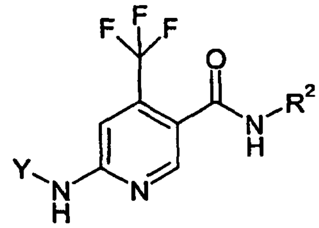

| Ex. No | Chemical name | Method | Y | $R^2$ | $^1H$ NMR (solvent) ppm and/or MS |
|---|---|---|---|---|---|
| 107 | N-Cyclohexylmethyl-6-phenylamino-4-trifluoromethyl-nicotinamide | B | 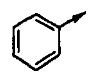 | 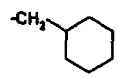 | ESI Pos: AQA; Spray 3.5kV; Skimmer 30V; Probe 250 °C: 378 (MH+). |
| 108 | N-Cyclopentylmethyl-6-phenylamino-4-trifluoromethyl-nicotinamide | B | 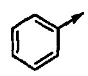 | 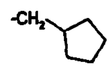 | ESI Pos: AQA; Spray 3.5kV; Skimmer 30V; Probe 250 °C: 364 (MH+). |
| 109 | N-Cyclobutylmethyl-6-phenylamino-4-trifluoromethyl-nicotinamide | B | 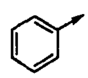 | 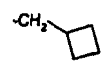 | ESI Pos: AQA; Spray 3.5kV; Skimmer 30V; Probe 250 °C: 350 (MH+). |
| 110 | N-Cyclobutyl-6-(3-chloro-phenylamino)-4-trifluoromethyl-nicotinamide | B | 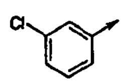 | 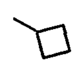 | $^1H$ NMR (300 MHz, DMSO-$d_6$) δ: 9.87 (s, 1H); 8.66 (d br, 1H); 8.40 (s, 1H); 8.01 (dd, 1H); 7.49 (dd, 1H); 7.34 (dd, 1H); 7.16 (s, 1H); 7.02 (dd, 1H); 4.31 (m, 1H); 2.22 (m, 2H); 1.99 (m, 2H); 1.67 (m, 2H).<br>ESI Pos: AQA; Spray 3 kV; Source 20 V; Probe 250°C: 370 (MH+). |
| 111 | N-(Tetrahydropyran-4-ylmethyl)-6-(3-chloro-phenylamino)-4-trifluoromethyl-nicotinamide | B | 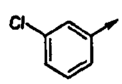 | 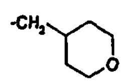 | ESI Pos: AQA; Spray 3.5kV; Skimmer 30V; Probe 250°C: 414 (MH+). |
| 112 | N-Cyclobutylmethyl-6-(3-chloro-phenylamino)-4-trifluoromethyl-nicotinamide | B | 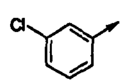 | 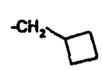 | ESI Pos: AQA; Spray 3.5kV; Skimmer 30V; Probe 250°C: 383 (MH+). |
| 113 | N-Isobutyl-6-(3-chloro-phenylamino)-4-trifluoromethyl-nicotinamide | B | 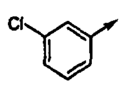 | 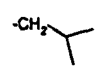 | ESI Pos: AQA; Spray 3.5kV; Skimmer 30V; Probe 250°C: 372 (MH+). |
| 114 | N-Cyclopentylmethyl-6-(3-chloro-phenylamlno)-4-trifluoromethyl-nicotinamide | B | 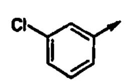 | 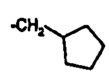 | ESI Pos: AQA; Spray 3.5kV; Skimmer 30V; Probe 250°C: 398 (MH+). |
| 115 | N-Cyclopropylmethyl-6-(3-chloro-phenylamino)-4-trifluoromethyl-nicotinamide | B | 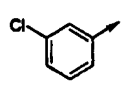 | 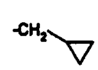 | ESI Pos: AQA; Spray 3.5kV; Skimmer 30V; Probe 250°C: 370 (MH+). |

(continued)

| Ex. No | Chemical name | Method | Y | $R^2$ | $^1H$ NMR (solvent) ppm and/or MS |
|---|---|---|---|---|---|
| 116 | N-Cyclohexylmethyl-6-(3-bromo-phenylamino)-4-trifluoromethyl-nicotinamide | B | 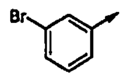 | 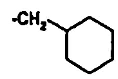 | ESI Pos: AQA; Spray 3.5kV; Skimmer 30V; Probe 250˚C: 456 (MH+). |
| 117 | N-Cycloheptylmethyl-6-(3-bromo-phenylamino)-4-trifluoromethyl-nicotinamide | B | 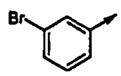 | 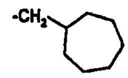 | ESI Pos: AQA; Spray 3.5kV; Skimmer 30V; Probe 250˚C: 470 (MH+). |
| 118 | N-(Tetrahydropyran-4-ylmethyl)-6-(3-bromo-phenylamino)-4-trifluoromethyl-nicotinamide | B | 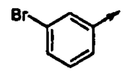 | 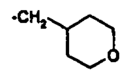 | ESI Pos: AQA; Spray :3.5kV; Skimmer 30V; Probe 250˚C: 458 (MH+). |
| 119 | N-Cyclobutyl-6-(3-bromo-phenylamino)-4-trifluoromethyl-nicotinamide | B | 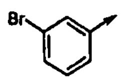 | 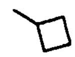 | ESI Pos: AQA; Spray 3.5kV; Skimmer 30V; Probe 250˚C: 414 (MH+). |
| 120 | N-Cyclobutylmethyl-6-(3-bromo-phenylamino)-4-trifluoromethyl-nicotinamide | B | 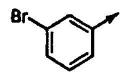 | 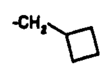 | ESI Pos: AQA; Spray 3.5kV; Skimmer 30V; Probe 250˚C: 427 (MH+). |
| 121 | N-Isobutyl-6-(3-bromo-phenylamino)-4-trifluoromethyl-nicotinamide | B | 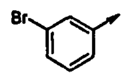 | 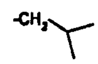 | ESI Pos: AQA; Spray 3.5kV; Skimmer 30V; Probe 250˚C: 415 (MH+). |
| 122 | N-Cyclopentylmethyl-6-(3-bromo-phenylamino)-4-trifluoromethyl-nicotinamide | B | 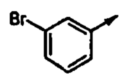 | 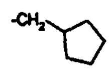 | ESI Pos: AQA; Spray 3.5kV; Skimmer 30V; Probe 250˚C: 442 (MH+). |
| 123 | N-Cyclopropylmethyl-6-(3-bromo-phenylamino)-4-trifluoromethyl-nicotinamide | B | 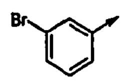 | 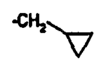 | ESI Pos: AQA; Spray 3.5kV; Skimmer 30V; Probe 250˚C: 414 (MH+). |
| 124 | N-Cyclobutylmethyl-6-(2-fluoro-phenylamino)-4-trifluoromethyl-nicotinamide | B | 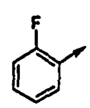 | 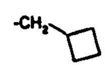 | ESI Pos: AQA; Spray 3.5kV; Skimmer 30V; Probe 250˚C: 368 (MH+). |
| 125 | N-Cycloheptylmethyl-6-(3-fluoro-phenylamino)-4-trifluoromethyl-nicotinamide | B | 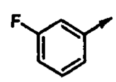 | 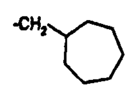 | ESI Pos: AQA; Spray 3.5kV; Skimmer 30V; Probe 250˚C: 410 (MH+). |
| 126 | N-(Tetrahydropyran-4-ylmethyl)-6-(3-fluoro-phenylamino)-4-trifluoromethyl-nicotinamide | B | 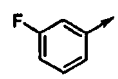 | 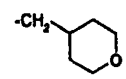 | ESI Pos: AQA; Spray 3.5kV; Skimmer 30V; Probe 250˚C: 398 (MH+). |
| 127 | N-Cyclobutyl-6-(3-fluoro-phenylamino)4-trifluoromethyl-nicotinamide | B | 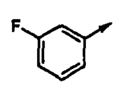 | 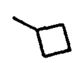 | ESI Pos: AQA; Spray 3.5kV; Skimmer 30V; Probe 250˚C: 354 (MH+). |
| 128 | N-Cyclohexylmethyl-6-(3-fluoro-phenylamino)-4-trifluoromethyl-nicotinamide | B | 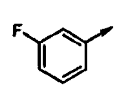 | 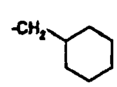 | ESI Pos: AQA; Spray 3.5kV; Skimmer 30V; Probe 250˚C: 396 (MH+). |
| 129 | N-Cyclobutylmethyl-6-(3-fluoro-phenylamino)-4-trifluoromethyl-nicotinamide | B | 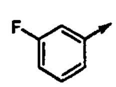 | 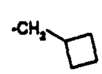 | ESI Pos: AQA; Spray 3.5kV; Skimmer 30V; Probe 250˚C: 367 (MH+). |

(continued)

| Ex. No | Chemical name | Method | Y | $R^2$ | $^1$H NMR (solvent) ppm and/or MS |
|---|---|---|---|---|---|
| 130 | N-Cyclopentylmethyl-6-(3-fluoro-phenylamino)-4-trifluoromethyl-nicotinamide | B | 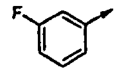 | 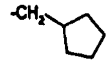 | ESI Pos: AQA; Spray 3.5kV; Skimmer 30V; Probe 250˚C: 382 (MH+). |
| 131 | N-Isobutyl-6-(3-fluoro-phenylamino)-4-trifluoromethyl-nicotinamide | B | 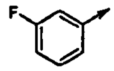 | 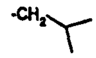 | ESI Pos: AQA; Spray 3.5kV; Skimmer 30V; Probe 250˚C: 356 (MH+). |
| 132 | N-Cyclopropylmethyl-6-(3-fluoro-phenylamino)-4-trifluoromethyl-nicotinamide | B | 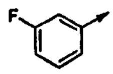 | 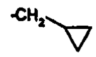 | ESI Pos: AQA; Spray 3.5kV; Skimmer 30V; Probe 250˚C: 354 (MH+). |
| 133 | N-(1,1-Dioxo-tetrahydro-thiophen-3-ylmethyl)-6-(3-fluoro-phenylamino)-4-trifluoromethyl-nicotinamide | C | 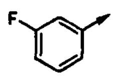 | 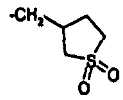 | ESI Pos: AQA; Spray 3.5kV; Skimmer 30V; Probe 250˚C: 432 (MH+). |
| 134 | N-Cyclobutylmethyl-6-(4-fluoro-phenylamino)-4-trifluoromethyl-nicotinamide | B | 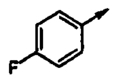 | 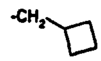 | ESI Pos: AQA; Spray 3.5kV; Skimmer 30V; Probe 250˚C: 368 (MH+). |
| 135 | N-(Tetrahydropyran-4-ylmethyl)-6-(2,3-dichloro-phenylamino)-4-trifluoromethyl-nicotinamide | B | 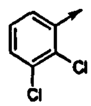 | 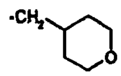 | ESI Pos: AQA; Spray 3.5kV; Skimmer 30V; Probe 250˚C: 448 (MH+). |
| 136 | N-Cyclohexylmethyl-6-(2,3-dichloro-phenylamino)-4-trifluoromethyl-nicotinamide | B | 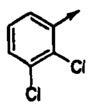 | 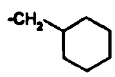 | ESI Pos: AQA; Spray 3.5kV; Skimmer 30V; Probe 250˚C: 446 (MH+). |
| 137 | N-Cycloheptylmethyl-6-(2,3-dichloro-phenylamino)-4-trifluoromethyl-nicotinamide | B | 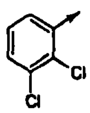 | 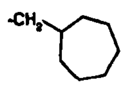 | ESI Pos: AQA; Spray 3.5kV; Skimmer 30V; Probe 250˚C: 460 (MH+). |
| 138 | N-Cyclohexylmethyl-6-(2,4-dichloro-phenylamino)-4-trifluoromethyl-nicotinamide | B | 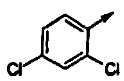 | 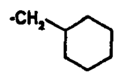 | ESI Pos: AQA; Spray 3.5kV; Skimmer 30V; Probe 250˚C: 447 (MH+). |
| 139 | N-Cycloheptylmethyl-6-(2,4-dichloro-phenylamino)-4-trifluoromethyl-nicotinamide | B | 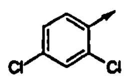 | 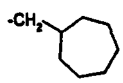 | ESI Pos: AQA; Spray 3.5kV; Skimmer 30V; Probe 250˚C: 460 (MH+). |
| 140 | N-Cyclobutyl-6-(2,4-dichloro-phenylamino)-4-trifluoromethyl-nicotinamide | B | 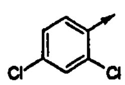 | 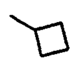 | ESI Pos: AQA; Spray 3.5kV; Skimmer 30V; Probe 250˚C: 404 (MH+). |
| 141 | N-Cyclopentylmethyl-6-(2,4-dichloro-pheny(amino)-4-trifluoromethyl-nicotinamide | B | 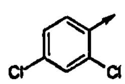 | 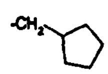 | ESI Pos: AQA; Spray 3.5kV; Skimmer 30V; Probe 250˚C: 432 (MH+). |
| 142 | N-Cyclobutylmethyl-6-(2,4-dichloro-phenylamino)-4-trifluoromethyl-nicotinamide | B | 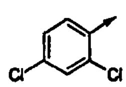 | 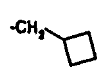 | ESI Pos: AQA; Spray 3.5kV; Skimmer 30V; Probe 250˚C: 418 (MH+). |

(continued)

| Ex. No | Chemical name | Method | Y | $R^2$ | $^1H$ NMR (solvent) ppm and/or MS |
|---|---|---|---|---|---|
| 143 | N-Isobutyl-6-(2,4-dichloro-phenylamino)-4-trifluoromethyl-nicotinamide | B | 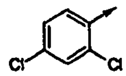 | 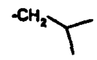 | ESI Pos: AQA; Spray 3.5kV; Skimmer 30V; Probe 250˚C: 406 (MH+). |
| 144 | N-Cyclopropylmethyl-6-(2,4-dichloro-phenylamino)-4-trifluoromethyl-nicotinamide | B | 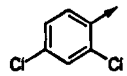 | 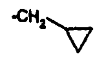 | ESI Pos: AQA; Spray 3.5kV; Skimmer 30V; Probe 250˚C: 404 (MH+). |
| 145 | N-(1,1-Dioxo-tetrahydro-thiophen-3-ylmethyl)-6-(2,4-dichloro-phenylamino)-4-trifluoromethyl-nicotinamide | C | 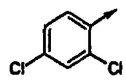 | 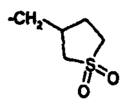 | $^1H$ NMR (300 MHz, $CDCl_3$) δ: 8.38 (s, 1H); 8.08 (d, 1H); 7.47 (s, 1H); 7.41 (t br, 1H); 7.40 (d, 1H); 7.23 (dd, 1H); 7.04 (s, 1H); 3.60-3.39 (m, 2H); 3.24-3.12 (m, 2H); 3.01 (ddd, 1H); 2.90-2.72 (m, 2H); 2.38-2.26 (m, 1H); 2.09-1.87 (m,1H).<br>EI+; TSQ 700; source 180˚C; 70 V; 200 uA: 481 ($M^+$); 446; 333; 270. |
| 146 | N-Cyclohexylmethyl-6-(3,5-dichloro-phenylamino)-4-trifluoromethyl-nicotinamide | B | 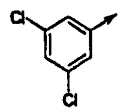 | 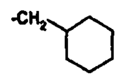 | ESI Pos: AQA; Spray 3.5kV; Skimmer 30V; Probe 250˚C: 446 (MH+). |
| 147 | N-(Tetrahydropyran-4-ylmethyl)-6-(3,5-dichloro-phenylamino)-4-trifluoromethyl-nicotinamide | B | 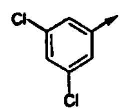 | 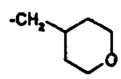 | ESI Pos: AQA; Spray 3.5kV; Skimmer 30V; Probe 250˚C: 448 (MH+). |
| 148 | N-Cyclobutyl-6-(3,5-dichloro-phenylamino)-4-trifluoromethyl-nicotinamide | B | 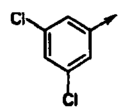 | 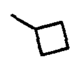 | ESI Pos: AQA; Spray 3.5kV; Skimmer 30V; Probe 250˚C: 404 (MH+). |
| 149 | N-Cyclopentylmethyl-6-(3,5-dichloro-phenylamino)-4-trifluoromethyl-nicotinamide | B | 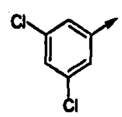 | 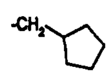 | ESI Pos: AQA; Spray 3.5kV; Skimmer 30V; Probe 250˚C: 432 (MH+). |
| 150 | N-Cyclobutylmethyl-6-(3,5-dichloro-phenylamino)-4-trifluoromethyl-nicotinamide | B | 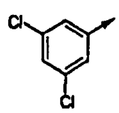 | 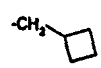 | ESI Pos: AQA; Spray 3.5kV; Skimmer 30V; Probe 250˚C: 418 (MH+). |
| 151 | N-Isobutyl-6-(3,5-dichloro-phenylamino)-4-trifluoromethyl-nicotinamide | B | 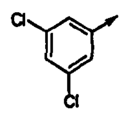 | 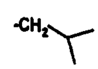 | ESI Pos: AQA; Spray 3.5kV; Skimmer 30V; Probe 250˚C: 406 (MH+). |
| 152 | N-Cyclopropylmethyl-6-(3,5-dichloro-phenylamino)-4-trifluoromethyl-nicotinamide | B | 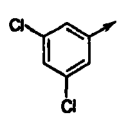 | 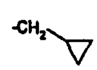 | ESI Pos: AQA; Spray 3.5kV; Skimmer 30V; Probe 250˚C: 404 (MH+). |

(continued)

| Ex. No | Chemical name | Method | Y | $R^2$ | $^1$H NMR (solvent) ppm and/or MS |
|---|---|---|---|---|---|
| 153 | N-isobutyl-6-(3,4-dichloro-phenylamino)-4-trifluoromethyl-nicotinamide | B | 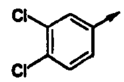 | 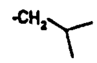 | $^1$H NMR (300 MHz, DMSO-$d_6$) δ: 9.98 (s br, 1H); 8.47 (t br, 1H); 8.41 (s, 1H); 8.20 (s, 1H); 7.55 (s, 2H); 7.17 (s, 1H); 3.05 (dd, 2H); 1.80 (m, 1H); 0.90 (d, 6H). ESI Pos: AQA; Spray 3 kV; Source 20 V; Probe 250˚C: 406(MH+). |
| 154 | N-Cyclobutyl-6-(3,4-dichloro-phenylamino)-4-trifluoromethyl-nicotinamide | B | 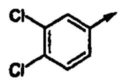 | 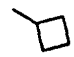 | ESI Pos: AQA; Spray 3.5kV; Skimmer 30V; Probe 250˚C: 404 (MH+). |
| 155 | N-(Tetrahydropyran-4-ylmethyl)-6-(3,4-dichloro-phenylamino)-4-trifluoromethyl-nicotinamide | B | 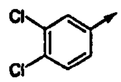 | 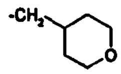 | ESI Pos: AQA; Spray :3.5kV; Skimmer 30V; Probe 250˚C: 448 (MH+). |
| 156 | N-Cyclopentylmethyl-6-(3,4-dichloro-phenylamino)-4-trifluoromethyl-nicotinamide | B | 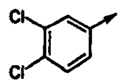 | 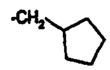 | ESI Pos: AQA; Spray :3.5kV; Skimmer 30V; Probe 250˚C: 432 (MH+). |
| 157 | N-Cyclobutylmethyl-6-(3,4-dichloro-phenylamino)-4-trifluoromethyl-nicotinamide | B | 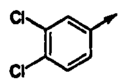 | 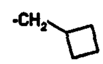 | ESI Pos: AQA; Spray 3.5kV; Skimmer 30V; Probe 250˚C: 418 (MH+). |
| 158 | N-Cyclopropylmethyl-6-(3,4-dichloro-phenylamino)-4-trifluoromethyl-nicotinamide | B | 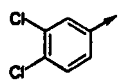 | 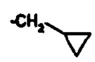 | ESI Pos: AQA; Spray 3.5kV; Skimmer 30V; Probe 250˚C: 404 (MH+). ESI Pos: AQA; Spray 3.5kV; Skimmer 30V; Probe 250˚C: 446 (MH+). |
| 159 | N-Cyclohexylmethyl-6-(3,4-dichloro-phenylamino)-4-trifluoromethyl-nicotinamide | B | 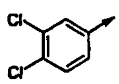 | 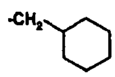 | |
| 160 | N-Cyclobutylmethyl-6-(2-fluoro-4-chloro-phenylamino)-4-trifluoromethyl-nicotinamide | A | 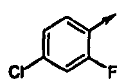 | 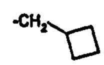 | $^1$H NMR (300 MHz, DMSO-$d_6$) δ: 9.42 (s, 1H); 8.42 (t br, 1H); 8.28 (s, 1H); 8.17 (dd, 1H); 7.48 (dd, 1H); 7.35 (s, 1H); 7.27 (d br, 1H); 3.23 (dd, 2H); 2.48 (m, 1H); 2.04-1.91 (m, 2H); 1.89-1.64 (m, 4H). EI+; TSQ 700; source 180˚C; 70 V; 200 uA: 401 ($M^+$) 366, 333, 317. |
| 161 | N-Cyclopentylmethyl-6-(2-fluoro-4-chloro-phenylamino)-4-trifluoromethyl-nicotinamide | A | 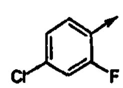 | 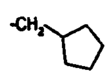 | $^1$H NMR (300 MHz, DMSO-$d_6$) δ: 9.42 (s, 1H); 8.47 (t br, 1H); 8.29 (s, 1H); 8.17 (dd, 1H); 7.48 (dd, 1H); 7.35 (s, 1H); 7.27 (d br, 1H); 3.14 (dd, 2H); 2.08 (m, 1H); 1.75-1.42 (m, 6H); 1.29-1.15 (m, 2H). EI+; TSQ 700; source 180˚C; 70 V; 200 uA: 415 ($M^+$) 346,333,317. |

(continued)

| Ex. No | Chemical name | Method | Y | $R^2$ | $^1$H NMR (solvent) ppm and/or MS |
|---|---|---|---|---|---|
| 162 | N-(Tetrahydropyran-4-ylmethyl)-6-(2-fluoro-4-chloro-phenylamino)-4-trifluoromethyl-nicotinamide | A | 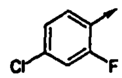 | 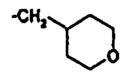 | $^1$H NMR (300 MHz, DMSO-$d_6$) δ: 9.44 (s, 1H); 8.50 (t br, 1H); 8.32 (s, 1H); 8.17 (dd, 1H); 7.48 (dd, 1H); 7.36 (s, 1H); 7.29 (d br, 1H); 3.84 (dd, 2H); 3.26 (dd, 2H); 3.11 (dd, 2H); 1.74 (m, 1H); 1.60 (m, 2H); 1.19 (m, 2H). EI+; TSQ 700; source 180˚C; 70 V; 200 uA: 431 ($M^+$); 346; 333; 317. |
| 163 | N-Cyclobutylmethyl-6-(2-chloro-4-fluoro-phenylamino)-4-trifluoromethyl-nicotinamide | A | 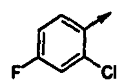 | 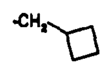 | $^1$H NMR (300 MHz, DMSO-$d_6$) δ: 9.13 (s, 1H); 8.39 (t br, 1H); 8.19 (s, 1H); 7.80 (dd, 1H); 7.51 (dd, 1H); 7.24 (dt, 1H); 7.20 (s, 1H); 3.22 (dd, 2H); 2.55-2.42 (m, 1H); 2.04-1.63 (m, 6H). EI+; TSQ 700; source 180˚C; 70 V; 200 uA: 401 ($M^+$); 366; 317; 298; 254. |
| 164 | N-Cyclopentylmethyl-6-(2-chloro-4-fluoro-phenylamino)-4-trifluoromethyl-nicotinamide | A | 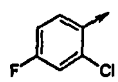 | 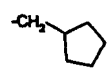 | $^1$H NMR (300 MHz, DMSO-$d_6$) δ: 9.13 (s, 1H); 8.42 (t br, 1H); 8.20 (s, 1H); 7.81 (dd, 1H); 7.52 (dd, 1H); 7.24 (dt, 1H); 7.20 (s, 1H); 3.13 (dd, 2H); 2.07 (m, 1H); 1.75-1.42 (m, 6H); 1.30-1.15 (m, 2H). EI+; TSQ 700; source 180˚C; 70 V; 200 uA: 415 ($M^+$); 380; 346; 317; 298; 254. |
| 165 | N-(Tetrahydropyran-4-ylmethyl)-6-(2-chloro-4-fluoro-phenylamino)-4-trifluoromethyl-nicotinamide | A | 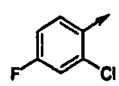 | 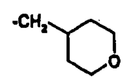 | $^1$H NMR (300 MHz, DMSO-$d_6$) δ: 9.14 (s, 1H); 8.45 (t br, 1H); 8.23 (s, 1H); 7.81 (dd, 1H); 7.51 (dd, 1H); 7.24 (dt, 1H); 7.20 (s, 1H); 3.84 (dd, 2H); 3.25 (dd, 2H); 3.10 (dd, 2H); 1.73 (m, 1H); 1.59 (m, 2H); 1.18 (m, 2H). EI+; TSQ 700; source 180˚C; 70 V; 200 uA: 431.1 ($M^+$), 346,333,317. |
| 166 | N-Cyclobutylmethyl-6-(2,4-difluoro-phenylamino)-4-trifluoromethyl-nicotinamide | A | 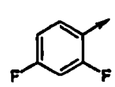 | 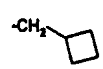 | $^1$H NMR (300 MHz, DMSO-$d_6$) δ: 9.28 (s, 1H); 8.39 (t br, 1H); 8.23 (s, 1H); 7.95 (m, 1H); 7.31 (ddd, 1H); 7.21 (s, 1H); 7.08 (t br, 1H); 3.24 (dd, 2H); 2.55-2.42 (m, 1H); 2.04-1.63 (m, 6H). EI+; TSQ 700; source 180˚C; 70 V; 200 uA: 385 ($M^+$); 366; 317; 301. |

(continued)

| Ex. No | Chemical name | Method | Y | $R^2$ | $^1H$ NMR (solvent) ppm and/or MS |
|---|---|---|---|---|---|
| 167 | N-Cyclopentylmethyl-6-(2,4-difluoro-phenylamino)-4-trifluoromethyl-nicotinamide | A | 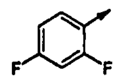 | 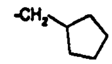 | $^1H$ NMR (300 MHz, DMSO-$d_6$) δ: 9.29 (s, 1H); 8.45 (t br, 1H); 8.24 (s, 1H); 7.96 (dt, 1H); 7.32 (ddd, 1H); 7.22 (s, 1H); 7.09 (t br, 1H); 3.13 (dd, 2H); 2.08 (m, 1H); 1.75-1.42 (m, 6H); 1.30-1.16 (m, 2H). EI+; TSQ 700; source 180˚C; 70 V; 200 uA: 399 ($M^+$); 380; 330; 317; 301; 298. |
| 168 | N-(Tetrahydropyran-4-ylmethyl)-6-(2-methoxy-5-chloro-phenylamino)-4-trifluoromethyl-nicotinamide | B | 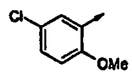 | 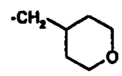 | ESI Pos: AQA; Spray 3.5kV; Skimmer 30V; Probe 250˚C: 445 (MH+). |
| 169 | N-Cyclobutylmethyl-6-(2-methoxy-5-chloro-phenylamino)-4-trifluoromethyl-nicotinamide | B | 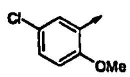 | 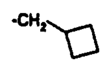 | ESI Pos: AQA; Spray 3.5kV; Skimmer 30V; Probe 250˚C: 415 (MH+). |
| 170 | N-(Tetrahydropyran-4-ylmethyl)-6-(2-hydroxy-5-chloro-phenylamino)-4-trifluoromethyl-nicotinamide | A | 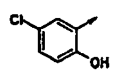 | 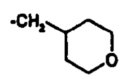 | ESI Pos: AQA; Spray 3.5kV; Skimmer 30V; Probe 250˚C: 430 (MH+). |
| 171 | N-Cyclohexylmethyl-6-(2-methyl-4-chloro-phenylamino)-4-trifluoromethyl-nicotinamide | A | 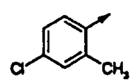 | 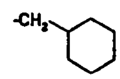 | $^1H$ NMR (300 MHz, DMSO-$d_6$) δ: 8.89 (s br,1H); 8.36 (t br, 1H); 8.21 (s, 1H); 7.62 (d, 1H); 7.33 (d, 1H); 7.24 (dd, 1H); 7.12 (s, 1H); 3.04 (dd, 2H); 2.23 (s, 3H); 1.76-1.39 (m, 6H); 1.29-1.05 (m, 3H); 0.99-0.83 (m, 2H). EI+; TSQ 700; source 180˚C; 70 V; 200 uA: 425 ($M^+$); 410; 342;329;313. |
| 172 | N-(Tetrahydropyran-4-ylmethyl)-6-(2-methyl-4-chloro-phenylamino)-4-trifluoromethyl-nicotinamide | A | 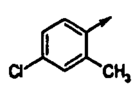 | 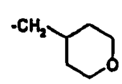 | $^1H$ NMR (300 MHz, DMSO-$d_6$) δ: 8.91 (s br, 1H); 8.42 (t br, 1H); 8.23 (s, 1H); 7.63 (d, 1H); 7.32 (d, 1H); 7.24 (dd, 1H); 7.12 (s, 1H); 3.84 (m, 2H); 3.26 (m, 2H); 3.09 (dd, 2H); 2.23 (s, 3H); 1.82-1.65 (m, 1H); 1.58 (d br, 2H); 1.18 (dq, 2H). EI+; TSQ 700; source 180˚C; 70 V; 200 uA: 427 ($M^+$); 412; 313. |

**Table 11.**

Compounds of Examples 173 to 177 described in Table 11 were prepared as described in Example 104 (Method A), Example 105 (Method B) and Example 106 (Method C). The method used is indicated in the third column.

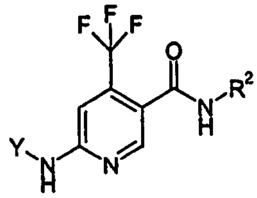

| Ex. No. | Chemical name | Method | Y | $R^2$ | $^1H$ NMR (solvent) ppm and/or MS |
|---|---|---|---|---|---|
| 173 | N-(Tetrahydropyran-4-ylmethyl)-6-phenylamino-4-trifluoromethyl-nicotinamide | B | 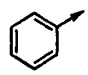 | 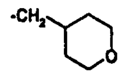 | ESI Pos: AQA; Spray 3.5kV; Skimmer 30V; Probe 250°C: 380 (MH+). |
| 174 | N-Cyclopropylmethyl-6-phenylamino-4-trifluoromethyl-nicotinamide | B | 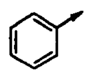 | 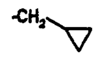 | ESI Pos: AQA; Spray 3.5kV; Skimmer 30V; Probe 250°C: 336 (MH+). |
| 175 | N-Cycloheptylmethyl-6-(3,5-dichloro-phenylamino)-4-trifluoromethyl-nicotinamide | B | 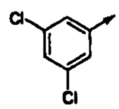 | 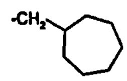 | ESI Pos: AQA; Spray 3.5kV; Skimmer 30V; Probe 250°C: 460 (MH+). |
| 176 | N-(Tetrahydropyran-4-ylmethyl)-6-(2-methyl-5-chloro-phenylamino)-4-trifluoromethyl-nicotinamide | A | 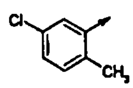 | 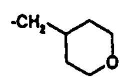 | $^1H$ NMR (300 MHz, DMSO-$d_6$) δ: 8.89 (s br, 1H); 8.45 (t br, 1H); 8.30 (s, 1H); 7.86 (d, 1H); 7.26 (s, 1H); 7.25 (d, 1H); 7.07 (dd, 1H); 3.84 (m, 2H); 3.27 (m, 2H); 3.10 (dd, 2H); 2.23 (s, 3H); 1.83-1.68 (m, 1H); 1.60 (m, 2H); 1.27-1.10 (m, 2H). EI+; TSQ 700; source 180°C; 70 V; 200 uA: 427 ($M^+$); 412; 313. |
| 177 | N-Cyclobutylmethyl-6-(2-hydroxy-5-chloro-phenylamino)-4-trifluoromethyl-nicotinamide | A | 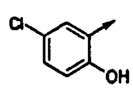 | 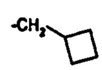 | ESI Pos: AQA; Spray 3.5kV; Skimmer 30V; Probe 250°C: 400 (MH+). |

**Example 196: N-(5-Oxo-pyrrolidin-3-ylmethyl)-6-(2,4-dichloro-phenylamino)-4-trifluoromethyl-nicotinamide**

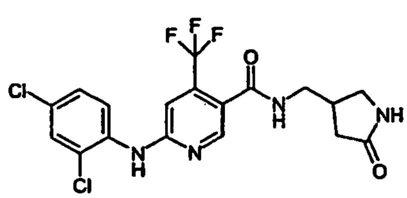

PS-carbodiimide (0.305 g, 0.4 mmol, loading 1.31 mmol/g, ex Argonaut Technologies) and 1-hydroxy-7-azabenzotriazole (0.046 g, 0.34 mmol) were added to a solution of 6-(2,4-dichlorophenylamino)-4-(trifluoromethyl)-nicotinic acid (Description 20) (0.08 g. 0.22 mmol) in dry dichloromethane (5 mL) and the mixture was stirred at room temperature overnight. The resin was filtered and washed repeatedly with dichloromethane, the solvent was then removed in vacuo. The solid residue was dissolved in anhydrous N-methylpyrrolidone (1 mL) and 4-aminomethyl-pyrrolidin-2-one (23 mg, 0.20 mmol) was added. The solution was heated in a sealed tube under microwaves irradiation for 30 min at 140˚C (power = 50 W). The reaction mixture was diluted with dichloromethane, washed with an aqueous solution of $K_2CO_3$ 10%, dried over magnesium sulphate and evaporated under reduced pressure. Chromatographic purification through preparative HPLC on a Symmetry $C_{18}$ column, by gradient elution with a solvent system water / TFA 99.9:0.1 respectively (A) and $CH_3CN$ / TFA 99.9:0.1 respectively (B) with the following gradient: 5% B (3 min); 5% B → 95 % B (11 min); 95 % B (1 min); 95 % B → 5 % B (2 min) afforded the title compound as its trifluoroacetate salt that was suspended in dichloromethane and treated with NaOH 0.5 N. The organic layer was dried over $Na_2SO_4$ and evaporated under reduced pressure to give the title compound (42 mg, yield = 47%).
ESI Pos: AQA; Spray 3.5kV; Skimmer 30V; Probe 250˚C: 447 (MH+).

**Table 12.**

Compounds of Examples 178 to 201 described in Table 12 were prepared as described in Example 104 (Method A), Example 105 (Method B) and Example 196 (Method D). The method used is indicated in the third column.

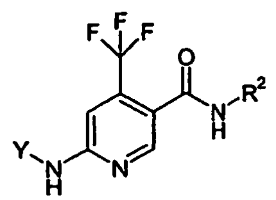

| Ex No | Chemical name | Method | Y | $R^2$ | 1h NMR(Solvent) ppm and/or MS |
|---|---|---|---|---|---|
| 178 | N-Cycloheptylmethyl-6-phenylamino-4-trifluoromethyl-nicotinamide | B | 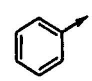 | 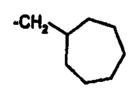 | ESI Pos: AQA; Spray 3.5kV; Skimmer 30V; Probe 250˚C: 392 (MH+). |
| 179 | N-Cyclobutyl-6-phenylamino-4-trifluoromethyl-nicotinamide | B | 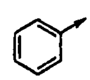 | 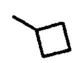 | ESI Pos: AQA; Spray 3.5kV; Skimmer 30V; Probe 250˚C: 336 (MH+). |
| 180 | N-Isobutyl-6-phenylamino-4-trifluoromethyl-nicotinamide | B | 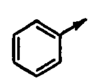 | 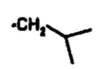 | ESI Pos: AQA; Spray 3.5kV; Skimmer 30V; Probe 250˚C: 338 (MH+). |
| 181 | N-(3-Dimethylamino-2,2-dimethyl-propyl)-6-(3-chloro-phenylamino)-4-trifluoromethyl-nicotinamide | B | 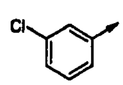 | 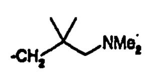 | ESI Pos: AQA; Spray 3.5kV; Skimmer 30V; Probe 250˚C: 429 (MH+). |

(continued)

| Ex No | Chemical name | Method | Y | $R^2$ | 1h NMR(Solvent) ppm and/or MS |
|---|---|---|---|---|---|
| 182 | N-(3-Hydroxy-2,2-dimethyl-propyl)-6-(3-chloro-phenylamino)-4-trifluoromethyl-nicotinamide | B | 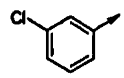 | 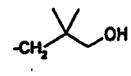 | ESI Pos: AQA; Spray 3.5kV; Skimmer 30V; Probe 250˚C: 402 (MH+). |
| 183 | N-(2-Methoxy-2-methyl-propyl)-6-(3-chloro-phenylamino)-4-trifluoromethyl-nicotinamide | D | 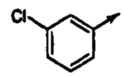 | 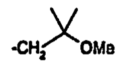 | ESI Pos: AQA; Spray 3.5kV; Skimmer 30V; Probe 250˚C: 402 (MH+). |
| 184 | N-([1,4]dioxan-2-ylmethyl)-6-(3-chloro-phenylamino)-4-trifluoromethyl-nicotinamide | B | 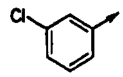 | 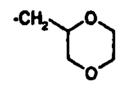 | ESI Pos: AQA; Spray 3.5kV; Skimmer 30V; Probe 250˚C: 416 (MH+). |
| 185 | N-(Piperidin-2-ylmethyl)-6-(3-chloro-phenylamino)-4-trifluoromethyl-nicotinamide | B | 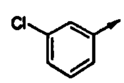 | 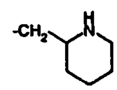 | ESI Pos: AQA; Spray 3.5kV; Skimmer 30V; Probe 250˚C: 427 (MH+). |
| 186 | N-(1-Benzyl-5-oxo-pyrrolidin-3-ylmethyl)-6-(3-chloro-phenylamino)-4-trifluoromethyl-nicotinamide | D | 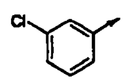 | 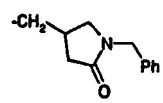 | ESI Pos: AQA; Spray 3.5kV; Skimmer 30V; Probe 250˚C: 503 (MH+). |
| 187 | N-(5-Oxo-pyrrolidin-3-ylmethyl)-6-(3-chloro-phenylamino)-4-trifluoromethyl-nicotinamide | D | 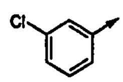 | 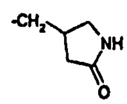 | ESI Pos: AQA; Spray 3.5kV; Skimmer 30V; Probe 250˚C: 413 (MH+). |
| 188 | N-Methylcarbamoylmethyl-6-(3-chloro-phenylamino)-4-trifluoromethyl-nicotinamide | D | 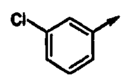 | 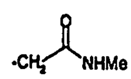 | ESI Pos: AQA; Spray 3.5kV; Skimmer 30V; Probe 250˚C: 387 (MH+). |
| 189 | N-(1-Ethyl-pyrrolidin-2-ylmethyl)-6-(3-chloro-phenylamino)-4-trifluoromethyl-nicotinamide | D | 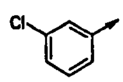 | 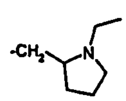 | ESI Pos: AQA; Spray 3.5kV; Skimmer 30V; Probe 250˚C: 427 (MH+). |
| 190 | N-(2,2,6,6-Tetramethyl-piperidin-4-ylmethyl)-6-(3-chloro-phenylamino)-4-trifluoromethyl-nicotinamide | D | 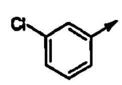 | 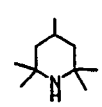 | ESI Pos: AQA; Spray 3.5kV; Skimmer 30V; Probe 250˚C: 455 (MH+). |

(continued)

| Ex No | Chemical name | Method | Y | $R^2$ | 1h NMR(Solvent) ppm and/or MS |
|---|---|---|---|---|---|
| 191 | N-(2,2-Dimethyl-[1,3] dioxolan-4-ylmethyl)-6-(3-chloro-phenylamino)-4-trifluoromethyl-nicotinamide | B | 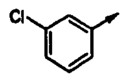 | 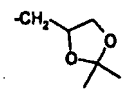 | ESI Pos: AQA; Spray 3.5kV; Skimmer 30V; Probe 250˚C: 430 (MH+). |
| 192 | N-(Tetrahydropyran-4-ylmethyl)-6-(2-fluoro-phenylamino)-4-trifluoromethyt-nicotinamide | B | 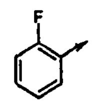 | 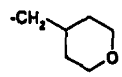 | ESI Pos: AQA; Spray 3.5kV; Skimmer 30V; Probe 250˚C: 398 (MH+). |
| 193 | N-(Tetrahydropyran-4-ylmethyl)-6-(4-fluoro-phenylamino)-4-trifluoromethyl-nicotinamide | B | 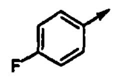 | 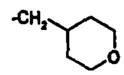 | ESI Pos: AQA; Spray 3.5kV; Skimmer 30V; Probe 250˚C: 398 (MH+). |
| 194 | N-(3-Dimethylamino-2,2-dimethyl-propyl)-6-(2,4-dichloro-phenylamino)-4-trifluoromethyl-nicotinamide | B | 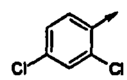 | 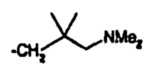 | ESI Pos: AQA; Spray 3.5kV; Skimmer 30V; Probe 250˚C: 463 (MH+). |
| 195 | N-([1,4]dioxan-2-ylmethyl)-6-(2,4-dichloro-phenylamino)-4-trifluoromethyl-nicotinamide | B | 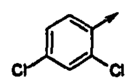 | 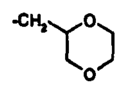 | ESI Pos: AQA; Spray 3.5kV; Skimmer 30V; Probe 250˚C: 450 (MH+). |
| 196 -see above for full write up | N-(5-Oxo-pyrrolidin-3-ylmethyl)-6-(2,4-dichloro-phenylamino)-4-trifluoromethyl-nicotinamide | D | 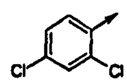 | 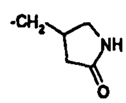 | ESI Pos: AQA; Spray 3.5kV; Skimmer 30V; Probe 250˚C: 447 (MH+). |
| 197 | N-Methylcarbamoylmethyl-6-(2,4-dichloro-phenylamino)-4-trifluoromethyl-nicotinamide | D | 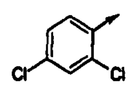 | 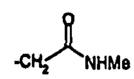 | ESI Pos: AQA; Spray 3.5kV; Skimmer 30V; Probe 250˚C: 421 (MH+). |
| 198 | N-(2,2-Dimethyl-[1,3] dioxolan-4-ylmethyl)-6-(2,4-dichloro-pheny (amino)-4-trifluoromethyl-nicotinamide | B | 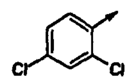 | 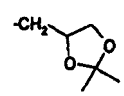 | ESI Pos: AQA; Spray 3.5kV; Skimmer 30V; Probe 250˚C: 464 (MH+). |
| 199 | N-Cycloheptylmethyl-6-(3,4-dichloro-phenylamino)-4-trifluoromethyl-nicotinamide | B | 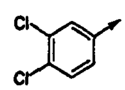 | 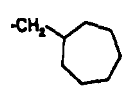 | ESI Pos: AQA; Spray 3.5kV; Skimmer 30V; Probe 250˚C: 460 (MH+). |

(continued)

| Ex No | Chemical name | Method | Y | $R^2$ | 1h NMR(Solvent) ppm and/or MS |
|---|---|---|---|---|---|
| 200 | N-(Tetrahydropyran-4-ylmethyl)-6-(2,4-difluoro-phenylamino)-4-trifluoromethyl-nicotinamide | A | 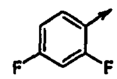 | 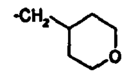 | EI+; TSQ 700; source 180°C; 70 V; 200 uA: 415 ($M^+$). |
| 201 | N-Cyclohexylmethyl-6-(2-methyl-5-chloro-phenylamino)-4-trifluoromethyl-nicotinamide | A | 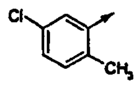 | 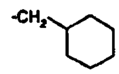 | ESI Pos: AQA; Spray 3.5kV; Skimmer 30V; Probe 250°C: 431 (MH+). |

**Example 202: N-(2,3-Dihydroxy-propyl)-6-(3-chloro-phenylamino)-4-trifluoromethyl-nicotinamide**

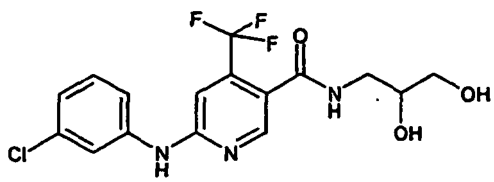

N-(2,2-dimethyl-[1,3]dioxolan-4-ylmethyl)-6-(3-chloro-phenylamino)-4-trifluoromethyl-nicotinamide (Example 191) (30 mg, 0.07 mmol), was dissolved in tetrahydrofuran (4mL) and stirred overnight at ambient temperature in the presence of $Et_2O$/HCl (3 mL). Evaporation of the solvent in vacuo afforded the title compound as a white solid (27 mg, yield=99%).
$^1$H NMR (300 MHz, DMSO-$d_6$) δ: 9.90 (s, 1H); 8.45 (s, 1H); 8.41 (t br, 1H); 8.02 (dd, 1H); 7.50 (ddd, 1H); 7.34 (dd, 1H); 7.17 (s, 1H); 7.03 (ddd, 1H); 3.65-3.30 (m, 7H); 3.14 (ddd, 1H).
MS m/z (ESI+): AQA; Spray 3.5kV; Skimmer 30V; Probe 250°C: 390 (MH+).

**Example 203: N-(2,3-Dihydroxy-propyl)-6-(2,4-dichloro-phenylamino)-4-trifluoromethyl-nicotinamide**

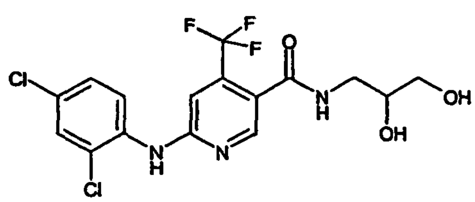

The title compound was prepared in a similar manner to that described in the Example 202, starting from N-(2,2-dimethyl-[1,3]dioxolan-4-ylmethyl)-6-(2,4-dichloro-phenylamino)-4-trifluoromethyl-nicotinamide (Example 199) (40 mg, 0.09 mmol) and the title compound was obtained as a white solid (35 mg, yield=96%).
$^1$H NMR (300 MHz, $CDCl_3$) δ: 8.36 (s, 1H); 8.02 (d, 1H); 7.66 (s br, 1H); 7.35 (d, 1H); 7.18 (dd, 1H); 7.11 (t br, 1H); 7.05 (s, 1H); 3.89 (s br, 1H); 3.77 (s br, 1H); 3.59-3.47 (m, 3H); 3.42 (ddd, 1H).
MS m/z (ESI+): AQA; Spray 3.5kV; Skimmer 30V; Probe 250°C: 424 (MH+).

**Example 204: 6-(3-Chloro-phenylamino)-N-(tetrahydro-pyran-4-ylmethyl)-2-trifluoromethyl-nicotinamide**

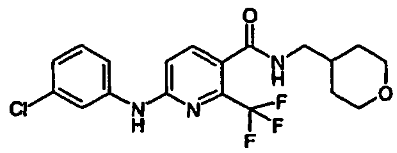

N-methyl morpholine (0.14 mL, 1.27 mmol, 2.5 eq), 1-hydroxy-benzotriazole (110 mg, 0.76 mmol, 1.5 eq), N-(3-dimethylaminopropyl)-N-ethylcarbodiimide hydrochloride (120 mg, 0.61 mmol, 1.2 eq) and tetrahydropyran-4-ylmethyl amine (77 mg, 0.66 mmol, 1.3 eq) were subsequently added to a solution of 6-(3-chloro-phenylamino)-2-trifluoromethyl-nicotinic acid hydrochloride (180 mg, 0.51 mmol, 1.0 eq) in anhydrous DCM (12 mL) and stirred at ambient temperature for 12h. After evaporation of the solvent in vacuo, the mixture was diluted with ethyl acetate (50 mL) and washed subsequently with a saturated aqueous solution of $NaHCO_3$ (20 mL x 2 times) and brine (25 mL). The organic phase was dried over sodium sulphate and concentrated in vacuo to afford a black residue that was purified by flash chromatography (silica gel; eluent gradient: from hexane / ethyl acetate 1:9 to pure ethyl acetate). The title compound was obtained as a brown solid (130 mg, yield = 61%).
EI; TSQ 700; source 180 C; 70 V; 200 uA: 413 (M+.); 315; 299.
$^1H$ NMR (300 MHz, DMSO-$d_6$) δ: 9.80(s, 1H); 8.48(t br, 1H); 8.02(dd, 1H); 7.72(d, 1H); 7.51 (dd, 1H); 7.31 (dd, 1H); 7.09(d, 1H); 7.00(dd, 1H); 3.89(m, 2H); 3.27(m, 2H); 3.09(dd, 2H); 1.75(m, 1H); 1.60(m, 2H); 1.20(m, 2H).

**Table 13.**

Compounds of Example 205 to 209 were prepared as described Example 204, from the appropriate starting materials via similar intermediates, prepared in a similar manner to the intermediates described in Descriptions 25 to 29.

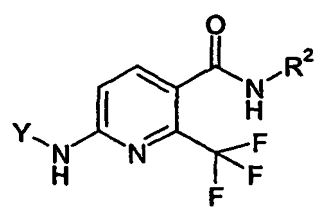

| Ex. No | Chemical Name | Y | $R^2$ | $^1H$ NMR (Solvent) ppm and/or MS |
|---|---|---|---|---|
| 205 | 6-(3-Chloro-phenylamino)-N-cyclohexylmethyt-2-trifluoromethyl-nicotinamide | 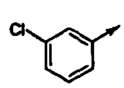 | 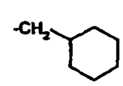 | EI; TSQ 700; source 180 C; 70 V; 200 uA: 411 (M+.), 315, 299. $^1H$ NMR (300 MHz, DMSO-$d_6$) δ: 9.80(s, 1H); 8.38(t br, 1H); 8.01 (dd, 1H); 7.72(d, 1H); 7.51 (dd, 1H); 7.32 (dd, 1H); 7.08(d, 1H); 7.00(dd, 1H); 3.05(dd, 2H); 1.77-1.57(m, 5H); 1.57-1.41 (m, 1H); 1.30-1.10(m, 3H); 1.02-0.83(m, 2H). |

(continued)

| Ex. No | Chemical Name | Y | $R^2$ | $^1H$ NMR (Solvent) ppm and/or MS |
|---|---|---|---|---|
| 206 | 6-(3-Chloro-phenylamino)-N-cyclobutylmethyl-2-trifluoromethyl-nicotinamide | 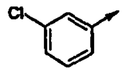 | 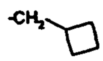 | EI; TSQ 700; source 180 C; 70 V; 200 uA: 383 (M+.); 315; 299. $^1H$ NMR (300 MHz, DMSO-$d_6$) δ: 9.80(s, 1H); 8.40(t br, 1H); 8.00(dd, 1H); 7.71 (d, 1H); 7.50(dd, 1H); 7.30 (dd, 1H); 7.08(d, 1H); 7.00(dd, 1H); 3.21 (dd, 2H); 2.50(m, 1H); 2.00(m, 2H); 1.95-1.68(m, 4H). |
| 207 | 6-(3-Chloro-phenylamino)-N-cyclopentylmethyl-2-trifluoromethyl-nicotinamide | 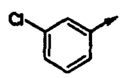 | 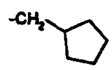 | EI; TSQ 700; source 180 C; 70 V; 200 uA: 397 (M+.); 315; 299. $^1H$ NMR (300 MHz, DMSO-$d_6$) δ: 9.80(s, 1H); 8.42(t br, 1H); 8.02(dd, 1H); 7.71 (d, 1H); 7.52(dd, 1H); 7.33 (dd, 1H); 7.09(d, 1H); 7.00(dd, 1H); 3.14(dd, 2H); 2.08(m, 1H); 1.76-1.43(m, 6H); 1.32-1.16(m, 2H). |

**Example 208: 6-(3-Chloro-phenylamlno)-2-isopropyl-N-(tetrahydro-pyran-4-ylmethyl)-nicotinamide**

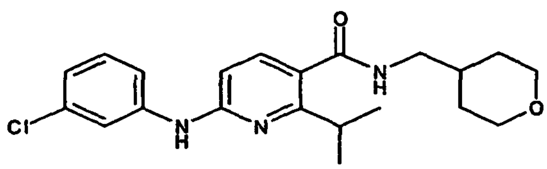

N-methyl morpholine (0.14 mL, 1.27 mmol, 2.5 eq), 1-hydroxy-benzotriazole (100 mg, 0.74 mmol, 1.5 eq), N-(3-dimethylaminopropyl)-N-ethylcarbodiimide hydrochloride (115 mg, 0.6 mmol, 1.2 eq) were subsequently added to a solution of 6-(3-chloro-phenylamino)-2-isopropyl-nicotinic acid hydrochloride (Description 35) (0.16 g, 0.49 mmol, 1.0 eq) in anhydrous DCM (5 mL). After stirring 1h at room temperature, tetrahydropyran-4-ylmethyl amine (77 mg, 0.66 mmol, 1.3 eq) was added and the resulting solution was stirred at room temperature overnight. Solvent was evaporated in vacuo, the residue was dissolved in ethyl acetate (50 mL) and washed with a saturated aqueous solution of $NaHCO_3$ and with brine: the organic phase was dried over $Na_2SO_4$ and concentrated in vacuo to yield a solid that was triturated with hexane / diethyl ether 9:1 and filtered. The title compound was obtained as a white solid (170 mg, yield = 89%).
EI; TSQ 700; source 180 C; 70 V; 200 uA: 387(M+.), 289, 273, 243.
$^1H$ NMR (300 MHz, DMSO-$d_6$) δ: 9.39(s, 1H); 8.29(dd, 1H); 8.21 (t br, 1H); 7.50(d, 1H); 7.46(dd, 1H); 7.27(dd, 1H); 6.91 (dd, 1H); 6.65(d, 1H); 3.86(m, 2H); 3.45(m, 1H); 3.27(m, 2H); 3.10(dd, 2H); 1.76(m, 1H); 1.60(m, 2H); 1.22(d, 6H); 1.29-1.12(m, 2H).

**Example 209: 6-(3-Chloro-phenylamino)-N-(1,1-dioxo-tetrahydrothiophen-3-ylmethyl)-2-isopropyl-nicotinamide**

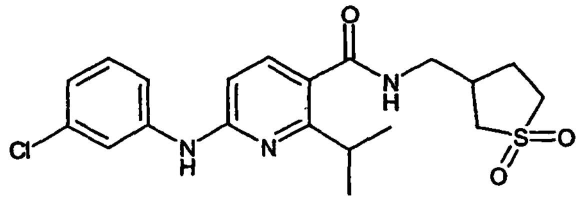

A mixture of 6-(3-chloro-phenylamino)-2-isopropyl-nicotinic acid hydrochloride (Description 35) (166 mg, 0.5 mmol, 1.0 eq), 1-hydroxy-benzotriazole (100 mg, 0.74 mmol, 1.5 eq), PS-dicyclohexylcarbodiimide (760 mg, 1.0 mmol, 2.0 eq, loading = 1.31 mmol/g) and PS-diisopropylethylamine (154 mg, 0.6 mmol, 1.2 eq, loading = 3.88 mmol/g) was stirred at room temperature overnight. The resins were filtered, washed with DCM and tetrahydrofuran (30 mL) and the filtrate was concentrated in vacuo. The residue was dissolved in 2.5 mL of anhydrous THF and C-(1,1-dioxo-tetrahydro-1/6-thiophen-3-ylmethyl amine (108 mg, 0.72 mmol, 1.44 eq) and 1-butyl-3-methylimidazolium hexafluorophosphate (53 uL) were then added. The mixture was heated under microwaves irradiation at 140°C for 20 min, the solvent was removed in vacuo, the residue diluted with ethyl acetate (30 mL) and 5% $Na_2CO_3$ (aq) (20 mL). The organic phase was then washed with brine (20 mL) and evaporated in vacuo to afford a solid that was purified by flash chromatography (silica gel, eluent: DCM / MeOH / $NH_4OH$ 97:3:0.3). The title compound was obtained as a solid (140 mg, yield = 66%).
EI; TSQ 700; source 180 C; 70 V; 200 uA: 421 (M+.); 273.
$^1$H NMR (300 MHz, DMSO-$d_6$) δ: 9.41 (s, 1H); 8.36(t br, 1H); 8.28(dd, 1H); 7.55(d, 1H); 7.45(dd, 1H); 7.27(dd, 1H); 6.91 (dd, 1H); 6.67(d, 1H); 3.49-3.15(m, 5H); 3.07(m, 1H); 2.85(dd, 1H); 2.63(m, 1H); 2.23(m, 1H); 1.86(m, 1H); 1.09(d, 6H).

**Table 14.**

All the Examples described in Table 14 were prepared as described for the Example 208 and 209, from the appropriate starting materials via similar intermediates, prepared in a similar manner to the intermediates described in Descriptions 30 to 35. In particular, the compounds of the Examples 210 to 214 and 216 to 218 were prepared according to the same experimental procedure as described for the Example 208, whereas the compounds of the Examples 215 and 219 were prepared according to the same experimental procedure as described for the Example 210

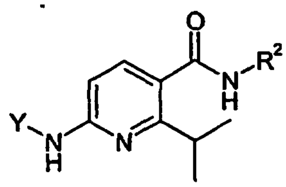

| Ex. No | Chemical Name | Y | $R^2$ | $^1$H NMR (Solvent) ppm and/or MS |
|---|---|---|---|---|
| 210 | 6-(3-Chloro-phenylamino)-N-cyclopentylmethyl-2-isopropyl-nicotinamide | 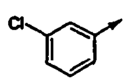 | 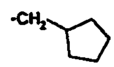 | EI; TSQ 700; source 180 C; 70 V; 200 uA: 371 (M+.), 289, 273. $^1$H NMR (300 MHz, DMSO-$d_6$) δ: 9.38(s, 1H); 8.29(dd, 1H); 8.19(t br, 1H); 7.48(d, 1H); 7.45(dd, 1H); 7.27(dd, 1H); 6.91 (dd, 1H); 6.66 (d, 1H); 3.44(m, 1H); 3.13(dd, 2H); 2.16-2.04(m, 1H); 1.76-1.42(m, 6H); 1.32-1.19(m, 2H); 1.22(d, 6H). |

(continued)

| Ex. No | Chemical Name | Y | $R^2$ | $^1H$ NMR (Solvent) ppm and/or MS |
|---|---|---|---|---|
| 211 | 6-(3-Chloro-phenylamino)-N-cyclohexylmethyl-2-isopropyl-nicotinamide | 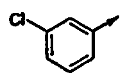 | 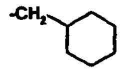 | El; TSQ 700; source 180 C; 70 V; 200 uA: 385(M+.), 289, 273. $^1H$ NMR (300 MHz, DMSO-$d_6$) δ: 9.37(s, 1H); 8.28(dd, 1H); 8.14(t br, 1H); 7.49(d, 1H); 7.46(dd, 1H); 7.27(dd, 1H); 6.90(dd, 1H); 6.65(d, 1H); 3.45(m, 1H); 3.05(dd, 2H); 1.76-1.56(m, 4H); 1.57-1.43(m, 1H); 1.22(d, 6H); 1.22-1.10(m, 4H); 0.94(m, 2H). |
| 212 | 6-(2,4-Dichloro-phenylamino)-N-cyclobutylmethyl-2-isopropyl-nicotinamide | 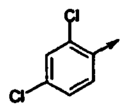 | 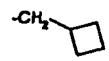 | El; TSQ 700; source 180 C; 70 V; 200 uA: 391 (*M*+.); 356; 322; 307. $^1H$ NMR (300 MHz, DMSO-$d_6$) δ: 8.52(s, 1H); 8.23(d, 1H); 8.15(t br, 1H); 7.58(d, 1H); 7.47(d, 1H); 7.37 (dd, 1H); 6.86(d, 1H); 3.39(m, 1H); 3.23(dd, 2H); 2.50(m, 1H); 2.06-1.63(m, 6H); 1.13(d, 6H). |
| 213 | 6-(2,4-Dichloro-phenylamino)-N-cyclopentylmethyl-2-isopropyl-nicotinamide | 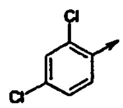 | 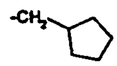 | El; TSQ 700; source 180 C; 70 V; 200 uA: 405 (M+.); 370; 307; 288. $^1H$ NMR (300 MHz, DMSO-$d_6$) δ: 8.53(s, 1H); 8.23(d, 1H); 8.19(t br, 1H); 7.58(d, 1H); 7.48(d, 1H); 7.37 (dd, 1H); 6.87(d, 1H); 3.39(m, 1H); 3.13(dd, 2H); 2.11 (m, 1H); 1.75-1.41 (m. 6H); 1.23(m, 2H); 1.14(d, 6H). |
| 214 | 6-(2,4-Dichloro-phenylamino)-N-(tetrahydro-pyran-4-ylmethyl)-2-isopropyl-nicotinamide | 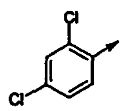 | 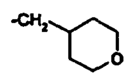 | El; TSQ 700; source 180 C; 70 V; 200 uA: 421 (M+.); 386; 307; 288; 271. $^1H$ NMR (300 MHz, DMSO-$d_6$) δ: 8.53(s, 1H); 8.23(d, 1H); 8.20(t br, 1H); 7.58(d, 1H); 7.51 (d, 1H); 7.37 (dd, 1H); 6.87(d, 1H); 3.85(m, 2H); 3.39(m, 1H); 3.26(m, 2H); 3.10(dd, 2H); 1.75(m, 1H); 1.60(m, 2H); 1.28-1.07(m, 2H); 1.13(d, 6H). |
| 215 | 6-(2,4-Dichloro-phenylamino)-N-(1,1-dioxo-tetrahydrothiophen-3-ylmethyl)-2-isopropyl-nicotinamide | 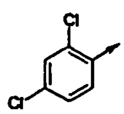 | 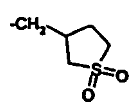 | El; TSQ 700; source 180 C; 70 V; 200 uA: 455 (M+.), 420, 307. $^1H$ NMR (300 MHz, DMSO-$d_6$) δ: 8.14(d, 1H); 7.51 (d, 1H); 7.49(d, 1H); 7.32(d, 1H); 6.78(d, 1H); 3.40-3.10(m, 5H); 3.04(m, 1H); 2.80(dd, 1H); 2.63(m, 1H); 2.23(m, 1H); 1.82(m, 1H); 1.09(d, 6H). |

(continued)

| Ex. No | Chemical Name | Y | $R^2$ | $^1H$ NMR (Solvent) ppm and/or MS |
|---|---|---|---|---|
| 216 | 6-(3-Fluoro-phenylamino)-N-cyclobutylmethyl-2-isopropyl-nicotinamide | 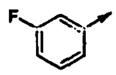 | 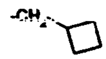 | EI; TSQ 700; source 180 C; 70 V; 200 uA: 341 (M+.); 257. $^1H$ NMR (300 MHz, DMSO-$d_6$) δ: 9.38(s, 1H); 8.15(t br, 1H); 8.00(d. 1H); 7.46(d, 1H); 7.34-7.21 (m, 2H); 6.67(m, 1H); 6.65(d, 1H); 3.44 (m, 1H); 3.23(dd, 2H); 2.50(m, 1H); 2.07-1.64(m, 6H); 1.21 (d, 6H). |
| 217 | 6-(3-Fluoro-phenylamino)-N-cyclopentylmethyl-2-isopropyl-nicotinamide | 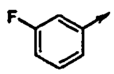 | 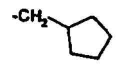 | EI; TSQ 700; source 180 C; 70 V; 200 uA: 355 (M+.); 273; 257; 227. $^1H$ NMR (300 MHz, DMSO-$d_6$) δ: 9.38(s, 1H); 8.19(t br, 1H); 8.01 (ddd, 1H); 7.47(d, 1H); 7.34-7.22 (m, 2H); 6.67(m, 1H); 6.66(d, 1H); 3.44(m, 1H); 3.14(dd, 2H); 2.11 (m, 1H); 1.76-1.43(m, 6H); 1.25(m, 2H); 1.22(d, 6H). |
| 218 | 6-(3-Fluoro-phenylamino)-N-(tetrahydro-pyran-4-ylmethyl)-2-isopropyl-nicotinamide | 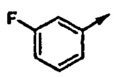 | 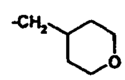 | EI; TSQ 700; source 180 C; 70 V; 200 uA: 371 (M+.); 273; 257; 227. $^1H$ NMR (300 MHz, DMSO-$d_6$) δ: 9.39(s, 1H); 8.20(t br, 1H); 8.00(d, 1H); 7.50(d, 1H); 7.34-7.20(m, 2H); 6.67(m, 1H); 6.66(d, 1H); 3.84 (m, 2H); 3.45(m, 1H); 3.36-3.00(m, 2H); 3.11(dd, 2H); 1.76(m, 1H); 1.61 (m, 2H); 1.33-1.04(m, 2H); 1.21(d, 6H). |
| 219 | 6-(3-Fluoro-phenylamino)-N-(1,1-dioxo-tetrahydrothiophen-3-ylmethyl)-2-isopropyl-nicotinamide | 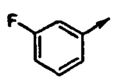 | 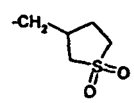 | ESI POS, spray 3,5 KV / source: 30V / PROBE: 250 C: 406 (MH+). $^1H$ NMR (300 MHz, DMSO-$d_6$) δ: 9.44(s, 1H); 8.36(t br, 1H); 8.00 (ddd, 1H); 7.55(d, 1H); 7.35-7.22 (m, 2H); 6.68(m, 1H); 6.67(d, 1H); 3.35-3.14(m, 5H); 3.07(m, 1H); 2.85(dd, 1H); 2.64(m, 1H); 2.23(m, 1H); 1.86(m, 1H); 1.22(d, 6H). |

**Example 220: 6-(4-Cyano-2-methyl-phenylamino)-4-isopropyl-N-(tetrahydro-pyran-4-ylmethyl)-nicotinamide**

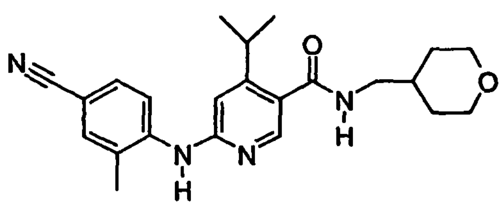

A mixture of 6-chloro-4-isopropyl-N-(tetrahydro-pyran-4-ylmethyl)-nicotinamide (Description 8) (100mg), 4-amino-3-methyl benzonitrile (2eq), cesium carbonate (168mg), tris(dibenzylideneacetone)palladium(0) ($Pd_2(dba)_3$) (3.4mg), 4,5-bis(diphenylphosphino)-9,9-dimethylxanthene (Xantphos) (2.3mg) in 1,4-dioxane(1ml) was irradiated under

microwave conditions at 150˚C for 30 minutes. Further quantities of cesium carbonate (168mg), $Pd_2(dba)_3$ (3.4mg) and Xantphos (2.3mg) were added and the mixture was again subjected to microwave conditions at 150˚C for 30 minutes. Ethyl acetate was added and the mixture was washed with water. The ethyl acetate layer was dried (sodium sulphate) and the solvent was removed under reduced pressure. The residue was purified using MDAP to give the title compound (20mg)

NMR (MeOD) δ 1.25(6H, d), 1.29-1.43(2H, m), 1.70(2H, d), 1.81-1.93(1H, m), 2.3393H, s), 3.21-3.50 (5H, m), 3.98 (2H, dd), 7.01 (1H, s), 7.49 (1H, dd), 7.55 (1H, bs), 8.02 (1H, d), 8.09 (1H, s)

LC/MS, t = 2.89 min, Molecular ion observed [$MH^+$] = 393 consistent with the molecular formula $C_{23}H_{28}N_4O_2$

**Example 221: 6-(5-Chloro-2-cyano-phenylamino)-4-isopropyl-N-(tetrahydro-pyran-4-ylmethyl)-nicotinamide**

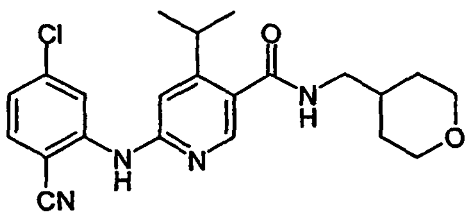

A mixture of 6-chloro-4-isopropyl-N-(tetrahydro-pyran-4-ylmethyl)-nicotinamide (Description 8) (100 mg), 2-amino-4-chlorobenzonitrile (61 mg), cesium carbonate (154 mg), tris(dibenzylideneacetone)palladium(0) (3.2 mg), 4,5-bis(diphenylphosphino)-9,9-dimethyl xanthene (Xantphos) (2.2 mg) and dioxan (1 ml) was stirred under reflux under nitrogen for 24 hours. The mixture was allowed to cool and insoluble material filtered and washed with ethyl acetate. The filtrate was evaporated under reduced pressure and the residue purified by trituration with ether followed by recrystallisation from methanol to give the title compound as a yellow solid (53 mg).

NMR (DMSO-d6) δ 1.2-1.3 (2H, m), 1.21 (6H, d), 1.62 (2H, d), 1.77 (1H, m), 3.15 (2H, t), 3.29 (2H, t), 3.33 (1H, m), 3.86 (2H, d), 7.05 (1H, s), 7.36 (1H, d), 7.46 (1H, s), 8.36 (1H, d), 8.79 (1H, t), 9.00 (1H, s), 9.74 (1H, s).

LC/MS t = 2.3 min, [$MH^+$] 413 consistent with the molecular formula $C_{22}H_{25}{}^{35}ClN_4O_2$.

**Example 222: 6-(2-cyano-5-methyl-phenylamino)-4-isopropyl-N-(tetrahydro-pyran-4-ylmethyl)-nicotinamide**

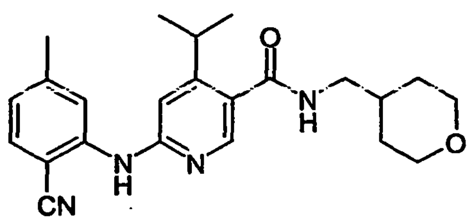

In a manner similar to Example 221, 6-chloro-4-isopropyl-N-(tetrahydro-pyran-4-ylmethyl)-nicotinamide (Description 8) (100 mg) and 2-amino-4-methylbenzonitrile (44.5 mg) afforded the title compound (38 mg).

LC/MS t = 1.9 min, [$MH^+$] 393 consistent with the molecular formula $C_{23}H_{28}N_4O_2$.

**Example 223: 6-(3-Chloro-phenylamino)-N-(1,1-dioxo-tetrahydro-1/6-thiophen-3-ylmethyl)-4-isopropyl-nicotinamide**

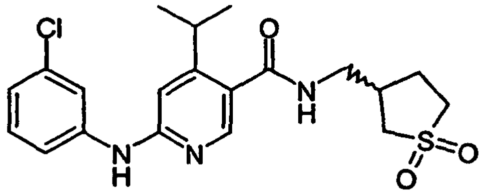

In a manner similar to that described in Example 50, 6-(3-chloro-phenylamino)-4-isopropyl-nicotinic acid (Description 13) (30 mg) and C-(1,1-dioxo-tetrahydro-1/$^6$-thiophen-3-yl)-methylamine hydrochloride (Argyle et al, J Chem Soc (C), 1967, 2156) (23 mg) afforded the title compound (32 mg).
LC/MS t = 3.0 min, [$MH^+$] 422 consistent with $C_{20}H_{24}{}^{35}ClN_3O_3S$

**Example 224: N-Cyclobutylmethyl-4-isopropyl-6-(3-trifluoromethoxy-phenylamino)-nicotinamide**

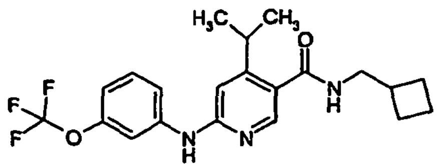

In a manner similar to Example 6, 6-chloro-N-cyclobutylmethyl-4-isopropyl-nicotinamide (Description 6) (80mg) and 3-trifluoromethoxyaniline (0.5ml) gave the title compound (41 mg).
LC/MS, t = 3.73 min, Molecular ion observed [$MH^+$] = 408 consistent with the molecular formula $C_{21}H_{24}F_3N_3O_2$

**Table 15.**

Examples 225 to 233 were prepared by the method given in column 4 and purified by the procedure given in column 5
Preparation method G: As for the preparation of Example 75
Preparation method J: As for the preparation of Example 46
Purification method E: Mass-directed autopreparative technique
Purification method H: Biotage Horizon

| Example no | Compound Name | Compound Structure | Preparation method | Purification Method | 1.Retention time(min).<br>2.[MH+]<br>3.Molecular Formula |
|---|---|---|---|---|---|
| 225 | 6-(2,3-Difluoro-phenylamino)-4-isopropyl-N-(tetrahydro-pyran-4-ylmethyl)-nicotinamide | 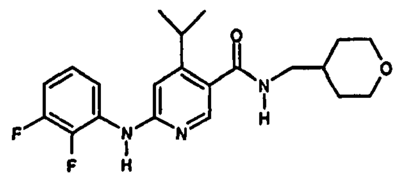 | G | E | 2.82min<br>390<br>$C_{21}H_{25}F_2N_3O_2$ |
| 226 | 6-(3,5-Bis-trifluoromethyl-phenylamino)-4-isopropyl-N-(tetrahydro-pyran-4-ylmethyl)-nicotinamide | 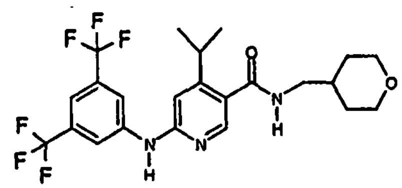 | G | E | 3.60min<br>490<br>$C_{23}H_{25}F_6N_3O_2$ |
| 227 | 6-(2,4-Difluoro-phenylamino)-4-isopropyl-N-(tetrahydro-pyran-4-ylmethyl)-nicotinamide | 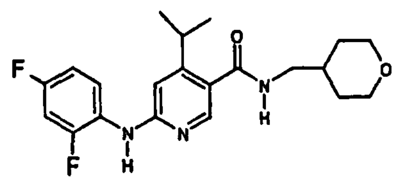 | G | E | 2.70min<br>390<br>$C_{21}H_{25}F_2N_3O_2$ |
| 228 | 6-(3-Ethynyl-phenylamino)-4-isopropyl-N-(tetrahydro-pyran-4-ylmethyl)-nicotinamide | 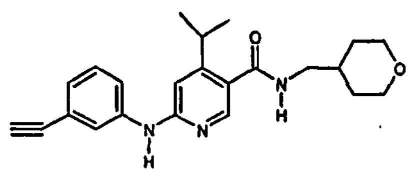 | G | E | 2.88min<br>378<br>$C_{23}H_{27}N_3O_2$ |
| 229 | 6-(2-Fluoro-4-trifluoromethyl-phenylamino)-N-cyclopentylmethyl-4-isopropyl-nicotinamide | 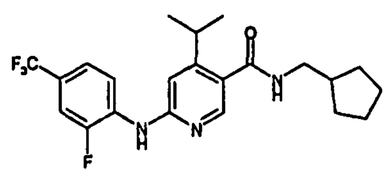 | G | E | 3.82<br>424<br>$C_{22}H_{25}F_4N_3O$ |

(continued)

| Example no | Compound Name | Compound Structure | Preparation method | Purification Method | 1.Retention time(min). 2.[MH+] 3.Molecular Formula |
|---|---|---|---|---|---|
| 230 | 6-(3-cyano-4-methyl-phenylamino)-4-isopropyl-N-(tetrahydropyran-4-ylmethyl)-nicotinamide | 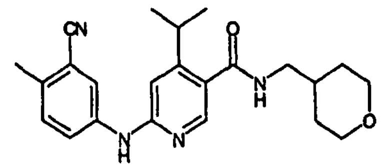 | J | H | 2.90<br>393<br>$C_{23}H_{28}$ $N_4O_2$ |
| 231 | 6-(3-cyano-4-fluoro-phenylamino)- 4-isopropyl-N-(tetrahydropyran-4-ylmethyl)-nicotinamide | 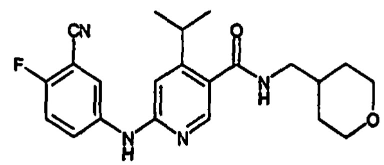 | J | Ethyl acetate trituration of crude product | 2.80<br>397<br>$C_{22}H_{25}$ $FN_4O_2$ |
| 232 | 6-(3-bromo-4-trifluoromethoxy-phenylamino)-4-isopropyl-N-(tetrahydropyran-4-ylmethyl)-nicotinamide | 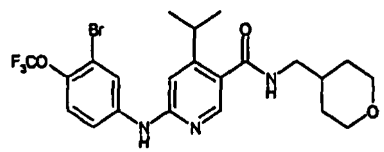 | J | Ether trituration of the crude product | 3.60<br>516<br>$C_{22}H_{25}{}^{79}Br$ $F_3N_3O_3$ |
| 233 | 6-(4-Chloro-2-fluoro-phenylamino)-N-cyclobutylmethyl-4-isopropyl-nicotinamide | 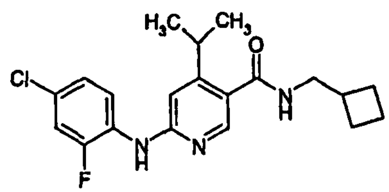 | J | H | 3.58<br>376<br>$C_{20}H_{23}{}^{35}ClFN_3O$ |

**Table 16:**

Examples 234 to 279 in this table were prepared by the method and reaction time given in column 4 and purified by the procedure given in column 5.
Method G: Examples were prepared as for Example 75
Method K: Examples were prepared as for Example 221.
Purification method E: mass-directed auto-preparative technique
Purification method H: Biotage Horizon
Purification method L: the reaction was evaporated, taken up in 1:1 DCM/MeOH, filtered, evaporated, and the residue triturated with MeOH

| Example No. | Name | Structure | Method/ Reaction Time | Purification Method | RT (min), (MH+) Consistent with molecular formula |
|---|---|---|---|---|---|
| 234 | 6-(5-Bromo-2-methyl-phenylamino)-4-isopropyl-N-(tetrahydro-pyran-4-ylmethyl)-nicotinamide | 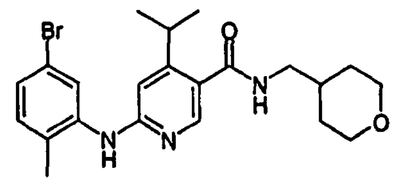 | G<br>30 min | E | 3.0<br>446<br>$C_{22}H_{28}{}^{79}BrN_3O_2$ |
| 235 | 6-(2-Bromo-5-fluoro-phenylamino)-4-isopropyl-N-(tetrahydro-pyran-4-ylmethyl)-nicotinamide | 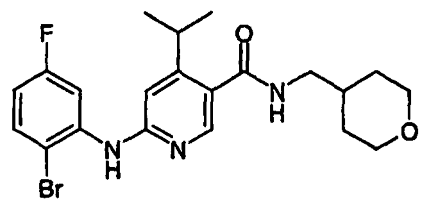 | G<br>1 hour | E | 3.0<br>450<br>$C_{21}H_{25}{}^{79}BrFM_3O_2$ |
| 236 | 6-(2-Fluoro-5-trifluoromethyl-phenylamino)-4-isopropyl-N-(tetrahydro-pyran-4-ylmethyl)-nicotinamide | 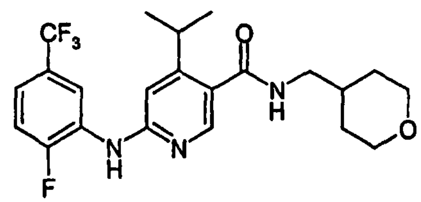 | G<br>30 min | E | 3.2<br>440<br>$C_{22}H_{25}F_4N_3O_2$ |
| 237 | 6-(2-Chloro-5-trifluoromethyl-phenylamino)-4-isopropyl-N-(tetrahydro-pyran-4-ylmethyl)-nicotinamide | 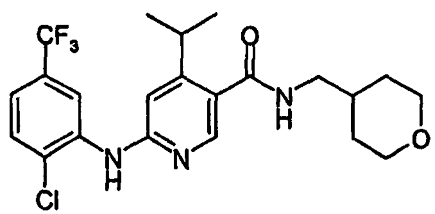 | G<br>1 hour | E | 3.4<br>456<br>$C_{22}H_{25}{}^{35}ClF_3N_3O_2$ |

(continued)

| Example No. | Name | Structure | Method/ Reaction Time | Purification Method | RT (min), (MH+) Consistent with molecular formula |
|---|---|---|---|---|---|
| 238 | 6-(2-Bromo-5-trifluoromethyl-phenylamino)-4-isopropyl-N-(tetrahydro-pyran-4-ylmethyl)-nicotinamide | 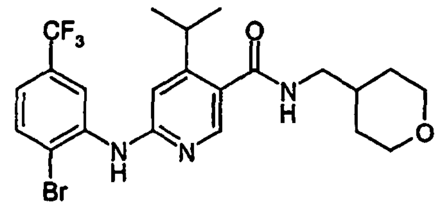 | G 1 hour | E | 3.4 500 $C_{22}H_{25}{}^{79}BrF_3N_3O_2$ |
| 239 | 6-(3-Bromo-4-cyano-phenylamino)-4-isopropyl-N-(tetrahydro-pyran-4-ylmethyl)-nicotinamide | 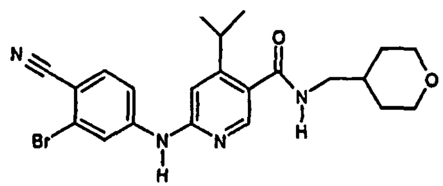 | G 30 min | E | 3.10 459 $C_{22}H_{25}{}^{81}BrN_4O_2$ |
| 240 | 6-(2-Bromo-4-trifluoromethoxy-phenylamino)-4-isopropyl-N-(tetrahydro-pyran-4-ylmethyl)-nicotinamide | 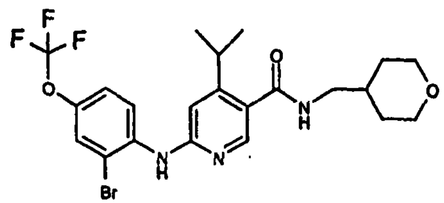 | G 30 min | E | 3.40 518 $C_{22}H_{25}{}^{81}BrF_3N_3O_3$ |
| 241 | 6-(3-Chloro-2-methyl-phenylamino)-4-isopropyl-N-(tetrahydro-pyran-4-ylmethyl)-nicotinamide | 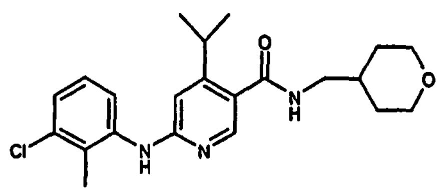 | G 30 min | E | 2.29 402 $C_{22}H_{28}{}^{35}ClN_3O_2$ |

(continued)

| Example No. | Name | Structure | Method/ Reaction Time | Purification Method | RT (min), (MH+) Consistent with molecular formula |
|---|---|---|---|---|---|
| 242 | 6-(3,5-Difluoro-phenylamino)-4-isopropyl-N-(tetrahydro-pyran-4-ylmethyl)-nicotinamide | 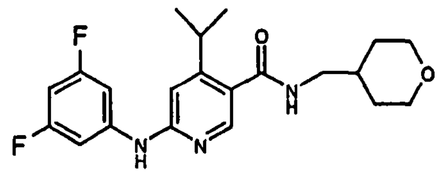 | G 30 min | E | 3.06 390 $C_{21}H_{25}F_2N_3O_2$ |
| 243 | 6-(2-Chloro-4-fluoro-phenylamino)-4-isopropyl-N-(tetrahydro-pyran-4-ylmethyl)-nicotinamide | 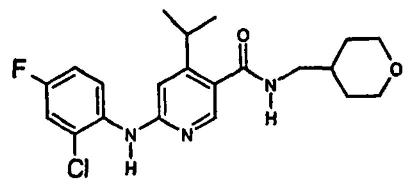 | G 30 min | E | 2.86 406 $C_{21}H_{25}{}^{35}ClFN_3O_2$ |
| 244 | 6-(4-Chloro-2-methyl-phenylamino)-4-isopropyl-N-(tetrahydro-pyran-4-ylmethyl)-nicotinamide | 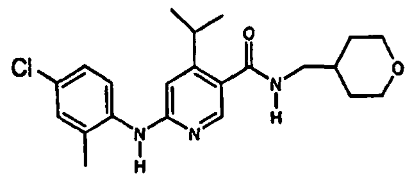 | G 30 min | E | 2.90 402 $C_{22}H_{28}{}^{35}ClN_3O_2$ |
| 245 | 6-(2-Fluoro-3-trifluoromethyl-phenylamino)-N-cyclopentylmethyl-4-isopropyl-nicotinamide | 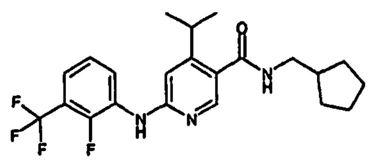 | G 30 Min | H | 3.72 424 $C_{22}H_{25}F_4N_3O$ |
| 246 | 6-(2-Methyl-4-chloro-phenylamino)-N-cyclopentylmethyl-4-isopropyl-nicotinamide | 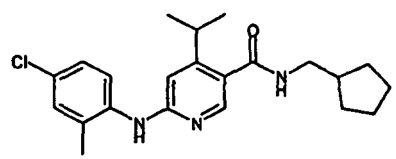 | G 30 Min | H | 3.50 386 $C_{22}H_{28}{}^{35}ClN_3O$ |

(continued)

| Example No. | Name | Structure | Method/ Reaction Time | Purification Method | RT (min), (MH+) Consistent with molecular formula |
|---|---|---|---|---|---|
| 247 | 6-(3-Chloro-4-cyano-phenylamino)-N-cyclopentylmethyl-4-isopropyl-nicotinamide | 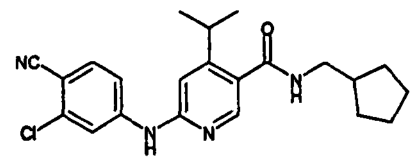 | G<br>30 Min | H | 3.68<br>397<br>$C_{22}H_{25}{}^{35}ClN_4O$ |
| 248 | 6-(4-bromo-2-chloro phenylamino)-N-cyclopentylmethyl-4-isopropyl-nicotinamide | 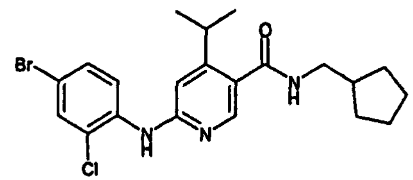 | G<br>30 Min | E | 3.91<br>450<br>$C_{21}H_{25}{}^{79}Br^{35}ClN_3O$ |
| 249 | N-Cyclobutylmethyl-6-(2,4-difluoro-phenylamino)-4-isopropyl-nicotinamide | 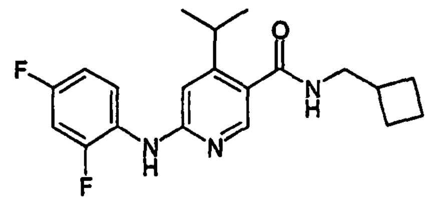 | G<br>1hour | F | 3.24<br>360<br>$C_{20}H_{23}F_2N_3O$ |
| 250 | N-Cyclobutylmethyl-6-(2,4-dichloro-phenylamino)-4-isopropyl-nicotinamide | 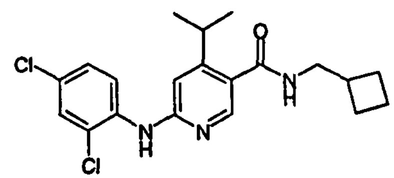 | G<br>1hour | F | 3.75<br>392<br>$C_{20}H_{23}{}^{35}Cl_2N_3O$ |
| 251 | N-Cyclobutylmethyl-6-(3,4-dichloro-phenylamino)-4-isopropyl-nicotinamide | 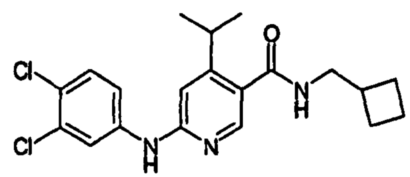 | G<br>1 hour | Crude product purified by trituration with 1:1 DCM/Ether | 3.89<br>392<br>$C_{20}H_{23}{}^{35}Cl_2N_3O$ |

(continued)

| Example No. | Name | Structure | Method/ Reaction Time | Purification Method | RT (min), (MH+) Consistent with molecular formula |
|---|---|---|---|---|---|
| 252 | N-Cyclobutylmethyl-6-(2,3-dichloro-phenylamino)-4-isopropyl-nicotinamide | 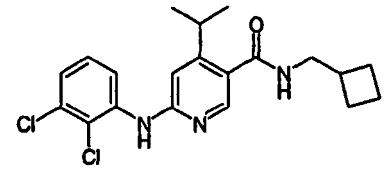 | G<br>1hour | H | 3.68<br>392<br>$C_{20}H_{23}{}^{35}Cl_2N_3O$ |
| 253 | 6-(2-Chloro-4-fluoro-phenylamino)-N-cyclobutylmethyl-4-isopropyl-nicotinamide | 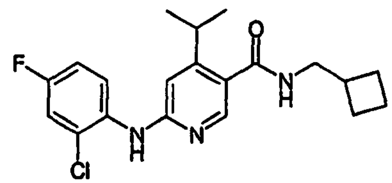 | G<br>1hour | F | 3.37<br>376<br>$C_{20}H_{23}{}^{35}ClFN_3O$ |
| 254 | 6-(3-Chloro-4-fluoro-phenylamino)-N-cyclobutylmethyl-4-isopropyl-nicotinamide | 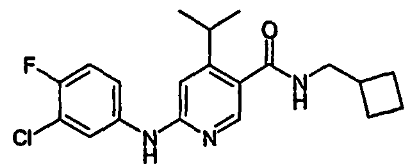 | G<br>1hour | H | 3.63<br>376<br>$C_{20}H_{23}{}^{35}ClFN_3O$ |
| 255 | 6-(4-Bromo-2-chloro-phenytamino)-N-cyclobutylmethyl-4-isopropyl-nicotinamide | 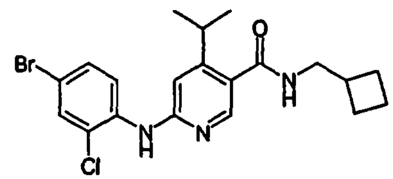 | G<br>1hour | H | 3.81<br>436<br>$C_{20}H_{23}{}^{79}Br^{35}ClN_3O$ |
| 256 | 6-(2-Bromo-4-chloro-phenylamino)-N-cyclobutylmethyl-4-isopropyl-nicotinamide | 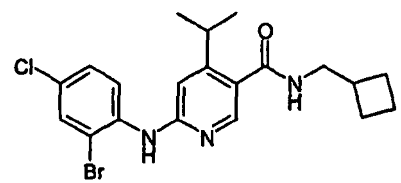 | G<br>1hour | H | 3.75<br>436<br>$C_{20}H_{23}{}^{79}Br^{35}ClN_3O$ |

(continued)

| Example No. | Name | Structure | Method/ Reaction Time | Purification Method | RT (min), (MH+) Consistent with molecular formula |
|---|---|---|---|---|---|
| 257 | N-cyclobutylmethyl-6-(2-fluoro-3-trifluoromethyl-phenylamino)-4-isopropyl-nicotinamide | 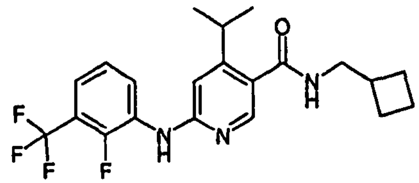 | G<br>1 hour | H | 3.64<br>410<br>$C_{21}H_{23}F_4N_3O$ |
| 258 | 6-(4-Chloro-2-methyl-phenylamino)-N-cyctobutylmethyl-4-isopropyl-nicotinamide | 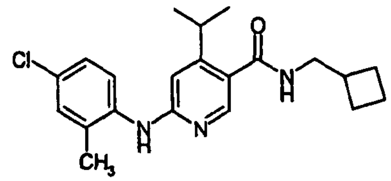 | G<br>1 hour | H | 3.35<br>372<br>$C_{21}H_{26}{}^{35}ClN_3O$ |
| 259 | 6-(2-Chloro-4-cyano-phenylamino)-N-cyclobutylmethyl-4-isopropyl-nicotinamide | 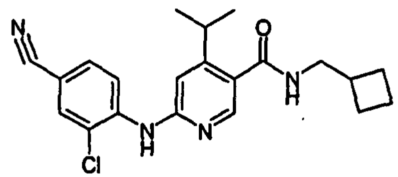 | K<br>3 hours | L | 3.41<br>383<br>$C_{21}H_{23}{}^{35}ClN_4O$ |
| 260 | 6-(4-Cyano-2-fluoro-phenylamino)-N-cyclobutylmethyl-4-isopropyl-nicotinamide | 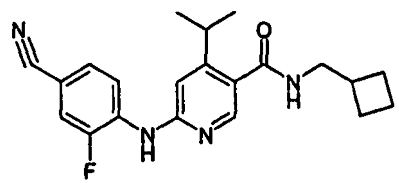 | K<br>4 hours | L | 3.32<br>367<br>$C_{21}H_{23}FN_4O$ |
| 261 | 6-(4-Cyano-2-methyl-phenylamino)-N-cyclobutylmethyl-4-isopropyl-nicotinamide | 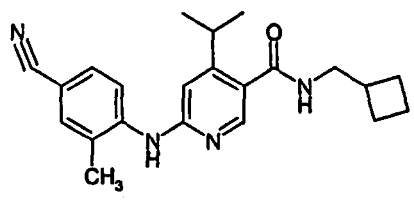 | K<br>4 hours | L | 3.24<br>363<br>$C_{22}H_{26}N_4O$ |

(continued)

| Example No. | Name | Structure | Method/ Reaction Time | Purification Method | RT (min), (MH+) Consistent with molecular formula |
|---|---|---|---|---|---|
| 262 | 6-(2-Chloro-4-trifluoromethyl-phenylamino)-N-cyclobutylmethyl-4-isopropyl-nicotinamide | 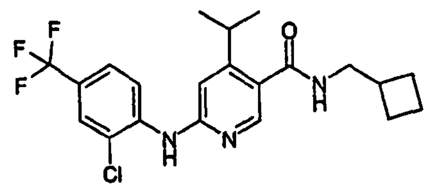 | K<br>4 hours | E | 3.86<br>426<br>$C_{21}H_{23}{}^{35}ClF_3N_3O$ |
| 263 | N-Cyclobutylmethyl-6-(3,5-dichloro-phenylamino)-4-isopropyl-nicotinamide | 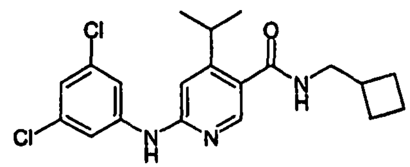 | G<br>1hour | H | 4.01<br>392<br>$C_{20}H_{23}{}^{35}Cl_2N_3O$ |
| 264 | N-Cyclobutylmethyl-6-(2,5-dichloro-phenylamino)-4-isopropyl-nicotinamide | 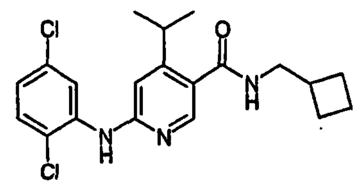 | G<br>1hour | H | 3.78<br>392<br>$C_{20}H_{23}{}^{35}Cl_2N_3O$ |
| 265 | N-Cyclobutylmethyl-6-(3,5-difluoro-phenylamino)-4-isopropyl-nicotinamide | 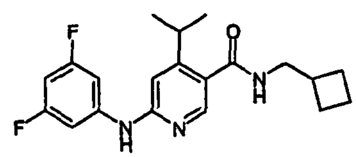 | G<br>1 hour | H | 3.57<br>360<br>$C_{20}H_{23}F_2N_3O$ |
| 266 | 6-(5-Chloro-2-fluoro-phenylamino)-N-cyclobutylmethyl-4-isopropyl-nicotinamide | 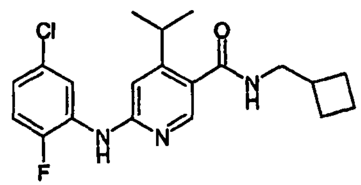 | G<br>1hour | H | 3.62<br>376<br>$C_{20}H_{23}{}^{35}ClFN_3O$ |

(continued)

| Example No. | Name | Structure | Method/ Reaction Time | Purification Method | RT (min), (MH+) Consistent with molecular formula |
|---|---|---|---|---|---|
| 267 | 6-(2-Chloro-5-fluoro-phenylamino)-N-cyclobutylmethyl-4-isopropyl-nicotinamide | 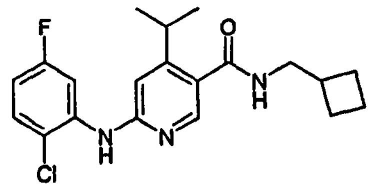 | G<br>1 hour | H | 3.56<br>376<br>$C_{20}H_{23}{}^{35}ClFN_3O$ |
| 268 | 6-(3-Chloro-phenylamino)-N-isobutyl-4-isopropyl-nicotinamide | 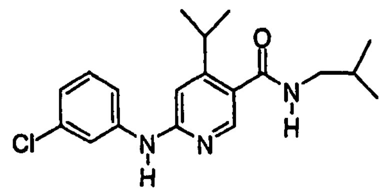 | G<br>30 min | H | 3.28<br>346<br>$C_{19}H_{24}{}^{35}ClN_3O$ |
| 269 | N-Isobutyl-4-isopropyl-6-(3-trifluoromethyl-phenylamino)-nicotinamide | 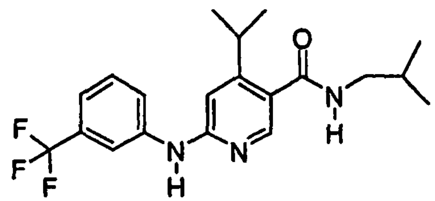 | G<br>30 min | H | 3.53<br>380<br>$C_{20}H_{24}F_3N_3O$ |
| 270 | 6-(3,4-Dichloro-phenylamino)-N-isobutyl-4-isopropyl-nicotinamide | 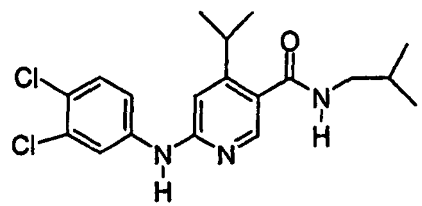 | G<br>30 min | H | 3.72<br>380<br>$C_{19}H_{23}{}^{35}Cl_2N_3O$ |

(continued)

| Example No. | Name | Structure | Method/ Reaction Time | Purification Method | RT (min), (MH+) Consistent with molecular formula |
|---|---|---|---|---|---|
| 271 | 6-(2-Fluoro-3-trifluoromethyl-phenylamino)-N-isobutyl-4-isopropyl-nicotinamide | 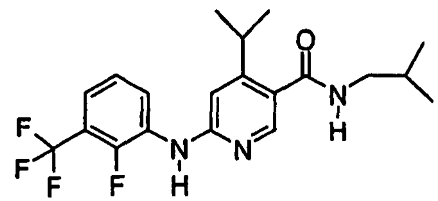 | G<br>30 min | H | 3.37<br>398<br>$C_{20}H_{23}F_4N_3O$ |
| 272 | 6-(3-Bromo-2-methyl-phenylamino)-N-isobutyl-4-isopropyl-nicotinamide | 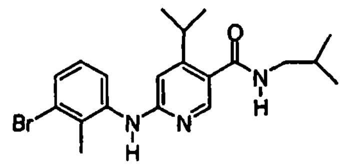 | G<br>30 min | H | 3.44<br>406<br>$C_{20}H_{26}{}^{81}BrN_3O$ |
| 273 | 6-(2,4-Dichloro-phenylamino)-N-isobutyl-4-isopropyl-nicotinamide | 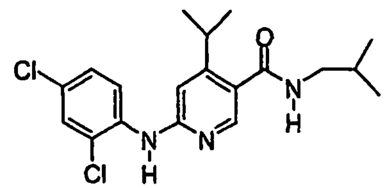 | G<br>30 min | H | 3.70<br>380<br>$C_{19}H_{23}{}^{35}Cl_2N_3O$ |
| 274 | 6-(2-Chloro-5-fluoro-phenylamino)-N-isobutyl-4-isopropyl-nicotinamide | 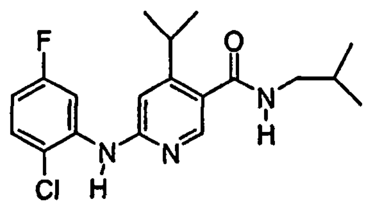 | G<br>30 min | H | 3.60<br>364<br>$C_{19}H_{23}{}^{35}ClFN_3O$ |

(continued)

| Example No. | Name | Structure | Method/ Reaction Time | Purification Method | RT (min), (MH+) Consistent with molecular formula |
|---|---|---|---|---|---|
| 275 | 6-(3,5-Difluoro-phenylamino)-N-isobutyl-4-isopropyl-nicotinamide | 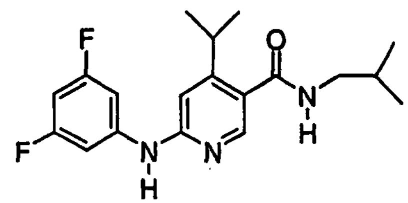 | G<br>30 min | H | 3.56<br>348<br>$C_{19}H_{23}F_2N_3O$ |
| 276 | 6-(5-Chloro-2-fluoro-phenylamino)-N-isobutyl-4-isopropyl-nicotinamide | 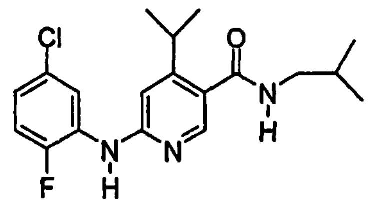 | G<br>30 min | H | 3.60<br>364<br>$C_{19}H_{23}{}^{35}ClFN_3O$ |
| 277 | 6-(3-Bromo-phenylamino)-N-isobutyl-4-isopropyl-nicotinamide | 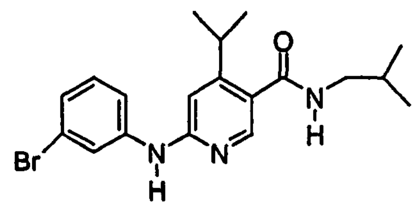 | G<br>30 min | H | 3.63<br>392<br>$C_{19}H_{24}{}^{81}BrN_3O$ |
| 278 | 6-(2,4-Dichloro-phenylamino)-N-(1,1-dioxo-tetrahydro-1/6-thiophen-3-ylmethyl)-4-isopropyl-nicotinamide | 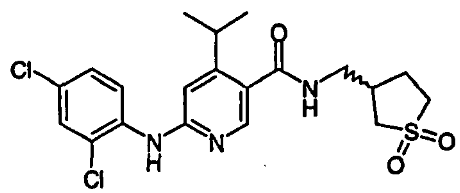 | G<br>30 min<br>From Descriptio n 38 | E | 3.2<br>456<br>$C_{20}H_{23}{}^{35}Cl_2N_3O_3S$ |

(continued)

| Example No. | Name | Structure | Method/ Reaction Time | Purification Method | RT (min), (MH+) Consistent with molecular formula |
|---|---|---|---|---|---|
| 279 | 6-(4-Bromo-3-fluoro-phenylamino)-N-(1,1-dioxo-tetrahydro-1/6-thiophen-3-ylmethyl)-4-isopropyl-nicotinamide | 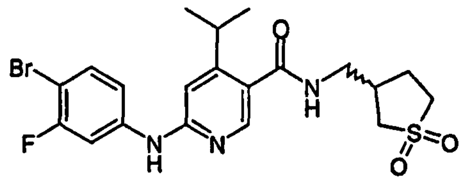 | G<br>30 min<br>From Descriptio n 38 | E | 3.2<br>484<br>$C_{20}H_{23}{}^{79}BrFN_3O_3S$ |

**Table 17.**

Examples in this table were prepared by the method and reaction time given in column 4 and purified by the procedure given in column 5.
Method G: Examples were prepared as for example 75
Method K: Examples were prepared as for Example 221.
Purification method E: mass-directed auto-preparative technique
Purification method H: Biotage Horizon

| Example No. | Name | Structure | Method / Reaction Time | Purification Method | RT (min), (MH+) Consistent with molecular formula |
|---|---|---|---|---|---|
| 280 | 6-(2-Chloro-5-fluoro-phenylamino)-4-isopropyl-N-(tetrahydro-pyran-4-ylmethyl)-nicotinamide | 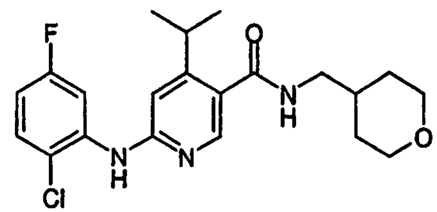 | G<br>1 hour | E | 3.1<br>406<br>$C_{21}H_{25}{}^{35}ClFN_3O_2$ |
| 281 | 6-(2-Chloro-5-methyl-phenylamino)-4-isopropyl-N-(tetrahydro-pyran-4-ylmethyl)-nicotinamide | 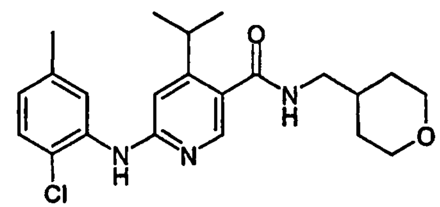 | G<br>30 min | E | 3.0<br>402<br>$C_{22}H_{28}{}^{35}ClN_3O_2$ |
| 282 | 6-(2-Fluoro-5-methyl-phenylamino)-4-isopropyl-N-(tetrahydro-pyran-4-ylmethyl)-nicotinamide | 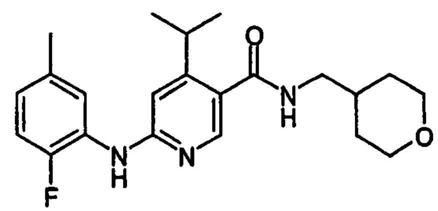 | G<br>30 min | E | 2.8<br>386<br>$C_{22}H_{28}FN_3O_2$ |
| 283 | 6-(5-Fluoro-2-methyl-phenylamino)-4-isopropyl-N-(tetrahydro-pyran-4-ylmethyl)-nicotinamide | 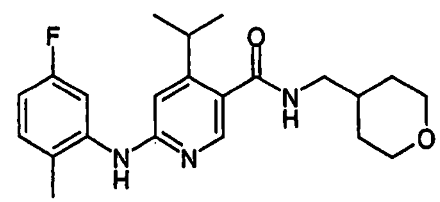 | G<br>1 hour | E | 2.7<br>386<br>$C_{22}H_{28}FN_3O_2$ |

(continued)

| Example No. | Name | Structure | Method / Reaction Time | Purification Method | RT (min), (MH+) Consistent with molecular formula |
|---|---|---|---|---|---|
| 284 | 6-(3-Bromo-2-methyl-phenylamino)-4-isopropyl-N-(tetrahydro-pyran-4-ylmethyl)-nicotinamide | 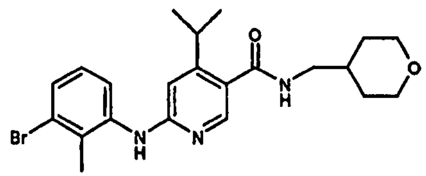 | G<br>30 min | E | 2.98<br>448<br>$C_{22}H_{28}{}^{81}BrN_3O_2$ |
| 285 | 4-Isopropyl-6-(2-methyl-4-trifluoromethoxy-phenylamino)-N-(tetrahydro-pyran-4-ylmethyl)-nicotinamide | 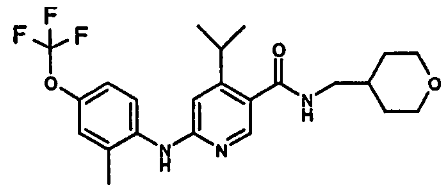 | G<br>30 min | E | 3.14<br>452<br>$C_{23}H_{28}F_3N_3O_3$ |
| 286 | 6-(3-Fluoro-2-methyl-phenylamino)-4-isopropyl-N-(tetrahydro-pyran-4-ylmethyl)-nicotinamide | 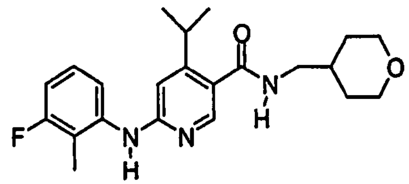 | G<br>30 min | E | 2.70<br>386<br>$C_{22}H_{28}FN_3O_2$ |
| 287 | 6-(3-Bromo-5-trifluoromethyl-phenylamino)-4-isopropyl-N-(tetrahydro-pyran-4-ylmethyl)-nicotinamide | 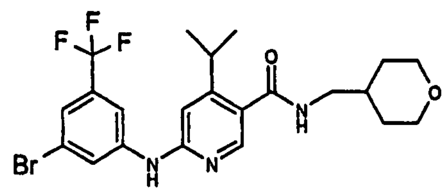 | G<br>30 min | E | 3.59<br>501<br>$C_{22}H_{25}{}^{81}BrF_3N_3O_2$ |
| 288 | 6-(4-Cyano-phenylamino)-4-isopropyl-N-(tetrahydro-pyran-4-ylmethyl)-nicotinamide | 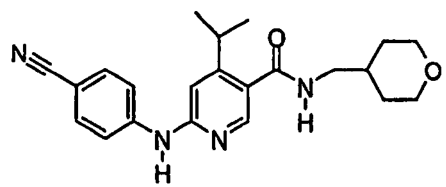 | G<br>30 min | E | 3.60<br>490<br>$C_{22}H_{26}N_4O_2$ |

(continued)

| Example No. | Name | Structure | Method / Reaction Time | Purification Method | RT (min), (MH+) Consistent with molecular formula |
|---|---|---|---|---|---|
| 289 | 6-(4-Chloro-2-fluoro-phenylamino)-4-isopropyl-N-(tetrahydro-pyran-4-ylmethyl)-nicotinamide | 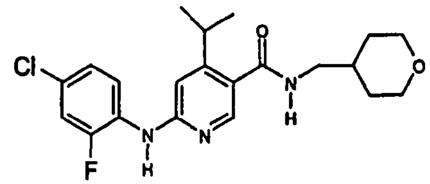 | G<br>30 min | E | 2.72<br>379<br>$C_{21}H_{25}{}^{35}ClFN_3O_2$ |
| 290 | 6-(4-bromo-2-fluoro-phenylamino)-N-cyclopentylmethyl-4-isopropyl-nicotinamide | 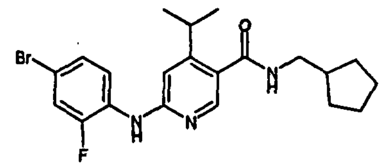 | G<br>30 min | E | 3.75<br>434<br>$C_{21}H_{25}{}^{79}BrFN_3O$ |
| 291 | 6-(2-Bromo-4-trifluoromethoxy-phenylamino)-N-cyclobutylmethyl-4-isopropyl-nicotinamide | 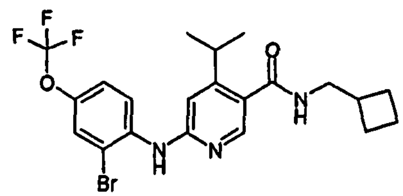 | G<br>1hour | H | 3.84<br>486<br>$C_{21}H_{23}{}^{79}BrF_3N_3O$ 2 |
| 292 | N-Cyclobutylmethyl-6-(2-fluoro-4-trifluoromethyl-phenylamino)-4-isopropyl-nicotinamide | 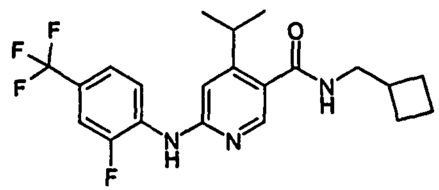 | K<br>8 hours | E | 3.71<br>410<br>$C_{21}H_{23}F_4N_3O$ |

**Example 293: 6-(4-Cyano-2-fluoro-phenylamino)-N-cyclopentylmethyl-4-isopropyl-nicotinamide**

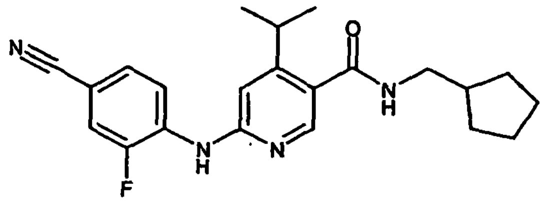

Prepared in a manner similar to Example 221 from 6-chloro-N-cyclopentylmethyl-4-isopropyl-nicotinamide (Description 10) and 4-cyano-2-fluoro-aniline, to give the title compound (16mg).
NMR (DMSO-d6) δ1.16 (6H, d), 1.23 (2H, m), 1.51-1.68 (6H, m), 2.11 (1H, m), 3.17 (2H, s), 4.11 (1H, s), 7.25 (1H, s), 7.61 (1H, d), 7.80 (1H, d), 8.12 (1H, s), 8.43 (1H, s), 8.72 (1H, t), 9.37 (1H, s).
LC/MS t = 3.4 min, [$MH^+$] 381, consistent with molecular formula $C_{22}H^{25}FN_4O$

**Example 294: 6-(4-Bromo-3-trifluoromethyl-phenylamino)-4-isopropyl-N-(tetrahydropyran-4-ylmethyl)-nicotinamide**

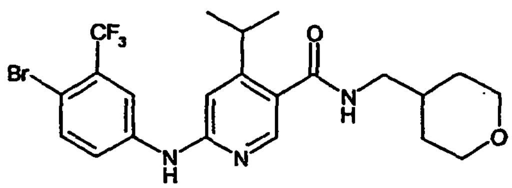

A mixture of 6-chloro-4-isopropyl-N-(tetrahydropyran-4-ylmethyl)-nicotinamide (Description 8) (100mg), 4-bromo-3-trifluoromethyl- (ex Lancaster,162mg), methanesulfonic acid (44μl) in 1,4-dioxane (1ml) was irradiated under microwave conditions at 180°for 30 minutes. After removal of the 1,4-dioxane under reduced pressure, the mixture was partitioned between ethyl acetate (5ml) and brine (2ml) and the aqueous layer separated. The organic layer was evaporated under reduced pressure and the residue purified using the Biotage Horizon system. Purification afforded the title compound as a white solid (47mg). NMR (DMSO-d6) δ1.16-1.23 (8H, d,m), 1.60-1.63 (2H, d), 1.75 (1H, m), 3.10 (2H, t), 3.28 (2H, t), 3.41 (1H, m), 3.85 (2H, d), 6.80 (1H, s), 7.73 (1H, d), 7.83 (1H, d), 8.16 (1H, s), 8.38-8.42 (2H, m), 9.70 (1H, s).
LC/MS t = 3.5 min, [$MH^+$] 500. consistent with molecular formula $C_{22}H_{25}{}^{79}Br\ F_3N_3O_2$

**Example 295: 6-(4-Fluoro-3-trifluoromethyl-phenylamino)-4-isopropyl-N-(tetrahydropyran-4-ylmethyl)-nicotinamide**

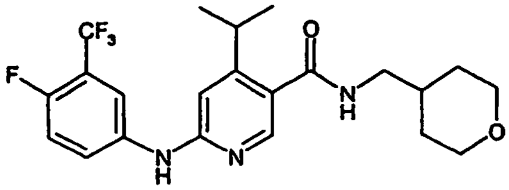

Prepared in a manner similar to Example 294 from 6-chloro-4-isopropyl-N-(tetrahydropyran-4-ylmethyl)-nicotinamide (Description 8) and 4-fluoro-3-trifluoromethylaniline (ex Lancaster, 120mg). Purified by trituration with ether to afford the title compound as a white solid (121 mg).

NMR (DMSO-d6) δ 1.09-1.24 (8H, d,m), 1.60-1.63 (2H, d), 1.76 (1H, m), 3.10 (2H, t), 3.28 (2H, t), 3.41 (1H, m), 3.85 (2H, d), 6.78 (1H, s), 7.42 (1H, t), 7.86 (1H, d), 8.13 (1H, s), 8.30 (1H, d), 8.40 (1H, t), 9.60 (1H, s).
LC/MS t = 3.3 min, [MH$^+$] 440, consistent with molecular formula $C_{22}H_{25}F_4N_3O_2$

**Example 296: 6-(3,4-Dibromo-phenylamino)-4-isopropyl-N-(tetrahydropyran-4-ylmethyl)-nicotinamide**

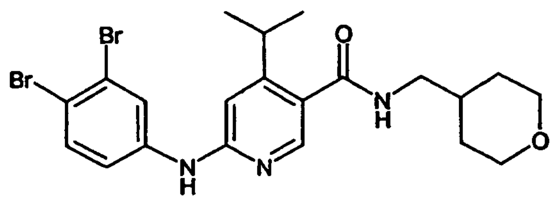

Prepared in a manner similar to Example 294 from 6-chloro-4-isopropyl-N-(tetrahydropyran-4-ylmethyl)-nicotinamide (Description 8) and 3,4-dibromoaniline (169mg). Purified using the Biotage Horizon system to afford the title compound as a white solid (76mg).
NMR (DMSO-d6) δ 1.09-1.23 (8H, d,m), 1.60-1.63 (2H, d), 1.76 (1H, m), 3.10 (2H, t), 3.28 (2H, t), 3.41 (1H, m), 3.85 (2H, d), 6.78 (1H, s), 7.48 (1H, d), 7.59 (1H, d), 8.15 (1H, s), 8.38 (2H, t), 9.52 (1H, s).
LC/MS t = 3.5 min, [MH$^+$] 510, consistent with molecular formula $C_{21}H_{25}{}^{79}Br_2N_3O_2$

**Example 297: 6-(4-Bromo-3-fluoro-phenylamino)-4-isopropyl-N-(tetrahydropyran-4-ylmethyl)-nicotinamide**

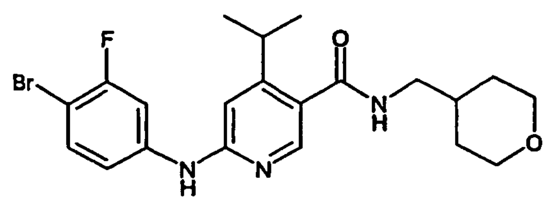

Prepared in a manner similar to Example 294 from 6-chloro-4-isopropyl-N-(tetrahydropyran-4-ylmethyl)-nicotinamide (Description 8) and 4-bromo-3-fluoro-aniline (128mg). Purified by trituration with ether to afford the title compound as a white solid (88mg).
NMR (DMSO-d6) δ 1.15-1.25 (8H, d,m), 1.60-1.63 (2H, d), 1.76 (1H, m), 3.10 (2H, t), 3.28 (2H, t), 3.41 (1H, m), 3.85 (2H, d), 6.81 (1H, s), 7.30 (1H, d), 7.54 (1H, t), 8.04 (1H, d), 8.15 (1H, s), 8.40 (1H, t), 9.64 (1H, s).
LC/MS t = 3.3 min, [MH$^+$] 450, consistent with molecular formula $C_{21}H_{25}F^{79}BrN_3O_2$

**Example 298: 6-(2-Chloro-4-trifluoromethyl-phenylamino)-N-cyclopentylmethyl-4-isopropyl-nicotinamide**

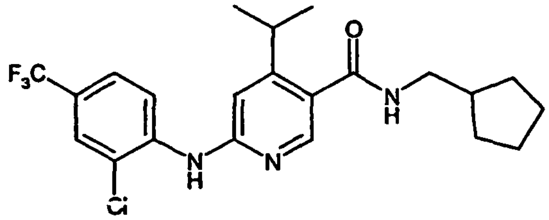

Prepared in a manner similar to Example 294 from 6-chloro-N-cyclopentylmethyl-4-isopropyl-nicotinamide and 2-chloro-4-trifluoromethylaniline, to give the title compound (30mg).

NMR (DMSO-d6) δ. 1.18 (8H, m), 1.50-1.68 (6H, m), 2.11 (1H, m), 3.16 (2H, s), 3.37 (1H, s), 7.29 (1H, s), 7.64 (1H, d), 7.83 (1H, s), 8.09 (1H, s), 8.43 (1H, s), 8.52 (1H, d), 8.80 (1H, s).
LC/MS t = 4.0 min, [MH+] 440, consistent with molecular formula $C_{22}H_{25}{}^{35}ClF_3N_3O$

**Example 299: 6-(3,4-Difluoro-phenylamino)-4-isopropyl-N-(tetrahydropyran-4-ylmethyl)-nicotinamide**

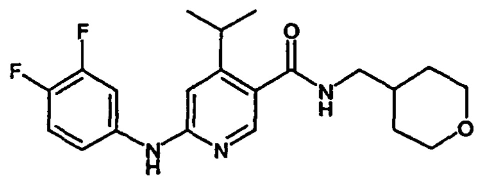

A mixture of 6-chloro-4-isopropyl-N-(tetrahydropyran-4-ylmethyl)-nicotinamide (Description 8) (100mg), 3,4-difluoroaniline (ex Lancaster,87mg), methanesulfonic acid (44μl) in 1,4-dioxane (1ml) was irradiated under microwave conditions at 180°for 30 minutes. The solid was dissolved in methanol then evaporated under reduced pressure. The mixture was partitioned between ethyl acetate (5ml) and brine (2ml) whereby a solid remained at the interface. The solid was filtered off and washed with water and ethyl acetate to afford the title compound (43mg).
NMR (DMSO-d6) δ 1.16-1.25 (8H, d,m), 1.60-1.62 (2H, d), 1.75 (1H, m), 3.10 (2H, t), 3.28 (2H, t), 3.41 (1H, m), 3.85 (2H, d), 6.85 (1H, s), 7.29 (1H, d), 7.37 (1H, q), 7.97 (1H, s), 8.08 (1H, s), 8.45 (1H, t), 9.80 (1H, s).
LC/MS t = 3.0 min, [MH+] 390, consistent with molecular formula $C_{21}H_{25}F_2N_3O_2$

**Example 300: 6-(4-Chloro-3-trifluoromethyl-phenylamino)-4-isopropyl-N-(tetrahydropyran-4-ylmethyl)-nicotinamide**

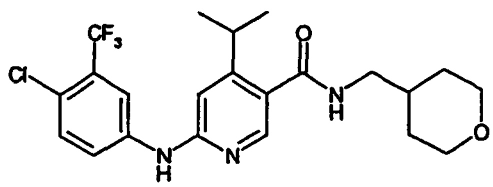

Prepared in a manner similar to Example 291 from 6-chloro-4-isopropyl-N-(tetrahydropyran-4-ylmethyl)-nicotinamide (Description 8) (100mg) and 4-chloro-3-trifluoromethyl-aniline (ex Lancaster,131mg). Purified by trituration with ether to afford the title compound as a white solid (79mg).
NMR (DMSO-d6) δ1.16-1.24 (8H, d,m), 1.60-1.63 (2H, d), 1.76 (1H, m), 3.11 (2H, t), 3.28 (2H, t), 3.41 (1H, m), 3.85 (2H, d), 6.80 (1H, s), 7.58 (1H, d), 7.91 (1H, d), 8.16 (1H, s), 8.39 (1H, s), 8.41 (1H, t), 9.70 (1H, s).
LC/MS t = 3.5 min, [MH+] 456, consistent with molecular formula $C_{22}H_{25}{}^{35}ClF_3N_3O_2$

**Example 301: 6-(4-Methyl-3-trifluoromethyl-phenylamino)-4-isopropyl-N-(tetrahydropyran-4-ylmethyl)-nicotinamide**

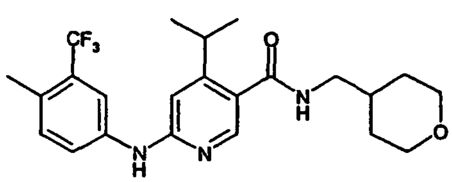

Prepared in a manner similar to Example 291 from 6-chloro-4-isopropyl-N-(tetrahydropyran-4-ylmethyl)-nicotinamide (Description 8) (100mg) and 4-methyl-3-trifluoromethylaniline (ex Lancaster, 118mg). Purified by trituration with ether to afford the title compound as a white solid (105mg).
NMR (DMSO-d6) δ1.15-1.24 (8H, d,m), 1.60-1.63 (2H, d), 1.76 (1H, m), 2.36 (3H, s), 3.11 (2H, t), 3.28 (2H, t), 3.41 (1H, m), 3.85 (2H, d), 6.76 (1H, s), 7.31 (1H, d), 7.76 (1H, d), 8.13 (1H, s), 8.18 (1H, s), 8.37 (1H, t), 9.45 (1H, s).
LC/MS t = 3.2 min, [$MH^+$] 436, consistent with molecular formula $C_{23}H_{28}F_3N_3O_2$

**Example 302: 6-(2-Chloro-4-trifluoromethoxy-phenylamino)-4-isopropyl-N-(tetrahydropyran-4-ylmethyl)-nicotinamide**

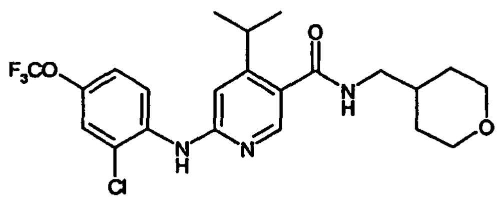

Prepared in a manner similar to Example 291 from 6-chloro-4-isopropyl-N-(tetrahydropyran-4-ylmethyl)-nicotinamide (Description 8) (100mg) and 2-chloro-4-trifluoromethoxyaniline (ex Acros,142mg). Purified using the Biotage Horizon system detailed at the beginning of the experimental section and by trituration with ether to afford the title compound as a white solid (20mg).
NMR (DMSO-d6) δ 1.16-1.23 (8H, d,m), 1.59-1.62 (2H, d), 1.75 (1H, m), 3.11 (2H, t), 3.28 (2H, t), 3.37 (1H, m), 3.84 (2H, d), 7.09 (1H, s), 7.34 (1H, d), 7.58 (1H, s), 8.04 (1H, s), 8.20 (1H, d), 8.38 (1H, t), 8.66 (1H, s).
LC/MS t = 3.4 min, [$MH^+$] 472, consistent with molecular formula $C_{22}H_{25}{}^{35}Cl\ F_3N_3O_3$

**Example 303: 6-(2-Cyano-3-methyl-phenylamino)-4-isopropyl-N-(tetrahydropyran-4-ylmethyl)-nicotinamide**

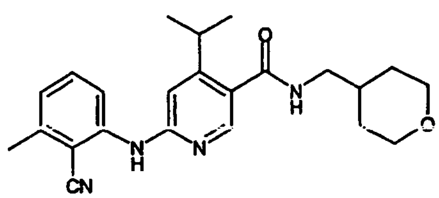

Prepared in a manner similar to Example 221 from 6-chloro-4-isopropyl-N-(tetrahydropyran-4-ylmethyl)-nicotinamide (Description 8) (100mg) and 2-cyano-3-methylaniline (ex Fluka, 44mg) to give the title compound (60mg).
NMR (DMSO-d6) δ1.16-1.23 (8H, d,m), 1.59-1.62 (2H, d), 1.75 (1H, m), 2.46 (3H, s), 3.11 (2H, t), 3.28 (2H, t), 3.37 (1H, m), 3.84 (2H, d), 6.96 (1H, s), 7.07 (1H, d), 7.48 (1H, t), 7.67 (1H, d), 8.03 (1H, s), 8.39 (1H, t), 9.13 (1H, s).
LC/MS t = 2.7 min, [$MH^+$] 393, consistent with molecular formula $C_{23}H_{28}N_4O_2$

**Example 304: 6-(3-Chloro-2-cyano-phenylamino)-4-isopropyl-N-(tetrahydropyran-4-ylmethyl)-nicotinamide**

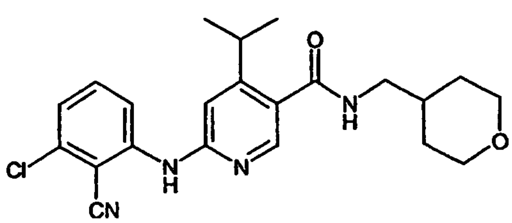

Prepared in a manner similar to Example 221 from 6-chloro-4-isopropyl-N-(tetrahydropyran-4-ylmethyl)-nicotinamide (Description 8) (100mg) and 3-chloro-2-cyanoaniline (ex Lancaster, 51 mg) to give the title compound (64mg). NMR (DMSO-$d_6$) δ 1.17-1.23 (8H, d,m), 1.59-1.62 (2H, d), 1.75 (1H, m), 3.11 (2H, t), 3.28 (2H, t), 3.37 (1H, m), 3.85 (2H, d), 7.03 (1H, s), 7.32 (1H, d), 7.60 (1H, t), 7.87 (1H, d), 8.07 (1H, s), 8.42 (1H, t), 9.41 (1H, s).
LC/MS t = 2.8 min, [$MH^+$] 413, consistent with molecular formula $C_{22}H_{25}{}^{35}ClN_4O_2$

**Example 305: 6-(3-Chloro-phenylamino)-4-(1-hydroxy-methyl-ethyl)-N-(tetrahydropyran-4-ylmethyl)-nicotinamide**

**1) 6-Chloro-1,1,dimethyl-1H-furo[3,4-c]pyridin-3-one**

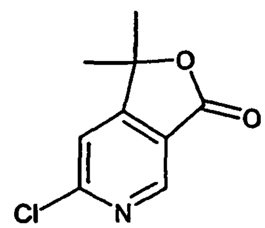

To a solution of 2,2,6,6,-tetramethylpiperidine (ex Aldrich, 13.44g) in tetrahydrofuran (90ml) at -55˚C under nitrogen was added dropwise 1.6M butyl lithium in hexane (ex Aldrich, 80ml). After 30 minutes a solution of 6-chloronicotinic acid (ex Aldrich, 5g) in tetrahydrofuran (40ml) was added dropwise and the solution stirred at -71˚C for 2 hours. The solution was treated with acetone (23ml) and then allowed to warm to room temperature. The solvent was removed under reduced pressure and the residue dissolved in water (100ml) and acidified to pH 3 with concentrated hydrochloric acid. The precipitated white solid was filtered off washed with water and dried to afford the title compound (4.42g).
NMR (DMSO-d6) δ1.65 (6H, s), 8.11 (1H, s), 8.91 (1H, s)
LC/MS t = 2.0 min, [$MH^+$] 198, consistent with molecular formula $C_9H_8{}^{35}ClNO_2$

**2) 6-(3-Chloro-phenylamino)-1,1,dimethyl-1H-furo[3,4-c]pyridin-3-one**

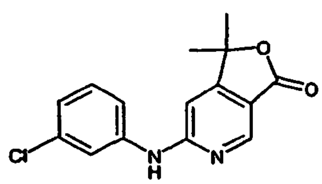

A mixture of 6-Chloro-1,1,dimethyl-1H-furo[3,4-c]pyridin-3-one (100mg), 3-chloroaniline (ex Lancaster,318mg), methanesulfonic acid (65μl) in 1,4-dioxane (1ml) was irradiated under microwave conditions at 180˚for 30 minutes. The solid was dissolved in methanol then evaporated under reduced pressure and the residue partitioned between ethyl acetate (5ml) and water (2ml) and the aqueous layer separated. The organic layer was dried over anhydrous magnesium sulphate, filtered and evaporated under reduced pressure. Purified by trituration with ether to afford the title compound as a white solid (30mg).
NMR (DMSO-d6) δ 1.61 (6H, s), 6.91 (1H, s), 7.04 (1H, d), 7.34 (1H, t), 7.55 (1H, d), 7.93 (1H, t), 8.69 (1H, s), 9.96 (1H, s).
LC/MS t = 3.3 min, [$MH^+$] 289, consistent with molecular formula $C_{15}H_{13}{}^{35}ClN_2O_2$

**3) 6-(3-Chloro-phenylamino)-4-(1-hydroxy-methyl-ethyl)-N-(tetrahydropyran-4-ylmethyl)-nicotinamide**

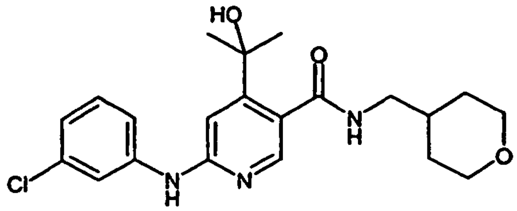

To a solution of 4-aminomethyltetrahydropyran (ex Combi-Blocks, Inc, 60mg) in dry dichloromethane (2ml) under nitrogen, was added dropwise 2.0M trimethylaluminium in hexane (ex Aldrich, 280µl) and the solution stirred for 15 minutes. Then a solution of 6-(3-Chloro-phenylamino)-1,1,dimethyl-1H-furo[3,4-c]pyridin-3-one (70mg) in dry dichloromethane (2ml) was added and the mixture stirred at 40˚C overnight. A further portion of 4-aminomethyltetrahydropyran (80mg) and 2.0M trimethylaluminium in hexane (380µl) in dry dichloromethane (3ml) was added and the mixture stirred for 48h.
The solvent was evaporated under reduced pressure and the residue partitioned between ethyl acetate (10ml) and water (5ml) and the aqueous layer separated. The organic layer was dried over anhydrous magnesium sulphate, filtered and evaporated under reduced pressure. Purified using the Biotage Horizon system detailed at the beginning of the experimental section to afford the title compound as a white solid (40mg).
NMR (DMSO-d6) δ 1.18-1.23 (2H, m), 1.47 (6H, s), 1.62-1.65 (2H, d), 1.80 (1H, m), 3.11 (2H, t), 3.28 (2H, t), 3.85 (2H, d), 6.06 (1H, s), 6.93 (1H, d), 7.05 (1H, s), 7.28 (1H, t), 7.48 (1H, d), 8.07 (1H, s), 8.17 (1H, s), 8.67 (1H, t), 9.53 (1H, s).
LC/MS t = 3.0 min, [MH$^+$] 404, consistent with molecular formula $C_{21}H_{26}{}^{35}ClN_3O_3$

**Example 306**

The compound below was prepared as for Example 75 from the intermediate of Description 15.

| Name | Structure | Method | Purification Method | RT (min), (MH+) Consistent with molecular formula |
|---|---|---|---|---|
| 4-*tert*-Butyl-6-(3,4-dichloro-phenylamino)-N-(tetrahydro-pyran-4-ylmethyl)-nicotinamide | 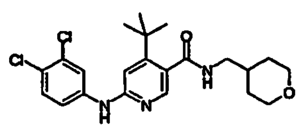 | G | E | 3.6<br>436<br>$C_{22}H_{27}{}^{35}Cl_2N_3O_2$ |

Formulations for pharmaceutical use incorporating compounds of the present invention can be prepared in various forms and with numerous excipients. Examples of such formulations are given below.

**Example 307: Inhalant Formulation**

A compound of formula (I) or a pharmaceutically acceptable derivative thereof, (1 mg to 100 mg) is aerosolized from a metered dose inhaler to deliver the desired amount of drug per use.

**Example 308: Tablet Formulation**

| Tablets/Ingredients | | Per Tablet |
|---|---|---|
| 1. | Active ingredient (Compound of formula (1) or pharmaceutically acceptable derivative) | 40 mg |
| 2. | Corn Starch | 20 mg |
| 3. | Alginic acid | 20 mg |
| 4. | Sodium Alginate | 20 mg |
| 5. | Mg stearate | 1.3 mg |

Procedure for tablet formulation:

Ingredients 1, 2. 3 and 4 are blended in a suitable mixer/blender, Sufficient water is added portion-wise to the blend with careful mixing after each addition until the mass is of a consistency to permit its conversion to wet granules. The wet mass is converted to granules by passing it through an oscillating granulator using a No. 8 mesh (2.38 mm) screen. The wet granules are then dried in an oven at 140˚F (60˚C) until dry. The dry granules are lubricated with ingredient No. 5, and the lubricated granules are compressed on a suitable tablet press.

**Example 309: Parenteral Formulation**

A pharmaceutical composition for parenteral administration is prepared by dissolving an appropriate amount of a compound of formula (I) in polyethylene glycol with heating. This solution is then diluted with water for injections Ph Eur. (to 100 ml). The solution is then rendered sterile by filtration through a 0.22 micron membrane filter and sealed in sterile containers.

It is to be understood that the present invention covers all combinations of particular and preferred groups described herein above

## Claims

**1.** A compound of formula (I):

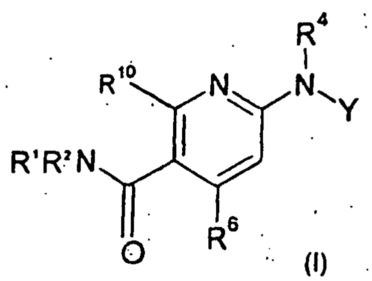

(I)

wherein:

Y is phenyl, unsubstituted or substituted with one, two or three substituents selected from $C_{1-6}$ alkyl, halosubstituted$C_{1-6}$ alkyl, $C_{1-6}$ alkoxy, a hydroxy group, a cyano group, halo, a $C_{1-6}$alkylsulfonyl group, $-CONH_2$, $-NHCOCH_3$, -COOH, halosubstituted$C_{1-6}$ alkoxy, $SO_2NR^{8a}R^{8b}$ wherein $R^{8a}$ and $R^{8b}$ are each independently H, $C_{1-6}$alkyl or $C_{1-6}$ alkynyl;
$R^1$ is selected from hydrogen, $C_{1-6}$ alkyl, $C_{3-6}$ cycloalkyl, or halosubstituted$C_{1-6}$ alkyl;
$R^2$ is $(CH_2)_mR^3$ where m is 0 or 1;
or $R^1$ and $R^2$ together with N to which they are attached form a 4- to 8-membered non-aromatic heterocyclyl ring which is unsubstituted or substituted with 1, 2 or 3 substituents selected from: $C_{1-6}$ alkyl, $C_{1-6}$ alkoxy, a hydroxy group, a cyano group; halo, a sulfonyl group, methylsulfonyl, $NR^{8a}R^{8b}$, $CH_2$phenyl, $NHCOCH_3$, (=O), $CONHCH_3$ or $NHSO_2CH_3$ wherein $R^{8a}$ and $R^{8b}$ are as defined above;
$R^3$ is a 4- to 8- membered non-aromatic heterocyclyl group, a $C_{3-8}$ cycloalkyl group, a straight or branched $C_{1-10}$ alkyl, a $C_{2-10}$alkenyl, a $C_{3-8}$cycloalkenyl, a $C_{2-10}$ alkynyl, or a $C_{3-8}$cycloalkynyl any of which can be unsubtituted or substituted with 1, 2 or 3 substituents preferably selected from: $C_{1-6}$ alkyl, $C_{1-6}$ alkoxy, a hydroxy group, a cyano group, halo, a sulfonyl group, methylsulfonyl, $NR^{8a}R^{8b}$, $CH_2$phenyl, $NHCOCH_3$, (=O), $CONHCH_3$ or $NHSO_2CH_3$ wherein $R^{8a}$ and $R^{8b}$ are as defined above or $R^5$;
$R^4$ is selected from hydrogen, $C_{1-6}$ alkyl, $C_{3-6}$ cycloalkyl, or halosubstituted$C_{1-6}$ alkyl, $COCH_3$, or $SO_2Me$;
$R^5$ is

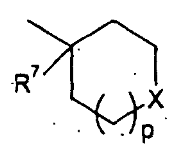

wherein p is 0, 1 or 2, and X is $CH_2$, O, or S;
$R^6$ is chloro or $(C_{1-6})$alkyl optionally substituted with 1, 2 or 3 substitutents selected from hydroxy, $C_{1-6}$alkyloxy, cyano, halo, $NR^{8a}R^{8b}$, $CONR^{8a}R^{8b}$, $SO_2NR^{8a}R^{8b}$, $NR^{8a}COR^{8b}$ or $NR^{8a}SO_2R^{8b}$ wherein $R^{8a}$ and $R^{8b}$ are as defined above and $R^{10}$ is hydrogen, or $R^{10}$ is chloro or $(C_{1-6})$alkyl optionally substituted with 1, 2 or 3 substitutents selected from hydroxy, $C_{1-6}$alkyloxy, cyano, halo, $NR^{8a}R^{8b}$, $CONR^{8a}R^{8b}$, $SO_2NR^{8a}R^{8b}$, $NR^{8a}COR^{8b}$ or $NR^{8a}SO_2R^{8b}$ wherein $R^{8a}$ and $R^{8b}$ are as defined above and $R^6$ is hydrogen;
$R^7$ is OH, $C_{1-6}$alkoxy, $NR^{8a}R^{8b}$, $NHCOR^9$, $NHSO_2R^9$ or $SO_qR^9$;
$R^{8a}$ is H or $C_{1-6}$alkyl;
$R^{8b}$ is H or $C_{1-6}$alkyl;
$R^9$ is $C_{1-6}$alkyl;
q is 0, 1 or 2;

or a pharmaceutically acceptable derivative thereof.

**2.** A compound as claimed in claim 1 of formula (Ia):

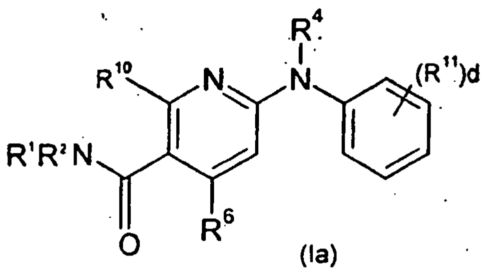

(Ia)

$R^1$ is selected from hydrogen, $C_{1-6}$ alkyl, $C_{3-6}$ cycloalkyl, or halosubstituted$C_{1-6}$ alkyl;
$R^2$ is $(CH_2)_mR^3$ where m is 0 or 1;
or $R^1$ and $R^2$ together with N to which they are attached form a non-aromatic heterocyclyl ring selected from azetidinyl, pyrrolidinyl, morpholinyl, piperazinyl, piperidinyl, tetrahydropyridinyl, azapine, oxapine, azacyclooctanyl, azaoxacyclooctanyl and azathiacyclooctanyl, any of which can be unsubstituted or substituted with 1, 2 or 3 substituents selected from; $C_{1-6}$ alkyl, $C_{1-6}$ alkoxy, hydroxy, cyano, halo, sulfonyl, methylsulfonyl, $NR^{8a}R^{8b}$, $CH_2$phenyl, $NHCOCH_3$, (=O), $CONHCH_3$ and $NHSO_2CH_3$;
$R^3$ is 2- or 3- azetidinyl, oxetanyl, thioxetanyl, thioxetanyl-S-oxide, thioxetanyl-S,S-dioxide, dioxalanyl, pyrrolidinyl, tetrahydrofuranyl, tetrahydrothiophenyl, tetrahydrothiophenyl-S,S-dioxide, morpholinyl, piperidinyl, piperazinyl, tetrahydropyranyl, tetrahydrothiopyranyl, thiomorpholinyl, thiomorpholinyl-S,S-dioxide, tetrahydropyridinyl, dioxanyl, tetrahydro-thiopyran-1,1-dioxide, azapine, oxapine, azacyclooctanyl, azaoxacyclooctanyl, azathiacyclooctanyl, oxacylcooctanyl, thiacyclooctanyl, a $C_{3-8}$ cycloalkyl group, a straight or branched $C_{1-10}$ alkyl, a $C_{2-10}$ alkenyl, a $C_{3-8}$cycloalkenyl, a $C_{2-10}$alkynyl, or a $C_{3-8}$cycloalkynyl; any of which can be unsubstituted or substituted with 1, 2 or 3 substituents selected from $C_{1-6}$ alkyl, $C_{1-6}$ alkoxy, hydroxy, cyano, halo, sulfonyl, methylsulfonyl, $NR^{8a}R^{8b}$, $CH_2$phenyl, $NHCOCH_3$, (=O), $CONHCH_3$ and $NHSO_2CH_3$; or $R^3$ is $R^5$;
$R^4$ is selected from hydrogen, $C_{1-6}$ alkyl, $C_{3-6}$ cycloalkyl, or halosubstituted$C_{1-6}$ alkyl, $COCH_3$, or $SO_2Me$;
$R^5$ is

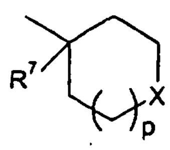

wherein p is 0, 1 or 2, and X is $CH_2$, O or S;
$R^6$ is chloro or $(C_{1-6})$alkyl optionally substituted with 1, 2 or 3 substitutents selected from hydroxy, $C_{1-6}$alkyloxy, cyano, halo, $NR^{8a}R^{8b}$, $CONR^{8a}R^{8b}$, $SO_2NR^{8a}R^{8b}$, $NR^{8a}COR^{8b}$ or $NR^{8a}SO_2R^{8b}$ wherein $R^{8a}$ and $R^{8b}$ are as defined above and $R^{10}$ is hydrogen, or $R^{10}$ is chloro or $(C_{1-6})$alkyl optionally substituted with 1, 2 or 3 substitutents selected from hydroxy, $C_{1-6}$alkyloxy, cyano, halo, $NR^{8a}R^{8b}$, $CONR^{8a}R^{8b}$, $SO_2NR^{8a}R^{8b}$, $NR^{8a}COR^{8b}$ or $NR^{8a}SO_2R^{8b}$ wherein $R^{8a}$ and $R^{8b}$ are as defined above and $R^6$ is hydrogen;
$R^7$ is OH, $C_{1-6}$alkoxy, $NR^{8a}R^{8b}$, $NHCOR^9$, $NHSO_2R^9$ or $SOqR^9$;

$R^{8a}$ is H or $C_{1-6}$alkyl;
$R^{8b}$ is H or $C_{1-6}$alkyl;
$R^9$ is $C_{1-6}$alkyl;
$R^{11}$ is $C_{1-6}$ alkyl, halosubstituted$C_{1-6}$ alkyl, $C_{1-6}$ alkoxy, hydroxy, cyano, halo, $C_{1-6}$alkylsulfonyl group, $-CONH_2$, $-NHCOCH_3$. -COOH, halosubstituted $C_{1-6}$ alkoxy, $SO_2NR^{8a}R^{8b}$ or $C_{1-6}$ alkynyl;
q is 0, 1 or 2;
d is 0, 1, 2, or 3;

or a pharmaceutically acceptable derivative thereof.

**3.** A compound as claimed in claim 1 or 2 wherein $R^1$ is hydrogen.

**4.** A compound as claimed in any preceding claim wherein $R^4$ is $C_{1-6}$ alkyl or hydrogen.

**5.** A compound as claimed in any preceding claim wherein $R^6$ is *t*-butyl, isopropyl or $CF_3$.

**6.** A compound as described in any one of Examples 1 to 306, namely a compound selected from:

2-(3-Chlorophenylamino)-4-trifluoromethylpyridine-5-carboxylic acid cyclohexylmethyl amide,
6-(3-Chlorophenylamino)-*N*-cyclohexylmethyl-4-isopropylnicotinamide,
N-Cyclohexylmethyl-6-(3,4-dichloro-phenylamino)-4-isopropyl-nicotinamide,
6-(3-Bromo-phenylamino)-N-cyclohexylmethyl-4-isopropyl-nicotinamide,
N-Cyclohexylmethyl-6-(2,4-dichloro-phenylamino)-4-isopropyl-nicotinamide,
6-(3-Chloro-phenylamino)-N-cyclobutylmethyl-4-isopropyl-nicotinamide,
6-(3-Chloro-phenyl-amino)-4-isopropyl-N-(tetrahydro-pyran-4-yimethyl)-nicotinamide,
6-(3-Bromo-phenyl-amino)-4-isopropyl-N-(tetrahydro-pyran-4-ylmethyl)-nicotinamide,
N-Cyclohexylmethyl-4-isopropyl-6-(3-methoxy-phenylamino)-nicotinamide,
N-Cyclohexylmethyl-6-(3-fluoro-phenylamino)-4-isopropyl-nicotinamide,
1-[6-(3-Chloro-phenylamino)-4-isopropyl-pyridin-3-yl]-1-morpholin-4-yl-methanone,
6-(3-Bromo-phenylamino)-N-cyclohexylmethyl-4-isopropyl-nicotinamide,
N-Cyclohexylmethyl-4-isopropyl-6-m-tolylamino-nicotinamide,
N-Cyclohexytmethyl-4-isopropyl-6-(3-trifluoromethyl-phenylamino)-nicotinamide.
N-Cyclohexylmethyl-4-isopropyl-6-(3-trifluoromethoxy-phenylamino)-nicotinamide,
6-(2,3-Dichloro-phenylamino)-4-isopropyl-N-(tetrahydro-pyran-4-ylmethyl)-nicotinamide,
6-(2,4-Dichloro-phenylamino)-4-isopropyl-N-(tetrahydro-pyran-4-ylmethyl)-nicotinamide,
6-(3,4-Dichloro-phenylamino)-4-isopropyl-N-(tetrahydro-pyran-4-ylmethyl)-nicotinamide,
4-Isopropyl-N-(tetrahydro-pyran-4-ylmethyl)-6-(3-trifluoromethyl-phenylamino)-nicotinamide,
4-Isopropyl-N-(tetrahydro-pyran-4-ylmethyl)-6-(3-trifluoromethoxy-phenylamino)-nicotinamide,
6-[(3-Chloro-phenyl)amino]-N-(cyclopentylmethyl)-4-isopropyl-nicotinamide,
N-Cyclopentylmethyl-6-(3-fluorophenylamino)-4-isopropyl-nicotinamide,
N-Cyclopentylmethyl-4-isopropyl-6-(3-trifluoromethyl-phenylamino)-nicotinamide,
N-Cyclopentylmethyl-4-isopropyl-6-m-tolylamino-nicotinamide,
N-Cyclopentylmethyl-4-isopropyl-6-(3-trifluoromethoxy-phenylamino)-nicotinamide,
6-(3-Bromophenylamino)-N-cyclopentylmethyl-4-isopropyl-nicotinamide,
N-Cyclopentylmethyl-4-isopropyl-6-(3-methoxyphenylamino)nicotinamide,
6-(3-Cyano-phenylamino)-N-cyclopentylmethyl-4-isopropyl-nicotinamide,
6-(2-Chloro-4-fluoro-phenylamino)-N-cyclopentylmethyl-4-isopropyl-nicotinamide,
6-(2-Chloro-4-cyano-phenylamino)-N-cyclopentylmethyl-4-isopropyl-nicotinamide,
N-Cyclopentylmethyl-6-(2,4-dichloro-phenylamino)-4-isopropyl-nicotinamide,
N-Cyclopentylmethyl-6-(3,4-dichlorophenylamino)-4-isopropyl-nicotinamide,
6-(3-Bromo-phenylamino)-N-cyclobutylmethyl-4-isopropyl-nicotinamide,
N-Cyclobutylmethyl-6-(3-fluoro-phenylamino)-4-isopropyl-nicotinamide,
N-Cyclobutylmethyl-6-(3-trifluoromethyl-phenylamino)-4-isopropyl-nicotinamide,
6-(3-Cyano-phenylamino)-N-cyclobutylmethyl-4-isopropyl-nicotinamide,
N-Cyclobutylmethyl-4-isopropyl-6-m-tolylamino-nicotinamide,
N-Cyclobutylmethyl-4-isopropyl-6-(3-methoxy-phenylamino)-nicotinamide,
6-(3-Fluoro-phenylamino)-4-isopropyl-N-(tetrahydro-pyran-4-ylmethyl)-nicotinamide,
1-[6-(3-Fluoro-phenylamino)-4-isopropyl-pyridin-3-yl]-1-morpholin-4-yl-methanone,

4-Isopropyl-6-(3-methoxy-phenylamino)-N-(tetrahydro-pyran-4-ylmethyl)-nicotinamide,
4-Isopropyl-N-(tetrahydro-pyran-4-ylmethyl)-6-m-tolylamino-nicotinamide,
6-(3-Cyano-phenylamino)-4-isopropyl-N-(tetrahydro-pyran-4-ylmethyl)-nicotinamide,
6-[(3.4-Dichloro-phenyl)-methyl-amino]-4-isopropyl-N-(tetrahydro-pyran-4-ylmethyl)-nicotinamide,
6-[(3-Bromo-phenyl)-methyl-amino]-4-isopropyl-N-(tetrahydro-pyran-4-ylmethyl)-nicotinamide,
6-[(3-Fluoro-phenyl)-methyl-amino]-4-isopropyl-N-(tetrahydro-pyran-4-ylmethyl)-nicotinamide,
4-Isopropyl-6-(methyl-phenyl-amino)-N-(tetrahydro-pyran-4-ylmethyl)-nicotinamide,
6-[(3-Chloro-phenyl)-methyl-amino]-4-isopropyl-N-(tetrahydro-pyran-4-ylmethyl)-nicotinamide.
6-[(4-Chloro-phenyl)-methyl-amino]-4-isopropyl-N-(tetrahydro-pyran-4-ylmethyl)-nicotinamide.
6-(3-Chloro-phenylamino)-N-cyclobutyl-4-isopropyl-nicotinamide,
6-(3-Chloro-phenylamino)-N-cyclopropylmethyl-4-isopropyl-nicotinamide,
6-(3-Chloro-phenylamino)-N-(2-ethyl-butyl)-4-isopropyl-nicotinamide,
6-(3-Chloro-phenylamino)-N-cyclohexyl-4-isopropyl-nicotinamide,
6-(3-Chloro-phenylamino)-N-(1-hydroxy-cyclohexylmethyl)-4-isopropyl-nicotinamide,
1-[6-(3-Chloro-phenylamino)-4-isopropyl-pyridin-3-yl]-1-piperidin-1-yl-methanone,
6-(3-Chloro-phenylamino)-N-(2,2-dimethyl-propyl)-4-isopropyl-nicotinamide,
6-(3-Chloro-phenylamino)-4-isopropyl-N-(2-methoxy-ethyl)-nicotinamide,
6-(3-Chloro-phenylamino)-4-isopropyl-N-(tetrahydro-pyran-4-yl)-nicotinamide,
6-(3-Chloro-phenylainino)-4-isopropyl-N-[(R)-1-(tetrahydro-furan-2-yl)methyl]-nicotinamide,
N-((R)-1-{1-[6-(3-Chloro-phenylamino)-4-isopropyl-pyridin-3-yl]-methanoyl}-pyrrolidin-3-yl)-acetamide,
1-[6-(3-Chloro-phenylamino)-4-isopropyl-pyridin-3-yl]-1-(4-methane-sulfonyl-piperazin-1-yl)-methanone,
6-(3-Chloro-phenylamino)-N-(1,1-dioxo-tetrahydro-1/$^{6}$-thiophen-3-yl)-4-isopropyl-nicotinamide,
6-(3-Chloro-phenylamino)-4-isopropyl-N-[(S)-1-(tetrahydro-furan-2-yl)methyl]-nicotinamide;
6-(3-Chloro-phenylamino)-N-(1,1-dioxo-hexahydro-1/$^{6}$-thiopyran-4-yl)-4-isopropyl-nicotinamide,
1-[6-(3-Chloro-phenylamino)-4-isopropyl-pyridin-3-yl]-1-(4-methyl-piperazin-1-yl)-methanone,
6-(3-Chloro-phenylamino)-N-(2-dimethylamino-ethyl)-4-isopropo-'nicotinamide,
N-((S)-1-{1-[6-(3-Chloro-phenylamino)-4-isopropyl-pyridin-3-yl]-methanoyl}-pyrrolidin-3-yl)-acetamide,
N-(1-{1-[6-(3-Chloro-phenylamino)-4-isopropyl-pyridin-3-yl]-methanoyl}-piperidin-4-yl)-methanesulfonamide,
4-*tert*-Butyl-6-(3-chloro-phenylamino)-N-cyclohexylmethyl-nicotinamide,
4-*tert*-Butyl-6-(2,4-dichloro-phenylamino)-N-cyclohexylmethyl-nicotinamide,
4-*tert*-Butyl-6-(3-chloro-phenylamino)-N-(tetrahydro-pyran-4-ylmethyl)-picotinamide,
4-*tert*-Butyl-6-(3-fluoro-phenylamino)-N-(tetrahydro-pyran-4-ylmethyl)-nicotinamide,
4-*tert*-Butyl-6-(2-chloro-3-fluorophenylamino)-N-(tetrahydro-pyran-4-ylmethyl)-nicotinamide,
4-*tert*-Butyl-6-(2,4-di-chloro-phenylamino)-N-(tetrahydro-pyran-4-ylmethyl)-nicotinamide,
6-(3,5-Dichloro-phenylamino)-4-isopropyl-N-(tetrahydro-pyran-4-ylmethyl)-nicotinamide,
6-(5-Chloro-2-fluoro-phenylamino)-4-isopropyl-N-(tetrahydro-pyran-4-ylmethyl)-nicotinamide,
6-(3-Chloro-4-fluoro-phenylamino)-4-isopropyl-N-(tetrahydro-pyran-4-ylmethyl)-nicotinamide,
6-(3-Chloro-4-trifluoromethoxy-phenylamino)-4-isopropyl-N-(tetrahydro-pyran-4-ylmethyl)-nicotinamide,
6-(3-Chloro-4-cyano-phenylamino)-4-isopropyl-N-(tetrahydro-pyran-4-ylmethyl)-nicotinamide,
6-(3-Fluoro-5-trifluoromethyl-phenylamino)-4-isopropyl-N-(tetrahydro-pyran-4-ylmethyl)-nicotinamide,
6-(2-Fluoro-3-trifluoromethyl-phenylamino)-4-isopropyl-N-(tetrahydro-pyran-4-ylmethyl)-nicotinamide,
6-(4-Bromo-2-chloro-phenylamino)-4-isopropyl-N-(tetrahydro-pyran-4-ylmethyl)-nicotinamide,
6-(2-Bromo-4-chloro-phenylamino)-4-isopropyl-N-(tetrahydro-pyran-4-ylmethyl)-nicotinamide,
4-Isopropyl-6-(2-methyl-3-trifluoromsthyl-phenylamino)-N-(tetrahydro-pyran-4-ylmethyl)-nicotinamide,
6-(3-chloro-4-methyl-phenylamino)-4-isopropyl-N-(tetrahydro-pyran-4-ylmethyl)-nicotinamide,
6-(4-Bromo-3-methyl-phenylamino)-4-isopropyl-N-(tetrahydro-pyran-4-ylmethyl)-nicotinamide,
6-(2,5-Dichloro-phenylamino)-4-isopropyl-N-(tetrahydro-pyran-4-ylmethyl)-nicotinamide,
4-Isopropyl-6-(2-methyl-5-trifluoromethyl-phenylamino)-N-(tetrahydro-pyran-4-ylmethyl)-nicotinamide,
6-(2-Bromo-4-chloro-phenylamino)-N-cyclopentylmethyl-4-isopropyl-nicotinamide,
6-(4-Bromo-3-chloro-phenylamino)-4-isopropyl-N-(tetrahydro-pyran-4-ylmethyl)-nicotinamide,
6-(4-Chloro-2-fluoro-phenylamino)-N-cyclopentylmethyl-4-isopropyl-nicotinamide,
N-Cyclopentylmethyl-6-(3-fluoro-4-trifluoromethyl)-phenylamino)-4-isopropyl-nicotinamide,
6-(4-Cyano-2-methyl-phenylamino)-N-cyclopentylmethyl-4-isopropyl-nicotinamide,
6-(3-Chloro-2-fluoro-phenylamino)-4-isopropyl-N-(tetrahydro-pyran-4-ylmethyl)-nicotinamide,
6-(3-Fluoro-4-trifluoromethyl-phenylamino)-4-isopropyl-N-(tetrahydro-pyran-4-ylmethyl)-nicotinamide,
6-(4-Cyano-3-trifluoromethyl-phenylamino)-4-isopropyl-N-(tetrahydro-pyran-4-ylmethyl)-nicotinamide,
6-(4-Cyano-2-fluoro-phenylamino)-4-isopropyl-N-(tetrahydro-pyran-4-ylmethyl)-nicotinamide,
6-(4-fluoro-3-methyl-phenylamino)-4-isopropyl-N-(tetrahydro-pyran-4-ylmethyl)-nicotinamide,

6-(5-Chloro-2-methyl-phenylamino)-4-isopropyl-N-(tetrahydro-pyran-4-ylmethyl)-nicotinamide,
6-(3-Fluoro-4-methyl-phenylamino)-4-isopropyl-N-(tetrahydro-pyran-4-ylmethyl)-nicotinamide,
6-(3,4-Dimethyl-phenylamino)-4-isopropyl-N-(tetrahydro-pyran-4-ylmethyl)-nicotinamide,
6-(3-Bromo-4-methyl-phenylamino)-4-isopropyl-N-(tetrahydro-pyran-4-ylmethyl)-nicotinamide,
6-(3-Chloro-phenylamino)-N-(4-hydroxy-tetrahydro-pyran-4-ylmethyl)-4-isopropyl-nicotinamide,
6-(2,3-Dichloro-phenylamino)-N-(cyclobutyl)-4-trifluoromethyl-nicotinamide,
6-(2,4-Dichloro-phenylamino)-N-(tetrahydropyran-4-ylmethyl)-4-trifluoromethyl-nicotinamide,
6-(3-Chloro-phenylamino)-N-(1,1-dioxo-tetrahydrothiophen-3-ylmethyl)-4-trifluoromethyl-nicotinamide,
N-Cyclohexylmethyl-6-phenylamino-4-trifluoromethyl-nicotinamide,
N-Cyclopentylmethyl-6-phenylamino-4-trifluoromethyl-nicotinamide,
N-Cyclobutylmethyl-6-phenylamino-4-trifluoromethyl-nicotinamide.
N-Cyclobutyl-6-(3-chloro-phenylamino)-4-trifluoromethyl-nicotinamide,
N-(Tetrahydrqpyran-4-ylmethyl)-6-(3-chloro-phenylamino)-4-trifluoromethyl-nicotinamide,
N-Cyclobutylmethyl-6-(3-chloro-phenylamino)-4-trifluoromethyl-nicotinamide,
N-Isobutyl-6-(3-chloro-phenylamino)-4-trifluoromethyl-nicotinamide,
N-Cyclopentylmethyl-6-(3-chloro-phenylamino)-4-trifluoromethyl-nicotinamide,
N-Cyclopropylmethyl-6-(3-chloro-phenylamino)-4-trifluoromethyl-nicotinamide,
N-Cyclohexylmethyl-6-(3-bromo-phenylamino)-4-trifluoromethyl-nicotinamide,
N-Cycloheptylmethyl-6-(3-bromo-phenylamino)-4-trifluoromethyl-nicotinamide,
N-(Tetrahydropyran-4-ylmethyl)-6-(3-bromo-phenylamino)-4-trifluoromethyl-nicotinamide,
N-Cyclobutyl-6-(3-bromo-phenylamino)-4-trifluoromethyl-nicotinamide,
N-Cyclobutylmethyl-6-(3-bromo-phenylamino)-4-trifluoromethyl-nicotinamide,
N-Isobutyl-6-(3-bromo-phenylamino)-4-trifluoromethyl-nicotinamide,
N-Cyclopentylmethyl-6-(3-bromo-phenylamino)-4-trifluoromethyl-nicotinamide,
N-Cyclopropylmethyl-6-(3-bromo-phenylamino)-4-trifluoromethyl-nicotinamide,
N-Cyclobutylmethyl-6-(2-fluoro-phenylamino)-4-trifluoromethyl-nicotinamtde,
N-Cycloheptylmethyl-6-(3-fluoro-phenylamino)-4-trifluoromethyl-nicotinamide,
N-(Tetrahydropyran-4-ylmethyl)-6-(3-fluoro-phenylamino)-4-trifluoromethyl-nicotinamide,
N-Cyclobutyl-6-(3-fluoro-phenylamino)-4-trifluorornethyl-nicotinamide,
N-Cyclohexylmethyl-6-(3-fluoro-phenylamino)-4-trifluoromethyl-nicotinamide,
N-Cyclobutylmethyl-6-(3-tluoro-phenylamino)-4-trifluoromethyl-nicotinamide,
N-Cyclopentylmethyl-6-(3-fluoro-phenylamino)-4-trifluoromethyl-nicotinamide,
N-Isobutyl-6-(3-fluoro-phenylamino)-4-trifluoromethyl-nicotinamide,
N-Cyclopropylmethyl-6-(3-fluoro-phenylamino)-4-trifluoromethyl-nicotinamide,
N-(1,1-Dioxo-tetrahydro-thiophen-3-ylmethyl)-6-(3-fluoro-phenylamino)-4-trifluoromethyl-nicotinamide,
N-Cyclobutylmethyl-6-(4-fluoro-phenylamino)-4-trifluoromethyl-nicotinamide,
N-(Tetrahydropyran-4-ylmethyl)-6-(2,3-dichloro-phenylamino)-4-trifluoromethyl-nicotinamide.
N-Cyclohexylmethyl-6-(2,3-dichloro-phenylamino)-4-trifluoromethyl-nicotinamide,
N-Cyclohexylmethyl-6-(2,3-dichloro-phenylamino)-4-trifluoromethyl-nicotinamide,
N-Cycohexylmethyl-6-(2,4-dichloro-phenylamino)-4-trifluoromethyl-nicotinamide.
N-Cycloheptylmethyl-6-(2,4-dichloro-phenylamino)-4-tritluoromethyl-nicotinamide,
N-Cyclobutyl-6-(2,4-dichloro-phenylamino)-4-trifluoromethyl-nicotinamide.
N-Cyclopentylmethyl-6-(2,4-dichloro-phenylamino)-4-trifluoromethyl-nicotinamide,
N-Cyclobutylmethyl-6-(2,4-dichloro-phenylamino)-4-trifluoromethyl-nicotinamide,
N-lsobutyl-6-(2,4-dichloro-phenylamino)-4-trifluoromethyl-nicotinamide,
N-Cyclopropylmethyl-6-(2,4-dichloro-phenylamino)-4-trifluoromethyl-nicotinamide,
N-(1,1-Dioxo-tetrahydro-thiophen-3-ylmethyl)-6-(2,4-dichloro-phenylamino)-4-trifluoromethyl-nicotinamide,
N-Cyclohexylmethyl-6-(3,5-dichloro-phenylamino)-4-trifluoromethyl-nicotinamide,
N-(Tetrahydropyran-4-ylmethyl)-6-(3,5-dichloro-phenylamino)-4-trifluoromethyl-nicotinamide,
N-Cyclobutyl-6-(3,5-dichloro-phenylamino)-4-trifluoromethyl-nicotinamide,
N-Cyclopentylmethyl-6-(3,5-dichloro-phenylamino)-4-trifluoromethyl-nicotinamide,
N-Cyclobutylmethyl-6-(3,5-dichloro-phenylamino)-4-trifluoromethyl-nicotinamide,
N-Isobutyl-6-(3,5-dichloro-phenylamino)-4-trifluoromethyl-nicotinamide,
N-Cyclopropylmethyl-6-(3,5-dichloro-phenylamino)-4-trifluoromethyl-nicotinamide,
N-Isobutyl-6-(3,4-dichloro-phenylamino)-4-trifluoromethyl-nicotinamide,
N-Cyclobutyl-6-(3,4-dichloro-phenylamino)-4-trifluoromethyl-nicotinamide,
N-(Tetrahydropyran-4-ylmethyl)-6-(3,4-dichloro-phenylamino)-4-trifluoromethyl-nicotinamide,
N-Cyclopentylmethyl-6-(3,4-dichloro-phenylamino)-4-trifluoromethyl-nicotinamide,

N-Cyclobutylmethyl-6-(3,4-dichloro-phenylamino)-4-trifluoromethyl-nicotinamide,
N-Cyclopropylmethyl-6-(3,4-dichloro-phenylamino)-4-trifluoromethyl-nicotinamide,
N-Cyclohexylmethyl-6-(3,4-dichloro-phenylamino)-4-trifluoromethyl-nicotinamide,
N-Cyclobutylmethyl-6-(2-fluoro-4-chloro-phenylamino)-4-trifluoromethyl-nicotinamide,
N-Cyclopentylmethyl-6-(2-fluoro-4-chloro-phenylamino)-4-trifluoromethyl-nicotinamide,
N-(Tetrahydropyran-4-ylmethyl)-6-(2-fluoro-4-chloro-phenylamino)-4-trifluoromethyl-nicotinamide,
N-Cyclobutylmethyl-6-(2-chloro-4-fluoro-phenylamino)-4-trifluoromethyl-nicotinamide.
N-Cyclopentylmethyl-6-(2-chloro-4-fluoro-phenylamino)-4-trifluoromethyl-nicotinamide,
N-(Tetrahydropyran-4-ylmethyl)-6-(2-chloro-4-fluoro-phenylamino)-4-trifluoromethyl-nicotinamide,
N-Cyclobutylmethyl-6-(2,4-difluoro-phenylamino)-4-trifluoromethyl-nicotinamide,
N-Cyclopentylmethyl-6-(2,4-difluoro-phenylamino)-4-trifluoromethyl-nicotinamide,
N-(Tetrahydropyran-4-ylmethyl)-6-(2-methoxy-5-chloro-phenylamino)-4-trifluoromethyl-nicotinamide,
N-Cyclobutylmethyl-6-(2-methoxy-5-chloro-phenylamino)-4-trifluoromethyl-nicotinamide,
N-(Tetrahydropyran-4-ylmethyl)-6-(2-hydroxy-5-chloro-phenylamino)-4-trifluoromethyl-nicotinamide,
N-Cyclohexylmethyl-6-(2-methyl-4-chloro-phenylamino)-4-trifluoromethyl-nicotinamide,
N-(Tetrahydropyran4-ylmethyl)-6-(2-methyl-4-chloro-phenylamino)-4-trifluoromethyl-nicotinamide,
N-(Tetrahydropyran-4-ylmethyl)-6-phenylamino4-trifluoromethyl-nicotinamide,
N-Cyclopropylmethyl-6-phenylamino-4-trifluoromethyl-nicotinamide,
N-Cycloheptylmethyl-6-(3,5-dichloro-phenylamino)-4-trifluoromethyl-nicotinamide,
N-(Tetrahydropyran-4-ylmethyl)-6-(2-methyl-5-chloro-phenylamino)-4-trifluoromethyl-nicotinamide,
N-Cyclobutylmethyl-6-(2-hydroxy-5-chloro-phenylamino)-4-trifluoromethyt-nicotinamide,
N-(5-Oxo-pyrrolidin-3-ylmethyl)-6-(2,4-dichloro-phenylamino)-4-trifluoromethyl-nicotinamide,
N-Cycloheptylmethyl-6-phenylamino-4-trifluoromethyl-nicotinamide,
N-Cyclobutyl-6-phenylamino-4-trifluoromethyl-nicotinamide,
N-lsobutyl-6-phenylamino-4-trifluoromettiyl-nicotinamids,
N-(3-Dimethylamino-2,2-dimethyl-propyl)-6-(3-chloro-phenylamino)-4-trifluoromethyl-nicotinamide,
N-(3-Hydroxy-2,2-dimethyl-propyl)-6-(3-chloro-phenylamino)-4-trifluoromethyl-nicotinamide,
N-(2-Methoxy-2-methyl-propyl)-6-(3-chloro-phenylamino)-4-trifluoromethyl-nicotinamide,
N-([1,4]dioxan-2-ylmethyl)-6-(3-chloro-phenylamino)-4-trifluoromethyl-nicotinamide,
N-(Piperidin-2-ylmethyl)-6-(3-chloro-phenylamino)-4-trifluoromethyl-nicotinamide,
N-(1-Benzyl-5-oxo-pyrrolidin-3-ylmethyl)-6-(3-chloro-phenylamino)-4-trifluoromethyl-nicotinamide,
N-(5-Oxo-pyrrolidin-3-ylmethyl)-6-(3-chloro-phenylamino)-4-trifluoromethyl-nicotinamide,
N-Methylcarbamoylmethyl-6-(3-chloro-phenylamino)-4-trifluoromethyl-nicotinamide,
N-(1-Ethyl-pyrrolidin-2-ylmethyl)-6-(3-chloro-phenylamino)-4-trifluoromethyl-nicotinamide,
N-(2,2,6,6-Tetramethyl-piperidin-4-ylmethyl)-6-(3-chloro-phenylamino)4-trifluoromethyl-nicotinamide,
N-(2,2-Dimethyl-[1,3]dioxolan-4-ylmethyl)-6-(3-chloro-phenylamino)-4-trifluoromethyl-nicotinamide,
N-(Tetrahydropyran-4-ylmethyl)-6-(2-fluoro-phenylamino)-4-trifluoromethyl-nicotinamide,
N-(Tetrahydropyran-4-ylmethyl)-6-(4-fluoro-phenylamino)-4-trifluoromethyl-nicotinamide,
N-(3-Dimethylamino-2,2-dimethyl-propyl)-6-(2,4-dichloro-phenylamino)-4-trifluoromethyl-nicotinamide,
N-([1,4]dioxan-2-ylmethyl)-6-(2,4-dichloro-phenylamino)-4-trifluoromethyl-nicotinamide,
N-(5-Oxo-pyrrolidin-3-ylmethyl)-6-(2,4-dichloro-phenylamino)-4-trifluoromethyl-nicotinamide,
N-Methylcarbamoylmethyl-6-(2,4-dichloro-phenylamino)-4-trifluoromethyl-nicotinamide,
N-(2,2-Dimethyl-[1,3]dioxolan-4-ylmethyl)-6-(2,4-dichloro-phenylamino)-4-trifluoromethyl-nicotinamide,
N-Cycloheptylmethyl-6-(3,4-dichloro-phenylamino)-4-trifluoromethyl-nicotinamide,
N-(Tetrahydropyran-4-ylmethyl)-6-(2,4-difluoro-phenylamino)-4-trifluoromethyl-nicotinamide,
N-Cyclohexylmethyl-6-(2-methyl-5-chloro-phenylamino)-4-trifluoromethyl-nicotinamide,
N-(2,3-Dihydroxy-propyl)-6-(3-chloro-phenylamino)-4-trifluoromethyl-nicotinamide,
N-(2,3-Dihydroxy-propyl)-6-(2,4-dichloro-phenylamino)-4-trifluoromethyl-nicotinamide,
6-(3-Chloro-phenylamino)-N-(tetrahydro-pyran-4-ylmethyl)-2-trifluoromethyl-nicotinamide,
6-(3-Chloro-phenylamino)-N-cyclohexylmethyl-2-trifluoromethyl-nicotinamide,
6-(3-Chloro-phenylamino)-N-cyclobutylmethyl-2-trifluoromethyl-nicotinamide,
6-(3-Chloro-phenylamino)-N-cyclopentylmethyl-2-trifluoromethyl-nicotinamide,
6-(3-Chloro-phenylamino)-2-isopropyl-N-(tetrahydro-pyran-4-ylmethyl)-nicotinamide,
6-(3-Chloro-phenylamino)-N-(1,1-dioxo-tetrahydrothiophen-3-ylmethyl)-2-isopropyl-nicotinamide,
6-(3-Chloro-phenylamino)-N-cyclopentylmethyl-2-isopropyl-nicotinamide,
6-(3-Chloro-phenylamino)-N-cydohexylmetoyl-2-isopropyl-nicotinamide,
6-(2,4-Dichloro-pheriylamino)-N-cydobutylmethyl-2-isopropyl-nicotinamide,
6-(2,4-Dichloro-phenylamino)-N-cyclopentylmethyl-2-isopropyl-nicotinamide,

6-(2,4-Dichloro-phenylamino)-N-(tetrahydro-pyran-4-ylmethyl)-2-isopropyl-nicotinamide,
6-(2,4-Dichloro-phenylamino)-N-(1,1-dioxo-tetrahydrothiophen-3-ylmethyl)-2-isopropyl-nicotinamide,
6-(3-Fluoro-phenylamino)-N-cyclobutylmethyl-2-isopropyl-nicotinamide,
6-(3-Fluoro-phenylamino-)-N-cyclopentylmethyl-2-isopropyl-nicotinamide,
6-(3-Fluoro-phenylamino)-N-(tetrahydro-pyran-4-ylmethyl)-2-isopropyl-nicotinamide,
6-(3-Fluoro-phenylamino)-N-(1,1-dioxo-tetrahydrothiophen-3-ylmethyl)-2-isopropyl-nicotinamide.
6-(4-Cyano-2-methyl-phenylamino)-4-isopropyl-N-(tetrahydro-pyran-4-ylmethyl)-nicotinamide,
6-(5-Chloro-2-cyano-phenylamino)-4-isopropyl-N-(tetrahydro-pyran-4-ylmethyl)-nicotinamide,
6-(2-cyano-5-methyl-phenylamino)-4-isopropyl-N-(tetrahydro-pyran-4-ylmethyl)-nicotinamide,
6-(3-Chloro-phenylamino)-N-(1,1-dioxo-tetrahydro-1/$^{6}$-thiophen-3-ylmethyl)-4-isopropyl-nicotinamide,
N-Cyclobutylmethyl-4-isopropyl-6-(3-trifluoromethoxy-phenylamino)-nicotinamide,
6-(2,3-Difluoro-phenylamino)-4-isopropyl-N-(tetrahydro-pyran-4-ylmethyl)-nicotinamide,
6-(3,5-Bis-trifluoromethyl-phenylamino)-4-isopropyl-N-(tetrahydro-pyran-4-ylmethyl)-nicotinamide.
6-(2,4-Difluoro-phenylamino)-4-isopropyl-N-(tetrahydro-pyran-4-ylmethyl)-nicotinamide,
6-(3-Ethynyl-phenylamino)-4-isopropyl-N-(tetrahydro-pyran-4-ylmethyl)-nicotinamide,
6-(2-Fluoro-4-trifluoromethyl-phenylamino)-N-cyclopentylmethyl-4-isopropyl-nicotinamide,
6-(3-cyano-4-methyl-phenylamino)-4-isopropyl-N-(tetrahydropyran-4-ylmethyl)-nicotinamide,
6-(3-cyano-4-fluoro-phenylamino)-4-isopropyl-N-(tetrahydropyran-4-ylmethyl)-nicotinamide,
6-(3-bromo-4-trifluoromethoxy-phenylamino)-4-isopropyl-N-(tetrahydropyran-4-ylmethyl)-nicotinamide,
6-(4-Chloro-2-fluoro-phenylamino)-N-cyclobutylmethyl-4-isopropyl-nicotinamide,
6-(5-Bromo-2-methyl-phenylamino)-4-isopropyl-N-(tetrahydro-pyran-4-ylmethyl)-nicotinamide,
6-(2-Bromo-5-fluoro-phenylamino)-4-isopropyl-N-(tetrahydro-pyran-4-ylmethyl)-nicotinamide,
6-(2-Fluoro-5-trifluoromethyl-phenylamino)-4-isopropyl-N-(tetrahydro-pyran-4-ylmethyl)-nicotinamide,
6-(2-Chloro-5-trifluoromethyl-phenylamino)-4-isopropyl-N-(tetrahydro-pyran-4-ylmethyl)-nicotinamide,
6-(2-Bromo-5-trifluoromethyl-phenylamino)-4-isopropyl-N-(tetrahydro-pyran-4-ylmethyl)-nicotinamide,
6-(3-Bromo-4-cyano-phenylamino)-4-isopropyl-N-(tetrahydro-pyran-4-ylmethyl)-nicotinamide,
6-(2-Bromo-4-trifluoromethoxy-phenylamino)-4-isopropyl-N-(tetrahydro-pyran-4-ylmethyl)-nicotinamide,
6-(3-Chloro-2-methyl-phenylamino)-4-isopropyl-N-(tetrahydro-pyran-4-ylmethyl)-nicotinamide,
6-(3,5-Difluoro-phenyfamino)-4-isopropyl-N-(tetrahydro-pyran-4-ylmethyl)-nicotinamide,
6-(2-Chloro-4-fluoro-phenylamino)-4-isopropyl-N-(tetrahydro-pyran-4-ylmethyl)-nicotinamide,
6-(4-Chloro-2-methyl-phenylamino)-4-isopropyl-N-(tetrahydro-pyran-4-ylmethyl)-nicotinamide,
6-(2-Fluoro-3-trifluoromethyl-phenylamino)-N-cyclopentylmethyl-4-isopropyl-nicotinamide,
6-(2-Methyl-4-chloro-phenylamino)-N-cyclopentylmethyl-4-isopropyl-nicotinamide,
6-(3-Chloro-4-cyano-phenylamino)-N-cyclopentylmethyl-4-isopropyl-nicotinamide,
6-(4-bromo-2-chloro-phenylamino)-N-cyclopentylmethyl-4-isopropyl-nicotinamide,
N-Cyclobutylmethyl-6-(2,4-difluoro-phenylamino)-4-isopropyl-nicotinamide,
N-Cyclobutylmethyl-6-(2,4-dichloro-phenylamino)-4-isopropyl-nicotinamide,
N-Cyclobutylmethyl-6-(3,4-dichloro-phenylamino)-4-isopropyl-nicotinamide,
N-Cyclobutylmethyl-6-(2,3-dichloro-phenylamino)-4-isopropyl-nicotinamide,
6-(2-Chloro-4-fluoro-phenylamino)-N-cyclobutylmethyl-4-isopropyl-nicotinamide.
6-(3-Chloro-4-fluoro-phenylamino)-N-cyclobutylmethyl-4-isopropyl-nicotinamide,
6-(4-Bromo-2-chloro-phenylamino)-N-cyclobutylmethyl-4-isopropyl-nicotinamide,
6-(2-Bromo-4-chloro-phenylamino)-N-cyclobutylmethyl-4-isopropyl-nicotinamide,
N-cyclobutylmethyl-6-(2-fluoro-3-trifluoromethyl-phenylamino)-4-isopropyl-nicotinamide,
6-(4-Chloro-2-methyl-phenylamino)-N-cyclobutylmethyl-4-isopropyl-nicotinamide,
6-(2-Chloro-4-cyano-phenylamino)-N-cyclobutylmethyl-4-isopropyl-nicotinamide,
6-(4-Cyano-2-fluoro-phenylamino)-N-cyclobutylmethyl-4-isopropyl-nicotinamide,
6-(4-Cyano-2-methyl-phenylamino)-N-cyclobutylmethyl-4-isopropyl-nicotinamide,
6-(2-Chloro-4-trifluoromethyl-phenylamino)-N-cyclobutylmethyl-4-isopropyl-nicotinamide,
N-Cyclobutylmethyl-6-(3,5-dichloro-phenylamino)-4-isopropyl-nicotinamide,
N-Cyclobutylmethyl-6-(2,5-dichloro-phenylamino)-4-isopropyl-nicotinamide,
N-Cyclobutylmethyl-6-(3,5-difluoro-phenylamino)-4-isopropyl-nicotinamide,
6-(5-Chloro-2-fluoro-phenylamino)-N-cyclobutylmethyl-4-isopropyl-nicotinamide,
6-(2-Chloro-5-fluoro-phenylamino)-N-cyclobutylmethyl-4-isopropyl-nicotinamide,
6-(2-Chloro-5-fluoro-phenylamino)-4-isopropyl-N-(tetrahydro-pyran-4-ylmethyl)-nicotinamide,
6-(2-Chloro-5-methyl-phenylamino)-4-isopropyl-N-(tetrahydro-pyran-4-ylmethyl)-nicotinamide,
6-(2-Fluoro-5-methyl-phenylamino)-4-isopropyl-N-(tetrahydro-pyran-4-ylmethyl)-nicotinamide,
6-(5-Fluoro-2-methyl-phenylamino)-4-isopropyl-N-(tetrahydro-pyran-4-ylmethyl)-nicotinamide,

6-(3-Bromo-2-methyl-phenylamino)-4-isopropyl-N-(tetrahydro-pyran-4-ylmethyl)-nicotinamide,
4-Isopropyl-6-(2-methyl-4-trifluoromethoxy-phenylamino)-N-(tetrahydro-pyran-4-ylmethyl)-nicotinamide,
6-(3-Fluoro-2-methyl-phenylamino)-4-isopropyl-N-(tetrahydro-pyran-4-ylmethyl)-nicotinamide,
6-(3-Bromo-5-trifluoromethyl-phenylamino)-4-isopropyl-N-(tetrahydro-pyran-4-ylmethyl)-nicotinamide,
6-(4-Cyano-phenylamino)-4-isopropyl-N-(tetrahydro-pyran-4-ylmethyl)-nicotinamide,
6-(4-Chloro-2-fluoro-phenylamino)-4-isopropyl-N-(tetrahydro-pyran-4-ylmethyl)-nicotinamide,
6-(4-bromo-2-fluoro-phenylamino)-N-cyclopentylmethyl-4-isopropyl-nicotinamide,
6-(2-Bromo-4-trifluoromethoxy-phenylamino)-N-cyclobutylmethyl-4-isopropyl-nicotinamide,
N-Cyclobutylmethyl-6-(2-fluoro-4-trifluoromethyl-phenylamino)-4-isopropyl-nicotinamide,
6-(4-Cyano-2-fluoro-phenylamino)-N-cyclopentylmethyl-4-isopropyl-nicotinamide,
6-(4-Bromo-3-trifluoromethyl-phenylamino)-4-isopropyl-N-(tetrahydropyran-4-ylmethyl)-nicotinamide,
6-(4-Fluoro-3-trifluoromethyl-phenylamino)-4-isopropyl-N-(tetrahydropyran-4-ylmethyl)-nicotinamide,
6-(3,4-Dibromo-phenylamino)-4-isopropyl-N-(tetrahydropyran-4-ylmethyl)-nicotinamide,
6-(4-Bromo-3-fluoro-phenylamino)-4-isopropyl-N-(tetrahydropyran-4-ylmethyl)-nicotinamide,
6-(2-Chloro-4-trifluoromethyl-phenylamino)-N-cyclopentylmethyl-4-isopropyl-nicotinamide,
6-(3,4-Difluoro-phenylamino)-4-isopropyl-N-(tetrahydropyran-4-ylmethyl)-nicotinamide,
6-(4-Chloro-3-trifluoromethyl-phenylamino)-4-isopropyl-N-(tetrahydropyran-4-ylmethyl)-nicotinamide,
6-(4-Methyl-3-trifluoromethyl-phenylamino)-4-isopropyl-N-(tetrahydropyran-4-ylmethyl)-nicotinamide,
6-(2-Chloro-4-trifluoromethoxy-phenylamino)-4-isopropyl-N-(tetrahydropyran-4-ylmethyl)-nicotinamide,
6-(2-Cyano-3-methyl-phenylamino)-4-isopropyl-N-(tetrahydropyran-4-ylmethyl)-nicotinamide,
6-(3-Chloro-2-cyano-phenylamino)-4-isopropyl-N-(tetrahydropyran-4-ylmethyl)-nicotinamide,
6-(3-Chloro-phenylamino)-4-(1-hydroxy-methyl-ethyl)-N-(tetrahydropyran-4-ylmethyl)-nicotinamide, and
4-*tert*-Butyl-6-(3,4-dichloro-phenylamino)-N-(tetrahydro-pyran-4-ylmethyl)-nicotinamide,

and pharmaceutically acceptable derivatives thereof.

**7.** A compound as claimed in claim 1 selected from:

6-(3-Chloro-phenyl-amino)-4-isopropyl-N-(tetrahydro-pyran-4-ylmethyl)-nicotinamide;
6-(3-Bromo-phenyl-amino)-4-isopropyl-N-(tetrahydro-pyran-4-ylmethyl)-nicotinamide;
6-(2,4-Dichloro-phenylamino)-4-isopropyl-N-(tetrahydro-pyran-4-ylmethyl)-nicotinamide;
4-Isopropyl-N-(tetrahydro-pyran-4-ylmethyl)-6-(3-trifluoromethoxy-phenylamino)-nicotinamide;
4-*tert*-Butyl-6-(2,4-di-chloro-phenylamino)-N-(tetrahydro-pyran-4-ylmethyl)-nicotinamide;
6-(3-Chloro-4-cyano-phenylamino)-4-isopropyl-N-(tetrahydro-pyran-4-ylmethyl)-nicotinamide;
6-(2-Fluoro-3-trifluoromethyl-phenylamino)-4-isopropyl-N-(tetrahydro-pyran-4-ylmethyl)-nicotinamide;
6-(4-Bromo-2-chloro-phenylamino)-4-isopropyl-N-(tetrahydro-pyran-4-ylmethyl)-nicotinamide;
6-(3,4-Dichloro-phenylamino)-4-isopropyl-N-(tetrahydro-pyran-4-ylmethyl)-nicotinamide;
6-(2-Bromo-4-trifluoromethoxy-phenylamino)-4-isopropyl-*N*-(tetrahydro-pyran-4-ylmethyl)-nicotinamide;
6-(3,5-Difluoro-phenylamino)-4-isopropyl-*N*-(tetrahydro-pyran-4-ylmethyl)-nicotinamide;
6-(2,4-Dichloro-phenylamino)-N-(tetrahydro-pyran-4-ylmethyl)-4-trifluoromethyl-nicotinamide;

or a pharmaceutically acceptable derivative thereof.

**8.** A pharmaceutical composition comprising a compound as claimed in any preceding claim or a pharmaceutically acceptable derivative thereof.

**9.** A pharmaceutical composition as claimed in claim 8 further comprising a pharmaceutical carrier or diluent thereof.

**10.** Use of a therapeutically effective amount of a compound of formula (I) as claimed in any one of claims 1 to 7 or a pharmaceutically acceptable derivative thereof for the manufacture of a medicament for the treatment of a condition which is mediated by the activity of cannabinoid 2 receptors.

**11.** The use as claimed in claim 10 wherein the condition is an immune disorder, an inflammatory disorder, pain, rheumatoid arthritis, multiple sclerosis, osteoarthritis or osteoporosis.

**12.** The use as claimed in claim 11 wherein the pain is selected from inflammatory pain, visceral pain, cancer pain, neuropathic pain, lower back pain, muscular skeletal pain, post operative pain, acute pain and migraine.

## Patentansprüche

1. Verbindung der Formel (I):

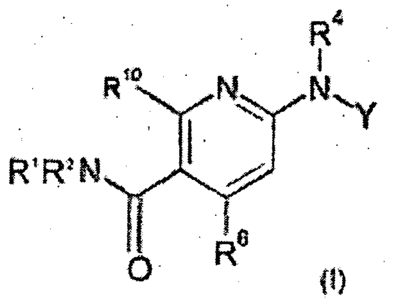

(I)

wobei:

Y Phenyl ist, unsubstituiert oder substituiert mit 1, 2 oder 3 Substituenten, ausgewählt aus $C_{1-6}$-Alkyl, Halogen-substituiertem $C_{1-6}$-Alkyl, $C_{1-6}$-Alkoxy, einer Hydroxylgruppe, einer Cyanogruppe, Halogen, einem $C_{1-6}$-Alkylsulfonylrest, -$CONH_2$, -$NHCOCH_3$, -COOH, Halogen-substituiertem $C_{1-6}$-Alkoxy, $SO_2NR^{8a}R^{8b}$, wobei $R^{8a}$ und $R^{8b}$ jeweils unabhängig ausgewählt sind aus H, $C_{1-6}$-Alkyl oder $C_{1-6}$-Alkinyl;
$R^1$ ausgewählt ist aus Wasserstoff, $C_{1-6}$-Alkyl, $C_{3-6}$-Cycloalkyl oder Halogensubstitüiertem $C_{1-6}$-Alkyl;
$R^2$ $(CH_2)_mR^3$ ist, wobei m 0 oder 1 ist;
oder $R^1$ und $R^2$ zusammen mit dem N, an welches sie gebunden sind, einen 4- bis 8-gliedrigen nicht-aromatischen heterocyclischen Ring bilden, welcher unsubstituiert oder substituiert mit 1, 2 oder 3 Substituenten, ausgewählt aus: $C_{1-6}$-Alkyl, $C_{1-6}$-Alkoxy, einer Hydroxylgruppe, einer Cyanogruppe, Halogen, einer Sulfonylgruppe, Methylsulfonyl, $NR^{8a}R^{8b}$, $CH_2$-Phenyl, $NHCOCH_3$, (=O), $CONHCH_3$ oder $NHSO_2CH_3$, wobei $R^{8a}$ und $R^{8b}$ wie oben definiert sind, ist;
$R^3$ ein 4- bis 8-gliedriger nicht-aromatischer heterocyclischer Rest, ein $C_{3-8}$-Cycloalkylrest, ein geradkettiges oder verzweigtes $C_{1-10}$-Alkyl, ein $C_{2-10}$-Alkenyl, ein $C_{3-8}$-Cycloalkenyl, ein $C_{2-10}$-Alkinyl oder ein $C_{3-8}$-Cycloalkinyl ist, von denen jedes unsubstituiert oder substituiert sein kann mit 1, 2 oder 3 Substituenten, vorzugsweise ausgewählt aus: $C_{1-6}$-Alkyl, $C_{1-6}$-Alkoxy, einer Hydroxylgruppe, einer Cyanogruppe, Halogen, einer Sulfonylgruppe, Methylsulfonyl, $NR^{8a}R^{8b}$, $CH_2$-Phenyl, $NHCOCH_3$, (=O), $CONHCH_3$ oder $NHSO_2CH_3$, wobei $R^{8a}$ und $R^{8b}$ wie oben definiert sind, oder $R^5$;
$R^4$ ausgewählt ist aus Wasserstoff, $C_{1-6}$-Alkyl, $C_{3-6}$-Cycloalkyl oder Halogen-substituiertem $(C_{1-6})$-Alkyl, $COCH_3$ oder $SO_2Me$; $R^5$

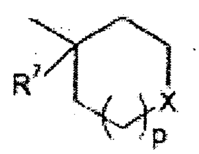

ist, wobei p 0, 1 oder 2 ist und X $CH_2$, O oder S ist;
$R^6$ Chlor oder $(C_{1-6})$-Alkyl ist, welches gegebenenfalls mit 1, 2 oder 3 Substituenten, ausgewählt aus Hydroxyl, $C_{1-6}$-Alkyloxy, Cyano, Halogen, $NR^{8a}R^{8b}$, $CONR^{8a}R^{8b}$, $SO_2NR^{8a}R^{8b}$, $NR^{8a}COR^{8b}$ oder $NR^{8a}SO_2R^{8b}$, substituiert ist, wobei $R^{8a}$ und $R^{8b}$ wie oben definiert sind und $R^{10}$ Wasserstoff ist oder $R^{10}$ Chlor oder $(C_{1-6})$-Alkyl ist, welches gegebenenfalls mit 1, 2 oder 3 Substituenten, ausgewählt aus Hydroxyl, $C_{1-6}$-Alkyloxy, Cyano, Halogen, $NR^{8a}R^{8b}$, $CONR^{8a}R^{8b}$, $SO_2NR^{8a}R^{8b}$, $NR^{8a}COR^{8b}$ oder $NR^{8a}SO_2R^{8b}$, substituiert ist, wobei $R^{8a}$ und $R^{8b}$ wie oben definiert sind und $R^6$ Wasserstoff ist;
$R^7$ OH, $C_{1-6}$-Alkoxy, $NR^{8a}R^{8b}$, $NHCOR^9$, $NHSO_2R^9$ oder $SOqR^9$ ist;
$R^{8a}$ H oder $C_{1-6}$-Alkyl ist;
$R^{8b}$ H oder $C_{1-6}$-Alkyl ist,
$R^9$ $C_{1-6}$-Alkyl ist;
q 0, 1 oder 2 ist;

oder ein pharmazeutisch verträgliches Derivat davon.

**2.** Verbindung gemäß Anspruch 1 der Formel (Ia):

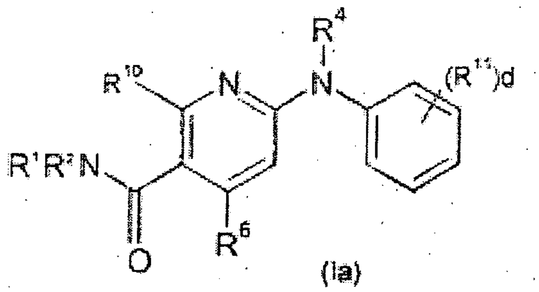

(Ia)

wobei $R^1$ ausgewählt ist aus Wasserstoff, $C_{1-6}$-Alkyl, $C_{3-6}$-Cycloalkyl oder Halogen-substituiertem $C_{1-6}$-Alkyl;
$R^2$ $(CH_2)_mR^3$ ist, wobei m 0 oder 1 ist;
oder $R^1$ und $R^2$ zusammen mit dem N, an welches sie gebunden sind, einen nicht-aromatischen heterocyclischen Ring bilden, ausgewählt aus Azetidinyl, Pyrrolidinyl, Morpholinyl, Piperazinyl, Piperidinyl, Tetrahydropyridinyl, Azapin, Oxapin, Azacyclooctanyl, Azaoxacyclooctanyl und Azathiacyclooctanyl, von denen jedes unsubstituiert oder substituiert sein kann mit 1, 2 oder 3 Substituenten, ausgewählt aus $C_{1-6}$-Alkyl, $C_{1-6}$-Alkoxy, Hydroxyl, Cyano, Halogen, Sulfonyl, Methylsulfonyl, $NR^{8a}R^{8b}$, $CH_2$-Phenyl, $NHCOCH_3$, (=O), $CONHCH_3$ und $NHSO_2CH_3$;
$R^3$ 2- oder 3-Azetidinyl, Oxetanyl, Thioxetanyl, Thioxetanyl-S-oxid, Thioxetanyl-S,S-dioxid, Dioxalanyl, Pyrrolidinyl, Tetrahydrofuranyl, Tetrahydrothiophenyl, Tetrahydrothiophenyl-S,S-dioxid, Morpholinyl, Piperidinyl, Piperazinyl, Tetrahydropyranyl, Tetrahydrothiopyranyl, Thiomorpholinyl, Thiomorpholinyl-S,S-dioxid, Tetrahydropyridinyl, Dioxanyl, Tetrahydrothiopyran-1,1-dioxid, Azapin, Oxapin, Azacyclooctanyl, Azaoxacyclooctanyl, Azathiacyclooctanyl, Oxacyclooctanyl, Thiacyclooctanyl, ein $C_{3-8}$-Cycloalkylrest, ein geradkettiges oder verzweigtes $C_{1-10}$-Alkyl, ein $C_{2-10}$-Alkenyl, ein $C_{3-8}$-Cycloalkenyl, ein $C_{2-10}$-Alkinyl oder ein $C_{3-8}$-Cycloalkinyl ist, von denen jedes unsubstituiert oder substituiert sein kann mit 1, 2 oder 3 Substituenten, ausgewählt aus $C_{1-6}$-Alkyl, $C_{1-6}$-Alkoxy, Hydroxyl, Cyano, Halogen, Sulfonyl, Methylsulfonyl, $NR^{8a}R^{8b}$, $CH_2$-Phenyl, $NHCOCH_3$, (=O), $CONHCH_3$ und $NHSO_2CH_3$;
oder $R^3$ $R^5$ ist;
$R^4$ ausgewählt ist aus Wasserstoff, $C_{1-6}$-Alkyl, $C_{3-6}$-Cycloalkyl oder Halogen-substituiertem $C_{1-6}$-Alkyl, $COCH_3$ oder $SO_2Me$;
$R^5$

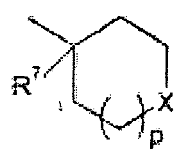

ist, wobei p 0, 1 oder 2 ist und X $CH_2$, O oder S ist;
$R^6$ Chlor oder $(C_{1-6})$-Alkyl ist, welches gegebenenfalls mit 1, 2 oder 3 Substituenten substituiert ist, ausgewählt aus Hydroxyl, $C_{1-6}$-Alkyloxy, Cyano, Halogen, $NR^{8a}R^{8b}$, $CONR^{8a}R^{8b}$, $SO_2NR^{8a}R^{8b}$, $NR^{8a}COR^{8b}$ oder $NR^{8a}SO_2R^{8b}$, wobei $R^{8a}$ und $R^{8b}$ wie oben definiert sind und $R^{10}$ Wasserstoff ist oder $R^{10}$ Chlor oder $(C_{1-6})$-Alkyl ist, welches gegebenenfalls mit 1, 2 oder 3 Substituenten, ausgewählt aus Hydroxyl, $C_{1-6}$-Alkyloxy, Cyano, Halogen, $NR^{8a}R^{8b}$, $CONR^{8a}R^{8b}$, $SO_2NR^{8a}R^{8b}$, $NR^{8a}COR^{8b}$ oder $NR^{8a}SO_2R^{8b}$ substituiert ist, wobei $R^{8a}$ und $R^{8b}$ wie oben definiert sind und $R^6$ Wasserstoff ist;
$R^7$ OH, $C_{1-6}$-Alkoxy, $NR^{8a}R^{8b}$, $NHCOR^9$, $NHSO_2R^9$ oder $SOqR^9$ ist;
$R^{8a}$ H oder $C_{1-6}$-Alkyl ist;
$R^{8b}$ H oder $C_{1-6}$-Alkyl ist;
$R^9$ $C_{1-6}$-Alkyl ist;
$R^{11}$ $C_{1-6}$-Alkyl Halogen-substituiertes $C_{1-6}$-Alkyl, $C_{1-6}$-Alkoxy, Hydroxyl, Cyano, Halogen, $C_{1-6}$-Alkylsulfonylrest, $-CONH_2$, $-NHCOCH_3$, -COOH, Halogen-substituiertes
$C_{1-6}$-Alkoxy, $SO_2NR^{8a}R^{8b}$ oder $C_{1-6}$-Alkinyl ist;
q 0, 1 oder 2 ist;

d 0, 1,2 oder 3 ist;
oder ein pharmazeutisch verträgliches Derivat davon.

**3.** Verbindung gemäß Anspruch 1 oder 2, wobei $R^1$ Wasserstoff ist.

**4.** Verbindung gemäß einem der vorstehenden Ansprüche, wobei $R^4$ $C_{1-6}$-Alkyl oder Wasserstoff ist.

**5.** Verbindung gemäß einem der vorstehenden Ansprüche, wobei $R^6$ tert-Butyl, Isopropyl oder $CF_3$ ist.

**6.** Verbindung wie beschrieben in einem der Beispiele 1 bis 306, nämlich eine Verbindung, ausgewählt aus:

2-(3-Chlorphenylamino)-4-trifluormethylpyridin-5-carbonsäurecyclohexylmethylamid,
6-(3-Chlorphenylamino)-N-cyclohexylmethyl-4-isopropylnicotinamid,
N-Cyclohexylmethyl-6-(3,4-dichlorphenylamino)-4-isopropylnicotinamid,
6-(3-Bromphenylamino)-N-cyclohexylmethyl-4-isopropylnicotinamid,
N-Cyclohexylmethyl-6-(2,4-dichlorphenylamino)-4-isopropylnicotinamid,
6-(3-Chlorphenylamino)-N-cyclobutylrriethyl-4-isopropylnicotinamid,
6-(3-Chlorphenylamino)-4-isopropyl-N-(tetrahydropyrä.n-4-ylmethyl)-nicotinamid,
6-(3-Bromphenylamino)-4-isopropyl-N-(tetrahydropyran-4-ylmethyl)-nicotinamid,
N-Cyclohexylmethyl-4-isopropyl-6-(3-methoxyphenylamino)-nicotinamid,
N-Cyclohexylmethyl-6-(3-fluorphenylamino)-4-isopropylnicotinamid,
1-[6-(3-Chlorphenylamino)-4-isopropylpyridin-3-yl]-1-morpholin-4-yl-methanon,
6-(3-Bromphenylamino)-N-cyclohexylmethyl-4-isopropylnicotinamid,
N-Cyclohexylmethyl-4-isopropyl-6-m-tolylaminonicotinamid,
N-Cyclohexylmethyl-4-isopropyl-6-(3-trifluormethylphenylamino)-nicotinamid,
N-Cyclohexylmethyl-4-isopropyl-6-(3-trifluormethoxyphenylamino)-nicotinamid,
6-(2,3-Dichlorphenylamino)-4-isopropyl-N-(tetrahydropyran-4-ylmethyl)-nicotinamid,
6-(2,4-Dichlorphenylamino)-4-isopropyl-N-(tetrahydropyran-4-ylmethyl)-nicotinamid
6-(3,4-Dichlorphenylamino)-4-isopropyl-N-(tetrahydropyran-4-ylmethyl)-nicotinamid,
4-Isopropyl-N-(tetrahydropyran-4-ylmethyl)-6-(3-trifluormethylphenylamino)-nicotinamid,
4-Isopropyl-N-(tetrahydropyran-4-ylmethyl)-6-(3-trifluormethoxyphenylamino)-nicotinamid,
6-[(3-Chlorphenyl)amino]-N-(cyclopentylmethyl)-4-isopropylnicotinamid,
N-Cyclopentylmethyl-6-(3-fluorphenylamino)-4-isopropylnicotinamid,
N-Cyclopentylmethyl-4-isopropyl-6-(3-trifluormethylphenylamino)-nicotinamid,
N-Cyclopentylmethyl-4-isopropyl-6-m-tolylaminonicotinamid,
N-Cyclopentylmethyl-4-isopropyl-6-(3-trifluormethoxyphenylamino)-nicotinamid,
6-(3-Bromphenylamino)-N-cyclopentylmethyl-4-isopropylnicotinamid,
N-Cyclopentylmethyl-4-isopropyl-6-(3-methoxyphenylamino)-nicotinamid,
6-(3-Cyanophenylamino)-N-cyclopentylmethyl-4-isopropylnicotinamid,
6-(2-Chlor-4-fluorphenylamino)-N,-cyclopentylmethyl-4-isopropylnlcotinamid,
6-(2-Chlor-4-cyanophenylamino)-N-cyclopentylmethyl-4-isopropylnicotinamid,
N-Cyclopentylmethyl-6-(2,4-dichlorphenylamino)-4-isopropylnicotinamid,
N-Cyclopentylmethyl-6-(3,4-dichlorphenylamino)-4-isopropylnicotinamid,
6-(3-Bromphenylamino)-N-cyclobutylmethyl-4-isopropylnicotinamid,
N-Cyclobutylmethyl-6-(3-fluorphenylamino)-4-isopropylnicotinamid,
N-Cyclobutylmethyl-6-(3-trifluormethylphenylamino)-4-isopropylnicotinamid,
6-(3-Cyanophenylamino)-N-cyclobutylmethyl-4-isopropylnicotinamid,
N-Cyclobutylmethyl-4-isopropyl-6-m-tolylaminonicotinamid,
N-Cyclobutylmethyl-4-isopropyl-6-(3-methoxyphenylamino)-nicotinamid,
6-(3-Fluorphenylamino)-4-isopropyl-N-(tetrahydropyran-4-ylmethyl)-nicotinamid,
1-[6-(3-Fluorphenylamino)-4-isopropylpyridin-3-yl]-1-morpholin-4-ylmethanon,
4-Isopropyl-6-(3-methoxyphenylamino)-N-(tetrahydropyran-4-ylmethyl)-nicotinamid,
4-Isopropyl-N-(tetrahydropyran-4-ylmethyl)-6-m-tolylaminonicotinamid,
6-(3-Cyanophenylamino)-4-isopropyl-N-(tetrahydropyran-4-ylmethyl)-nicotinamid,
6-[(3,4-Dichlorphenyl)methylamino]-4-isopropyl-N-(tetrahydropyran-4-ylmethyl)-nicotinamid,
6-[(3-Bromphenyl)methylamino]-4-isopropyl-N-(tetrahydropyran-4-ylmethyl)-nicotinamid,
6-[(3-Fluorphenyl)methylamino]-4-isopropyl-N-(tetrahydropyran-4-ylmethyl)-nicotinamid,
4-Isopropyl-6-(methylphenylamino)-N-(tetrahydropyran-4-ylmethyl)-nicotinamid,

6-[(3-Chlorphenyl)methylamino]-4-isopropyl-N-(tetrahydropyran-4-ylmethyl)-nicotinamid,
6-[(4-Chlorphenyl)methylamino]-4-isopropyl-N-(tetrahydropyran-4-ylmethyl)-nicotinamid,
6-(3-Chlorphenylamino)-N-cyclobutyl-4-isopropylnicotinamid,
6-(3-Chlorphenylamino)-N-cyclopropylmethyl-4-isopropylnicotinamid,
6-(3-Chlorphenylamino)-N-(2-ethylbutyl)-4-isopropylnicotinamid,
6-(3-Chlorphenylamino)-N-cyclohexyl-4-isopropylnicotinamid,
6-(3-Chlorphenylamino)-N-(1-hydroxy-cyclohexylmethyl)-4-isopropylnicotinamid;
1-[6-(3-Chlorphenylamino)-4-isopropylpyridin-3-yl]-1-piperidin-1-ylmethanon,
6-(3-Chlorphenylamino)-N-(2,2-dimethylpropyl)-4-isopropylnicotinamid,
6-(3-Chlorphenylamino)-4-isopropyl-N-(2-methoxyethyl)-nicotinamid,
6-(3-Chlorphenylamino)-4-isopropyl-N-(tetrahydropyran-4-yl)-nicotinamid,
6-(3-Chlorphenylamino)-4-isopropyl-N-[(R)-1-(tetrahydrofuran-2-yl)methyl]nicotinamid,
N((R)-1-{1-[6-(3-Chlorphenylamino)-4-isopropylpylidin-3-yl]-methanoyl}-pyrrolidin-3-yl)-acetamid,
1-[6-(3-Chlorphenylamino)-4-isopropylpyridin-3-yl]-1-(4-methansulfonylpiperazin-1-yl)methanon,
6-(3-Chlorphenylamino)-N-(1,1-dioxotetrahydro-$1/^6$-thiophen-3-yl)-4-isopropylnicotinamid,
6-(3-Chlorphenylamino)-4-isopropyl-N-[(S)-1-(tetrahydrofuran-2-yl)methyl]nicotinamid,
6-(3-Chlorphenylamino)-N-(1,1-dioxohexahydro-$1/^6$-thiopyran-4-yl)-4-isopropylnicotinamid,
1-[6-(3-Chlorphenylamino)-4-isopropylpyridin-3-yl]-1-(4-methylpiperazin-1-yl)methanon,
6-(3-Chlorphenylamino)-N-(2-dimethylaminoethyl)-4-isopropylnicotinamid,
N-((S)-1-{1-[6-(3-Chlorphenylamino)-4-isopropylpyridin-3-yl]-methanoyl}-pyrrolidin-3-yl)-acetamid,
N-(1-{1-[6-(3-Chlorphenylamino)-4-isopropyl-pyridin-3-yl]-methanoyl}-piperidin-4-yl)-methansulfonamid,
4-tert-Butyl-6-(3-chlorphenylamino)-N-cyclohexylmethylnicotinamid,
4-tert-Butyl-6-(2,4-dichlorphenylamino)-N-cyclohexylmethylnicotinamid,
4-tert-Butyl-6-(3-chlorphenylamino)-N-(tetrahydropyran-4-ylmethyl)-nicotinamid,
4-tert-Butyl-6-(3-fluorphenylamino)-N-(tetrahydropyran-4-ylmethyl)-nicotinamid,
4-tert-Butyl-6-(2-chlor-3-fluorphenylamino)-N-(tetrahydropyran-4-ylmethyl)-nicotinamid,
4-tert-Butyl-6-(2,4-dichlorphenylamino)-N-(tetrahydropyran-4-ylmethyl)-nicotinamid,
6-(3,5-Dichlorphenylamino)-4-isopropyl-N-(tetrahydropyran-4-ylmethyl)-nicotinamid,
6-(5-Chlor-2-fluorphenylamino)-4-isopropyl-N-(tetrahydropyran-4-ylmethyl)-nicotinamid,
6-(3-Chlor-4-fluorphenylamino)-4-isopropyl-N-(tetrahydropyran-4-ylmethyl)-nicotinamid,
6-(3-Chlor-4-trifluormethoxyphenylamino)-4-isopropyl-N-(tetrahydropyran-4-ylmethyl)-nicotinamid,
6-(3-Chlor-4-cyano-phenylamino)-4-isopropyl-N-(tetrahydropyran-4-ylmethyl)-nicotinamid,
6-(3-Fluor-5-trifluormethylpheriylamino)-4-isopropyl-N-(tetrahydropyran-4-ylmethyl)-nicotinamid,
6-(2-Fluor-3-trifluormethylphenylamino)-4-isopropyl-N-(tetrahydropyran-4-ylmethyl)-nicotinamid,
6-(4-Brom-2-chlorphenylamino)-4-isopropyl-N-(tetrahydropyran-4-ylmethyl)-nicotinamid,
6-(2-Brom-4-chlorphenylamino)-4-isopropyl-N-(tetrahydropyran-4-ylmethyl)-nicotinamid,
4-Isopropyl-6-(2-methyl-3-trifluormethylphenylamino)-N-(tetrahydropyran-4-ylmethyl)-nicotinamid,
6-(3-Chlor-4-methylphenylamino)-4-isopropyl-N-(tetrahydropyran-4-ylmethyl)-nicotinamid,
6-(4-Brom-3-methylphenylamino)-4-isopropyl-N-(tetrahydropyran-4-ylmethyl)-nicotinamid,
6-(2,5-Dichlorphenylamino)-4-isopropyl-N-(tetrahydropyran-4-ylmethyl)-nicotinamid,
4-Isopropyl-6-(2-methyl-5-trifluormethylphenylamino)-N-(tetrahydropyran-4-ylmethyl)-nicotinamid,
6-(2-Brom-4-chlorphenylamino)-N-cyclopentylmethyl-4-isopropylnicotinamid,
6-(4-Brom-3-chlorphenylamino)-4-isopropyl-N-(tetrahydropyran-4-ylmethyl)-nicotinamid,
6-(4-Chlor-2-fluorphenylamino)-N-cyclopentylmethyl-4-isopropylnicotinamid,
N-Cyclopentylmethyl-6-(3-fluor-4-trifluormethylphenylamino)-4-isopropylnicotinamid,
6-(4-Cyano-2-methylphenylamino)-N-cyclopentylmethyl-4-isopropylnicotinamid,
6-(3-Chlor-2-fluorphenylamino)-4-isopropyl-N-(tetrahydropyran-4-ylmethyl)-nicotinamid,
6-(3-Fluor-4-trifluorrnethylphenylamino)-4-isopropyl-N-(tetrahydropyran-4-ylmethyl)-nicotinamid,
6-(4-Cyano-3-trifluormethylphenylamino)-4-isopropyl-N-(tetrahydropyran-4-ylmethyl)-nicotinamid,
6-(4-Cyano-2-fluorphenylamino)-4-isopropyl-N-(tetrahydropyran-4-ylmethyl)-nicotinamid,
6-(4-Fluor-3-methylphenylamino)-4-isopropyl-N-(tetrahydropyran-4-ylmethyl)-nicotinamid,
6-(5-Chlor-2-methylphenylamino)-4-isopropyl-N-(tetrahydropyran-4-ylmethyl)-nicotinamid,
6-(3-Fluor-4-methylphenylamino)-4-isopropyl-N-(tetrahydropyran-4-ylmethyl)-nicotinamid,
6-(3,4-Dimethylphenylamino)-4-isopropyl-N-(tetrahydropyran-4-ylmethyl)-nicotinamid,
6-(3-Brom-4-methylphenylamino)-4-isopropyl-N-(tetrahydropyran-4-ylmethyl)-nicotinamid,
6-(3-Chlorphenylamino)-N-(4-hydroxytetrahydropyran-4-ylmethyl)-4-isopropylnicotinamid,
6-(2,3-Dichlorphenylamino)-N-(cyclobutyl)-4-trifluormethylnicotinamid,
6-(2,4-Dichlorphenylamino)-N-(tetrahydropyran-4-ylmethyl)-4-trifluormethylnicotinamid,

6-(3-Chlorphenylamino)-N-(1,1-dioxotetrahydrothiophen-3-ylmethyl)-4-trifluormethylnicotinamid,
N-Cyclohexylmethyl-6-phenylamino-4-trifluormethylnicotinamid,
N-Cyclopentylmethyl-6-phenylamino-4-trifluormethylnicotinamid,
N-Cyclobutylmethyl-6-phenylamino-4-trifluonnethylnicotinamid,
N-Cyclobutyl-6-(3-chlorphenylamino)-4-trifluornlethylnicotinamid,
N-(Tetrahydropyran-4-methyl)-6-(3-chlorphenylamino)-4-trifluonnethylnicotinamid,
N-Cyclobutylmethyl-6-(3-chlorphenylamino)-4-trifluormethylnicotinamid,
N-Isobutyl-6-(3-chlorphenylamino)-4-trifluormethylnicotinamid,
N-Cyclopentylmethyl-6-(3-chlorphenylamino)-4-trifluonnethylnicotinamid,
N-Cyclopropylmethyl-6-(3-chlorphenylamino)-4-trifluonnethylnicotinamid,
N-Cyclohexylmethyl-6-(3-bromphenylamino)-4-trifluormethylnicotinamid,
N-Cycloheptylmethyl-6-(3-bromphenylamino)-4-trifluormethylnicotinamid,
N-(Tetrahydropyran-4-ylmethyl)-6-(3-bromphenylamino)-4-trifluormethylnicotinamid,
N-Cyclobutyl-6-(3-bromphenylamino)-4-trifluormethylnicotinamid,
N-Cyclobutylmethyl-6-(3-bromphenylamino)-4-trifluormethylnicotinamid,
N-Isobutyl-6-(3-bromphenylamino)-4-trifluormethylnicotinamid,
N-Cyclopentylmethyl-6-(3-bromphenylamino)-4-trifluormethylnicotinamid,
N-Cyclopropylmethyl-6-(3-bromphenylamino)-4-trifluormethylnicotinamid,
N-Cyclobutylmethyl-6-(2-fluorphenylamino)-4-trifluormethylnicotinamid,
N-Cycloheptylmethyl-6-(3-fluorphenylamino)-4-trifluormethylnicotinamid,
N-(Tetrahydropyran-4-ylmethyl)-6-(3-fluorphenylamino)-4-trifluormethylnicotinamid,
N-Cyclobutyl-6-(3-fluorphenylamino)-4-trifluormethylnicotinamid,
N-Cyclohexylmethyl-6-(3-fluorphenylamino)-4-trifluormethylnicotinamid,
N-Cyclobutylmethyl-6-(3-fluorphenylamino)-4-trifluormethylnicotinamid,
N-Cyclopentylmethyl-6-(3-fluorphenylamino)-4-trifluormethylnicotinamid,
N-Isobutyl-6-(3-fluorphenylamino)-4-trifluormethylnicotinamid,
N-Cyclopropylmethyl-6-(3-fluorphenylamino)-4-trifluormethylnicotinamid,
N-(1,1-Dioxotetrahydrothiophen-3-ylmethyl)-6-(3-fluorphenylamino)-4-trifluormethylnicotinamid,
N-Cyclobutylmethyl-6-(4-fluorphenylamino)-4-trifluormethylnicotinamid,
N-(Tetrahydropyran-4-ylmethyl)-6-(2,3-dichlorphenylamino)-4-trifluormethylnicotinamid,
N-Cyclohexylmethyl-6-(2,3-dichlorphenylamino)-4-trifluormethylnicotinamid,
N-Cycloheptylmethyl-6-(2,3-dichlorphenylamino)-4-trifluormethylnicotinamid,
N-Cyclohexylmethyl-6-(2,4-dichlorphenylamino)-4-trifluon-nethylnicotinamid,
N-Cycloheptylmethyl-6-(2,4-dichlorphenylamino)-4-trifluormethylnicotinamid,
N-Cyclobutyl-6-(2,4-dichlorphenylamino)-4-trifluormethylnicotinamid,
N-Cyclopentylmethyl-6-(2,4-dichlorphenylamino)-4-trifluormethylnicotinamid,
N-Cyclobutylmethyl-6-(2,4-dichlorphenylamino)-4-trifluormethylnicotinamid,
N-Isobutyl-6-(2,4-dichlorphenylamino)-4-trifluormethylnicotinamid,
N-Cyclopropylmethyl-6-(2,4-dichlorphenylamino)-4-trifluormethylnicotinamid,
N-(1,1-Dioxotetrahydrothiophen-3-ylmethyl)-6-(2,4-dichlorphenylamino)-4-trifluormethylnicotinamid,
N-Cyclohexylmethyl-6-(3,5-dichlorphenylamino)-4-trifluormethylnicotinamid,
N-(Tetrahydropyran-4-ylmethyl)-6-(3,5-dichlorphenylamino)-4-trifluormethylnicotinamid,
N-Cyclobutyl-6-(3,5-dichlorphenylamino)-4-trifluorinethylnicotinamid,
N-Cyclopentylmethyl-6-(3,5-dichlorphenylamino)-4-trifluormethylnicotinamid,
N-Cyclobutylmethyl-6-(3,5-dichlorphenylamino)-4-trifluormethylnicotinamid,
N-Isobutyl-6-(3,5-dichlorphenylamino)-4-trifluormethylnicotinamid,
N-Cyclopropylmethyl-6-(3,5-dichlorphenylamino)-4-trifluormethylnicotinamid,
N-Isobutyl-6-(3,4-dichlorphenylamino)-4-trifluorrnethylnicotinamid,
N-Cyclobutyl-6-(3,4-dichlorphenylamino)-4-trifluorrnethylnicotinamid,
N-(Tetrahydropyran-4-ylmethyl)-6-(3,4-dichlorphenylamino)-4-trifluormethylnicotinamid,
N-Cyclopentylmethyl-6-(3,4-dichlorphenylamino)-4-trifluormethylnicotinamid,
N-Cyclobutylmethyl-6-(3,4-dichlorphenylamino)-4-trifluorinethylnicotinamid,
N-Cyclopropylmethyl-6-(3,4-dichlorphenylamino)-4-trifluormethylnicotinamid,
N-Cyclohexylmethyl-6-(3,4-dichlorphenylamino)-4-trifluormethylnicotinamid,
N-Cyclobutylmethyl-6-(2-fluor-4-chlorphenylamino)-4-trifluormethylnicotinamid,
N-Cyclopentylmethyl-6-(2-fluor-4-chlorphenylamino)-4-trifluormethylnicotinamid,
N-(Tetrahydropyran-4-ylmethyl)-6-(2-fluor-4-chlorphenylamino)-4-trifluormethylnicotinamid,
N-Cyclobutylmethyl-6-(2-chlor-4-fluorphenylamino)-4-trifluormethylnicotinamid,

N-Cyclopentylmethyl-6-(2-chlor-4-fluorphenylamino)-4-trifluormethylnicotinamid,
N-(Tetrahydropyran-4-ylmethyl)-6-(2-chlor-4-fluorphenylamino)-4-trifluormethylnicotinamid,
N-Cyclobutylmethyl-6-(2,4-difluorphenylamino)-4-trifluormethylnicotinamid,
N-Cyclopentylmethyl-6-(2,4-difluorphenylamino)-4-trifluormethylnicotinamid,
N-(Tetrahydropyran-4-ylmethyl)-6-(2-methoxy-5-chlor-phenylamino)-4-trifluormethylnicotinamid,
N-Cyclobutylmethyl-6-(2-methoxy-5-chlorphenylamino)-4-trifluormethylnicotinamid,
N-(Tetrahydropyran-4-ylmethyl)-6-(2-hydroxy-S-chlorphenylamino)-4-trifluormethylnicotinamid,
N-Cyclohexylmethyl-6-(2-methyl-4-chlorphenylamino)-4-trifluormethylnicotinamid,
N-(Tetrahydropyran-4-ylmethyl)-6-(2-methyl-4-chlorphenylamino)-4-trifluormethylnicotinamid,
N-(Tetrahydropyran-4-ylmethyl)-6-phenylamino-4-trifluormethylnicotinamid,
N-Cyclopropylmethyl-6-phenylamino-4-trifluormethylnicotinamid,
N-Cycloheptylmethyl-6-(3,5-dichlorphenylamino)-4-trifluormethylnicotinamid,
N-(Tetrahydropyran-4-ylmethyl)-6-(2-methyl-5-chlorphenylamino)-4-trifluormethylnicotinamid,
N-Cyclobutylmethyl-6-(2-hydroxy-5-chlorphenylamino)-4-trifluormethylnicotinamid,
N-(5-Oxopyrrolidin-3-ylmethyl)-6-(2,4-dichlorphenylamino)-4-trifluormethylnicotinamid,
N-Cycloheptylmethyl-6-phenylamino-4-trifluomiethylnicotinamid,
N-Cyclobutyl-6-phenylamino-4-trifluormethylnicotinamid,
N-Isobutyl-6-phenylamino-4-trifluormethylnicotinamid,
N-(3-Dimethylamino-2,2-dimethylpropyl)-6-(3-chlorphenylamino)-4-trifluormethylnicotinamid,
N-(3-Hydroxy-2,2-dimethylpropyl)-6-(3-chlorphenylamino)-4-trifluormethylnicotinamid,
N-(2-Methoxy-2-methylpropyl)-6-(3-chlorphenylamino)-4-trifluormethylnicotinamid,
N-([1,4]dioxan-2-ylmethyl)-6-(3-chlorphenylamino)-4-trifluormethylnicotinamid,
N-(Piperidin-2-ylmethyl)-6-(3-chlorphenylamino)-4-trifluormethylnicotinamid,
N-(1-Benzyl-5-oxo-pyrrolidin-3-ylmethyl)-6-(3-chlorphenylamino)-4-trifluormethylnlcotinamid,
N-(5-Oxo-pyrrolidin-3-ylmethyl)-6-(3-chlorphenylamino)-4-trifluormethylnicotinamid,
N-Methylcarbamoylmethyl-6-(3-chlorphenylamino)-4-trifluormethylnicotinamid,
N-(1-Ethylpyrrolidin-2-ylmethyl)-6-(3-chlorphenylamino)-4-trifluormethylnicotinamid,
N-(2,2,6,6-Tetramethylpiperidin-4-ylmethyl)-6-(3-chlorphenylamirio)-4-trifluormethylnicotinamid,
N-(2,2-Dimethyl-[1,3]-dioxolan-4-ylmethyl)-6-(3-chlorphenylamino)-4-trifluormethylnicotinamid,
N-(Tetrahydropyran-4-ylmethyl)-6-(2-fluorphenylamino)-4-trifluormethylnicotinamid,
N-(Tetrahydropyran-4-ylmethyl)-6-(4-fluorphenylamino)-4-trifluormethylnicotinamid,
N-(3-Dimethylamino-2,2-dimethylpropyl)-6-(2,4-dichlorphenylamino)-4-trifluormethylnicotinamid,
N-([1,4]-dioxan-2-ylmethyl)-6-(2,4-dichlorphenylamino)-4-trifluormethylnicotinamid,
N-(5-Oxo-pyrrolidin-3-ylmethyl)-6-(2,4-dichlorphenylamino)-4-trifluormethylnicotinamid,
N-Methylcarbamoylmethyl-6-(2,4-dichlorphenylamino)-4-trifluormethylnicotinamid,
N-(2,2-Dimethyl[1,3]-dioxolan-4-ylmethyl)-6-(2,4-dichlorphenylamino)-4-trifluormethylnicotinamid,
N-Cycloheptylmethyl-6-(3,4-dichlorphenylamino)-4-trifluormethylnicotinamid,
N-(Tetrahydropyran-4-ylmethyl)-6-(2,4-difluorphenylamino)-4-trifluormethylnicotinamid,
N-Cyclohexylmethyl-6-(2-methyl-5-chlorphenylamino)-4-trifluormethylnicotinamid,
N-(2,3-Dihydroxypropyl)-6-(3-chlorphenylamino)-4-trifluormethylnicotinamid;
N-(2,3-Dihydroxypropyl)-6-(2,4-dichlorphenylamino)-4-, trifluormethylnicotinamid,
6-(3-Chlorphenylamino)-N-(tetrahydropyran-4-ylmethyl)-2-trifluormethylnicotinamid,
6-(3-Chlorphenylamino)-N-cyclohexylmethyl-2-trifluormethylnicotinamid,
6-(3-Chlorphenylamino)-N-cyclobutylmethyl-2-trifluormethylnicotinamid,
6-(3-Chlorphenylamino)-N-cyclopentylmethyl-2-trifluormethylnicotinamid,
6-(3-Chlorphenylamino)-2-isopropyl-N-(tetrahydropyran-4-ylmethyl)-nicotinamid,
6-(3-Chlorphenylamino)-N-(1,1-dioxotetrahydrothlophen-3-ylmethyl)-2-isopropylnicotinamid,
6-(3-Chlorphenylamino)-N-cyclopentylmethyl-2-isopropylnicotinamid,
6-(3-Chlorphenylamino)-N-cyclohexylmethyl-2-isopropylnicotinamid,
6-(2,4-Dichlorphenylamino)-N-cyclobutylmethyl-2-isopropylnicotinamide,
6-(2,4-Dichlorphenylamino)-N-cyclopentylmethyl-2-isopropylnicotinamid,
6-(2,4-Dichlorphenylamino)-N-(tetrahydropyran-4-ylmethyl)-2-isopropylnicotinamid,
6-(2,4-Dichlorphenylamino)-N-(1,1-dioxotetrahydrothiophen-3-ylmethyl)-2-isopropylnicotinamid,
6-(3-Fluorphenylamino)-N-cyclobutylmethyl-2-isopropylnicotinamid,
6-(3-Fluorphenylamino)-N-cyclopentylmethyl-2-isopropylnicotinamid,
6-(3-Fluorphenylamino)-N-(tetrahydropyran-4-ylmethyl)-2-isopropylnicotinamid,
6-(3-Fluorphenylamino)-N-(1,1-dioxotetrahydrothiophen-3-ylmethyl)-2-isopropylnicotinamid,
6-(4-Cyano-2-methylphenylamino)-4-isopropyl-N-(tetrahydropyran-4-ylmethyl)-nicotinamid,

6-(5-Chlor-2-cyanophenylamino)-4-isopropyl-N-(tetrahydropyran-4-ylmethyl)-nicotinamid,
6-(2-Cyano-5-methylphenylamino)-4-isopropyl-N-(tetrahydropyran-4-ylmethyl)-nicotinamid,
6-(3-Chlorphenylamino)-N-(1,1-dioxotetrahydro-1/$^6$-thiophen-3-ylmethyl)-4-isopropylnicotinamid,
N-Cyclobutylmethyl-4-isopropyl-6-(3-trifluormethoxyphenylamino)-nicotinamid,
6-(2,3-Difluorphenylamino)-4-isopropyl-N-(tetrahydropyran-4-ylmethyl)-nicotinamid,
6-(3,5-Bistrifluormethylphenylamino)-4-isopropyl-N-(tetrahydropyran-4-ylmethyl)-nicotinamid,
6-(2,4-Difluorphenylamino)-4-isopropyl-N-(tetrahydropyran-4-ylmethyl)-nicotinamid,
6-(3-Ethinylphenylamino)-4-isopropyl-N-(tetrahydropyran-4-ylmethyl)-nicotinamid,
6-(2-Fluor-4-trifluormethylphenylamino)-N-cyclopentylmethyl-4-isopropylnicotinamid,
6-(3-Cyano-4-methylphenylamino)-4-isopropyl-N-(tetrahydropyran-4-ylmethyl)-wicotinamid,
6-(3-Cyano-4-fluorphenylamino)-4-isopropyl-N-(tetrahydropyran-4-ylmethyl)-nicotinamid,
6-(3-Brom-4-trifluormethoxyphenylamino)-4-isopropyl-N-(tetrahydropyran-4-ylmethyl)-nicotinamid,
6-(4-Chlor-2-fluorphenylamino)-N-cyclobutylmethyl-4-isopropylnicotinamid,
6-(5-Brom-2-methylphenylamino)-4-isopropyl-N-(tetrahydropyran-4-ylmethyl)-nicotinamid,
6-(2-Brom-5-fluorphenylamino)-4-isopropyl-N-(tetrahydropyran-4-ylmethyl)-nicotinamid,
6-(2-Fluor-5-trifluormethylphenylamino)-4-isopropyl-N-(tetrahydropyran-4-ylmethylnicotinamid,
6-(2-Chlor-5-trifluormethylphenylamino)-4-isopropyl-N-(tetrahydropyran-4-ylmethylnicotinamid,
6-(2-Brom-5-trifluormethylphenylamino)-4-isopropyl-N-(tetrahydropyran-4-ylmethyl)-nicotinamid,
6-(3-Brom-4-cyanophenylamino)-4-isopropyl-N-(tetrahydropyran-4-ylmethyl)-nicotinamid,
6-(2-Brom-4-trifluomiethoxwhenylamino)-4-isopropyl-N-(tetrahydropyran-4-ylmethyl)-nicotinamid,
6-(3-Chlor-2-methylphenylamino)-4-isopropyl-N-(tetrahydropyran-4-ylmethyl)-nicotinamid,
6-(3,5-Difluorphenylamino)-4-isopropyl-N-(tetrahydropyran-4-ylmethyl)-nicotinamid,
6-(2-Chlor-4-fluorphenylamino)-4-isopropyl-N-(tetrahydropyran-4-ylmethyl)-nicotinamid,
6-(4-Chlor-2-methylphenylamino)-4-isopropyl-N-(tetrahydropyran-4-ylmethyl)-nicotinamid,
6-(2-Fluor-3-trifluormethylphenylamino)-N-cyclopentylmethyl-4-isopropylnicotinamid,
6-(2-Methyl-4-chlorphenylamino)-N-cyclopentylmethyl-4-isopropylnicotinamid,
6-(3-Chlor-4-cyanophenylamino)-N-cyclopentylmethyl-4-isopropylnicotinamid,
6-(4-Brom-2-chlorphenylamino)-N-cyclopentylmethyl-4-isopropylnicotinamid,
N-Cyclobutylmethyl-6-(2,4-difluorphenylamino)-4-isopropylnicotinamid,
N-Cyclobutylmethyl-6-(2,4-dichlorphenylamino)-4-isopropylnicotinamid,
N-Cyclobutylmethyl-6-(3,4-dichlorphenylamino)-4-isopropylnicotinamid,
N-Cyclobutylmethyl-6-(2,3-dichlorphenylamino)-4-isopropylnicotinamid,
6-(2-Chlor-4-fluorphenylamino)-N-cyclobutylmethyl-4-isopropylnicotinamid,
6-(3-Chlor-4-fluorphenylamino)-N-cyclobutylmethyl-4-isopropylnicotinamid,
6-(4-Brom-2-chlorphenylamino)-N-cyclobutylmethyl-4-isopropylnicotinamid,
6-(2-Brom-4-chlorphenylamino)-N-cyclobutylmethyl-4-isopropylnicotinamid,
N-Cyclobutylmethyl-6-(2-fluor-3-trifluormethylphenylamino)-4-isopropylnicotinamid,
6-(4-Chlor-2-methylphenylamino)-N-cyclobutylmethyl-4-isopropylnicotinamid,
6-(2-Chlor-4-cyanophenylamino)-N-cyclobutylmethyl-4-isopropylnicotinamid,
6-(4-Cyano-2-fluoiphenylamino)-N-cydobutylmethyl-4-isopropylnicotinamid,
6-(4-Cyano-2-methylphenylaminö)-N-cyclobutylmethyl-4-isopropylnicotinamid,
6-(2-Chlor-4-trifluormethylphenylamino)-N-cyclobutylmethyl-4-isopropylnicotinamid,
N-Cyclobutylmethyl-6-(3,5-dichlorphenylamino)-4-isopropylnicotinamid,
N-Cyclobutylmethyl-6-(2,5-dichlorphenylamino)-4-isopropylnicotinamid,
N-Cyclobutylmethyl-6-(3,5-difluorphenylamino)-4-isopropylnicotinamid,
6-(5-Chlor-2-fluorphenylamino)-N-cyclobutylmethyl-4-isopropylnicotinamid,
6-(2-Chlor-5-fluorphenylamino)-N-cyclobutylmethyl-4-isopropylnicotinamid,
6-(2-Chlor-5-fluorphenylamino)-4-isopropyl-N-(tetrahydropyran-4-ylmethyl)-nicotinamid,
6-(2-Chlor-5-methylphenylamino)-4-isopropyl-N-(tetrahydropyran-4-ylmethyl)-nicotinamid,
6-(2-Fluor-5-methylphenylamino)-4-isopropyl-N-(tetrahydropyran-4-ylmethyl)-' nicotinamid,
6-(5-Fluor-2-methylphenylamino)-4-isopropyl-N-(tetrahydropyran-4-ylmethyl)-nicotinamid,
6-(3-Brom-2-methylphenylamino)-4-isopropyl-N-(tetrahydropyran-4-ylmethyl)-nicotinamid,
4-Isopropyl-6-(2-methyl-4-trifluormethoxyphenylamino)-N-(tetrahydropyran-4-ylmethylnicotinamid,
6-(3-Fluor-2-methylphenylamino)-4-isopropyl-N-(tetrahydropyran-4-ylmethyl)-nicotinamid,
6-(3-Brom-5-trifluormethylphenylamino)-4-isopropyl-N-(tetrahydropyran-4-ylmethyl)-nicotinamid,
6-(4-Cyanophenylamino)-4-isopropyl-N-(tetrahydropyran-4-ylmethyl)-nicotinamid,
6-(4-Chlor-2-fluorphenylamino)-4-isopropyl-N-(tetrahydropyran-4-ylmethyl)-nicotinamid,
6-(4-Brom-2-fluorphenylamino)-N-cyclopentylmethyl-4-isopropylnicotinamid,

6-(2-Brom-4-trifluormethoxyphenylamino)-N-cyclobutylmethyl-4-isopropylnicotinamid,
N-Cyclobutylmethyl-6-(2-fluor-4-trifluormethylphenylamino)-4-isopropylnicotinamid,
6-(4-Cyano-2-fluorphenylamino)-N-cyclopentylmethyl-4-isopropylnicotinamid,
6-(4-Brom-3-trifluormethylphenylamino)-4-isopropyl-N-(tetrahydropyran-4-ylmethyl)-nicotinamid,
6-(4-Fluor-3-trifluormethylphenylamino)-4-isopropyl-N-(tetrahydropyran-4-ylmethyl)-nicotinamid,
6-(3,4-Dibromphenylamino)-4-isopropyl-N-(tetrahydropyran-4-ylmethyl)-nicotinamid,
6-(4-Brom-3-fluorphenylamino)-4-isopropyl-N-(tetrahydropyran-4-ylmethyl)-nicotinamid,
6-(2-Chlor-4-trifluormethylphenylamino)-N-cyclopentylmethyl-4-isopropylnicotinamid,
6-(3,4-Difluorphenylamino)-4-isopropyl-N-(tetrahydropyran-4-ylmethyl)-nicotinamid,
6-(4-Chlor-3-trifluormethylphenylamino)-4-isopropyl-N-(tetrahydropyran-4-ylmethyl)-nicotinamid,
6-(4-Methyl-3-trifluormethylphenylamino)-4-isopropyl-N-(tetrahydropyran-4-ylmethyl-nicotinamid,
6-(2-Chlor-4-trifluormethoxyphenylamino)-4-isopropyl-N-(tetrahydropyran-4-ylmethyl)-nicotinamid,
6-(2-Cyano-3-methylphenylamino)-4-isopropyl-N-(tetrahydropyran-4-ylmethyl)-nicotinamid,
6-(3-Chlor-2-cyanophenylamino)-4-isopropyl-N-(tetrahydropyran-4-ylmethyl)-nicotinamid,
6-(3-Chlorphenylamino)-4-(1-hydroxymethylethyl)-N-(tetrahydropyran-4-ylmethyl)-nicotinamid, and
4-tert-Butyl-6-(3,4-dichlorphenylamino)-N-(tetrahydropyran-4-ylmethyl)-nicotinamid,

und pharmazeutisch verträgliche Derivate davon.

7. Verbindung nach Anspruch 1, ausgewählt aus:

6-(3-Chlorphenylamino)-4-isopropyl-N-(tetrahydropyran-4-ylmethyl)-nicotinamid,
6-(3-Bromphenylamino)-4-isopropyl-N-(tetrahydropyran-4-ylmethyl)-nicotinamid,
6-(2,4-Dichlorphenylamino)-4-isopropyl-N-(tetrahydropyran-4-ylmethyl)-nicotinamid,
4-Isopropyl-N-(tetrahydropyran-4-ylmethyl)-6-(3-trifluormethoxyphenylamino)-nicotinamid,
4-tert-Butyl-6-(2,4-dichlorphenylamino)-N-(tetrahydropyran-4-ylmethyl)-nicotinamid,
6-(3-Chlor-4-cyanophenylamino)-4-isopropyl-N-(tetrahydropyran-4-ylmethyl)-nicotinamid,
6-(2-Fluor-3-trifluormethylphenylamino)-4-isopropyl-N-(tetrahydropyran-4-ylmethyl)-nicotinamid,
6-(4-Brom-2-chlorphenylamino)-4-isopropyl-N-(tetrahydropyran-4-ylmethyl)-nicotinamid,
6-(3,4-Dichlorphenylamino)-4-isopropyl-N-(tetrahydropyran-4-ylmethyl)-nicotinamid,
6-(2-Brom-4-trifluormethoxyphenylamino)-4-isopropyl-N-(tetrahydropyran-4-ylmethyl)-nicotinamid,
6-(3,5-Difluorphenylamino)-4-isopropyl-N-(tetrahydropyran-4-ylmethyl)-nicotinamid,
6-(2,4-Dichlorphenylamino)-N-(tetrahydropyran-4-ylmethyl)-4-trifluormethylnicotinamid,

oder ein pharmazeutisch verträgliches Derivat davon.

8. Arzneimittel, umfassend eine Verbindung gemäß einem der vorstehenden Ansprüche oder ein pharmazeutisch verträgliches Derivat davon.

9. Arzneimittel gemäß Anspruch 8, ferner umfassend einen pharmazeutischen Träger oder ein Verdünnungsmittel davon.

10. Verwendung einer therapeutisch wirksamen Menge einer Verbindung der Formel (I) gemäß einem der Ansprüche 1 bis 7 oder eines pharmazeutisch verträglichen Derivats davon zur Herstellung eines Medikaments zur Behandlung einer Erkrankung, welche durch die Aktivität von Cannabinoid-2-Rezeptoren vermittelt wird.

11. Verwendung gemäß Anspruch 10, wobei die Erkrankung eine Immunstörung, eine Entzündungsstörung, Schmerz, rheumatoide Arthritis, Multiple Sklerose, Osteoarthritis oder Osteoporose ist.

12. Verwendung gemäß Anspruch 11, wobei der Schmerz ausgewählt ist aus Entzündungsschmerz, viszeralem Schmerz, Krebsschmerz, neuropathischem Schmerz, Rückenschmerz, Muskelschmerz, Skelettschmerz, postoperativem Schmerz, akutem Schmerz und Migräne.

## Revendications

1. Composé de formule (I) :

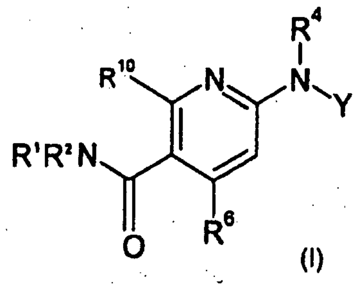

(I)

dans laquelle :

Y représente un groupe phényle, non substitué ou substitué avec un, deux ou trois substituants choisis entre des substituants alkyle en $C_1$ à $C_6$, alkyle en $C_1$ à $C_6$ à substituant halogéno, alkoxy en $C_1$ à $C_6$, hydroxy, cyano, halogéno, alkylsulfonyle en $C_1$ à $C_6$, $-CONH_2$, $-NHCOCH_3$, -COOH, alkoxy en $C_1$ à $C_6$ à substituant halogéno, $SO_2NR^{8a}R^{8b}$ dans lequel $R^{8a}$ et $R^{8b}$ représentent chacun indépendamment H, un groupe alkyle en $C_1$ à $C_6$ ou alcynyle en $C_1$ à $C_6$ ;
$R^1$ est choisi entre un atome d'hydrogène, des groupes alkyle en $C_1$ à $C_6$, cycloalkyle en $C_3$ à $C_6$ et alkyle en $C_1$ à $C_6$ à substituant halogéno ;
$R^2$ représente un groupe $(CH_2)_mR^3$ dans lequel m est égal à 0 ou 1 ;
ou bien $R^1$ et $R^2$, conjointement avec 1'atome de N auquel ils sont fixés, forment un noyau hétérocyclyle non aromatique tétra- à octogonal qui est non substitué ou substitué avec 1, 2 ou 3 substituants choisis entre des substituants : alkyle en $C_1$ à $C_6$, alkoxy en $C_1$ à $C_6$, hydroxy, cyano, halogéno, sulfonyle, méthylsulfonyle, $NR^{8a}R^{8b}$, $CH_2$phényle, $NHCOCH_3$, (=O), $CONHCH_3$ et $NHSO_2CH_3$ dans lesquels $R^{8a}$ et $R^{8b}$ répondent aux définitions précitées ;
$R^3$ représente un groupe hétérocyclyle non aromatique tétra- à octogonal, cycloalkyle en $C_3$ à $C_8$, alkyle en $C_1$ à $C_{10}$ droit ou ramifié, alcényle en $C_2$ à $C_{10}$, cycloalcényle en $C_3$ à $C_8$, alcynyle en $C_2$ à $C_{10}$ ou cycloalcynyle en $C_3$ à $C_8$, n'importe lequel de ces groupes pouvant être non substitué ou substitué avec 1, 2 ou 3 substituants choisis de préférence entre des substituants : alkyle en $C_1$ à $C_6$, alkoxy en $C_1$ à $C_6$, hydroxy, cyano, halogéno, sulfonyle, méthylsulfonyle, $NR^{8a}R^{8b}$, $CH_2$phényle, $NHCOCH_3$, (=O), $CONHCH_3$ et $NHSO_2CH_3$ dans lesquels $R^{8a}$ et $R^{8b}$ répondent aux définitions précitées, ou un groupe $R^5$ ;
$R^4$ est choisi entre un atome d'hydrogène, des groupes alkyle en $C_1$ à $C_6$, cycloalkyle en $C_3$ à $C_6$ et alkyle en $C_1$ à $C_6$ à substituant halogéno, $COCH_3$ et $SO_2Me$ ;
$R^5$ représente un groupe
$R^5$ is

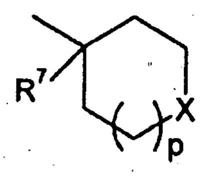

dans lequel p est égal à 0, 1 ou 2 et X représente un groupe $CH_2$, O ou S ;
$R^6$ représente un groupe chloro ou alkyle en $C_1$ à $C_6$ facultativement substitué avec 1, 2 ou 3 substituants choisis entre des substituants hydroxy, alkoxy en $C_1$ à $C_6$, cyano, halogéno, $NR^{8a}R^{8b}$, $CONR^{8a}R^{8b}$, $SO_2NR^{8a}R^{8b}$, $_{NR}{}^{8a}{}_{COR}{}^{8b}$ et $NR^{8a}SO_2R^{8b}$, dans lesquels $_R{}^{8a}$ et $_R{}^{8b}$ répondent aux définitions précitées et $R^{10}$ représente un atome d'hydrogène, ou bien $R^{10}$ représente un groupe chloro ou alkyle en $C_1$ à $C_6$ facultativement substitué avec 1, 2 ou 3 substituants choisis entre des substituants hydroxy, alkoxy en $C_1$ à $C_6$, cyano, halogéno, $NR^{8a}R^{8b}$, $CONR^{8a}R^{8b}$, $SO_2NR^{8a}R^{8b}$, $NR^{8a}COR^{8b}$ et $NR^{8a}SO2R^{8b}$, dans lesquels $R^{8a}$ et $R^{8b}$ répondent aux définitions précitées, et $R^6$ représente un atome d'hydrogène ;
$R^7$ représente un groupe OH, alkoxy en $C_1$ à $C_6$, $NR^{8a}R^{8b}$ ; $NHCOR^9$, $NHSO_2R^9$ ou $SOqR^9$ ;
$R^{8a}$ représente H ou un groupe alkyle en $C_1$ à $C_6$ ;
$R^{8b}$ représente H ou un groupe alkyle en $C_1$ à $C_6$ ;
$R^9$ représente un groupe alkyle en $C_1$ à $C_6$ ;
q est égal à 0, 1 ou 2 ;

ou un de ses dérivés pharmaceutiquement acceptables.

**2.** Composé suivant la revendication 1, de formule (Ia) :

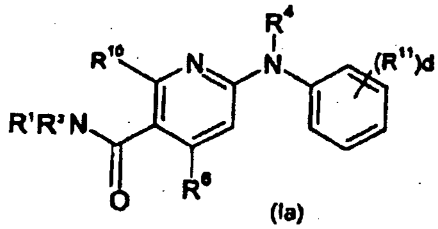

(Ia)

$R^1$ est choisi entre un atome d'hydrogène, des groupes alkyle en $C_1$ à $C_6$, cycloalkyle en $C_3$ à $C_6$ et alkyle en $C_1$ à $C_6$ à substituant halogéno ;
$R^2$ représente un groupe $(CH_2)_mR^3$ dans lequel m est égal à 0 ou 1 ;
ou bien $R^1$ et $R^2$, conjointement avec l'atome de N auquel ils sont fixés, forment un noyau hétérocyclyle non aromatique choisi entre les noyaux azétidinyle, pyrrolidinyle, morpholinyle, pipérazinyle, pipéridinyle, tétrahydropyridinyle, azapine, oxapine, azacyclooctanyle, azaoxacyclooctanyle et azathiacyclooctanyle, n'importe lequel de ces noyaux pouvant être non substitué ou substitué avec 1, 2 ou 3 substituants choisis entre des substituants : alkyle en $C_1$ à $C_6$, alkoxy en $C_1$ à $C_6$, hydroxy, cyano, halogéno, sulfonyle, méthylsulfonyle, $NR^{8a}R^{8b}$, $CH_2$phényle, $NHCOCH_3$, (=O), $CONHCH_3$ et $NHSO_2CH_3$ ;
$R^3$ représente un groupe 2- ou 3-azétidinyle, oxétannyle, thioxétannyle, thioxétannyl-s-oxyde, thioxétannyl-s,s-dioxyde, dioxalanyle, pyrrolidinyle, tétrahydrofurannyle, tétrahydrothiophényle, tétrahydrothiophényl-s,s-dioxyde, morpholinyle, pipéridinyle, pipérazinyle, tétrahydropyrannyle, tétrahydrothiopyrannyle, thiomorpholinyle, thiomorpholinyl-s,s-dioxyde, tétrahydropyridinyle, dioxannyle, tétrahydro-thiopyranne-1,1-dioxyde, azapine, oxapine, azacyclooctanyle, azaoxacyclooctanyle, azathiacyclooctanyle, oxacyclooctanyle, thiacyclooctanyle, cycloalkyle en $C_3$ à $C_8$, alkyle en $C_1$ à $C_{10}$ droit ou ramifié, alcényle en $C_2$ à $C_{10}$, cycloalcényle en $C_3$ à $C_8$, alcynyle en $C_2$ à $C_{10}$ ou cycloalcynyle en $C_3$ à $C_8$ ; n'importe lequel de ces groupes pouvant être non substitué ou substitué avec 1, 2 ou 3 substituants choisis entre des substituants alkyle en $C_1$ à $C_6$, alkoxy en $C_1$ à $C_6$, hydroxy, cyano, halogéno, sulfonyle, méthylsulfonyle, $NR^{8a}R^{8b}$, $CH_2$phényle, $NHCOCH_3$, (=O), $CONHCH_3$ et $NHSO_2CH_3$ ; ou bien $R^3$ représenté un groupe $R^5$ ;
$R^4$ est choisi entre un atome d'hydrogène, des groupes alkyle en $C_1$ à $C_6$, cycloalkyle en $C_3$ à $C_6$ et alkyle en $C_1$ à $C_6$ à substituant halogéno, $COCH_3$ et $SO_2Me$ ;
$R^5$ représente un groupe

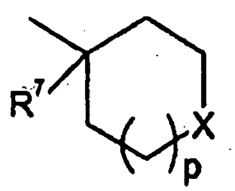

dans lequel p est égal à 0, 1 ou 2 et X représente un groupe $CH_2$, O où S ;
$R^6$ représente un groupe chloro ou alkyle en $C_1$ à $C_6$ facultativement substitué avec 1, 2 ou 3 substituants choisis entre des substituants hydroxy, alkoxy en $C_1$ à $C_6$, cyano, halogéno, $NR^{8a}R^{8b}$, $CONR^{8a}R^{8b}$, $SO_2NR^{8a}R^{8b}$, $NR^{8a}COR^{8b}$ et $NR^{8a}SO_2R^{8b}$, dans lesquels $R^{8a}$ et $R^{8b}$ répondent aux définitions précitées et $R^{10}$ représente un atome d'hydrogène, ou bien $R^{10}$ représente un groupe chloro ou alkyle en $C_1$ à $C_6$ facultativement substitué avec 1, 2 ou 3 substituants choisis entre des substituants hydroxy, alkoxy en $C_1$ à $C_6$, cyano, halogéno, $NR^{8a}R^{8b}$, $CONR^{8a}R^{8b}$, $SO_2NR^{8a}R^{8b}$, $NR^{8a}COR^{8b}$ et $NR^{8a}SO_2R^{8b}$, dans lesquels $R^{8a}$ et $R^{8b}$ répondent aux définitions précitées, et $R^6$ représente un atome d'hydrogène ;
$R^7$ représente un groupe OH, alkoxy en $C_1$ à $C_6$, $NR^{8a}R^{8b}$, $NHCOR^9$, $NHSO_2R^9$ ou $SOqR^9$ ;
$R^{8a}$ représente H ou un groupe alkyle en $C_1$ à $C_6$ ;
$R^{8b}$ représente H ou un groupe alkyle en $C_1$ à $C_6$ ;
$R^9$ représente un groupe alkyle en $C_1$ à $C_6$ ;
$R^{11}$ représente un groupe alkyle en $C_1$ à $C_6$, alkyle en $C_1$ à $C_6$ à substituant halogéno, alkoxy en $C_1$ à $C_6$, hydroxy, cyano, halogéno, alkylsulfonyle en $C_1$ à $C_6$, $-CONH_2$,- $NHCOCH_3$, -COOH, alkoxy en $C_1$ à $C_6$ à

substituant halogéno, $SO_2NR^{8a}R^{8b}$ ou alcynyle en $C_1$ à $C_6$ ;
q est égal à 0, 1 ou 2 ;
d est égal à 0, 1, 2 ou 3 ;

ou un de ses dérivés pharmaceutiquement acceptables.

**3.** Composé suivant la revendication 1 ou 2, dans lequel $R^1$ représente un atome d'hydrogène.

**4.** Composé suivant l'une quelconque des revendications précédentes, dans lequel $R^4$ représente un groupe alkyle en $C_1$ à $C_6$ ou un atome d'hydrogène.

**5.** Composé suivant l'une quelconque des revendications précédentes, dans lequel $R^6$ représente un groupe tertio-butyle, isopropyle ou $CF_3$.

**6.** Composé tel que décrit dans l'un quelconque des exemples 1 à 306, à savoir un composé choisi entre les suivants :

cyclohexylméthylamide d'acide 2-(3-chlorophénylamino)-4-trifluorométhylpyridine-5-carboxylique,
6-(3-chlorophénylamino)-*N*-cyclohexylméthyl-4-isopropylnicotinamide,
N-cyclohexylméthyl-6-(3,4-dichloro-phénylamino)-4-isopropyl-nicotinamide,
6-(3-bromo-phénylamino)-N-cyclohexylméthyl-4-isopropyl-nicotinamide,
N-cyclohexylméthyl-6-(2,4-dichloro-phénylamino)-4-isopropyl-nicotinamide,
6-(3-chloro-phénylamino)-N-cyclobutylméthyl-4-isopropyl-nicotinamide,
6-(3-chloro-phényl-amino)-4-isopropyl-N-(tétrahydro-pyranne-4-ylméthyl)-nicotinamide,
6-(3-bromo-phényl-amino)-4-isopropyl-N-(tétrahydro-pyranne-4-ylméthyl)-nicotinamide,
N-cyclohexylméthyl-4-isopropyl-6-(3-méthoxyphénylamino)-nicotinamide,
N-cyclohexylméthyl-6-(3-fluoro-phénylamino)-4-isopropyl-nicotinamide,
1-[6-(3-chloro-phénylamino)-4-isopropyl-pyridine-3-yl]-1-morpholine-4-yl-méthanone,
6-(3-bromo-phénylamino)-N-cyclohexylméthyl-4-isopropyl-nicotinamide,
N-cyclohexylméthyl-4-isopropyl-6-m-tolylamino-nicotinamide,
N-cyclohexylméthyl-4-isopropyl-6-(3-trifluorométhyl-phénylamino)-nicotinamide,
N-cyclohexylméthyl-4-isopropyl-6-(3-trifluorométhoxy-phénylamino)-nicotinamide,
6-(2,3-dichloro-phénylamino)-4-isopropyl-N-(tétrahydro-pyranne-4-ylméthyl)-nicotinamide,
6-(2,4-dichloro-phénylamino)-4-isop1-N-(tétrahydro-pyranne-4-ylméthyl)-nicotinamide,
6-(3,4-dichloro-phénylamino)-4-isopropyl-N-(tétrahydro-pyranne-4-ylméthyl)-nicotinamide,
4-isopropyl-N-(tétrahydro-pyranne-4-ylméthyl)-6-(3-trifluorométhyl-phénylamino)-nicotinamide,
4-isopropyl-N-(tétrahydro-pyranne-4-ylméthyl)-6-(3-trifluorométhoxy-phénylamino)-nicotinamide,
6-[(3-chloro-phényl)amino]-N-(cyclopentylméthyl)-4-isopropyl-nicotinamide,
N-cyclopentylméthyl-6-(3-fluorophénylamino)-4-isopropyl-nicotinamide,
N-cyclopentylméthyl-4-isopropyl-6-(3-trifluorométhyl-phénylamino)-nicotinamide,
N-cyclopentylméthyl-4-isopropyl-6-m-tolylamino-nicotinamide,
N-cyclopentylméthyl-4-isopropyl-6-(3-trifluorométhoxy-phénylamino)-nicotinamide,
6-(3-bromophénylamino)-N-cyclopentylméthyl-4-isopropyl-nicotinamide,
N-cyclopentylméthyl-4-isopropyl-6-(3-méthoxyphénylamino)-nicotinamide,
6-(3-cyano-phénylamino)-N-cyclopentylméthyl-4-isopropyl-nicotinamide,
6-(2-chloro-4-fluoro-phénylamino)-N-cyclopentylméthyl-4-isopropyl-nicotinamide,
6-(2-chloro-4-cyano-phénylamino)-N-cyclopentylméthyl-4-isopropyl-nicotinamide,
N-cyclopentylméthyl-6-(2,4-dichloro-phénylamino)-4-isopropyl-nicotinamide,
N-cyclopentylméthyl-6-(3,4-dichlorophényl amino)-4-isopropyl-nicotinamide,
6-(3-bromo-phénylamino)-N-cyclobutylméthyl-4-isopropyl-nicotinamide,
N-cyclobutylméthyl-6-(3-fluoro-phénylamino)-4-isopropyl-nicotinamide,
N-cyclobutylméthyl-6-(3-trifluorométhyl-phénylamino)-4-isopropyl-nicotinamide,
6-(3-cyano-phénylamino)-N-cyclobutylméthyl-4-isopropyl-nicotinamide,
N-cyclobutylméthyl-4-isopropyl-6-m-tolylamino-nicotinamide,
N-cyclobutylméthyl-4-isopropyl-6-(3-méthoxy-phénylamino)-nicotinamide,
6-(3-fluoro-phénylamino)-4-isopropyl-N-(tétrahydro-pyranne-4-ylméthyl)-nicotinamide,
1-[6-(3-fluoro-phénylamino)-4-isopropyl-pyridine-3-yl]-1-morpholine-4-yl-méthanone,
4-isopropyl-6-(3-méthoxy-phénylamino)-N-(tétrahydro-pyranne-4-ylméthyl)-nicotinamide,
4-isopropyl-N-(tétrahydro-pyranne-4-ylméthyl)-6-m-tolylamino-nicotinamide,

6-(3-cyano-phénylamino)-4-isopropyl-N-(tétrahydro-pyranne-4-ylméthyl)-nicotinamide,
6-[(3,4-dichloro-phényl)-méthyl-amino]-4-isopropyl-N-(tétrahydro-pyranne-4-ylméthyl)-nicotinamide,
6-[(3-bromo-phényl)-méthyl-amino]-4-isopropyl-N-(tétrahydro-pyranne-4-ylméthyl)-nicotinamide,
6-[(3-fluoro-phényl)-méthyl-amino]-4-isopropyl-N-(tétrahydro-pyranne-4-ylméthyl)-nicotinamide,
4-isopropyl-6-(méthyl-phényl-amino)-N-(tétrahydro-pyranne-4-ylméthyl)-nicotinamide,
6-[(3-chloro-phényl)-méthyl-amino]-4-isopropyl-N-(tétrahydro-pyranne-4-ylméthyl)-nicotinamide,
6-[(4-chloro-phényl)-méthyl-amino]-4-isopropyl-N-(tétrahydro-pyranné-4-ylméthyl)-nicotinamide,
6-(3-chloro-phénylamino)-N-cyclobutyl-4-isopropyl-nicotinamide,
6-(3-chloro-phénylamino)-N-cyclopropylméthyl-4-isopropyl-nicotinamide,
6-(3-chloro-phénylamino)-N-(2-éthyl-butyl)-4-isopropyl-nicotinamide,
6-(3-chloro-phénylamino)-N-cyclohexyl-4-isopropyl-nicotinamide,
6-(3-chloro-phénylamino)-N-(1-hydroxy-cyclohexylméthyl)-4-isopropyl-nicotinamide,
1-[6-(3-chloro-phénylamino)-4-isopropyl-pyridine-3-yl]-1-pipéridine-1-yl-méthanone,
6-(3-chloro-phénylamino)-N-(2,2-diméthyl-propyl)-4-isopropyl-nicotinamide,
6-(3-chloro-phénylamino)-4-isopropyl-N-(2-méthoxy-éthyl)-nicotinamide,
6-(3-chloro-phénylamino)-4-isopropyl-N-(tétrahydro-pyranne-4-yl)-nicotinamide,
6-(3-chloro-phénylamino)-4-isopropyl-N-[(R)-1-(tétrahydro-furanne-2-yl)méthyl]-nicotinamide,
N-((R)-1-{1-[6-(3-chloro-phénylamino)-4-isopropyl-pyridine-3-yl]-méthanoyl}-pyrrolidine-3-yl)-acétamide,
1-[6-(3-chloro-phénylamino)-4-isopropyl-pyridine-3-yl]-1-(4-méthane-sulfonyl-pipérazine-1-yl)-méthanone,
6-(3-chloro-phénylamino)-N-(1,1-dioxo-tétrahydro-1/$^{6}$-thiophène-3-yl)-4-isopropyl-nicotinamide,
6-(3-chloro-phénylamino)-4-isopropyl-N-[(S)-1-(tétrahydro-furanne-2-yl)méthyl]-nicotinamide,
6-(3-chloro-phénylamino)-N-(1,1-dioxo-hexahydro-1/$^{6}$-thiopyranne-4-yl)-4-isopropyl-nicotinamide,
1-[6-(3-chloro-phénylamino)-4-isopropyl-pyridine-3-yl]-1-(4-méthyl-pipérazine-1-yl)-méthanone,
6-(3-chloro-phénylamino)-N-(2-diméthylamino-éthyl)-4-isopropyl-nicotinamide,
N-((S)-1-{1-[6-(3-chloro-phénylamino)-4-isopropyl-pyridine-3-yl]-méthanoyl}-pyrrolidine-3-yl)-acétamide,
N-(1-{1-[6-(3-chloro-phénylamino)-4-isopropyl-pyridine-3-yl]-méthanoyl}-pipéridine-4-yl)-méthanesulfonamide,
4-tertiobutyl-6-(3-chloro-phénylamino)-N-cyclohexylméthyl-nicotinamide,
4-tertiobutyl-6-(2,4-dichloro-phénylamino)-N-cyclohexylméthyl-nicotinamide,
4-tertiobutyl-6-(3-chloro-phénylamino)-N-(tétrahydro-pyranne-4-ylméthyl)-nicotinamide,
4-tertiobutyl-6-(3-fluoro-phénylamino)-N-(tétrahydro-pyranne-4-ylméthyl)-nicotinamide,
4-tertiobutyl-6-(2-chloro-3-fluorophénylamino)-N-(tétrahydro-pyranne-4-ylméthyl)-nicotinamide,
4-tertiobutyl-6-(2,4-di-chloro-phénylamino)-N-(tétrahydro-pyranne-4-ylméthyl)-nicotinamide,
6-(3,5-dichloro-phénylamino)-4-isopropyl-N-(tétrahydro-pyranne-4-ylméthyl)-nicotinamide,
6-(5-chloro-2-fluoro-phénylamino)-4-isopropyl-N-(tétrahydro-pyranne-4-ylméthyl)-nicotinamide,
6-(3-chloro-4-fluoro-phénylamino)-4-isopropyl-N-(tétrahydro-pyranne-4-ylméthyl)-nicotinamide,
6-(3-chloro-4-trifluorométhoxy-phénylamino)-4-isopropyl-N-(tétrahydro-pyranne-4-ylméthyl)-nicotinamide,
6-(3-chloro-4-cyano-phénylamino)-4-isopropyl-N-(tétrahydro-pyranne-4-ylméthyl)-nicotinamide,
6-(3-fluoro-5-trifluorométhyl-phénylamino)-4-isopropyl-N-(tétrahydro-pyranne-4-ylméthyl)-nicotinamide,
6-(2-fluoro-3-trifluorométhyl-phénylamino)-4-isopropyl-N-(tétrahydro-pyranne-4-ylméthyl)-nicotinamide,
6-(4-bromo-2-chloro-phénylamino)-4-isopropyl-N-(tétrahydro-pyranne-4-ylméthyl)-nicotinamide,
6-(2-bromo-4-chloro-phénylamino)-4-isopropyl-N-(tétrahydro-pyranne-4-ylméthyl)-nicotinamide,
4-isopropyl-6-(2-méthyl-3-trifluorométhyl-phénylamino)-N-(tétrahydro-pyranne-4-ylméthyl)-nicotinamide,
6-(3-chloro-4-méthyl-phénylamino)-4-isopropyl-N-(tétrahydro-pyranne-4-ylméthyl)-nicotinamide,
6-(4-bromo-3-méthyl-phénylamino)-4-isopropyl-N-(tétrahydro-pyranne-4-ylméthyl)-nicotinamide,
6-(2,5-dichloro-phénylamino)-4-isopropyl-N-(tétrahydro-pyranne-4-ylméthyl)-nicotinamide,
4-isopropyl-6-(2-méthyl-5-trifluorométhyl-phénylamino)-N-(tétrahydro-pyranne-4-ylméthyl)-nicotinamide,
6-(2-bromo-4-chloro-phénylamino)-N-cyclopentylméthyl-4-isopropyl-nicotinamide,
6-(4-bromo-3-chloro-phénylamino)-4-isopropyl-N-(tétrahydro-pyranne-4-ylméthyl)-nicotinamide,
6-(4-chloro-2-fluoro-phénylamino)-N-cyclopentylméthyl-4-isopropyl-nicotinamide,
N-cyclopentylméthyl-6-(3-fluoro-4-trifluorométhyl-phénylamino)-4-isopropyl-nicotinamide,
6-(4-cyano-2-méthyl-phénylamino)-N-cyclopentylméthyl-4-isopropyl-nicotinamide,
6-(3-chloro-2-fluoro-phénylamino)-4-isopropyl-N-(tétrahydro-pyranne-4-ylméthyl)-nicotinamide,
6-(3-fluoro-4-trifluorométhyl-phénylamino)-4-isopropyl-N-(tétrahydro-pyranne-4-ylméthyl)-nicotinamide,
6-(4-cyano-3-trifluorométhyl-phénylamino)-4-isopropyl-N-(tétrahydro-pyranne-4-ylméthyl)-nicotinamide,
6-(4-cyano-2-fluoro-phénylamino)-4-isopropyl-N-(tétrahydro-pyranne-4-ylméthyl)-nicotinamide,
6-(4-fluoro-3-méthyl-phénylamino)-4-isopropyl-N-(tétrahydro-pyranne-4-ylméthyl)-nicotinamide,
6-(5-chloro-2-méthyl-phénylamino)-4-isopropyl-N-(tétrahydro-pyranne-4-ylméthyl)-nicotinamide,

6-(3-fluoro-4-méthyl-phénylamino)-4-isopropyl-N-(tétrahydro-pyranne-4-ylméthyl)-nicotinamide,
6-(3,4-diméthyl-phénylamino)-4-isopropyl-N-(tétrahydro-pyranne-4-ylméthyl)-nicotinamide,
6-(3-bromo-4-méthyl-phénylamino)-4-isopropyl-N-(tétrahydro-pyranne-4-ylméthyl)-nicotinamide,
6-(3-chloro-phénylamino)-N-(4-hydroxy-tétrahydro-pyranne-4-ylméthyl)-4-isopropyl-nicotinamide,
6-(2,3-dichloro-phénylamino)-N-(cyclobutyl)-4-trifluorométhyl-nicotinamide,
6-(2,4-dichloro-phénylamino)-N-(tétrahydropyranne-4-ylméthyl)-4-trifluorométhyl-nicotinamide,
6-(3-chloro-phénylamino)-N-(1,1-dioxo-tétrahydrothiophène-3-ylméthyl)-4-trifluorométhyl-nicotinamide,
N-cyclohexylméthyl-6-phénylamino-4-trifluorométhyl-nicotinamide,
N-cyclopentylméthyl-6-phénylamino-4-trifluorométhyl-nicotinamide,
N-cyclobutylméthyl-6-phénylamino-4-trifluorométhyl-nicotinamide,
N-cyclobutyl-6-(3-chloro-phénylamino)-4-trifluorométhyl-nicotinamide,
N-(tétrahydropyranne-4-ylméthyl)-6-(3-chloro-phénylamino)-4-trifluorométhyl-nicotinamide,
N-cyclobutylméthyl-6-(3-chloro-phénylamino)-4-trifluorométhyl-nicotinamide,
N-isobutyl-6-(3-chloro-phénylamino)-4-trifluorométhyl-nicotinamide,
N-cyclopentylméthyl-6-(3-chloro-phénylamino)-4-trifluorométhyl-nicotinamide,
N-cyclopropylméthyl-6-(3-chloro-phénylamino)-4-trifluorométhyl-nicotinamide,
N-cyclohexylméthyl-6-(3-bromo-phénylamino)-4-trifluorométhyl-nicotinamide,
N-cycloheptylméthyl-6-(3-bromo-phénylamino)-4-trifluorométhyl-nicotinamide,
N-(tétrahydropyranne-4-ylméthyl)-6-(3-bromo-phénylamino)-4-trifluorométhyl-nicotinamide,
N-cyclobutyl-6-(3-bromo-phénylamino)-4-trifluorométhyl-nicotinamide,
N-cyclobutylméthyl-6-(3-bromo-phénylamino)-4-trifluorométhyl-nicotinamide,
N-isobutyl-6-(3-bromo-phénylamino)-4-trifluorométhyl-nicotinamide,
N-cyclopentylméthyl-6-(3-bromo-phénylamino)-4-trifluorométhyl-nicotinamide,
N-cyclopropylméthyl-6-(3-bromo-phénylamino)-4-trifluorométhyl-nicotinamide,
N-cyclobutylméthyl-6-(2-fluoro-phénylamino)-4-trifluorométhyl-nicotinamide,
N-cycloheptylméthyl-6-(3-fluoro-phénylamino)-4-trifluorométhyl-nicotinamide,
N-(tétrahydropyranne-4-ylméthyl)-6-(3-fluoro-phénylamino)-4-trifluorométhyl-nicotinamide,
N-cyclobutyl-6-(3-fluoro-phénylamino)-4-trifluorométhyl-nicotinamide,
N-cyclohexylméthyl-6-(3-fluoro-phénylamino)-4-trifluoronéthyl-nicotinamide,
N-cyclobutylméthyl-6-(3-fluoro-phénylamino)-4-trifluorométhyl-nicotinamide,
N-cyclopentylméthyl-6-(3-fluoro-phénylamino)-4-trifluorométhyl-nicotinamide,
N-isobutyl-6-(3-fluoro-phénylamino)-4-trifluorométhyl-nicotinamide,
N-cyclopropylméthyl-6-(3-fluoro-phénylamino)-4-trifluorométhyl-nicotinamide,
N-(1,1-dioxo-tétrahydro-thiophène-3-ylméthyl)-6-(3-fluoro-phénylamino)-4-trifluorométhyl-nicotinamide,
N-cyclobutylméthyl-6-(4-fluoro-phémylamino)-4-trifluorométhyl-nicotinamide,
N-(tétrahydropyranne-4-ylméthyl)-6-(2,3-dichloro-phénylamino)-4-trifluorométhyl-nicotinamide,
N-cyclohexylméthyl-6-(2,3-dichloro-phénylamino)-4-trifluorométhyl-nicotinamide,
N-cycloheptylméthyl-6-(2,3-dichloro-phénylamino)-4-trifluorométhyl-nicotinamide,
N-cyclohexylméthyl-6-(2,4-dichloro-phénylamino)-4-trifluorométhyl-nicotinamide,
N-cycloheptylméthyl-6-(2,4-dichloro-phénylamino)-4-trifluorométhyl-nicotinamide,
N-cyclobutyl-6-(2,4-dichloro-phénylamino)-4-trifluorométhyl-nicotinamide,
N-cyclopentylméthyl-6-(2,4-dichloro-phénylamino)-4-trifluorométhyl-nicotinamide,
N-cyclobutylméthyl-6-(2,4-dichloro-phénylamino)-4-trifluorométhyl-nicotinamide,
N-isobutyl-6-(2,4-dichloro-phénylamino)-4-trifluorométhyl-nicotinamide,
N-cyclopropylméthyl-6-(2,4-dichloro-phénylamino)-4-trifluorométhyl-nicotinamide,
N-(1,1-dioxo-tétrahydro-thiophène-3-ylméthyl)-6-(2,4-dichloro-phénylamino)-4-trifluorométhyl-nicotinamide,
N-cyclohexylméthyl-6-(3,5-dichloro-phénylamino)-4-trifluorométhyl-nicotinamide,
N-(tétrahydropyranne-4-ylméthyl)-6-(3,5-dichloro-phénylamino)-4-trifluorométhyl-nicotinamide,
N-cyclobutyl-6-(3,5-dichloro-phénylamino)-4-trifluorométhyl-nicotinamide,
N-cyclopentylméthyl-6-(3,5-dichloro-phénylamino)-4-trifluorométhyl-nicotinamide,
N-cyclobutylméthyl-6-(3,5-dichloro-phénylamino)-4-trifluorométhyl-nicotinamide,
N-isobutyl-6-(3, 5-dichloro-phénylamino)-4-trifluorométhyl-nicotinamide,
N-cyclopropylméthyl-6-(3,5-dichloro-phénylamino)-4-trifluorométhyl-nicotinamide,
N-isobutyl-6-(3,4-dichloro-phénylamino)-4-trifluorométhyl-nicotinamide,
N-cyclobutyl-6-(3,4-dichloro-phénylamino)-4-trifluorométhyl-nicotinamide,
N-(tétrahydropyranne-4-ylméthyl)-6-(3,4-dichloro-phénylamino)-4-trifluorométhyl-nicotinamide,
N-cyclopentylméthyl-6-(3,4-dichloro-phénylamino)-4-trifluorométhyl-nicotinamide,
N-cyclobutylméthyl-6-(3,4-dichloro-phénylamino)-4-trifluorométhyl-nicotinamide,

N-cyclopropylméthyl-6-(3,4-dichloro-phénylamino)-4-trifluorométhyl-nicotinamide,
N-cyclohexylméthyl-6-(3,4-dichloro-phénylamino)-4-trifluorométhyl-nicotinamide,
N-cyclobutylméthyl-6-(2-fluoro-4-chloro-phénylamino)-4-trifluorométhyl-nicotinamide,
N-cyclopentylméthyl-6-(2-fluoro-4-chloro-phénylamino)-4-trifluorométhyl-nicotinamide,
N-(tétrahydropyranne-4-ylméthyl)-6-(2-fluoro-4-chloro-phénylamino)-4-trifluorométhyl-nicotinamide,
N-cyelobutylméthyl-6-(2-chloro-4-fluoro-phénylamino)-4-trifluorométhyl-nicotinamide,
N-cyclopentylméthyl-6-(2-chloro-4-fluoro-phénylamino)-4-trifluorométhyl-nicotinamide,
N-(tétrahydropyranne-4-ylméthyl)-6-(2-chloro-4-fluoro-phénylamino)-4-trifluorométhyl-nicotinamide,
N-cyclobutylméthyl-6-(2,4-difluoro-phénylamino)-4-trifluorométhyl-nicotinamide,
N-cyclopentylméthyl-6-(2,4-difluoro-phénylamino)-4-trifluorométhyl-nicotinamide,
N-(tétrahydropyranne-4-ylméthyl)-6-(2-méthoxy-5-chloro-phénylamino)-4-trifluorométhyl-nicotinamide,
N-cyclobutylméthyl-6-(2-méthoxy-5-chloro-phénylamino)-4-trifluorométhyl-nicotinamide,
N-(tétrahydropyranne-4-ylméthyl)-6-(2-hydroxy-5-chloro-phénylamino)-4-trifluorométhyl-nicotinamide,
N-cyclohexylméthyl-6-(2-méthyl-4-chloro-phénylamino)-4-trifluorométhyl-nicotinamide,
N-(tétrahydropyranne-4-ylméthyl)-6-(2-méthyl-4-chloro-phénylamino)-4-trifluorométhyl-nicotinamide,
N-(tétrahydropyranne-4-ylméthyl)-6-phénylamina-4-trifluorométhyl-nicotinamide,
N-cyclopropylméthyl-6-phénylamino-4-trifluorométhyl-nicotinamide,
N-cycloheptylméthyl-6-(3,5-dichloro-phénylamino)-4-trifluorométhyl-nicotinamide,
N-(tétrahydropyranne-4-ylméthyl)-6-(2-méthyl-5-chloro-phénylamino)-4-trifluorométhyl-nicotinamide,
N-cyclobutylméthyl-6-(2-hydroxy-5-chloro-phénylamino)-4-trifluorométhyl-nicotinamide,
N-(5-oxo-pyrrolidine-3-ylméthyl)-6-(2,4-dichloro-phénylamino)-4-trifluorométhyl-nicotinamide,
N-cycloheptylméthyl-6-phénylamino-4-trifluorométhyl-nicotinamide,
N-cyclobutyl-6-phénylamino-4-trifluorométhyl-nicotinamide,
N-isobutyl-6-phénylamino-4-trifluorométhyl-nicotinamide,
N-(3-diméthylamino-2,2-diméthyl-propyl)-6-(3-chloro-phénylamino)-4-trifluorométhyl-nicotinamide,
N-(3-hydroxy-2,2-diméthyl-propyl)-6-(3-chloro-phénylamino)-4-trifluorométhyl-nicotinamide,
N-(2-méthoxy-2-méthyl-propyl)-6-(3-chloro-phénylamino)-4-trifluorométhyl-nicotinamide,
N-([1,4]dioxanne-2-ylméthyl)-6-(3-chloro-phénylamino)-4-trifluorométhyl-nicotinamide,
N-(pipéridine-2-ylméthyl)-6-(3-chloro-phénylamino)-4-trifluorométhyl-nicotinamide,
N-(1-benzyl-5-oxo-pyrrolidine-3-ylméthyl)-6-(3-chloro-phénylamino)-4-trifluorométhyl-nicotinamide,
N-(5-oxo-pyrrolidine-3-ylméthyl)-6-(3-chloro-phénylamino)-4-trifluorométhyl-nicotinamide,
N-méthylcarbamoylméthyl-6-(3-chloro-phénylamino)-4-trifluorométhyl-nicotinamide,
N-(1-éthyl-pyrrolidine-2-ylméthyl)-6-(3-chloro-phénylamino)-4-trifluorométhyl-nicotinamide,
N-(2,2,6,6-tétraméthyl-pipéridine-4-ylméthyl)-6-(3-chloro-phénylamino)-4-trifluorométhyl-nicotinamide,
N-(2,2-diméthyl-[1,3]dioxolanne-4-ylméthyl)-6-(3-chloro-phénylamino)-4-trifluorométhyl-nicotinamide,
N-(tétrahydropyranne-4-ylméthyl)-6-(2-fluoro-phénylamino)-4-trifluorométhyl-nicotinamide,
N-(tétrahydropyranne-4-ylméthyl)-6-(4-fluoro-phénylamino)-4-trifluorométhyl-nicotinamide,
N-(3-diméthylamino-2,2-diméthyl-propyl)-6-(2,4-dichloro-phénylamino)-4-trifluorométhyl-nicotinamide,
N-([1,4]dioxanne-2-ylméthyl)-6-(2,4-dichloro-phénylamino)-4-trifluorométhyl-nicotinamide,
N-(5-oxo-pyrrolidine-3-ylméthyl)-6-(2,4-dichloro-phénylamino)-4-trifluorométhyl-nicotinamide,
N-méthylcarbamoylméthyl-6-(2,4-dichloro-phénylamino)-4-trifluorométhyl-nicotinamide,
N-(2,2-diméthyl-[1,3]dioxolanne-4-ylméthyl)-6-(2,4-dichloro-phénylamino)-4-trifluorométhyl-nicotinamide,
N-cycloheptylméthyl-6-(3,4-dichloro-phénylamino)-4-trifluorométhyl-nicotinamide,
N-(tétrahydropyranne-4-ylméthyl)-6-(2,4-difluoro-phénylamino)-4-trifluorométhyl-nicotinamide,
N-cyclohexylméthyl-6-(2-méthyl-5-chloro-phénylamino)-4-trifluorométhyl-nicotinamide,
N-(2,3-dihydroxy-propyl)-6-(3-chloro-phénylamino)-4-trifluorométhyl-nicotinamide,
N-(2,3-dihydroxy-propyl)-6-(2,4-dichloro-phénylamino)-4-trifluorométhyl-nicotinamide,
6-(3-chloro-phénylamino)-N-(tétrahydro-pyranne-4-ylméthyl)-2-trifluorométhyl-nicotinamide,
6-(3-chloro-phénylamino)-N-cyclohexylméthyl-2-trifluorométhyl-nicotinamide,
6-(3-chloro-phénylamino)-N-cyclobutylméthyl-2-trifluorométhyl-nicotinamide,
6-(3-chloro-phénylamino)-N-cyclopentylméthyl-2-trifluorométhyl-nicotinamide,
6-(3-chloro-phénylamino)-2-isopropyl-N-(tétrahydro-pyranne-4-ylméthyl)-nicotinamide,
6-(3-chloro-phénylamino)-N-(1,1-dioxo-tétrahydrothiophène-3-ylméthyl)-2-isopropyl-nicotinamide,
6-(3-chloro-phénylamino)-N-cyclopentylméthyl-2-isopropyl-nicotinamide,
6-(3-chloro-phénylamino)-N-cyclohexylméthyl-2-isopropyl-nicotinamide,
6-(2,4-dichloro-phénylamino)-N-cyclobutylméthyl-2-isopropyl-nicotinamide,
6-(2,4-dichloro-phénylamino)-N-cyclopentylméthyl-2-isopropyl-nicotinamide,
6-(2,4-dichloro-phénylamino)-N-(tétrahydro-pyranne-4-ylméthyl)-2-isopropyl-nicotinamide,

6-(2,4-dichloro-phénylamino)-N-(1,1-dioxo-tétrahydrothiophène-3-ylméthyl)-2-isopropyl-nicotinamide,
6-(3-fluoro-phénylamino)-N-cyclobutylméthyl-2-isopropyl-nicotinamide,
6-(3-fluoro-phénylamino)-N-cyclopentylméthyl-2-isopropyl-nicotinamide,
6-(3-fluoro-phénylamino)-N-(tétrahydro-pyranne-4-ylméthyl)-2-isopropyl-nicotinamide,
6-(3-fluoro-phénylamino)-N-(1,1-dioxo-tétrahydrothiophène-3-ylméthyl)-2-isopropyl-nicotinamide,
6-(4-cyano-2-méthyl-phénylamino)-4-isopropyl-N-(tétrahydro-pyranne-4-ylméthyl)-nicotinamide,
6-(5-chloro-2-cyano-phénylamino)-4-isopropyl-N-(tétrahydro-pyranne-4-ylméthyl)-nicotinamide,
6-(2-cyano-5-méthyl-phénylamino)-4-isopropyl-N-(tétrahydro-pyranne-4-ylméthyl)-nicotinamide,
6-(3-chloro-phénylamino)-N-(1,1-dioxo-tétrahydro-1/$^{6}$-thiophène-3-ylméthyl)-4-isopropyl-nicotinamide,
N-cyclobutylméthyl-4-isopropyl-6-(3-trifluorométhoxy-phénylamino)-nicotinamide,
6-(2,3-difluoro-phénylamino)-4-isopropyl-N-(tétrahydro-pyranne-4-ylméthyl)-nicotinamide,
6-(3,5-bis-trifluorométhyl-phénylamino)-4-isopropyl-N-(tétrahydro-pyranne-4-ylméthyl)-nicotinamide,
6-(2,4-difluoro-phénylamino)-4-isopropyl-N-(tétrahydro-pyranne-4-ylméthyl)-nicotinamide,
6-(3-éthynyl-phénylamino)-4-isopropyl-N-(tétrahydro-pyranne-4-ylméthyl)-nicotinamide,
6-(2-fluoro-4-trifluorométhyl-phénylamino)-N-cyclopéntyl-méthyl-4-isopropyl-nicotinamide,
6-(3-cyano-4-méthyl-phénylamino)-4-isopropyl-N-(tétrahydro-pyranne-4-ylméthyl)-nicotinamide,
6-(3-cyano-4-fluoro-phénylamino)-4-isopropyl-N-(tétrahydro-pyranne-4-ylméthyl)-nicotinamide,
6-(3-bromo-4-trifluorométhoxy-phénylamino)-4-iscpropyl-N-(tétrahydro-pyranne-4-ylméthyl)-nicotinamide,
6-(4-chloro-2-fluoro-phénylamino)-N-cyclobutylméthyl-4-isopropyl-nicotinamide,
6-(5-bromo-2-méthyl-phénylamino)-4-isopropyl-N-(tétrahydro-pyranne-4-ylméthyl)-nicotinamide,
6-(2-bromo-5-fluoro-phénylamino)-4-isopropyl-N-(tétrahydro-pyranne-4-ylméthyl)-nicotinamide,
6-(2-fluoro-5-trifluorométhyl-phénylamino)-4-isopropyl-N-(tétrahydro-pyranne-4-ylméthyl)-nicotinamide,
6-(2-chloro-5-trifluorométhyl-phénylamino)-4-isopropyl-N-(tétrahydro-pyranne-4-ylméthyl)-nicotinamide,
6-(2-bromo-5-trifluorométhyl-phénylamino)-4-isopropyl-N-(tétrahydro-pyranne-4-ylméthyl)-nicotinamide,
6-(3-bromo-4-cyano-phénylamino)-4-isopropyl-N-(tétrahydro-pyranne-4-ylméthyl)-nicotinamide,
6-(2-bromo-4-trifluorométhoxy-phénylamino)-4-isopropyl-N-(tétrahydro-pyranne-4-ylméthyl)-nicotinamide,
6-(3-chloro-2-méthyl-phénylamino)-4-isopropyl-N-(tétrahydro-pyranne-4-ylméthyl)-nicotinamide,
6-(3,5-difluoro-phénylamino)-4-isopropyl-N-(tétrahydro-pyranne-4-ylméthyl)-nicotinamide,
6-(2-chloro-4-fluoro-phénylamino)-4-isopropyl-N-(tétrahydro-pyranne-4-ylméthyl)-nicotinamide,
6-(4-chloro-2-méthyl-phénylamino)-4-isopropyl-N-(tétrahydro-pyranne-4-ylméthyl)-nicotinamide,
6-(2-fluoro-3-trifluorométhyl-phénylamino)-N-cyclopentylméthyl-4-isopropyl-nicotinamide,
6-(2-méthyl-4-chloro-phénylamino)-N-cyclopentylméthyl-4-isopropyl-nicotinamide,
6-(3-chloro-4-cyano-phénylamino)-N-cyclopentylméthyl-4-isopropyl-nicotinamide,
6-(4-bromo-2-chloro-phénylamino)-N-cyclopentylméthyl-4-isopropyl-nicotinamide,
N-cyclobutylméthyl-6-(2,4-difluoro-phénylamino)-4-isopropyl-nicotinamide,
N-cyclobutylméthyl-6-(2,4-dichloro-phénylamino)-4-isopropyl-nicotinamide,
N-cyclobutylméthyl-6-(3,4-dichloro-phénylamino)-4-isopropyl-nicotinamide,
N-cyclobutylméthyl-6-(2,3-dichloro-phénylamino)-4-isopropyl-nicotinamide,
6-(2-chloro-4-fluoro-phénylamino)-N-cyclobutylméthyl-4-isopropyl-nicotinamide,
6-(3-chloro-4-fluoro-phénylamino)-N-cyclobutylméthyl-4-isopropyl-nicotinamide,
6-(4-bromo-2-chloro-phénylamino)-N-cyclobutylméthyl-4-isopropyl-nicotinamide,
6-(2-bromo-4-chloro-phénylamino)-N-cyclobutylméthyl-4-isopropyl-nicotinamide,
N-cyclobutylméthyl-6-(2-fluoro-3-trifluorométhyl-phénylamino)-4-isopropyl-nicotinamide,
6-(4-chloro-2-méthyl-phénylamino)-N-cyclobutylméthyl-4-isopropyl-nicotinamide,
6-(2-chloro-4-cyano-phénylamino)-N-cyclobutylméthyl-4-isopropyl-nicotinamide,
6-(4-cyano-2-fluoro-phénylamino)-N-cyclobutylméthyl-4-isopropyl-nicotinamide,
6-(4-cyano-2-méthyl-phénylamino.)-N-cyclobutylméthyl-4-isopropyl-nicotinamide,
6-(2-chloro-4-trifluorométhyl-phénylamino)-N-cyclobutylméthyl-4-isopropyl-nicotinamide,
N-cyclobutylméthyl-6-(3,5-dichloro-phénylamino)-4-isopropyl-nicotinamide,
N-cyclobutylméthyl-6-(2,5-dichloro-phénylamino)-4-isopropyl-nicotinamide,
N-cyclobutylméthyl-6-(3,5-difluoro-phénylamino)-4-isopropyl-nicotinamide,
6-(5-chloro-2-fluoro-phénylamino)-N-cyclobutylméthyl-4-isopropyl-nicotinamide,
6-(2-chloro-5-fluoro-phénylamino)-N-cyclobutylméthyl-4-isopropyl-nicotinamide,
6-(2-chloro-5-fluoro-phénylamino)-4-isopropyl-N-(tétrahydro-pyranne-4-ylméthyl)-nicotinamide,
6-(2-chloro-5-méthyl-phénylamino)-4-isopropyl-N-(tétrahydro-pyranne-4-ylméthyl)-nicotinamide,
6-(2-fluoro-5-méthyl-phénylamino)-4-isopropyl-N-(tétrahydro-pyranne-4-ylméthyl)-nicotinamide,
6-(5-fluoro-2-méthyl-phénylamino)-4-isopropyl-N-(tétrahydro-pyranne-4-ylméthyl)-nicotinamide,
6-(3-bromo-2-méthyl-phénylamino)-4-isopropyl-N-(tétrahydro-pyranne-4-ylméthyl)-nicotinamide,

4-isopropyl-6-(2-méthyl-4-trifluorométhoxy-phénylamino)-N-(tétrahydro-pyranne-4-ylméthyl)-nicotinamide,
6-(3-fluoro-2-méthyl-phénylamino)-4-isopropyl-N-(tétrahydro-pyranne-4-ylméthyl)-nicotinamide,
6-(3-bromo-5-trifluorométhyl-phénylamino)-4-isopropyl-N-(tétrahydro-pyranne-4-ylméthyl)-nicotinamide,
6-(4-cyano-phénylamino)-4-isopropyl-N-(-tétrahydro-pyranne-4-ylméthyl)-nicotinamide,
6-(4-chloro-2-fluoro-phénylamino)-4-isopropyl-N-(tétrahydro-pyranne-4-ylméthyl)-nicotinamide,
6-(4-bromo-2-fluoro-phénylamino)-N-cyclopentylméthyl-4-isopropyl-nicotinamide,
6-(2-bromo-4-trifluorométhoxy-phénylamino)-N-cyclobutylméthyl-4-isopropyl-nicotinamide,
N-cyclobutylméthyl-6-(2-fluoro-4-trifluorométhyl-phénylamino)-4-isopropyl-nicotinamide,
6-(4-cyano-2-fluoro-phénylamino)-N-cyclopentylméthyl-4-isopropyl-nicotinamide,
6-(4-bromo-3-trifluorométhyl-phénylamino)-4-isopropyl-N-(tétrahydropyranne-4-ylméthyl)-nicotinamide,
6-(4-fluoro-3-trifluorométhyl-phénylamino)-4-isopropyl-N-(tétrahydropyranne-4-ylméthyl)-nicotinamide,
6-(3,4-dibromo-phénylamino)-4-isopropyl-N-(tétrahydropyranne-4-ylméthyl)-nicotinamide,
6-(4-bromo-3-fluoro-phénylamino)-4-isopropyl-N-(tétrahydropyranne-4-ylméthyl)-nicotinamide,
6-(2-chloro-4-trifluorométhyl-phénylamino)-N-cyclopentyl-méthyl-4-isopropyl-nicotinamide,
6-(3,4-difluoro-phénylamino)-4-isopropyl-N-(tétrahydropyranne-4-ylméthyl)-nicotinamide,
6-(4-chloro-3-trifluorométhyl-phénylamino)-4-isopropyl-N-(tétrahydropyranne-4-ylméthyl)-nicotinamide,
6-(4-méthyl-3-trifluorométhyl-phénylamino)-4-isopropyl-N-(tétrahydropyranne-4-ylméthyl)-nicotinamide,
6-(2-chloro-4-trifluorométhoxy-phénylamino)-4-isopropyl-N-(tétrahydropyranne-4-ylméthyl)-nicotinamide,
6-(2-cyano-3-méthyl-phénylamino)-4-isopropyl-N-(tétrahydropyranne-4-ylméthyl)-nicotinamide,
6-(3-chloro-2-cyano-phénylamino)-4-isopropyl-N-(tétrahydropyranne-4-ylméthyl)-nicotinamide,
6-(3-chloro-phénylamino)-4-(1-hydroxy-méthyl-éthyl)-N-(tétrahydropyranne-4-ylméthyl)-nicotinamide, et
4-tertiobutyl-6-(3,4-dichloro-phénylamino)-N-(tétrahydro-pyranne-4-ylméthyl)-nicotinamide,

et ses dérivés pharmaceutiquement acceptables.

**7.** Composé suivant la revendication 1, choisi entre les suivants :

6-(3-chloro-phényl-amino)-4-isopropyl-N-(tétrahydro-pyranne-4-ylméthyl)-nicotinamide ;
6-(3-bromo-phényl-amino)-4-isopropyl-N-(tétrahydro-pyranne-4-ylméthyl)-nicotinamide ;
6-(2,4-dichloro-phénylamino)-4-isopropyl-N-(tétrahydro-pyranne-4-ylméthyl)-nicotinamide ;
4-isopropyl-N-(tétrahydro-pyranne-4-ylméthyl)-6-(3-trifluorométhoxy-phénylamino)-nicotinamide ;
4-tertiobutyl-6-(2,4-di-chloro-phénylamino)-N-(tétrahydro-pyranne-4-ylméthyl)-nicotinamide ;
6-(3-chloro-4-cyano-phénylamino)-4-isopropyl-N-(tétrahydro-pyranne-4-ylméthyl)-nicotinamide ;
6-(2-fluoro-3-trifluorométhyl-phénylamino)-4-isopropyl-N-(tétrahydro-pyranne-4-ylméthyl)-nicotinamide ;
6-(4-bromo-2-chloro-phénylamino)-4-isopropyl-N-(tétrahydro-pyranne-4-ylméthyl)-nicotinamide ;
6-(3,4-dichloro-phénylamino)-4-isopropyl-N-(tétrahydro-pyranne-4-ylméthyl)-nicotinamide ;
6-(2-bromo-4-trifluorométhoxy-phénylamino)-4-isopropyl-N-(tétrahydro-pyranne-4-ylméthyl)-nicotinamide ;
6-(3,5-difluoro-phénylamino)-4-isopropyl-N-(tétrahydro-pyranne-4-ylméthyl)-nicotinamide ;
6-(2,4-dichloro-phénylamino)-N-(tétrahydro-pyranne-4-ylméthyl)-4-trifluorométhyl-nicotinamide ;

ou un ses dérivés pharmaceutiquement acceptables.

**8.** Composition pharmaceutique comprenant un composé suivant l'une quelconque des revendications précédentes ou un de ses dérivés pharmaceutiquement acceptables.

**9.** Composition pharmaceutique suivant la revendication 8, comprenant en outre un support ou diluant pharmaceutiquement acceptable.

**10.** Utilisation d'une quantité thérapeutiquement efficace d'un composé de formule (I) suivant l'une quelconque des revendications 1 à 7, ou d'un de ses dérivés pharmaceutiquement acceptables, pour la production d'un médicament destiné au traitement d'une affection qui est soumise à une médiation par l'activité des récepteurs de cannabinoides 2.

**11.** Utilisation suivant la revendication 10, dans laquelle l'affection est un trouble immunitaire, un trouble inflammatoire, la douleur, la polyarthrite rhumatoïde, la sclérose en plaques, l'arthrose ou l'ostéoporose.

**12.** Utilisation suivant la revendication 11, dans laquelle la douleur est choisie entre la douleur inflammatoire, la douleur viscérale, la douleur provoquée par le cancer, la douleur neuropathique, la douleur dans le bas du dos, la douleur musculo-squelettique, la douleur postopératoire, la douleur aiguë et la migraine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5112820 A
- EP 576357 A
- US 4826689 A, Violanto & Fischer
- US 5145684 A, Liversidge
- US 5298262 A, Na & Rajagopalan
- US 5302401 A, Liversidge
- US 5336507 A, Na & Rajagopalan
- US 5340564 A, Illig & Sarpotdar
- US 5346702 A, Na Rajagopalan
- US 5352459 A, Hollister
- US 5354560 A, Lovrecich
- US 5384124 A, Courteille
- US 5429824 A, June
- US 5503723 A, Ruddy
- US 5510118 A, Bosch
- US 5518 A, Bruno
- US 5518738 A, Eickhoff
- US 5534270 A, De Castro
- US 5536508 A, Canal
- US 5552160 A, Liversidge
- US 5560931 A, Eickhoff
- US 5560932 A, Bagchi
- US 5565188 A, Wong
- US 5571536 A, Eickhoff
- US 5573783 A, Desieno & Stetsko
- US 5580579 A, Ruddy
- US 5585108 A, Ruddy
- US 5587143 A, Wong
- US 5591456 A, Franson
- US 5622938 A, Wong
- US 5662883 A, Bagchi
- US 5665331 A, Bagchi
- US 5718919 A, Ruddy
- US 5747001 A
- WO 9325190 A
- WO 9624336 A
- WO 9714407 A
- WO 9835666 A
- WO 9965469 A
- WO 0018374 A
- WO 0027369 A
- WO 0030615 A
- WO 0141760 A
- WO 0200196 A
- US 5474995 A
- US 5633272 A
- US 5466823 A
- US 6310099 B
- US 6291523 B
- WO 9625405 A
- WO 9738986 A
- WO 9803484 A
- WO 9714691 A
- WO 9912930 A
- WO 0026216 A
- WO 0052008 A
- WO 0038311 A
- WO 0158881 A
- WO 0218374 A
- US 2002183309 A


### Non-patent literature cited in the description


- **L.E. HOLLISTER.** Health Aspects of Cannabis. *Pharmacological Reviews,* 1986, vol. 38, 1-20
- **WIRTH et al.** Antiinflammatory Properties of Cannabichrome. *Life Science,* 1980, vol. 26, 1991-1995
- **MATSUDA et al.** Structure of a Cannabinoid Receptor and Functional Expression of the Cloned cDNA. *Nature,* 1990, vol. 346, 561-564
- **MUNRO.** Molecular Characterization of a Peripheral Receptor for Cannabinoids. *Nature,* 1993, vol. 365, 61-65
- **BERGE ; BIGHLEY ; MONKHOUSE.** *J. Pharm. Sci.,* 1977, vol. 66, 1-19
- **BROWN et al.** *Yeast,* 2000, vol. 16, 11-22
- **GUTHRIE ; FINK.** *Methods in Enzymology,* 1991, vol. 194
- **EIDEN et al.** *Arch. Pharm.,* 1987, vol. 320, 348
- **ARGYLE et al.** *J Chem Soc (C,* 1967, 2156